# EUROPEAN PATENT APPLICATION

(11) **EP 4 488 366 A2**
(43) Date of publication of application: **08.01.2025**
(21) Application number: 24208814.4
(22) Date of filing: 18.10.2018
(51) Int. Cl.: C12N 5/0783

(54) **BITE-ACTIVATED CAR-T CELLS**

(30) Priority: 18.10.2017 US 201762573751 P
(62) Divisional of application: 18795957.2
(71) Applicant: Vivia Biotech, S.L., 28760 Tres Cantos, Madrid (ES)
(72) Inventor: PRIMO RAMOS, Daniel, 28760 Tres Cantos, Madrid (ES); BALLESTEROS NOBELL, Juan Antonio, 28670 Tres Cantos, Madrid (ES); BENNETT, Teresa Ann, 28670 Tres Cantos, Madrid (ES); GORROCHATEGUI GUILLÉN, Julián, 28670 Tres Cantos, Madrid (ES); MARTÍNEZ LÓPEZ, Joaquín, 28760 Tres Cantos, Madrid (ES); VALERI LOZANO, Antonio, 28850 Torrejón de Ardoz, Madrid (ES); LEIVAS ALDEA, Alejandra, 28016 Madrid (ES)
(74) Representative: Manuel Illescas y Asociados, S.L.

(57) **Abstract**

The CAR-T cells described herein can provide highly effective therapies for diverse cancer types, e.g., solid cancers, hematological cancers, and metastatic forms thereof. Provided herein are methods of generating CAR-T cells, compositions comprising such CAR-T cells, methods of treatment using the cells, methods of identifying subjects susceptible to immune checkpoint immunotherapy treatment and methods of evaluating susceptibility of a subject to develop Cytokine-Release Syndrome.

## Description

### Field of the Invention

The disclosure relates to three novel approaches using bispecific antibodies (BiTE)-activated T Cells. One is to generate chimeric antigen receptor (CAR) T cells using these BiTE-activated T cells as the source of T cells. These new CAR-T cells may be a better cellular therapy treatment for cancer patients. A second approach is a method to identify which immune check point inhibitors are responsible for resistance to these BiTE-activated T cells. This can be helpful to personalize immunotherapy treatments to cancer patients. This may also be helpful for other immunotherapy treatments, such as CAR-T cells, independently of the BiTE-activated T cells. A third approach is to identify patients less susceptible to suffer Cytokine-Release Syndrome. This can also be helpful to personalize immunotherapy treatments to cancer patients. This may also be helpful for other immunotherapy treatments, such as CAR-T cells, independently of the BiTE-activated T cells.

### Background of the Invention

Adoptive cell therapy (ACT) is a process involving collection of immune cells from a patient, expansion of the cells, and reintroduction of the cells into the same patient or a different patient. For example, ACT of donor-derived, *ex vivo* expanded human cytotoxic T lymphocytes (CTLs) has emerged as a promising approach to treat cancer. Examples of ACT include cultured tumor infiltrating lymphocytes (TILs), isolated and expanded T cell clones, and genetically engineered lymphocytes (e.g., T cells) that express conventional T cell receptors or chimeric antigen receptors. The genetically engineered lymphocytes are designed to eliminate cancer cells expressing specific antigen(s) and are expanded and delivered to a patient. Another example of an ACT is the isolation and use of T cells from a patient's blood after administration of a cancer vaccine. ACT can provide tumor specific lymphocytes (e.g., T cells) that lead to a reduction in tumor cells in a patient.

Despite the clinical efficacy of Chimeric Antigen Receptor (CAR)-T cells for cancer treatment, they still have major limitations related to toxicity or immune mechanism of resistance that could be overcome through the integration of these different approaches with the Cancer-Killing T Cells. Standard CAR-T cells are generated using peripheral blood naive T cells. A limitation of these standard CAR-T cells is that they can only recognize the tumor antigen of the CAR construct. However, tumor cells can be heterogeneous with some clones not expressing the CAR antigen leading to resistance to such CAR-T cells. Relapsed patients treated with CAR-T cells are showing this resistance mechanisms.

Document Borrello I *et* al., 2016 discloses utilization of marrow-infiltrating lymphocytes (MILs) for adoptive T-cell therapy. The document discloses activation of MILs with anti CD3/CD28 beads. Also disclosed in this document is the suggestion that MILs could potentially serve a better source of T-cells for CAR-based adoptive T-cell therapy. However, no experimental results are provided in the document supporting this hypothesis.

The method of producing CAR-T cells, often by transducing a CAR with a lentivirus, generates an heterogenous population of T Cells. The CAR construct may insert at different positions into the genome, resulting in different activity of the ensuing CAR-T cells; e.g. different levels of expression could affect activity, or disrupting different genes. Furthermore, the different types of T cells present in the mixed T cell population used as a source for producing CAR-T cells may result in different activities; e.g. memory T cells versus naive T cells, highly proliferating versus terminally proliferating T cells. It has been recently reported that expansion of a single CAR T-cell clone inside a patient with CLL resulted in complete remission (Fraietta et al. Nature. 2018 Jun;558(7709):307-312). This document discloses that at the peak of the response, 94% of CAR T cells originated from a single clone in which lentiviral vector-mediated insertion of the CAR transgene disrupted the methylcytosine dioxygenase TET2 gene. This genetic disruption was validated to confer an advantage to T cells for CAR-T expansion. Therefore, there are likely different, maybe thousands of different CAR-T cell clones produced when producing a CAR-T, which is in reality an heterogenous mixture of CAR-T clones. Methods to identify the best CAR-T clones would be beneficial to enhance the CAR-T activity and hence patient clinical responses.

Cytokine Storm, also called Cytokine Release Syndrome, has been recognized as a major toxicity challenge for CAR-T treatments (Park et al. N Engl J Med. 2018 Feb 1;378(5):449-459). It also a major toxicity for bispecific antibodies. However, there are no methods to identify patient most likely to suffer this toxicity when treated with CAR-T cells.

A key immunotherapy treatment often combined with CAR-T and BiTE treatments are immune check point inhibitors (ICHK). However, it is difficult to identify which patients would benefit from these new immunotherapies. Expression of PD1, PDL1, PDL2, are considered reasonable biomarkers to select patients for anti-PD1 or anti-PDL1 treatment. However, there is no similar guides for other ICHKs.

### Summary of the Invention

Bispecific T cell engager antibody (BiTE)-activated T-cells are potent and selective anti-tumor cells. In the present invention, BiTE-activated T cells are the target for grafting CAR molecules. BiTE-activated T cells combine the potency of the transfected CAR construct while retaining their ability to recognize and kill tumor cells expressing different, CAR-resistant antigens. In this sense, once the activated T Cells are generated by proximity with a bispecific T cell engager antibody (BiTE), the use of these T-Cells for Adoptive Cell Therapy can also be enhanced by using them as the source of CAR-T cells, transfecting CAR constructs into them prior to adoptive cell therapy. Using a bispecific T cell engager antibody (BiTE) to activate and thus identify these selective antitumor effector T-cells offers unique advantages for hematological malignancies. For these cancers, these selective antitumor effector T cells are part of the T cell population that consists of many sub-types of T cells that reside in hematological tissues such as bone marrow, and it is not known how to identify them in most of these malignancies.

T cell receptor (TCR) is a disulfide-linked heterodimer consisting of one α and one β chain expressed in complex with invariant CD3 chains (*γ*, *δ*, *ζ*, and *ε*). TCR recognizes intracellular or extracellular proteins presented as peptides by MHC molecules. Costimulation of CD28 through its ligands, CD80/CD86, is required for optimal activation of the receptor and for production of interleukin-2 (IL-2) and other cytokines. While most hematological tumors express costimulatory molecules, solid tumor cells as well as antigen presenting cells in the tumor microenvironment usually lack such molecules.

Chimeric Antigen Receptors (CARs) are recombinant receptors that recognize surface antigens in an MHC unrestricted manner. CARs are fusion proteins between single-chain variable fragments (scFv) from a monoclonal antibody and one or more T cell receptor intracellular signaling domains. Various hinges and transmembrane (TM) domains are used to link the recognition (antigen binding) and the signaling activation moiety. While first generation CARs signaled through the CD3 chain only, second generation CARs include a signaling domain from a costimulatory molecule, for example, CD28, 4-1BB, OX40, CD27, DAP10, or ICOS.

There are several strategies to improve CAR-T-cell therapy that involve higher safety, better trafficking of T-cells to tumor sites, increase persistence and overcome the immunosuppressive factors in the tumor microenvironment. Improvements in T-cell selections also represent a good approach to enhance the cancer treatment efficacy. Activated T cells generated after BiTE exposure represent a novel source of T cells that can be genetically engineered. There are many different types of genetic reengineering processes to produce CARs on their surface to recognize a tumor associated antigen (TAA) on the targeted tumor cells. These T cells would combine the advantages of both methods and should provide a highly effective cytotoxic T-cells that would be able to trigger a T cell mediated tumor cell lysis in a T cell receptor (TCR) and MHC-independent manner. Another approach exploits recent technologies through exome-guided neoantigen identification that can dissect the immune response to patient-specific neoantigens. Incorporation of these neoantigens expressed in cancer cells to the CAR, would enhance the selectively T cell reactivity against this class of antigens.

MILs in bone marrow of hematological malignancies is different than TILs in solid tumors, in that bone marrow always has T cells present and nobody knows which ones are TILs. The tumor-specific T cells, however, are believed to be present at much higher frequencies among MILs compared to peripheral blood but are often dysfunctional (exhausted/anergic) and require potent stimulation in order to recover their anti-cancer cytotoxic functions. These Tumor-Specific T cells in patient bone marrow samples can be identified pharmacologically, by activating them with bispecific antibodies (BiTEs). It is though that BiTEs induce T cells to kill tumor cells by proximity independent of the antigen recognition. The present invention provides that in many patient samples when the BiTE joins a tumor cell with an immunosuppressed TSA T Cell (TIL), it can also activate these TILs, which kills tumor cells independently of the BiTE. Cells may be sorted, BiTE may be washed, cells may be grown, and cells retain the cytotoxic efficacy against tumor cells of the same patient. These reactivated TILs can be identified because they have a great killing efficacy, where one activated T cell can kill on average 30-100 tumor cells. In contrast, normal T cells incubated with a BiTE can only kill tumor cells 1:1. Patient samples identified to kill >30 tumor cells per activated T cell are called superkillers; we hypothesize that these activated T cells are more highly enriched in Tumor-Specific T cells reactivated by BiTE proximity. Thus, these subset of BiTE-activated T cell samples are more likely to include the Tumor-Speciifc T cell clones that can provide the additional efficacy against heterogenous clones vs a single-antigen CAR-T. One advantage of CAR-T cells of the present invention is that they are more potent, and also that they can kill clonal populations that do not express the antigen on the CAR because they retain the native TCR recognition of other cancer antigens.

The CAR-T cells described herein can provide highly effective therapies for diverse cancer types, e.g., solid cancers, hematological cancers, and metastatic forms thereof. Therapies using the CAR-T cells disclosed herein are also suited for treating cancers that typically do not elicit a strong immune response in a subject, e.g., a cancer other than a melanoma. In embodiments, the cancer therapies disclosed herein can be tailored or personalized to a given subject, e.g., by generating CAR-T cells (e.g., autologous CAR-T cells) that selectively and effectively target the subject's cancer.

Accordingly, provided herein are methods of generating CAR-T cells that have enhanced target cell killing activity; compositions comprising such immune cells; methods of using the cells (e.g., methods of treatment); methods of selecting optimal agents for enhancing the target cell killing activity, e.g., by enhancing the proximity, e.g., spatial proximity, between the target cell and the immune cell, e.g., T cell; methods of selecting an optimized (e.g., highest activity fractions/clones) immune cell, e.g., T cell; and methods of using this approach to evaluate patient responsiveness to other cancer therapies.

### Method of Producing a CAR-T cell

In one aspect, an *in vitro* method of producing a genetically engineered T cell expressing Chimeric Antigen Receptors (a CAR-T cell) or a CAR-T cell preparation:
(a) providing a sample comprising at least one T cell from a subject having a cancer;
(b) providing a sample comprising at least one cancer cell;
(c) forming an ex *vivo* reaction mixture comprising the at least one T cell, the at least one cancer cell, and a bispecific T cell engager antibody (BiTE) under conditions and for a period of time sufficient to allow the at least one T cell to become activated and kill at least one cancer cell, thereby producing at least one activated T cell;
(d) selecting the activated T cell, wherein the activated T cell is defined by having an effective E:T ratio higher than 1:5 between the number of activated T cells (E) and the number of target cancer cells (T) after exposure to the bispecific T cell engager antibody (BiTE); and
(e) genetically engineering the activated T cell to produce Chimeric Antigen Receptors (CAR) on the surface of the activated T cell, thereby producing at least one CAR-T cell.

In another aspect, an *in vitro* method of producing a genetically engineered T cell expressing Chimeric Antigen Receptors (a CAR-T cell) or a CAR-T cell preparation:
(a) providing a sample comprising at least one T cell from a subject having a cancer;
(b) providing a sample comprising at least one cancer cell;
(c) forming an *ex vivo* reaction mixture comprising the at least one T cell, the at least one cancer cell, and a bispecific T cell engager antibody (BiTE) under conditions and for a period of time sufficient to allow the at least one T cell to acquire a surface marker from at least one cancer cell, thereby producing at least one activated T cell;
(d) selecting the activated T cell, wherein the activated T cell is defined by having acquired a cell surface marker from at least one cancer cell after exposure to the bispecific T cell engager antibody (BiTE); and
(e) isolating or enriching the activated T cells that have acquired a surface marker, using a fluorescently labeled molecule (e.g., antibody or fragment thereof) that binds to i) one or more cancer antigens ii) one or more markers of activated T cells, or both i) and ii); and
(f) genetically engineering the selected activated T cells to produce Chimeric Antigen Receptors (CAR) on the surface of the activated T cell, thereby producing at least one CAR-T cell.

In another aspect, an *in vitro* method of producing a genetically engineered T cell expressing Chimeric Antigen Receptors (a CAR-T cell) or a CAR-T cell preparation:
(a) providing a sample comprising at least one T cell from a subject having a cancer;
(b) providing a sample comprising at least one cancer cell;
(c) Isolating or enriching the cancer cells from the sample, adding a membrane dye or a cell tracker dye,
(d) forming an *ex vivo* reaction mixture comprising the at least one T cell, the at least one cancer cell, and a bispecific T cell engager antibody (BiTE) under conditions and for a period of time sufficient to allow the at least one T cell to acquire a surface marker from at least one cancer cell, thereby producing at least one activated T cell;
(e) selecting the activated T cell, wherein the activated T cell is defined by having acquired a cell surface marker from at least one cancer cell after exposure to the bispecific T cell engager antibody (BiTE); and
(f) isolating or enriching the activated T cells that have acquired a cancer surface marker, using the fluorescently membrane dye and one or more markers of activated T cells,; and
(g) genetically engineering the selected activated T cells to produce Chimeric Antigen Receptors (CAR) on the surface of the activated T cell, thereby producing at least one CAR-T cell.

In an embodiment, the selecting and/or enriching step (a) comprises using fluorescence activated cell sorting (FACS). In another embodiment, the selecting and/or enriching step (a) comprises using a bead (e.g., magnetic bead) coated with an antibody or fragment thereof that binds to i) one or more cancer antigens or ii) one or more markers of activated T cells, or both i) and ii). In another embodiment, the cancer-killing T cell preparation is enriched or purified and comprises trogocytotic cancer-killing T cells, e.g., at a concentration of at least 50% (e.g., at least 50%, 55%, 60%, 65%, 70%, 75%, 80%, 85%, 90%, 95%, 99%, or greater) of the total number of cells in the preparation.

In another aspect, the ex vivo reaction mixture further comprises one or multiple agents that enhance T cell activity. The agents that enhance T cell activity are selected from one or more of a chemotherapy drug, a targeted anti-cancer therapy, an oncolytic drug, a cytotoxic agent, an immune-based therapy, a cytokine, an agonist of T cells (e.g., agonistic antibody or fragment thereof or an activator of a costimulatory molecule), an inhibitor of an inhibitory molecule (e.g., immune checkpoint inhibitor), an immunomodulatory agent, a vaccine, or a cellular immunotherapy. In another embodiment, the agents enhancing T cell activity is selected from an agonist of T cells (e.g., an agonistic antibody or fragment thereof or an activator of a costimulatory molecule), and/or an inhibitor of an immune checkpoint inhibitor. In another embodiment, the inhibitors of the immune checkpoint inhibitor is an inhibitor of one or more of: PDL-1, PDL-2, B7-1 (CD80), B7-2 (CD86), 4-1BBL, Galectin, ICOSL, GITRL, OX40L, CD155, B7-H3, PD1, CTLA-4, 4-1BB, TIM-3, ICOS, GITR, LAG-3, KIR, OX40, TIGIT, CD160, 2B4, B7-H4 (VTCN1), HVEM (TNFRSF14 or CD270), BTLA, KIR, MHC class I, MHC class II, GAL9, VISTA, LAIR1, and A2aR. In another embodiment, the inhibitors of the immune checkpoint inhibitor comprise one or more of: ipilimumab, tremelimumab, MDX-1106, MK3475, CT-011, AMP-224, MDX-1105, IMP321, or MGA271. In another embodiment, the agents enhancing T cell activity comprises molecules (e.g. antibodies) constructed combining fragments of these molecules enhancing T cell activity, e.g. bispecific or multispecific antibody formats combining recognition arms of several immune checkpoint inhibitors, including but not limited to PD1-PDL1, PD1-PDL2, PD1-LAG3, PD1-TIM3. In another embodiment, the agonist of T cells comprises an antibody or fragment thereof to CD137, CD40, and/or glucocorticoid-induced TNF receptor (GITR). In another embodiment, the immunomodulatory agent comprises/is lenalidomide, ibrutinib or bortezomib. In another embodiment, the agent enhancing T cell activity enhances and/or restores the immunocompetence of T cells. In another embodiment, the immunomodulatory agent is an inhibitor of MDSCs and/or Treg cells. In another embodiment, the immunomodulatory agent activates an immune response to a tumor specific antigen, e.g., it is a vaccine (e.g., a vaccine against targets such as gp100, MUC1 or MAGEA3. In another embodiment, the immunomodulatory agent is a cytokine, e.g., a recombinant cytokine chosen from one or more of GM-CSF, IL-7, IL-12, IL-15, IL-18 or IL-21. In another embodiment, the immunomodulatory agent is a modulator of a component (e.g., enzyme or receptor) associated with amino acid catabolism, signalling of tumor-derived extracellular ATP, adenosine signalling, adenosine production, chemokine and chemokine receptor, recognition of foreign organisms, or kinase signalling activity. In another embodiment, the immunomodulatory agent is an inhibitor (e.g., small molecule inhibitor) of IDO, COX2, ARG1, ArG2, iNOS, or phosphodiesterase (e.g., PDE5); a TLR agonist, or a chemokine antagonist.

In some embodiments of any of the methods and compositions disclosed herein, the sample is a cancer sample chosen from a hematological cancer, a solid cancer, a metastatic cancer (e.g., a CTC, a primary, secondary or additional metastatic cancer), or a combination thereof.

In another embodiment of any of the methods and compositions disclosed herein, the sample is a T cell sample chosen from a blood sample (e.g., peripheral blood sample), a bone marrow sample, a lymph node sample, a spleen sample, a tumor sample comprising a CTL, a TIL, or a combination thereof.

In embodiments of any of the methods and compositions disclosed herein, substantially no components (e.g., cells) have been removed or isolated from the sample.

In embodiments of any of the methods and compositions disclosed herein, the sample substantially maintains the microenvironment from the tissue of origin, e.g., substantially maintains the structure of the tumor or immune microenvironment.

In embodiments of any of the methods and compositions disclosed herein, the sample comprises a tumor-specific T cell. Without being bound by theory, tumor-antigen specific T cells can be immunosuppressed, e.g., when present in the tumor microenvironment. In one embodiment, the immunosuppressed tumor-antigen specific T cell is activated under the conditions described herein, e.g., upon contact with the cancer cell and a bispecific T cell engager antibody (BiTE).

In some embodiments of any of the methods and compositions disclosed herein, the sample or samples comprise the cancer cell and the T cell. For example, the sample may be from a hematological cancer (e.g., bone marrow, lymph-node derived cancer) that includes a T cell (e.g., a tumor-antigen specific CTL). The hematological sample may also comprise cancer cells, e.g., leukemic or lymphoma blast cells (e.g., a blast cell expressing one or more markers chosen from CD19, CD123, CD20 or others). In embodiments, addition of the bispecific T cell engager antibody (BiTE) to the sample promotes an interaction between the T cell and the cancer cell that activates the T cell (e.g., activates the tumor-antigen specific CTL). In some embodiments, the activated T cell acquires a cell surface marker from the cancer cell, e.g., becomes a trogocytotic T cell.

In other embodiments of any of the methods and compositions disclosed herein, the cancer is a solid tumor. The sample may comprise a tumor-antigen specific T cell (e.g., a CTL or a TIL) as described herein and a cancer cell. In embodiments, addition of the bispecific T cell engager antibody (BiTE) to the sample promotes an interaction between the T cell and the cancer cell that activates the T cell (e.g., activates the tumor-antigen specific CTL or TIL). In some embodiments, the activated T cell acquires a cell surface marker from the cancer cell, e.g., becomes a trogocytotic T cell.

In other embodiments of any of the methods and compositions disclosed herein, the sample comprises a metastatic sample, e.g., a sample derived from a subject with a metastatic cancer. In one embodiment, the metastatic sample comprises a CTC. In embodiments, the CTC is a tumor cell found in the peripheral blood of a subject with a cancer, e.g., a solid tumor. An *ex vivo* reaction mixture can be formed comprising a metastatic cancer cell and a T cell. In embodiments, the T cell can be obtained from the metastatic cancer sample (e.g., a primary tumor sample or a secondary tumor sample, or a combination thereof). In some embodiments, the *ex vivo* reaction mixture comprises a tumor-antigen specific T cell (e.g., a CTL or a TIL) that targets the metastatic sample (e.g., that targets the CTC, the primary tumor sample or a secondary tumor sample, or a combination thereof). In embodiments, the tumor-antigen specific T cell is activated in the presence of the bispecific T cell engager antibody (BiTE) and the metastatic sample (e.g., the CTC, the primary tumor sample or the secondary tumor sample, or a combination thereof). For example, in a metastatic cancer, tumor growth may occur in tissues different from the primary tumor site, e.g., referred to herein as secondary tumors. Cancer cells from the primary tumor may be different from secondary or other metastatic sites. For example, bone marrow tumor infiltration may occur in a solid tumor. As another example, metastatic tumor cells from a solid cancer, e.g., pancreas or breast cancer, that grow in the bone marrow can be biologically different from the tumor cells in the primary tumor. In such embodiments, activation of a T cell in the presence of the bispecific T cell engager antibody (BiTE) can be repeated in every tissue affected by the tumor cells in the subject. In such embodiments, the activated T cells (e.g., the activated tumor-antigen specific T cells) can be selective against the primary and secondary tumors present in the subject.

In one embodiment, the sample comprises a CTC. An *ex vivo* reaction mixture can be formed with the CTC-containing sample with a sample from the primary and secondary tumors present in the subject, thereby producing activated T cells (e.g., the activated tumor-antigen specific T cells) selective against the CTCs, the primary and secondary tumors present in the subject.

### Method for testing cellular responsiveness of primary cell populations

In one aspect, provided herein is an ex *vivo* method for testing cellular responsiveness of primary cell populations to a genetically engineered T cell expressing Chimeric Antigen Receptors (a CAR-T cell) that comprises:
i) submit a whole sample from a subject selected from: peripheral blood (PB), or bone marrow (BN), or lymph node (LN) to a separation process to isolate an Artificial Environment (AE) consisting in a plasma fraction, an erythrocyte fraction or a combination thereof, free from leucocytes,
ii) mix the leucocyte-free AE obtained in the previous step with a primary cell population,
iii) add to the mixture of step ii) at least one genetically engineered T cell expressing Chimeric Antigen Receptors (a CAR-T cell) to be tested, obtainable according to the methods for producing CAR-T cells,
iv) incubate the mixture obtained in step iii) during from 2 hours to 14 days to allow the genetically engineered T cell expressing Chimeric Antigen Receptors (a CAR-T cell) tested to exert any activity it might have on the primary cell population,
v) assess the viability and/or proliferation of the primary cell population in the presence or absence of the genetically engineered T cell expressing Chimeric Antigen Receptors (a CAR-T cell) tested,
vi) produce comparative data on viability and/or on proliferation of the primary tumor cell population between the assessment made in presence and in absence of the genetically engineered T cell expressing Chimeric Antigen Receptors (a CAR-T cell) tested and relate the data obtained to values indicative of the genetically engineered T cell expressing Chimeric Antigen Receptors (a CAR-T cell) activity for reducing/increasing viability and/or proliferation of the primary cell population.

### Composition, Reaction Mixtures and Pharmaceutical Composition

For the purposes of the present specification, the term "composition" includes CAR-T cells, which term includes activated tumor antigen-specific T cells, including, but not limited to, effector memory T cells, cytotoxic T lymphocytes (CTLs), helper T cells, tumor infiltrating lymphocytes (TILs) and trogocytotic T cells, and pharmaceutical compositions thereof.

In one aspect, provided herein is a composition comprising a CAR-T cell or CAR-T cell preparation thereof obtainable according to the method of producing a CAR-T cell.

In an aspect, also featured herein is an *ex vivo* reaction mixture comprising a T cell, a cancer cell, and a bispecific T cell engager antibody (BiTE), where the T cell and the cancer cell are in a sample, e.g., a blood sample (e.g., whole blood, peripheral blood); a sample from a hematological cancer; a sample from a bone marrow, a sample from a lymph node; or a sample from a spleen, a sample from a solid tumor; a sample from a metastatic cancer (e.g., a CTC); where substantially no components (e.g., cells) have been removed or isolated from the sample.

In embodiments, the sample is from a subject having a cancer, e.g., a hematological cancer, a solid cancer or a metastatic cancer.

In embodiments, the sample substantially maintains the microenvironment, e.g., substantially maintains the structure of the tumor microenvironment.

In embodiments, the sample comprises a tumor-antigen specific T cell (e.g., a CTL or a TIL). Without being bound by theory, the tumor-antigen specific T cell can be immunosuppressed, e.g., when present in the tumor microenvironment. In one embodiment, the immunosuppressed tumor-antigen specific T cell can be activated under the conditions described herein, e.g., upon contact with the cancer cell and the bispecific T cell engager antibody (BiTE). In one embodiment, the immunosuppressed tumor-antigen specific T cell can be activated under conditions adding to the BiTE one of multiple agents enhancing T cell activity that further facilitate T cell activation, where such agents can be drugs or drug candidates or known biological agents, and they can be added one by one on in combination, especially where multiple are combined at the same time with the BiTE to further promote T cell activation. An example would be immune check point inhibitors, that we and other have shown that adding them to the incubation conditions results in more activated T cells and sometimes better cancer-cell killing. In this aspect, ex vivo assays can exploit the effects of multiple T cell enhancing agents, for example adding all possible immune check point inhibitors, to facilitate activation of the tumor-specific T cell, while in a patient only 1-3 immunotherapies can be combined given their toxicity. In another aspect, provided herein is a composition, e.g., a pharmaceutical composition, comprising a CAR-T cell produced by a method described herein and a pharmaceutically acceptable carrier, e.g., a Good Manufacturing Practices (GMP)- acceptable carrier.

In yet another aspect, the disclosure features a composition (e.g., a purified preparation). The composition includes:
(1) a CAR-T cell, which: (i) has cytotoxic activity toward a cancer cell, and (ii) comprises a cell surface marker derived from the cancer cell in an amount of 90 - 500 copies of a cell surface marker (+e.g., at least 90, 100, 200, 300, 400, or 500 copies) of e.g. one or more cancer cell surface markers; where said cell surface marker could also be a membrane fluorescent dye used to measure trogocytosis and
   (optionally) (2) bispecific T cell engager antibody (BiTE), e.g., a detectable (e.g., trace) amount of bispecific T cell engager antibody (BiTE), and
   (optionally) (3) immunotherapy agents such as immune check point inhibitors, e.g. a detectable (e.g., trace) amount of one or more immuno therapy molecules, including drug or drug candidates, such as immune check point inhibitors.

In embodiments, the composition further comprises a pharmaceutically acceptable carrier, e.g., a GMP-acceptable carrier.

In one embodiment, about 2 to 75% (e.g., about 2 to 70%, 2 to 60%, 2 to 50%, or 2 to 40%) of the total T cells in the reaction mixture express one or more cancer cell surface markers, including cell membrane dyes used to measure trogocytosis (e.g., one or more leukemic cell cancer markers).

In embodiments, the CAR-T cell is enriched or purified. In some embodiments, the enriched or purified CAR-T cell population comprises at least 80%, 90%, 95%, 99% or 100% CAR-T cells, wherein the CAR-T cells comprise one or more cancer cell surface markers.

In an aspect, also featured herein is a pharmaceutical composition comprising the composition and a pharmaceutically acceptable carrier.

### Method of Treatment

In one aspect, provided herein is a method for treating a subject having cancer comprising providing a CAR-T cell or a CAR-T cell preparation thereof obtainable according to the method of producing a CAR-T cell or the composition, and administering an effective amount of the CAR-T cell, the preparation or composition to the subject.

In another aspect, the disclosure features a method of treating a subject having cancer (e.g., a hematological cancer, a solid cancer, or a metastatic cancer as described herein). The method includes providing a preparation comprising CAR-T cells made by a method described herein; and administering the preparation to the subject.

In some embodiments, the CAR-T cells are administered without substantial expansion. In other embodiments, the CAR-T cells are administered after cell expansion, e.g., after expansion of individual cells.

In some embodiment of any of the aforesaid methods, the number of activated (e.g., cancer-killing) T cells, e.g., in the sample, administered to the subject is at least 5-1,000,000 (e.g., 5, 10, 100, 1000, 10,000, 100,000, 1,000,000 or more). In some embodiment of any of the aforesaid methods, the number of activated (e.g., cancer-killing) T cells, e.g., in the sample, administered to the subject is at least 1 billion (e.g., 10⁹, 10¹⁰, 10¹¹, 10¹², 10¹³ or more).

It is important to select when a patient sample can generate the right BiTE-activated T cells that are expected to be a good source to construct CAR-T cells. We distinguish 2 cases:
1. Most BiTE-activated T cells in immunosuppressed environments such as bone marrow, lymph nodes, or solid tumors, would be expected to be enriched in memory T cells. This is a difference from standard CAR-T cells generated from peripheral blood, composed mostly of naive T cells. This difference may provide an advantage in killing cancer cells because memory T cells are already trained to kill other cells. Based on this analysis, CAR-T from BiTE-activated T cells may have better killing activity than standard CAR-Ts.
2. Tumor-specific antigen T cells would represent some of the best T cell sources for CAR-Ts, because they may solve a key limitation of CAR-Ts. Standard CAR-T cells are generated using peripheral blood naive T cells. A limitation of these standard CAR-T cells is that they can only recognize the tumor antigen of the CAR construct. However, tumor cells can be heterogeneous with some clones not expressing the CAR antigen leading to resistance to such CAR-T cells. Relapsed patients after treatment with CAR-T cells often demonstrate this resistance mechanism. To circumvent this problem, the ideal T-cells in which to graft CAR genes could be Tumor-Specific T cells, potent effector T-cells with broader and more selective anti-tumor activity. Such T cells may combine the potency of the transfected CAR construct while retaining their ability to recognize and kill tumor cells expressing different CAR-resistant and tumor-specific antigens. The key property of these Tumor-specific T cells is their high activity against these tumor cells, and this if how we can identify them in these ex vivo assays. These tumor-specific T cells are created in the thymus and travel to the tumor tissues, and thus tumor tissues should be enriched with tumor-specific T cells. If those tumor tissues have an immunosuppressive microenvironment, the tumor-specific T cells may become immunosuppressed. In solid tumors, tumor-infiltrated lymphocytes (TILs) should be enriched in tumor-specific T cells. In hematological malignancies, there are many types of T cells in hematological tissues such as bone marrow, and identification of the tumor-specific T cells is very difficult, only achieved in multiple myeloma (Borrello I *et al.,* 2016). Thus, when we incubate a tumor tissue sample such as bone marrow with a BiTE, and the BiTE activated T cells that kill tumor cells, some of these T cells are expected to be tumor-specific T cells, and some other normal T cells. To identify these tumor-specific T cells, to be selected as a source to generate CAR-Ts, we can measure their cancer-killing activity and select the T cells with high cancer-killing activity, which are expected to be, or be enriched in, the tumor-specific T cells. We claim 2 novel methods to identify these high activity cancer-killing T cells in the BiTE-incubation assay:
   a. Trogocytosis: Tumor-specific antigen T cells recognize specific tumor antigens and thus would bind to these antigens with high affinity and very fast. Upon binding, they would kill the tumor cell quickly. Thus, the subset of activated T cells that kill tumor cells faster are likely to be tumor-specific T cells. We have discovered that when T cells kill tumor cells they extract some membrane surface markers that become part of the T cell membrane surface and can be identified by flow cytometry if these markers are fluorescent. This process is called trogocytosis. We have observed trogocytosis in BiTE-generated activated T cells that kill tumor cells suing fluorescently labelled antibodies on tumor cells, and also adding a membrane cell tracker fluorescent dye to the tumor cell. Both examples are shown in our patent application number 62/321 ,964 filed with the United States PTO included herein as reference. Isolating trogocytotic T cells generated in the BiTE assay at early time points would be expected to generate a pool of T cells enriched in tumor-specific antigen T cells for said tumor.
   b. Effective E:T Ratios: We can evaluate the cancer-killing activity of BiTE-generated activated T cells in our ex vivo assays. As shown before in our patent application number 62/321,964 filed with the United States PTO, included herein as reference, we have discovered algorithms to evaluate the cancer-killing activity of different patient samples. We measure how many tumor cells are killed by newly generated activated T cells, and we call this measurement Effective E:T Ratio. This measurement varies considerably across patients from 1:0.5 to 1:150, enabling the identification of patient samples that BiTE incubation generates high cancer-killing T cells. These T cells are expected to be enriched in tumor specific T cells. Thus this method can be used to select patient samples whose BiTE-activated T cells are a good source for CAR-Ts. This methodology is described below.

### Assays and Methods for Evaluating the Activity of BiTE-Generated Activated T Cells: E:T Ratios

In another aspect, provided herein is a method of, or assay for, evaluating the potency of a BiTE-generated activated T cell or preparation thereof. The method, or assay, includes:
(a) providing a T cell or a preparation thereof, e.g., produced according to a method described herein, e.g., from a subject (e.g., a subject with a cancer as described herein);
(b) providing a target cell (e.g., a cancer cell), e.g., wherein the cancer cell is from the subject;
(c) contacting the T cell or the preparation thereof with the target cell (e.g., cancer cells), under conditions (e.g., for a period of time) sufficient to allow the T cell to kill the cancer cell (in embodiments, the contacting step further comprises addition of a bispecific T cell engager antibody (BiTE) and/or an immunomodulatory agent as described herein, e.g., at different doses (e.g., increasing dosages);
(d) determining the level, e.g., number, of target cells that have been eliminated after step (c) (e.g. relative to a sample without adding a bispecific T cell engager antibody (BiTE) or immunomodulatory agent, e.g., a sample from the same subject without adding a bispecific T cell engager antibody (BiTE)), and (optionally) determining the level, e.g., number, of T cells produced (e.g., newly generated cells) after step (c) (e.g. relative to a sample without adding a bispecific T cell engager antibody (BiTE), e.g., a sample from the same subject without adding a bispecific T cell engager antibody (BiTE) or immunomodulatory agent) (in embodiments, the level, e.g., number, of target cells and/or T cells is determined at one or more time intervals after step (c)); and
(optionally) (e) determining the ratio of either target cell to T cell, or T cell to target cell, from step (d), at different doses (e.g., increasing ratios).

In some embodiments of any of the aforesaid methods or assays, a basal E:T ratio is obtained. In one embodiment, the basal E:T is the ratio between the cytotoxic T cells and the cancer cells before BiTE and/or immunomodulatory agent exposure.

In some embodiments of any of the aforesaid methods or assays, an Effective E:T ratio is obtained. In one embodiment, the Effective E:T ratio is the ratio between the activated T cells generated and the cancer cells killed after bispecific T cell engager antibody (BiTE) and/or immunomodulatory agent exposure.

In some embodiments of any of the aforesaid methods or assays, the Effective E:T ratio can be calculated at one or more predetermined concentrations of the bispecific T cell engager antibody (BiTE). In one embodiment, the predetermined concentration of the bispecific T cell engager antibody (BiTE) is optimized for calculating the Effective E:T ratio. In one embodiment, the E:T ratio is calculated using the numbers of tumor and activated T cells when exposed to the maximum concentration of bispecific T cell engager antibody (BiTE). In another embodiment, the E:T ratio is calculated using the numbers of tumor and activated T cells when exposed to the concentration of the bispecific T cell engager antibody (BiTE) that generate a maximum peak in the number of activated T cells. In a further embodiment, the E:T ratio is calculated using the numbers of tumor and activated T cells that correspond to the EC50 concentration of the respective dose response curves.

In some embodiments of any of the aforesaid methods or assays, the Effective E:T ratio can also be expressed as the Effective T:E ratio (e.g., ratio between cancer cells killed to the activated T cells generated).

In some embodiments, the CAR-T cell produced by a method described herein is provided. In some embodiments, the CAR-T cell is a trogocytotic T cell. In other embodiments, the CAR-T cell is a activated T cell with a high killing activity, e.g a high Effective E:T Ratio. In other embodiments, the CAR-T cell is a CD8+CD25+ T cell. In other embodiments, the CAR-T cell is a CD4+CD25+ T cell. The trogocytotic T cell is believed to be a more effective cancer cell killer, although the cytotoxic T cells, e.g., CD8+ T cells and activated CD4+ T cells also have cancer cell killing activity. Accordingly, all activated T cell types can be included in the Effective E:T ratio.

In some embodiments, the method or assay includes detecting, e.g., counting, the number of newly generated CAR cytotoxic T cells, and the number of targets cells that have been killed under the same conditions, e.g., in the same well. The ratio of these values is the Effective E:T ratio.

In some embodiments, the ratio is a ratio between two subtractions, one subtraction is the number of targets after incubation with a BiTE relative to control well without the BiTE also after incubation (i.e., to measure the number of target cells killed in such condition), and the other subtraction is the number of activated T cells after incubation with a BiTE relative to control wells without the BiTE also after incubation (i.e., to measure the number of cytotoxic t cells that kill the target cells in such condition). In some embodiments, there are zero (or no detectable) cancer killing T cells without the bispecific T cell engager antibody (BiTE), and thus the subtraction equals the total number for activated T cells (e.g., total number of CD8+CD25+ T cells or total number of CD4+CD25+ T cells).

In embodiments of any of the aforesaid methods or assays, a decrease in the level or amount of cancer cells, e.g., relative to a reference level without adding a bispecific T cell engager antibody (BiTE) and/or immunomodulatory agent, is indicative of increased cancer cell killing. In other embodiments, a reduced change or no substantial change in the level or amount of cancer cells, e.g., relative to a reference level, is indicative of decreased cancer cell killing.

In embodiments of any of the aforesaid methods or assays, a high level of target cell killing relative to the newly generated target killing T cells (e.g., a high Effective ratio of target cell to activated T cell) induced by the bispecific T cell engager antibody (BiTE) and/or immunomodulatory agent indicates that the activated T cell or preparation thereof is an effective killer of cancer cells. In one embodiment, the target to T cell ratio is compared to a reference ratio. For example, a ratio of 1 (T cell) to 10, 20, 30, 40, 50, 75, 100, 500 or higher (target cells) is indicative of potent T cell killing activity. In a preferred embodiment, the ratio T cell: target cells ranges 1:100, or higher. A subject having T cells having potent cell killing activity can be identified as being a strong responder to the bispecific T cell engager antibody (BiTE) and/or immunomodulatory agent.

In one embodiment, the reference ratios are the ratio between two subtractions:
- The number of target cells without bispecific T cell engager antibody (BiTE) minus the number of target cells adding bispecific T cell engager antibody (BiTE) (ΔT^{BiTE}), both sharing the same experimental incubation conditions,
   ∘ Wherein this number is calculated by subtracting the number of target cells at the dose of bispecific T cell engager antibody (BiTE) that induces the highest target cell killing, or alternatively the highest dose of the bispecific T cell engager antibody (BiTE),
   ∘ Wherein the maximum and minimum values are derived from mathematical fitting of the experimental values of a dose response curve of multiple doses of the bispecific T cell engager antibody (BiTE).
- The number of effector cancer killing T cells adding the bispecific T cell engager antibody (BiTE) minus the number of effector cancer killing T cells without the bispecific T cell engager antibody (BiTE) (ΔE^{BiTE}), both sharing the same incubation conditions,
   ∘ Wherein this number is calculated by subtracting the number of target cells at the dose of bispecific T cell engager antibody (BiTE) that induces the highest target cell killing, or alternatively the highest dose of bispecific T cell engager antibody (BiTE),
   ∘ Wherein the maximum and minimum values are derived from mathematical fitting of the experimental values of a dose response curve of multiple doses of the bispecific T cell engager antibody (BiTE).
- The ratio between these two variables is defined as the Effective E:T Ratio and equals ΔT^{BiTE} / ΔE^{BiTE}.
- This Effective E:T ratio measures the number of target cells that have been killed by a single cancer killing T cell in such conditions. This ratio can be similar for the same sample and bispecific T cell engager antibody (BiTE) in different incubation times, because it represents the activity of the same activated T cell, generated at different times.

In some embodiments, the Effective E:T Ratio represents an estimate of the activity of the generated activated T cell in killing cancer target cells. Without wishing to be bound by theory, it is equivalent to the activity of a drug in killing cancer cells, because the activated T cell is indeed an active medicament for treating a subject, e.g., a cancer patient. The Effective E:T Ratio can rank the activity of activated T cells from different patients thus stratifying those patients. This ranking or stratification can be very different than the ranking or stratification derived from the standard method of measuring the efficacy in killing cancer target cells. For example, a very efficacious activated T cell with a 1:100 Effective E:T Ratio, that eliminates 100 target cells per activated T cell, may not be able to kill all cancer cells if that patient has a very large density of cancer target cells. Leaving alive many cancer cells would normally be considered a sign of low activity for the activated T cell in a standard chemotherapy activity measurement; in this case, it would miss the true high activity of the activated T cell generated by the bispecific T cell engager antibody (BiTE), the problem being some cancer cells are immunosuppressed and resistant to the otherwise high activity CAR activated T cells generated. Hence, the Effective E:T ratio can identify the most active activated T cells, e.g., those activated T cells better suited to be administered to the patient, and to be used as a source to transfect a CAR making a CAR-T product.

Alternatively, a low level of Effective E:T Ratio is indicative of a poor T cell killing activity. In one embodiment, a ratio activated T cells:target cells of 1:1 (e.g., 1, 3, or 5 ) is indicative of poor T cell killing activity. A subject having T cells having reduced cell killing activity can be identified as being a poor responder to the bispecific T cell engager antibody (BiTE) and/or immunomodulatory agent.

There are alternative approaches to estimate the activity of activated T cells, besides the Effective E:T Ratios, basal E:T Ratios, EC50, Emax, kinetics, and association of these variables. More sophisticated mathematical calculations and different ways of fitting the experimental data, using different pharmacological operational models, can provide different ways to calculate how many cancer cells are killed by a activated T cells relative to the herein proposed Effective E:T Ratio. Those alternative approaches to calculate essentially the same concept to estimate the activity of the activated T cells are also considered covered by the definition of Effective E:T Ratios.

In embodiments of any of the aforesaid methods or assays, the level of target cells and/or activated T cells is determined at one or more time intervals after step (c). In exemplary embodiments, the level of target cells and/or activated T cells is determined at time 0, at time 1 - 168 hours (e.g., 1, 2, 4, 8, 16, 24, 48, 72, 96, 120, 144, or 168 hours) or several days or weeks after step (c).

In embodiments of any of the aforesaid methods or assays, the contacting step further comprises addition of a bispecific T cell engager antibody (BiTE) and/or immunomodulatory agent at different doses (e.g., increasing dosages) of the bispecific T cell engager antibody (BiTE) and/or immunomodulatory agent, e.g., to generate a dose-response curve. In one embodiment, the difference between the level of T cells or cancer cells at a dose zero or at control level (e.g., a threshold dose) and a saturated dose of the bispecific T cell engager antibody (BiTE) and/or immunomodulatory agent is determined. In embodiments, the difference in the level of T cells or cancer cells at the saturated dose vs. threshold dose is determined. In embodiments, the Effective E:T ratio as used herein is the ratio of the difference in the level of T cells relative to the difference in the level of cancer cells. In embodiments, the Effective E:T ratio as used herein is the ratio of the number of T cells and target cells at their respective EC50 concentration.

In embodiments of any of the aforesaid methods or assays, the method is performed using an automated platform, e.g., an automated fluorescence-based platform, e.g., the ExviTech^{®} platform described herein.

In embodiments of any of the aforesaid methods or assays, the activity of the bispecific T cell engager antibody (BiTE) and/or immunomodulatory agent is determined using an ex *vivo*/*in vitro* assay to measure dose response curves, whose mathematical fitting enable quantitative parameters to estimate the activity, selected from at least one from EC50, Effective E:T ratio, basal E:T ratios, Emax or kinetics.

In embodiments of any of the aforesaid methods or assays, the activity of the bispecific T cell engager antibody (BiTE) and/or immunomodulatory agent assessed by step (e) is different from an activity assessment using a dose response of the bispecific T cell engager antibody (BiTE) and/or immunomodulatory agent activity, e.g., compared to a standard depletion dose response curve.

In embodiments of any of the aforesaid methods or assays, the reference ratio is a predetermined ratio, e.g., about 1:3 to 1:10, e.g., about 1:3, 1:4, 1:5, 1:6, 1:7, 1:8, 1:9, or 1:10. In embodiments, the T cell to high target cell ratio from step (e) is about 1:4 - 1:500 (e.g., 1:4, 1:5, 1:6, 1:7, 1:8, 1:9, 1:10, 1:15, 1:20, 1:25, 1:30, 1:35, 1:40, 1:45, 1:50, 1:75, 1:100, 1:500, or higher).

In embodiments of any of the aforesaid methods or assays, step (c) comprises forming ex *vivo* mixtures of the activated T cell or the preparation thereof with target cells, e.g., cancer cells. In embodiments, the cancer cell is a cell chosen from a hematological cancer, a solid cancer, a metastatic cancer (e.g., a CTC, or a combination thereof). In embodiments, the cancer cell is a leukemic or lymphoma blast cell (e.g., a blast cell expressing one or more markers chosen from CD19, CD123, CD20 or others). In embodiments, the T cell is a cell chosen from a blood sample (e.g., peripheral blood sample), a bone marrow sample, a lymph node sample, a spleen sample, a tumor sample comprising a CTL and/or a TIL, or a combination thereof). In embodiments, the T cell expresses CD8 and/or CD25 (e.g., it is a CD8+CD25+ T cell). In other embodiments, the T cell expresses CD4 and/or CD25 (e.g. it is a CD4+CD25+ T cell).

In embodiments of any of the aforesaid methods or assays, the CAR-T cell or preparation thereof is produced using a method that comprises use of a bispecific T cell engager antibody (BiTE) and/or immunomodulatory agent, e.g., a bispecific T cell engager antibody (BiTE) and/or immunomodulatory agent described herein.

In embodiments of any of the aforesaid methods or assays, the CAR-T cell or preparation thereof comprises a T cell, e.g., CTL, that is CD8+ and CD25+, or a CD4+ and CD25+, or both.

In embodiments of any of the aforesaid methods or assays, the candidate bispecific T cell engager antibody (BiTE) and/or immunomodulatory agent is administered at different dosages (e.g., at increasing dosages).

In embodiments of any of the aforesaid methods or assays, an increase in the cell killing activity of the T cells in the presence of the candidate bispecific T cell engager antibody (BiTE) and/or immunomodulatory agent is indicative of high efficacy of the bispecific T cell engager antibody (BiTE) and/or immunomodulatory agent. Alternatively, a small change or no substantial change in the cell killing activity of the T cells in the presence of the candidate bispecific T cell engager antibody (BiTE) and/or immunomodulatory agent is indicative of low efficacy of the bispecific T cell engager antibody (BiTE) and/or immunomodulatory agent.

### Assays and Methods for Evaluating the Activity of CAR-T Cells and Other T Cell Therapies

In some embodiments, the cancer-killing activity of different T cell therapies can be evaluated on the same patient sample ex vivo, where the T cells can be selected from the group consisting of a tumor infiltrated lymphocyte (TIL), marrow infiltrated lymphocytes (MILs), a genetically engineered T cell, a CAR-T cell including comparing different CAR constructs, an activated T cell obtainable according to step (c) of the method of producing a CAR-T cell and a genetically engineered T cell expressing Chimeric Antigen Receptors obtainable according to step (e) of the method of producing a CAR-T cell.

To detect the lysis of the leukemic target population by CART cells in mononuclear cells from whole Bone Marrow or Peripheral Blood, serial dilutions of CART cells are incubated with a leukemic cells labelled with a membrane cell tracker dye such as PKH67, different time incubation times (6-12-24h-72h). The cells are then harvested and stained to recognize both target (Leukemic Cells) and Effector (CART) and the Annexin-V. Cell lysis is measured as number and % of surviving target cells with the ExviTech^{®} platform (detailed in the Experiment 2). The absolute cell count by the platform will allow to quantify from sample to sample the real effect of the CART cells and a direct comparison between CART and BITE-generated T-cells, or any other T cell therapy, on the same patient sample ex vivo.

In some embodiments, an important comparison is the activated T cell generated incubating with a BiTE, with the same activated T cells transfected with a CAR, because the BiTE-generated T cell would be safer and thus a preferred treatment than the CAR transfected T cell if the CAR transfected T cell is not substantially better.

In some embodiments, the activity of these different T cell therapies are first evaluated against at least 30 patient samples of the same cancer type that represent the patient population, and afterwards the activity of each T cell therapy is compared with the activity across the population of patient samples, deriving a sensitivity ranking. Combinations of these different T cell therapies with other drugs can be also evaluated to guide patient treatment, where drugs that can be combined for each disease include approved drugs for said disease, and especially other immunotherapies such as immune check point inhibitors, immunomodulatory drugs, etc... This methodology has been described for multiple drugs and combination treatments for AML in a publication (Bennett *et al.,* 2014), included herein as reference. It is reviewed here below.

Flow cytometry is the method chosen for the diagnosis and monitoring of patients with hematological malignances. Additionally, it has been validated for the study of cellular death or apoptosis processes induced by drugs. The ExviTech^{®} platform allows the escalation of flow cytometry technology, with the ability to measure the effect of a high number of drugs and combinations selectively in pathological cells (identified in a similar manner than in the diagnosis of the disease) of an individual patient's sample.

To perform the Precision Medicine (PM) Test, the patient's bone marrow sample is received, and a small aliquot is first analyzed to determine the number of live pathological cells present in the sample. The rest of the sample is diluted with a culture medium, and is divided into 96 well plates, containing the drug treatments (monotherapies and combinations) to be studied. 8 concentrations are studied for each treatment (drug or combination), duly adjusted to cover each treatment's range of pharmacological activity tested in multiple patient samples. The plates are later incubated at control temperature for certain time, from 12 to 48 hours. Subsequently, the sample is marked with the specific monoclonal antibodies to identify the leukemic cells, together with Annexin V. The presence of this last marker indicates that the cell has entered into apoptosis or programmed death. Therefore, cells that present the phenotype of a leukemic cell and the absence of Annexin V are identified as live leukemic cells (LLC).

The proportion of the number of live leukemic cells after the incubation present in the control wells (without drugs) compared to the wells containing each of the treatments or, which is equivalent, the percentage "survival index", is the measure of efficacy of the tested treatments for the specific patient that PM Test measures. PM Test then ranks treatments in order of efficacy based on the "survival index" measured for each treatment. The lower the "survival index" (the lesser number of leukemic cells alive), the more efficient the treatment will be.

PM incorporates modern pharmacokinetic and pharmacodynamic population modelling technologies, increasingly used in clinical trials for new drugs, to analyze the test's flow cytometry data. This enables making very accurate estimates in complex multiple-variable systems subject to high variability. In the case at hand, by using this technology ExviTech^{®} generates dose-response models that evaluate the patient's cellular response to increasing drug concentrations in the patient's bone marrow sample, measured as cellular death or depletion. The final model estimated is characterized by a set of pharmacological parameters that describe the effect of the drug or combination.

Additionally, to the estimation of these parameters, population models enables to analyze typical population values to put the patient's individual data in context of a patient population, inter-individual variability data associated to each parameter, and relative standard error individually associated to each estimation.

Graphically, pharmacodynamics models based on Hill equation are represented by typical sigmoidal curves of measured effect at increasing drug concentrations. These graphs allow a quick interpretation of drug biological effect and a direct comparison with population typical behavior. Individual model functions can be summarized with the value of the Area Under the Curve (AUC) that it is used as a general activity marker.

Treatments scores are calculated using the AUC values of dose-response model function of each individual drug included in a clinical treatment, together with the contribution of the synergy from binary combinations which is estimated from sophisticated drugs interaction surface models.

The estimation of accurate residual errors and confidence intervals associated to parameters allows the application of quality control criteria to the results provided by the test. Thus estimations associated to high error levels are automatically discarded.

The key to interpret the ex-vivo activity of individual drugs in a patient sample is not just the absolute value of the pharmacological variables, but their reference rank compared to a statistically representative patient population. This is why the results of PM Test are expressed in population terms, normalized to a reference activity range of the patient population.in terms of cellular efficacy of a treatment in terms of tumor cell killing for the individual patient compared with the cell killing efficacy of the same treatment in a reference patient population.

### Method of identifying immune checkpoint molecules

In one aspect, methods of identifying immune checkpoint molecules that would benefit an individual patient are described, for 3 different types of immunotherapy treatments:
- Monotherapy treatment with only one immune check point molecule. The ex vivo assay uses a BiTE as a reagent to activate T cells. In another embodiment, BiTE-activated T cells are isolated and then mixed with cancer cells.
- Combination treatment with a BiTE adding one immune check point molecule. The ex vivo assay uses the BiTE that the patient may be treated as a drug. In another embodiment, BiTE-activated T cells are isolated and then mixed with cancer cells.
- Combination treatment with a T Cell therapy such as CART (or other immunotherapy) adding one immune check point molecule. There is no BiTE added, because the T cells directly kill the tumor cells.

In another aspect, two different methods are used to identify the immune check point molecule appropriate for each patient:
∘ Functional ex vivo assay to measure activity of T cells killing cancer cells
∘ Expression of immune check point molecules at basal vs resistant population after incubation in ex vivo assay above.
∘ Both methods combined

In one aspect, provided herein is an *in vitro* method of identifying subjects susceptible to immune checkpoint immunotherapy treatment, comprising:
(a) providing a sample comprising at least one T cell from a subject having a cancer;
(b) providing a sample comprising at least one cancer cell, e.g., from the subject;
(c) forming an ex *vivo* reaction mixture comprising the at least one T cell, the at least one cancer cell, and a bispecific T cell engager antibody (BiTE), under conditions (e.g., for a period of time) sufficient to allow the T cell to kill cancer cells, thereby producing the cancer-killing T cell
(d) determining the pharmacological activity of the cancer-killing T cells obtained in step (c) by dose response and/or pharmacodynamic parameters of cancer-killing T cells and tumor cells, selected from EC50, Emax, AUC, Effective E:T Ratios, Basal E:T Ratios, or kinetic parameters;
(e) determining the pharmacological activity of the cancer-killing T cells repeating steps (c) and (d) by dose response or evaluating a single high saturating dose in combination with immune check point inhibitors, individually, or in combinations, or bispecific or multispecific antibody constructs combining immune checkpoint inhibitors, including the combination of all immune checkpoint inhibitors;
(f) determining the expression levels of immune checkpoint molecules in both the tumor cells and T cells in the reaction mixture of step (c), comparing basal levels with levels after incubation;
(g) identifying subjects susceptible to immune checkpoint immunotherapy treatment, whereby the bispecific T cell engager antibody (BiTE) incubation is only a reagent to activate T cells, by assessment of either of the following 2 criteria or a combination of them:
   i. step (d) reveals a resistant tumor cell population in the samples from the subject (i.e. incubation with the bispecific T cell engager antibody (BiTE) does not kill all tumor cells), and addition of one or more immune checkpoint inhibitors in (e) reverts resistance of tumor cell population;
   ii. step (f) reveals an increase in the expression level of an immune checkpoint molecule in either the tumor cells and/or T cells in the reaction mixture of step (c) after incubation, relative to basal levels prior incubation,
and wherein observance of both (i) and (ii) is indicative of a subject more susceptible to immune checkpoint immunotherapy treatment.

In another aspect, provided herein is an *in vitro* method of identifying subjects susceptible to immune checkpoint immunotherapy treatment, comprising:
(a) providing a sample comprising at least one T cell from a subject having a cancer;
(b) providing a sample comprising at least one cancer cell, e.g., from the subject;
(c) forming an *ex vivo* reaction mixture comprising the at least one T cell, the at least one cancer cell, and a bispecific T cell engager antibody (BiTE), under conditions (e.g., for a period of time) sufficient to allow the T cell to kill cancer cells, thereby producing the cancer-killing T cell
(d) Isolating the activated T cells, by FACS or magnetic-beads or other methods, adding them to a cancer cell, e.g., from the subject, forming an *ex vivo* reaction mixture comprising under conditions (e.g., for a period of time) sufficient to allow the activated T cells to kill cancer cells; and;
(e) determining the pharmacological activity of the cancer-killing T cells obtained in step (d) by dose response and/or pharmacodynamic parameters of cancer-killing T cells and tumor cells, selected from EC50, Emax, AUC, Effective E:T Ratios, Basal E:T Ratios, or kinetic parameters and;
(f) determining the pharmacological activity of the cancer-killing T cells repeating steps (d) and (e) by dose response or evaluating a single high saturating dose in combination with immune check point inhibitors, individually, or in combinations, or bispecific or multispecific antibody constructs combining immune checkpoint inhibitors, including the combination of all immune checkpoint inhibitors;
(g) determining the expression levels of immune checkpoint molecules in both the tumor cells and T cells in the reaction mixture of step (d), comparing basal levels with levels after incubation;
(h) identifying subjects susceptible to immune checkpoint immunotherapy treatment, whereby the bispecific T cell engager antibody (BiTE) incubation is only a reagent to activate T cells, by assessment of either of the following 2 criteria or a combination of them:
   i. step (e) reveals a resistant tumor cell population in the samples from the subject (i.e. incubation with BiTE-activated isolated T cells does not kill all tumor cells), and addition of one or more immune checkpoint inhibitors in (f) reverts resistance of tumor cell population;
   ii. step (g) reveals an increase in the expression level of an immune checkpoint molecule in either the tumor cells and/or T cells in the reaction mixture of step (d) after incubation, relative to basal levels prior incubation,
and wherein observance of both (i) and (ii) is indicative of a subject more susceptible to immune checkpoint immunotherapy treatment.

In another aspect, provided herein is an *in vitro* method of identifying subjects susceptible to immune checkpoint immunotherapy treatment to be combined with a bispecific T cell engager antibody (BiTE) immunotherapy, for decreasing resistance of said subject to said BiTE immunotherapy, comprising:
(a) providing a sample comprising at least one T cell from a subject having a cancer;
(b) providing a sample comprising at least one cancer cell, e.g., from the subject;
(c) forming an *ex vivo* reaction mixture comprising the at least one T cell, the at least one cancer cell, and the the bispecific T cell engager antibody (BiTE), being identical to BiTE of the immunotherapy, e.g., under conditions (e.g., for a period of time) sufficient to allow the T cell to kill cancer cells, thereby producing the cancer-killing T cell;
(d) determining the pharmacological activity of the cancer-killing T cells obtained in step (c) by dose response and/or pharmacodynamic parameters of cancer-killing T cells and tumor cells, selected from EC50, Emax, AUC, Effective E:T Ratios, Basal E:T Ratios, or kinetic parameters;
(e) determining the pharmacological activity of cancer-killing T cells obtained in step (c) by by dose response or evaluating a single high saturating dose in combination with immune check point inhibitors, individually, or in combinations, or bispecific or multispecific antibody constructs combining immune check point inhibitors, including the combination of all immune check point inhibitors;
(f) determining the expression levels of immune checkpoint molecules in both the tumor cells and T cells in the reaction mixture of step (c), comparing basal levels with levels after incubation,
(g) identifying subjects susceptible to immune checkpoint immunotherapy treatment to be combined with a bispecific T cell engager antibody (BiTE) immunotherapy, by assessment of either of the following 2 criteria or a combination of them:
   i. step (d) reveals a resistant tumor cell population in the samples from the subject (i.e. incubation with the bispecific T cell engager antibody (BiTE) does not kill all tumor cells), and addition of one or more immune checkpoint inhibitors in (e) reverts resistance of tumor cell population;
   ii. step (f) reveals an increase in the expression level of an immune checkpoint molecule in either the tumor cells and/or T cells in the reaction mixture of step (c) after incubation, relative to basal levels prior incubation;
and wherein observance of both (i) and (ii) is indicative of a subject more susceptible to immune checkpoint immunotherapy treatment to be combined with a bispecific T cell engager antibody (BiTE) immunotherapy.

In another aspect, provided herein is an *in vitro* method of identifying subjects susceptible to immune checkpoint immunotherapy treatment to be combined with a bispecific T cell engager antibody (BiTE) immunotherapy, for decreasing resistance of said subject to said BiTE immunotherapy, comprising:
(a) providing a sample comprising at least one T cell from a subject having a cancer;
(b) providing a sample comprising at least one cancer cell, e.g., from the subject;
(c) forming an *ex vivo* reaction mixture comprising the at least one T cell, the at least one cancer cell, and the bispecific T cell engager antibody (BiTE), being identical to the BiTE of the immunotherapy, e.g., under conditions (e.g., for a period of time) sufficient to allow the T cell to kill cancer cells, thereby producing the cancer-killing T cell;
(d) Isolating the activated T cells, by FACS or magnetic-beads or other methods, adding them to a cancer cell, e.g., from the subject, forming an *ex vivo* reaction mixture comprising under conditions (e.g., for a period of time) sufficient to allow the activated T cells to kill cancer cells; and;
(e) determining the pharmacological activity of the cancer-killing T cells obtained in step (d) by dose response and/or pharmacodynamic parameters of cancer-killing T cells and tumor cells, selected from EC50, Emax, AUC, Effective E:T Ratios, Basal E:T Ratios, or kinetic parameters and;
(f) determining the pharmacological activity of the cancer-killing T cells repeating steps (d) and (e) by dose response or evaluating a single high saturating dose in combination with immune check point inhibitors, individually, or in combinations, or bispecific or multispecific antibody constructs combining immune checkpoint inhibitors, including the combination of all immune checkpoint inhibitors;
(g) determining the expression levels of immune checkpoint molecules in both the tumor cells and T cells in the reaction mixture of step (d), comparing basal levels with levels after incubation;
(h) identifying subjects susceptible to immune checkpoint immunotherapy treatment, in combination with the BiTE, by assessment of either of the following 2 criteria or a combination of them:
   i. step (e) reveals a resistant tumor cell population in the samples from the subject (i.e. incubation with BiTE-activated isolated T cells does not kill all tumor cells), and addition of one or more immune checkpoint inhibitors in (f) reverts resistance of tumor cell population;
   ii. step (g) reveals an increase in the expression level of an immune checkpoint molecule in either the tumor cells and/or T cells in the reaction mixture of step (d) after incubation, relative to basal levels prior incubation,
and wherein observance of both (i) and (ii) is indicative of a subject more susceptible to immune checkpoint immunotherapy treatment for decreasing resistance of said subject to said BiTE immunotherapy.

In another aspect, provided herein is an *in vitro* method of identifying subjects susceptible to immune checkpoint immunotherapy treatment to be combined with a cellular immunotherapy such a CAR-T to treat a subject, for decreasing resistance of said subject to said cellular immunotherapy, comprising:
(a) providing a sample comprising at least one T cell selected from the group consisting of a tumor infiltrated lymphocyte (TIL), marrow infiltrated lymphocyte (MIL), a genetically engineered T cell, a CAR-T cell, or an activated T cell obtainable according to step (c) of the method of claim 1 or claim 2, or step (d) of the method of claim 3 and a genetically engineered T cell expressing Chimeric Antigen Receptors obtainable according to step (e) of the method of claim 1, step (f) of the method of claim 2, or step (g) of the method of claim 3, from a subject having a cancer;
(b) providing a cancer cell, e.g., from the subject;
(c) forming an *ex vivo* reaction mixture comprising (a) and (b), under conditions (e.g., for a period of time) sufficient to allow the T cells to kill cancer cells, thereby producing the cancer-killing T cell; and
(d) determining the pharmacological activity of cancer-killing T cells obtained in step (c) by dose response and/or pharmacodynamic parameters of cancer-killing T cells and tumor cells, selected from EC50, Emax, AUC, Effective E:T Ratios, Basal E:T Ratios, or kinetic parameters;
(e) determining the pharmacological activity of cancer-killing T cells obtained in step (c) by dose response or evaluating a single high saturating dose in combination with immune check point inhibitors, individually, or in combinations, or bispecific or multispecific antibody constructs combining immune check point inhibitors, including the combination of all immune check point inhibitors, either by full dose responses or evaluating a single high saturating dose.
(f) determining the expression levels of immune checkpoint molecules in both the tumor cells and T cells in the reaction mixture of step (c), comparing basal levels with levels after incubation,
(g) identifying subjects susceptible to immune checkpoint immunotherapy treatment in combination with the cellular therapy, by assessment of either of the following 2 criteria or a combination of them:
   i. step (d) reveals a resistant tumor cell population in the samples from the subject (i.e. incubation with T cell therapy does not kill all tumor cells), and addition of one or more immuno checkpoint inhibitors in (e) reverts resistance of tumor cell population;
   ii. step (f) reveals an increase in the expression level of an immune checkpoint molecule in either the tumor cells and/or T cells in the reaction mixture of step (c) after incubation, relative to basal levels prior incubation,
and wherein observance of both (i) and (ii) is indicative of a subject more susceptible to immune checkpoint immunotherapy treatment to be combined with a cellular immunotherapy.

In an embodiment, the immune check point molecules are added either from the beginning of the incubation or sequentially after a certain amount of time sufficient for the T cells to become activated killing tumor cells.

In an embodiment, different incubation times are evaluated, and any single incubation time can be used to identify subjects susceptible to immune check point immunotherapy, alone or in combination with other drugs.

In an embodiment, the immune check point molecules are added either from the beginning of the incubation or sequentially after a certain amount of time sufficient for the T cells to become activated killing tumor cells.

In an embodiment, different incubation times are evaluated, and any single incubation time can be used to identify subjects susceptible to immune check point immunotherapy, alone or in combination with other drugs.

### Further embodiments of methods of treatment

In one aspect, provided herein is a method for treating a subject having cancer comprising providing a bispecific T cell engager antibody (BiTE) or a T cell selected from the group consisting of a tumor infiltrated lymphocyte (TIL), a genetically engineered T cell, a CAR-T cell, an activated T cell obtainable according to step (c) of the method of producing a CAR-T cell and a genetically engineered T cell expressing Chimeric Antigen Receptors obtainable according to step (e) of the method of producing a CAR-T cell, in combination with an inhibitor of at least one immune checkpoint molecule selected in the method of identifying immune checkpoint molecules as target for decreasing resistance to a cancer therapy.

### Further embodiments of methods of producing CAR-T cells

In embodiments, the method (e.g., of producing) further comprises producing a CAR-T cell preparation, e.g., a pharmaceutical preparation.

In embodiments, the method (e.g., of producing) further comprises detecting the presence of the CAR-T cell.

In embodiments, the method (e.g., of producing) further comprises purifying the CAR-T cell from the bispecific T cell engager antibody (BiTE).

In embodiments, the bispecific T cell engager antibody (BiTE) is present, e.g., in the preparation, at a concentration of less than 10% by weight, e.g., less than 10%, 9%, 8%, 7%, 6%, 5%, 4%, 3%, 2%, 1%, 0.5%, 0.1%, 0.05%, 0.01%, 0.005 or less (e.g., but no less than 0.001%). In a preferred embodiment, the bispecific T cell engager antibody (BiTE) is present in the preparation at a concentration of 0.005% to 10% by weight.

In embodiments, the reaction mixture contains in volume a few nanoliters (e.g., less than 10 nl, about 1 to 5 nanoliters) of bispecific T cell engager antibody (BiTE) are added to over 50 microliters (e.g., about 60 microliters) of cell suspension.

In embodiments, the preparation comprises bispecific T cell engager antibody (BiTE), e.g., a level of bispecific T cell engager antibody (BiTE), detectable by immune assay.

In embodiments, the selecting and/or enriching step (e.g., step ii)-iii) or (d) of the method of producing above) comprises using a fluorescently labeled molecule (e.g., a cell surface label, e.g., a fluorescently labeled antibody or fragment thereof, or a cell tracker dye) that diffuses into the cancer cell membrane or binds to i) one or more cancer antigens or ii) one or more markers of activated T cells, or both i) and ii). In embodiments, the selecting and/or enriching step comprises using fluorescence activated cell sorting (FACS).

In embodiments, the selecting and/or enriching (e.g., step ii)-iii) or (d) of the method of producing above) comprises using a bead (e.g., magnetic bead) coated with an antibody or fragment thereof that binds to i) one or more cancer antigens or ii) one or more markers of activated T cells, or both i) and ii).

In embodiments, the selecting and/or enriching step (e.g., step ii)-iii) or (d) of the method of producing above) comprises the sequential addition of a low, e.g., an insufficient, number of cancer cells. In embodiments, the methods of producing described above can generate different clones of cytotoxic T cells. In embodiments, selection of the cytotoxic T cell clones that are the most efficient or most potent at killing cancer cells can be achieved by sequentially adding low, e.g., insufficient, amounts of cancer cells. In embodiments, a low, or insufficient, number or amount of cancer cells that can be added to a reaction comprising CAR-T cells is 50% or less, e.g., 30%, 10%, 1%, 0.1%, or 0.01% or less, of the number of activated T cells. In one embodiment, the low, or insufficient, number of cancer cells can be added to CAR-T cells (e.g., a reaction comprising cancer cells, T cells, and/or a bispecific T cell engager antibody (BiTE)) one or more times, e.g., 2, 3, 4, 5, 6, 7, 8, 9 or 10, times. In one embodiment, the low, or insufficient, number of cancer cells is added every 6 hours, 12 hours, 24 hours, 36 hours, or 48 hours. In an embodiment, the low, or insufficient, number of cancer cells that are added are cancer cells from the patient. In an embodiment, the low, or insufficient, number of cancer cells that are added are not cancer cells from the patient. In an embodiment, the low, or insufficient, number of cancer cells that are added are cancer cells from a cancer cell line.

In embodiments, the CAR-T cells are expanded. In embodiments, the expansion of the CAR-T cells comprises increasing the number of CAR-T cells, e.g., in a preparation, e.g., by at least about 2-fold (e.g., at least about 3-, 4-, 5-, 6-, 7-, 8-, 9-, 10-, 15-, 20-, 50-, 100-, 1000-, 10⁴-, 10⁵-, 10⁶-fold, or more).

In other embodiments, the CAR-T cells are not substantially expanded.

In embodiments, the CAR-T cell preparation comprises a fluorescently labeled molecule (e.g., a cell surface label, e.g., a fluorescently labeled antibody or fragment thereof or a cell tracker dye) and/or the bispecific T cell engager antibody (BiTE), e.g., wherein the fluorescently labeled molecule and/or the bispecific T cell engager antibody (BiTE) are present at trace amounts (e.g., less than 5% by weight, e.g., less than 5%, 4%, 3%, 2%, 1%, 0.5%, 0.25%, 0.1%, 0.05%, 0.01%, 0.005%, 0.001% by weight, or less).

In embodiments, the CAR-T cell preparation (prior to purification or expansion) comprises CAR-T cells at a concentration of 5% or less of the total number of cells in the preparation.

In embodiments, a purified or enriched CAR-T cell preparation comprises CAR-T cells at a concentration of at least 50% (e.g., at least 50%, 55%, 60%, 65%, 70%, 75%, 80%, 85%, 90%, 95%, 99%, or greater) of the total number of cells in the preparation.

In embodiments, a purified or enriched CAR-T cell preparation comprises activated CAR-T cells, e.g., at a concentration of at least 50% (e.g., at least 50%, 55%, 60%, 65%, 70%, 75%, 80%, 85%, 90%, 95%, 99%, or greater) of the total number of cells in the preparation.

In embodiments, a purified or enriched CAR-T cell preparation comprises trogocytotic CAR-T cells, e.g., at a concentration of at least 50% (e.g., at least 50%, 55%, 60%, 65%, 70%, 75%, 80%, 85%, 90%, 95%, 99%, or greater) of the total number of cells in the preparation.

In embodiments, the CAR-T cell or preparation comprises one or more CD8+ T cells. In embodiments, the CAR-T cell or preparation comprises one or more CD4+ T cells. In embodiments, the CAR-T cell or preparation comprises one or more CD25+ T cells. In embodiments, the CAR-T cell or preparation comprises one or more CD8+/CD25+ CTLs. In embodiments, the CAR-T cell or preparation comprises one or more CD4+/CD25+ T cells. In embodiments, the CAR-T cell or preparation comprises one or more cytotoxic T lymphocytes (CTLs), e.g., cancer antigen-specific CTLs. In embodiments, the CAR-T cell or preparation comprises one or more effector memory T cells. In embodiments, the CAR-T cell preparation does not comprise a substantial number of regulatory T cells (Tregs).

In embodiments, the method (e.g., of producing) further comprises reducing the number of Tregs in the CAR-T cell preparation. In one embodiment, the bispecific T cell engager antibody (BiTE) selectively expands the CAR-T cells, thus increasing the Effective E:T ratio of CAR-T cells:Tregs. In other embodiments, method further comprises removing (e.g., depleting) Tregs by physical separation, e.g., using a bead (e.g., a magnetic bead) attached to a Treg cell surface marker.

In embodiments, the CAR-T cell preparation comprises Tregs at a concentration of less than 10% (e.g., less than 9%, 8%, 7%, 6%, 5%, 4%, 3%, 2%, 1% or less) of the total number of cells in the preparation.

In embodiments, the CAR-T cell preparation does not comprise a substantial number of naive T cells.

In embodiments, the CAR-T cell preparation comprises naive T cells at a concentration of less than 10% (e.g., less than 9%, 8%, 7%, 6%, 5%, 4%, 3%, 2%, 1% or less) of the total number of cells in the preparation.

In embodiments, the naive T cells express CD45RA, CD62L, CCR7, CD27, CD28 and/or CD57.

In embodiments, the CAR-T cell preparation comprises more than one clone of CAR-T cells.

In embodiments, the method (e.g., of producing) further comprises separating individual clones from the CAR-T cell preparation.

In embodiments, the separating step comprises clonal expansion of single cells (e.g., (i) separating the preparation of CAR-T cells into single cells (e.g., a single cell per well or container) and (ii) expanding the single cells to generate one or more preparations of CAR-T cells, wherein each preparation comprises a single clone).

In embodiments, the separating step comprises flow cytometry or limited dilution.

In embodiments, the method (e.g., of producing) further comprises determining the cancer-killing activity of the CAR-T cell preparation, and optionally, selecting the preparation based on a parameter chosen from one or more of: increased cancer cell killing activity, reduced toxicity, reduced off-target effect, increased viability, increased proliferation, or Effective E:T ratio for cancer cell killing.

In embodiments, the CAR-T cell preparation comprises cells having high cancer-killing activity and/or low toxicity.

The cells comprised in the CAR-T cell preparation with low toxicity are cells which kill significantly less non-pathological cells, i.e. they kill more selectively. In embodiments, the CAR-T cell preparation comprises cells having low toxicity because they generate less cytokines in the supernatant and/or intracellularly. In embodiments, the CAR-T cell preparation comprises cells having both and simultaneously higher cancer-killing activity and low toxicity, because they generate less cytokines in the supernatant and/or intracellularly per unit of CAR-T cell, that is once the types and/or levels of cytokines released is normalized by the quantitative estimation of cancer cell killing activity such as Effective E:T Ratios, basal E:T ratios, EC50, Emax, kinetics, or a combination of these factors.

In embodiments, the CAR-T cell preparation comprises cells that effectively kill cancer cells at a high target cell per T cell. In embodiments, a T cell to high target cell ratio is about 1:4 to 1:100 (e.g., 1:4, 1:5, 1:6, 1:7, 1:8, 1:9, 1:10, 1:15, 1:20, 1:25, 1:30, 1:35, 1:40, 1:45, 1:50, 1:75, 1:100, or higher).

In embodiments, the CAR-T cell preparation comprises a population of cells consisting of less than 10 clones of CAR-T cells. In embodiments, 10, 9, 8, 7, 6, 5, 4, 3, 2 or 1 clone of CAR-T cells is present in the preparation. In one embodiment, 2-4 clones are present in the preparation. In other embodiments, a single clone of CAR-T cells.

In embodiments, the T cell or T cell sample of the method (e.g., of producing) and the cancer cell or cancer cell sample of the method (e.g., of producing) are from the same subject.

In embodiments, the T cell or T cell sample of the method (e.g., of producing) and the cancer cell or cancer cell sample of the method (e.g., of producing) are from a different subject.

In embodiments, the CAR-T cell or preparation is administered to the subject, e.g., wherein the subject is the same subject as the subject from whom the T cells (and/or the cancer cells) were obtained. For example, the CAR-T cell or preparation is autologous.

In embodiments, the CAR-T cell or preparation is administered to the subject, e.g., wherein the subject is a different subject from the subject from whom the T cells (and/or the cancer cells) were obtained. For example, the CAR-T cell or preparation is allogeneic.

In embodiments, the method (e.g., of producing) comprises providing a sample comprising the T cell. In embodiments, method (e.g., of producing) comprises providing a sample comprising the cancer cell.

In embodiments, the T cell and the cancer cell of the method (e.g., of producing) are from the same sample.

In embodiments, the T cell and the cancer cell of the method (e.g., of producing) are from different samples.

In embodiments, the sample is derived from a tissue with a microenvironment, e.g., a bone marrow, a lymph node, a primary tumor, or a metastasis.

In embodiments, the sample comprises blood (e.g., whole blood, peripheral blood, or bone marrow), a solid tumor (e.g., a sample resected from a primary tumor or a metastasis), a lymph node, or spleen of the subject. In embodiments, the sample is a blood sample e.g., whole blood, peripheral blood, or bone marrow, wherein substantially no components (e.g., cells or plasma) have been removed or isolated from the blood sample. In embodiments, the sample is diluted, e.g., with a physiologically compatible buffer or media, e.g., prior to and/or during step (c).

In embodiments, the method (e.g., of producing) comprises providing a T cell from a blood sample from the subject, e.g., where the T cell is not purified from other components, e.g., cells or plasma, in the blood sample. In embodiments, the blood sample is a bone marrow sample, a peripheral blood sample, or a whole blood sample.

In embodiments, the method (e.g., of producing) comprises providing a cancer cell from a blood sample from the subject, e.g., wherein the cancer cell is not purified from other components, e.g., cells or plasma, in the blood sample. In embodiments, the blood sample is a bone marrow sample, a whole blood sample, or a peripheral blood sample.

In embodiments, the cancer cell of the method (e.g., of producing) comprises a circulating cancer cell, e.g., from a blood sample, e.g., peripheral blood sample, of the subject.

In embodiments, the method (e.g., of producing) comprises providing a cancer cell from a tissue sample, e.g., a biopsy, e.g., of a tumor or metastasis, from the subject.

In embodiments, the method (e.g., of producing) comprises providing a sample, e.g., blood sample (e.g., bone marrow, peripheral blood, or whole blood sample), that comprises both the T cell and the cancer cell.

In embodiments, the subject is an adult or a pediatric subject.

In embodiments, the cancer is a hematological cancer, e.g., a B-cell or T cell malignancy.

In embodiments, the cancer is a Hodgkin's lymphoma, Non-Hodgkin's lymphoma (e.g., B cell lymphoma, diffuse large B cell lymphoma, follicular lymphoma, chronic lymphocytic leukemia, mantle cell lymphoma, marginal zone B-cell lymphoma, Burkitt lymphoma, lymphoplasmacytic lymphoma, hairy cell leukemia), acute myeloid leukemia, chronic myeloid leukemia, myelodysplastic syndrome, multiple myeloma, or acute lymphocytic leukemia.

In embodiments, the cancer is a solid cancer, e.g., wherein the solid cancer comprises ovarian cancer, rectal cancer, stomach cancer, testicular cancer, cancer of the anal region, uterine cancer, colon cancer, rectal cancer, renal-cell carcinoma, liver cancer, non-small cell carcinoma of the lung, cancer of the small intestine, cancer of the esophagus, melanoma, Kaposi's sarcoma, cancer of the endocrine system, cancer of the thyroid gland, cancer of the parathyroid gland, cancer of the adrenal gland, bone cancer, pancreatic cancer, skin cancer, cancer of the head or neck, cutaneous or intraocular malignant melanoma, uterine cancer, brain stem glioma, pituitary adenoma, epidermoid cancer, carcinoma of the cervix squamous cell cancer, carcinoma of the fallopian tubes, carcinoma of the endometrium, carcinoma of the vagina, sarcoma of soft tissue, cancer of the urethra, carcinoma of the vulva, cancer of the penis, cancer of the bladder, cancer of the kidney or ureter, carcinoma of the renal pelvis, spinal axis tumor, neoplasm of the central nervous system (CNS), primary CNS lymphoma, tumor angiogenesis, metastatic lesions of said cancers, or combinations thereof.

In embodiments, the cancer is not melanoma.

In embodiments, the method (e.g., of producing) does not comprise labelling the cancer cell (e.g., cancer cell membrane) with a fluorescent molecule prior to contacting the sample with the bispecific T cell engager antibody (BiTE).

In embodiments, the subject:
(i) has not received a prior treatment for the cancer;
(ii) has received one or more previous treatments for the cancer; or
(iii) has minimal residual disease (MRD).

In embodiments, the period of time is 12 to 120 hours (e.g., 12-24 hours, 24-48 hours, 48-36 hours, 36-60 hours, 60-90 hours, or 90-120 hours) or 1-7 days (e.g., 1, 2, 3, 4, 5, 6, or 7 days).

In embodiments, the method described here further comprises repeating the sample or cell providing step, *ex vivo* reaction formation step and/or the enrichment step (e.g., steps (a)-(d) of the methods of producing) using a different sample of T cells and cancer cells, e.g., wherein each repeat of steps uses a different sample of T cells and cancer cells. In embodiments, the different sample of T cells and cancer cells comprises a sample derived from a tissue with a microenvironment, e.g., a bone marrow, a lymph node, a primary tumor, or a metastasis.

In embodiments, the CAR-T cell produced from each repeat of steps is pooled to form a mixture of CAR-T cells.

In embodiments, the T cell comprises a CTC, and the T cell is from a sample (e.g., blood (e.g., whole blood, peripheral blood, or bone marrow), lymph node, primary tumor, or metastasis) from the subject. In embodiments, the T cell is enriched for the CTC. In embodiments, the T cell is purified, e.g., purified from other types of cells, e.g., from a blood sample from the subject (e.g., whole blood, peripheral blood, or bone marrow).

In embodiments, the method further comprises repeating the sample or cell providing step, ex *vivo* reaction formation step and/or the enrichment step (e.g., steps (a)-(d) of the methods of producing) using a different sample of T cells from the subject, e.g., wherein each repeat of steps uses a different sample of T cells from the subject.

In embodiments, the different sample of T cells comprises a sample derived from a cancer-containing tissue from the subject, e.g., a primary tumor, one or more metastases, a lymph node, a lymph sample, or a blood sample (e.g., whole blood, peripheral blood, or bone marrow).

In embodiments, the CAR-T cell produced from each repeat of the sample or cell providing step, ex *vivo* reaction formation step and/or the enrichment step (e.g., steps (a)-(d) of the methods of producing) is pooled to a form a mixture of CAR-T cells.

In embodiments, the method (e.g., of producing) further comprises evaluating the cancer-killing activity of the CAR-T cell. In embodiments, the evaluating comprises:
(a) contacting the CAR-T cells with target cells, wherein the target cells are derived from the cancer (e.g., wherein the target cells comprise a cell line derived from the cancer, e.g., wherein the target cells are not isolated from the subject) under conditions (e.g., for a period of time) sufficient to allow the CAR-T cells to kill the cancer cell;
(b) determining the number of target cells after step (a), and optionally determining the number of CAR-T cells after step (a);
where a decrease in the number of target cells compared to the number of target cells before the contacting step indicates that the CAR-T cells are effective in killing cancer cells. In embodiments, an increase in the number of CAR-T cells, e.g., compared to the number of CAR-T cells before the contacting step indicates that the CAR-T cells are effective in killing cancer cells.

In embodiments, the evaluating comprises:
(a) providing a CAR-T cell or a preparation thereof, e.g., produced according to a method described herein, e.g., from a subject (e.g., a subject with a cancer as described herein);
(b) providing a target cell (e.g., a cancer cell), e.g., wherein the cancer cell is from the subject;
(c) contacting the CAR-T cell or the preparation thereof with the target cell (e.g., cancer cells), under conditions (e.g., for a period of time) sufficient to allow the CAR-T cell to kill the cancer cell (in embodiments, the contacting step further comprises addition of a bispecific T cell engager antibody (BiTE), e.g., at different doses (e.g., increasing dosages);
(d) determining the level of target cells after step (c), and optionally determining the level of CAR-T cells after step (c) (in embodiments, the level of target cells and/or CAR-T cells is determined at one or more time intervals after step (c)); and
   (optionally) (e) determining the ratio of either target cell to T cell, or T cell to target cell, from step (d) (e.g., determining an Effective E:T ratio as described herein).

In embodiments, the evaluating comprises using a first patient sample, e.g., containing T cells and cancer cells, to generate a CAR-T cell, e.g., using a method described herein. In embodiments, the CAR-T cells are purified, sorted, enriched, expanded, and/or selected. In embodiments, the evaluating comprises subsequently mixing a second sample from the same patient with the CAR-T cells generated using the first patient sample. In embodiments, various concentrations of CAR-T cells can be mixed with the second sample, e.g., where the second sample is at a fixed concentration, e.g., to generate a dose response curve.

Accordingly, in embodiments, the evaluating comprises:
(a) producing a CAR-T cell or a preparation thereof, e.g., according to the method of claim or producing a CAR-T cell, wherein the T cell and the cancer cell are present in a patient sample,
(b) optionally expanding, selecting, enriching, and/or purifying the CAR-T cell from (a),
(c) contacting the CAR-T cell from (a) or (b) with a second sample, e.g., from the same patient, wherein the second sample comprises one or more cancer cells, and wherein the CAR-T cell is contacted with the cancer cells, and
(d) determining a dose response and/or pharmacodynamic parameter as described herein.

In an embodiment, the level of activity of the CAR-T cells (e.g., trogocytotic cells) in the ex *vivo* mixture is measured by Effective E:T Ratios, basal E:T ratios, EC50s, Emax, kinetics, or a combination of these factors.

In embodiments, step (c) comprises contacting the cancer cells with the CAR-T cells at a plurality of ratios, e.g., Effective E:T ratios.

In embodiments, step (c) comprises mixing different amounts of CAR-T cells with a fixed amount of cancer cells.

In some embodiments of any of the aforesaid methods, an Effective E:T ratio is obtained. In one embodiment, the Effective E:T is the ratio between the CAR-T cells and the cancer cells after bispecific T cell engager antibody (BiTE).

In embodiments of any of the aforesaid methods, a decrease in the level or amount of cancer cells, e.g., relative to a reference level, is indicative of increased cancer cell killing. In other embodiments, a reduced change or no substantial change in the level or amount of cancer cells, e.g., relative to a reference level, is indicative of decreased cancer cell killing.

In embodiments of any of the aforesaid methods, a high level of target cell relative to T cell (e.g., a high Effective E:T ratio of target cell to CAR-T cell) indicates that the CAR-T cell or preparation thereof is an effective killer of cancer cells. In one embodiment, the target to T cell ratio is compared to a reference ratio. For example, an Effective E:T ratio of 1 (CAR-T cell) to 100 (e.g., 10, 20, 30, 40, 50, 75, 100 or higher) (target cells) is indicative of potent T cell killing activity. A subject having T cells having potent cell killing activity can be identified as being a strong responder to the bispecific T cell engager antibody (BiTE).

Alternatively, a low level of target cell relative to T cell (e.g., a low Effective E:T ratio of target cell to CAR-T cell) is indicative of a poor T cell killing activity. In one embodiment, the target to T cell ratio is compared to a reference ratio. In one embodiment, an Effective E:T ratio of 1 (CAR-T cell) to 5 (target cells) (e.g., 1, 3, or 5) is indicative of poor T cell killing activity. A subject having T cells having reduced cell killing activity can be identified as being a poor responder to the bispecific T cell engager antibody (BiTE).

In embodiments of any of the aforesaid methods, the level of target cells and/or CAR-T cells is determined at one or more time intervals after step (c). In exemplary embodiments, the level of target cells and/or CAR-T cells is determined at time 0, at time of 1-75 hours (e.g., 1, 2, 4, 8, 16, 24, 36 or 72 hours) or several days after step (c).

In embodiments of any of the aforesaid methods, the contacting step further comprises addition of a bispecific T cell engager antibody (BiTE) at different doses (e.g., increasing dosages) of the bispecific T cell engager antibody (BiTE), e.g., to generate a dose response curve. In one embodiment, the difference between the level of CAR-T cells or cancer cells at a dose zero or at control level (e.g., a threshold dose) and a saturated dose of the bispecific T cell engager antibody (BiTE) is determined. In embodiments, the difference in the level of CAR-T cells or cancer cells at the saturated dose vs. threshold dose is determined. In embodiments, the Effective E:T ratio as used herein is the ratio of the difference in the level of CAR-T cells relative to the difference in the level of cancer cells.

In embodiments of any of the aforesaid methods, method is performed using an automated platform, e.g., an automated fluorescence-based platform, e.g., the ExviTech^{®} platform described herein.

In embodiments of any of the aforesaid methods or assays, the activity of the bispecific T cell engager antibody (BiTE) and/or immunomodulatory agent is determined using an *ex vivo*/*in vitro* assay to measure dose response curves, whose mathematical fitting enable quantitative parameters to estimate the activity, selected from at least one from EC50, Effective E:T ratio, basal E:T ratios, Emax or kinetics. In embodiments of any of the aforesaid methods, the activity of the bispecific T cell engager antibody (BiTE) assessed by step (e) is different from an activity assessment using a dose response of the bispecific T cell engager antibody (BiTE) activity, e.g., compared to a standard depletion dose response curve.

In embodiments of any of the aforesaid methods, the reference ratio is a predetermined ratio, e.g., about 1:3 to 1:10, e.g., about 1:3, 1:4, 1:5, 1:6, 1:7, 1:8, 1:9, or 1:10. In embodiments, the high target cell to T cell ratio from step (e) is about 1:4 to 1:100 (e.g., 1:4, 1:5, 1:6, 1:7, 1:8, 1:9, 1:10, 1:15, 1:20, 1:25, 1:30, 1:35, 1:40, 1:45, 1:50, 1:75, 1:100, or higher).

In embodiments of any of the aforesaid methods, step (c) comprises forming *ex vivo* mixtures of the CAR-T cell or the preparation thereof with target cells, e.g., cancer cells. In embodiments, the cancer cell is a cell chosen from a hematological cancer, a solid cancer, a metastatic cancer (e.g., a CTC, or a combination thereof). In embodiments, the cancer cell is a leukemic or lymphoma blast cell (e.g., a blast cell expressing one or more markers chosen from CD19, CD123, CD20 or others). In embodiment, the T cell is a cell chosen from a blood sample (e.g., peripheral blood sample), a bone marrow sample, a lymph node sample, a tumor sample comprising a CTL and/or a TIL, or a combination thereof). In embodiments, the T cell expresses CD8 and/or CD25 (e.g., it is a CD8+CD25+ T cell).

In embodiments of any of the aforesaid methods, the CAR-T cell or preparation thereof is produced using a method that comprises use of a bispecific T cell engager antibody (BiTE), e.g., a bispecific T cell engager antibody (BiTE) described herein.

In embodiments of any of the aforesaid methods, the CAR-T cell or preparation thereof comprises a T cell, e.g., CTL, that is CD8+ and CD25+. In some embodiments, the CAR-T cell is a trogocytotic T cell. In other embodiments, the CAR-T cell is a CD28+CD25+ T cell.

In embodiments of any of the aforesaid methods, the CAR-T cell: (i) has cytotoxic activity toward a cancer cell, and (ii) comprises a cell surface marker derived from the cancer cell, e.g., at least 90-500 copies of a cell surface marker (e.g., 90, 100, 200, 300, 400, or 500 copies, e.g., one or more cancer cell surface markers).

In embodiments of any of the aforesaid methods, about 2 to 75% (e.g., about 2 to 70%, 2 to 60%, 2 to 50%, or 2 to 40%) of the total T cells in the reaction mixture express one or more cancer cell surface markers (e.g., one or more leukemic cell cancers).

In embodiments, the CAR-T cell is enriched or purified. In some embodiments, the enriched or purified CAR-T cell population comprises at least 80%-100% CAR-T cells (e.g., 80%, 90%, 95%, 99% or 100%), wherein the CAR-T cells comprise one or more cancer cell surface markers.

In embodiments, the *ex vivo* reaction mixture is prepared according to Good Manufacturing Practice (GMP). In embodiments, one or more of the expansion, selection and/or enrichment of the CAR-T cells is according Good Manufacturing Practice (GMP). In embodiments, the method further comprises sending the produced CAR-T cell, e.g., to a hospital, a health care provider. In embodiments, the method further comprises receiving the T cell, the cancer cell, or both, e.g., from a hospital, a health care provider.

### Further embodiments of methods of treatment

In embodiments, the method (e.g., of treating) further comprises administering a second therapeutic agent or procedure. In embodiments, the second therapeutic agent or procedure is chosen from one or more of chemotherapy, a targeted anti-cancer therapy, an oncolytic drug, a cytotoxic agent, an immune-based therapy, a cytokine, a surgical procedure, a radiation procedure, an agonist of T cells (e.g., agonistic antibody or fragment thereof or an activator of a costimulatory molecule), an inhibitor of an inhibitory molecule (e.g., immune checkpoint inhibitor), an immunomodulatory agent, a vaccine, or a cellular immunotherapy.

In embodiments, the second therapeutic agent is an agonist of T cells (e.g., an agonistic antibody or fragment thereof or an activator of a costimulatory molecule) or an immune checkpoint inhibitor.

In embodiments, the immune checkpoint inhibitor is an inhibitor of one or more of: CTLA4, PD1, PDL1, PDL2, B7-H3, B7-H4, TIM3, LAG3, BTLA, CD80, CD86, or HVEM.

In embodiments, the immune checkpoint inhibitor comprises one or more of: ipilimumab, tremelimumab, MDX-1106, MK3475, CT-011, AMP-224, MDX-1105, IMP321, or MGA271.

In embodiments, the agonist of T cells comprises an antibody or fragment thereof to CD137, CD40, and/or glucocorticoid-induced TNF receptor (GITR).

In one embodiment, the immunomodulatory agent is an inhibitor of MDSCs and/or Treg cells. In embodiments, the immunomodulatory agent comprises/is lenalidomide.

In embodiments, the second therapeutic agent enhances and/or restores the immunocompetence of T cells.

In other embodiments, the immunomodulatory agent activates an immune response to a tumor specific antigen, e.g., it is a vaccine (e.g., a vaccine against targets such as gp100, MUC1 or MAGEA3). In other embodiments, the immunomodulatory agent is a cytokine, e.g., a recombinant cytokine chosen from one or more of GM-CSF, IL-7, IL-12, IL-15, IL-18 or IL-21. In other embodiments, the immunomodulatory agent is an autologous T cell, e.g., a tumor-targeted extracellular and intracellular tumor-specific antigen (e.g., a CAR-T cell or a TCR T cell). In yet other embodiments, the immunomodulatory agent is a modulator of a component (e.g., enzyme or receptor) associated with amino acid catabolism, signalling of tumor-derived extracellular ATP, adenosine signalling, adenosine production, chemokine and chemokine receptor, recognition of foreign organisms, or kinase signalling activity. Exemplary agents include an inhibitor (e.g., small molecule inhibitor) of IDO, COX2, ARG1, ArG2, iNOS, or phosphodiesterase (e.g., PDE5); a TLR agonist, or a chemokine antagonist. Additional examples of immunomodulatory agents are described herein.

### Further embodiments of compositions and reaction mixtures

In embodiments, the bispecific T cell engager antibody (BiTE) comprises an antibody molecule, e.g., a bi-specific antibody or fragment thereof, e.g., a bispecific immunoglobulin (BsIgG), an immunoglobulin operatively linked to additional antigen-binding molecule, a bispecific antibody (BsAb) fragment, a bispecific fusion protein, or a BsAb conjugate. Bispecific antibodies can also be named DART, DutaFab, Duobodies, Biparatopic, Adaptir. In embodiments, a BiTE includes multispecific constructs with more than 2 recognition arms, a common development in the field of bispecific antibodies, and a natural extension of the same concept. In embodiments, multispecific constructs can add more recognition fragments of the same type, or include fragments with different recognition properties.

In embodiments, the bispecific T cell engager antibody (BiTE) is a bi-specific antibody selected from the list consisting of BsMAb CD123/CD3, BsMAb CD19/CD3 and EpCAM/CD3.

In embodiments, the bispecific T cell engager antibody (BiTE) is present at a detectable amount, e.g., a concentration of less than 10% by weight, e.g., less than 10%, 9%, 8%, 7%, 6%, 5%, 4%, 3%, 2%, 1%, 0.5%, 0.1%, 0.05%, 0.01%, 0.001% or less (but no less than 0.0001%), e.g., in a composition described herein. In one embodiment, the bispecific T cell engager antibody (BiTE) is present at a level of less than 1%. In a preferred embodiment, the bispecific T cell engager antibody (BiTE) is present in the preparation at a concentration of 0.005% to 10% by weight.

In embodiments, the CAR-T cell comprises an activated T cell.

In embodiments, the CAR-T cell comprises a cell that has undergone trogocytosis, e.g., a cell that comprises a portion of a cell surface membrane from the cancer cell.

In embodiments, the CAR-T cell is a T cell, e.g., a cytotoxic T lymphocyte, e.g., a CD8+ T cell.

In embodiments, the composition or preparation does not comprise a substantial number of cancer cells, e.g., comprising cancer cells at a concentration of less than 30% (e.g., less than 30%, 25%, 20%, 15%, 10%, 5%, 2.5%, 1%, 0.5%, 0.1%, or less) of the total number of cells in the composition or preparation.

In embodiments, the composition or preparation does not comprise a substantial number of regulatory T cells (Tregs), e.g., comprising Tregs at a concentration of less than 30% (e.g., less than 30%, 25%, 20%, 15%, 10%, 5%, 2.5%, 1%, 0.5%, 0.1%, or less) of the total number of cells in the composition or preparation.

In embodiments, the composition or preparation does not comprise a substantial number of naive T cells, e.g., comprising naive T cells at a concentration of less than 30% (e.g., less than 30%, 25%, 20%, 15%, 10%, 5%, 2.5%, 1%, 0.5%, 0.1%, or less) of the total number of cells in the composition or preparation.

In embodiments, the composition or preparation does not comprise a substantial number of red blood cells, e.g., comprising red blood cells at a concentration of less than 30% (e.g., less than 30%, 25%, 20%, 15%, 10%, 5%, 2.5%, 1%, 0.5%, 0.1%, or less) of the total number of cells in the composition or preparation.

In embodiments, the composition or preparation does not comprise a substantial number of non-immune cells, e.g., comprising non-immune cells at a concentration of less than 30% (e.g., less than 30%, 25%, 20%, 15%, 10%, 5%, 2.5%, 1%, 0.5%, 0.1%, or less) of the total number of cells in the composition or preparation.

In embodiments, the composition or preparation comprises activated T cells at a concentration of at least 30%, (e.g., at least 30%, 40%, 50%, 60%, 70%, 80%, 90%, 95%, 99%, or more) of the total number of cells in the composition or preparation.

In embodiments, the composition or preparation comprises trogocytotic T cells at a concentration of at least 30%, (e.g., at least 30%, 40%, 50%, 60%, 70%, 80%, 90%, 95%, 99%, or more) of the total number of cells in the composition or preparation.

Also, provided herein is a preparation of CAR-T cells (e.g., made by a method described herein) for use in (e.g., use in preparation of a medicament for) treating a cancer (e.g., a hematological cancer, a solid cancer or a metastatic cancer) in a subject.

Also, provided herein is a CAR-T cell for use in (e.g., use in preparation of a medicament for) treating a cancer (e.g., a hematological cancer, a solid cancer or a metastatic cancer) in a subject, where the CAR-T cell is produced by a method comprising:
(a) providing a sample from the subject, wherein the sample comprises a T cell and a cancer cell;
(b) contacting the sample with a bispecific T cell engager antibody (BiTE), e.g., for a period of time; and
(c) enriching for activated T cells that have acquired a cell surface marker from the cancer cell.
(d) Generating a CAR-T cell from said activated T cells from (c)

Also, featured herein is a CAR-T cell for use in (e.g., use in preparation of a medicament for) treating a cancer (e.g., a hematological cancer or a solid cancer) in a subject, where the CAR-T cell is produced by a method comprising:
(a) providing a tumor sample from the subject;
(b) providing a blood sample (e.g., peripheral blood sample) from the subject, wherein the blood sample comprises a T cell;
(c) contacting the tumor sample with the blood sample and a bispecific T cell engager antibody (BiTE), e.g., for a period of time; and
(d) enriching for activated T cells that have acquired a cell surface marker from the cancer cell.
(e) Generating a CAR-T cell from said activated T cells from (d)

### Method of evaluating susceptibility to Cytokine-Release Syndrome (CRS)

In one aspect, provided herein is an *in vitro* method of evaluating susceptibility of a subject to develop Cytokine-Release Syndrome (CRS) for an immunotherapy treatment. In an embodiment, the immunotherapy treatment is a BiTE, and the ex vivo assay includes incubating with said BiTE. In another embodiment, the immunotherapy treatment is a T cell therapy, such as a CAR-T therapy, and the ex vivo assay does not include a BiTE. In another embodiment, the immunotherapy treatment is any other immunotherapy treatment that produces CRS in patients. In another embodiment, the treatment is a combination of immunotherapy treatments, or a combination of immunotherapy and non-immunotherapy treatments.

In one aspect, provided herein is an in vitro method of evaluating susceptibility of a subject to develop Cytokine-Release Syndrome (CRS) to a bispecific T cell engager antibody (BiTE) immunotherapy treatment, comprising:
(a) providing a sample comprising at least one T cell from a subject having a cancer;
(b) providing a sample comprising at least one cancer cell, e.g., from the subject;
(c) forming an ex vivo reaction mixture comprising the at least one T cell, the at least one cancer cell, and the bispecific T cell engager antibody (BiTE), being identical to BiTE of the immunotherapy treatment, e.g., under conditions (e.g., for a period of time) sufficient to allow the T cell to kill cancer cells, thereby producing the cancer-killing T cell; and
(d) determining the pharmacological activity of the cancer-killing T cells obtained in step (c) by dose response and/or pharmacodynamic parameters of cancer-killing T cells and tumor cells, selected from EC50, Emax, AUC, Effective E:T Ratios, Basal E:T Ratios, or kinetic parameters;
(e) determining the expression levels of multiple cytokines in the ex vivo reaction mixture, in supernatant and/or intracellular compartments, at basal and several time points; and
(f) evaluating susceptibility of a subject to develop Cytokine-Release Syndrome, by analyzing the results of (e) cytokine levels as a function of (d) cancer-killing activity, wherein a high expression value of anti-inflammatory cytokines in the sample, relative to (i.e. as a function of) its relative cancer-killing activity compared with other patient samples, is indicative of less susceptibility to develop Cytokine-Release Syndrome or wherein a low expression value of pro-inflammatory cytokines in the sample, relative to (i.e. as a function of) its relative cancer-killing activity compared with other patient samples, is indicative of less susceptibility to develop Cytokine-Release Syndrome.

In an aspect, provided herein is an in vitro method of evaluating susceptibility of a subject to develop Cytokine-Release Syndrome (CRS) to a Cellular therapy such as a CAR-T therapy, comprising:
(a) providing a sample comprising at least one T cell selected from the group consisting of a tumor infiltrated lymphocyte (TIL), marrow infiltrated lymphocyte (MIL), a genetically engineered T cell, a CAR-T cell, or an activated T cell obtainable according to the methods of producing CAR-T cells and a genetically engineered T cell expressing Chimeric Antigen Receptors obtainable according to the methods of producing CAR-T cells;
(b) providing a sample comprising at least one cancer cell from a subject having a cancer;
(c) forming an ex vivo reaction mixture comprising the sample of step (a) and the sample of step (b); e.g., under conditions (e.g., for a period of time) sufficient to allow said T cells to kill cancer cells; and
(d) determining the pharmacological activity of the cancer-killing T cells obtained in step (c) by dose response and/or pharmacodynamic parameters of cancer-killing T cells and tumor cells, selected from EC50, Emax, AUC, E:T Ratios, or kinetic parameters;
(e) determining the expression levels of multiple cytokines in the ex vivo reaction mixture, in supernatant and/or intracellular compartments, at basal and several time points; and
(f) evaluating susceptibility of a subject to develop Cytokine-Release Syndrome, by analyzing the results of (e) cytokine levels as a function of (d) cancer-killing activity, wherein a high expression value of anti-inflammatory cytokines in the sample, relative to (i.e. as a function of) its relative cancer-killing activity compared with other patient samples, is indicative of less susceptibility to develop Cytokine-Release Syndrome or wherein a low expression value of pro-inflammatory cytokines in the sample, relative to (i.e. as a function of) its relative cancer-killing activity compared with other patient samples, is indicative of less susceptibility to develop Cytokine-Release Syndrome.

In an embodiment, the cytokines are selected from the group consisting of IL-1a, IL1β, IL-1Ra, IL-2, IL-3, IL-4, IL-5, IL6, IL-7, IL-8, IL-9, IL-10, IL-12, IL12p70, IL-13, IL-15, IL-16, IL-17A, IL-17F, IL-18, IL-22, IP10, IFN-γ, TNF-α.

In a particular embodiment, the pharmacological parameter is Area Under the Curve (AUC) and levels of cytokine for IL-10 and/or INF-□, and their relationship is non-linear enabling selection of subjects with high cancer cell killing activity and moderate cytokine release. In another particular embodiment, the pharmacological parameter is Area Under the Curve (AUC) and levels of cytokine for IL-10 and/or INF-□, and their relationship is non-linear enabling selection of lower doses for subjects predicted with high cancer cell killing activity and high cytokine release, whereby such lower doses decrease the probability of suffering Cytokine Release Syndrome. IN another particular embodiment, the pharmacological parameter is high Effective E:T Ratio coinciding with high levels of cytokine IL-13, an anti-inflammatory cytokine, indicative of high cancer-killing activity and low probability of cytokine release syndrome, and high levels of IL-2.

In another embodiment, sequential time measurements identify dependent processes, such as cytokines induced by other cytokines, or short time vs longer time cytokine level variations, where any of these parameters (e.g. shorter time cytokines) may have higher clinical prediction capacity.

In another embodiment, the method is performed using an automated fluorescence-based platform. In another embodiment, the method is performed using flow cytometry.

In another embodiment, the bispecific T cell engager antibody (BiTE) has a first element providing affinity for the T cell and a second element having affinity for the cancer cell, wherein the first element binds to a T cell and does not bind to a substantial number of cancer cells and wherein the second element binds to a cancer cell and does not bind to a substantial number of T cells. In another embodiment, the first element binding to T cell comprises one or more of the following cell receptors: CD8, CD3, CD4, α/β T cell receptor (TCR), CD45RO, and/or CD45RA. In another embodiment, the second element binds to one or more of the following cell receptors: CD20, CD28, CD30, CD33, CD52; EpCAM, CEA, gpA33, mucin, TAG-72, carbonic anhydrase IX, PSMA, folate binding protein; one or more of a ganglioside selected from: GD2, GD3, or GM2; Lewis-Y2, VEGF, VEGFR, αVβ3, α5β1, ErbB1/EGFR, ErbB2/HER2, ERbB3, c-MET, IGF1R, EphA3, TRAIL-R1, TRAIL-R2, RANKL, FAP, tenascin, CD123, CD19, and/or BCMA. In another embodiment, the T cell engager antibody (BiTE) is selected from the group consisting of BsMAb CD19/CD3, BsMAb CD123/CD3, CD3/CD28 and EpCAM/CD3.

In another embodiment, Chimeric Antigen Receptors recognize a neoantigen of a cancer cell.

In another embodiment, the sample of step (a) and the sample of step (b) are from the same subject. In another embodiment, step (a) and step (b) comprise providing one sample comprising both the cancer cell and the T cell. In another embodiment, the sample (a) is derived from a tissue with a microenvironment, wherein substantially no components have been removed or isolated from the sample, selected from: whole blood, peripheral blood, bone marrow, lymph node, a biopsy of a primary tumor, or a biopsy of a metastasis or spleen.

In another embodiment, the subject is an adult or a pediatric subject.

In another embodiment, the cancer of sample (b) is a hematological cancer selected from: Hodgkin's lymphoma, Non-Hodgkin's lymphoma (B cell lymphoma, diffuse large B cell lymphoma, follicular lymphoma, chronic lymphocytic leukemia, mantle cell lymphoma, marginal zone B-cell lymphoma, Burkitt lymphoma, lymphoplasmacytic lymphoma, hairy cell leukemia), acute myeloid leukemia, chronic myeloid leukemia, myelodysplastic syndrome, multiple myeloma, or acute lymphocytic leukemia.

In another embodiment, the cancer is a solid cancer selected from: ovarian cancer, rectal cancer, stomach cancer, testicular cancer, cancer of the anal region, uterine cancer, colon cancer, rectal cancer, renal-cell carcinoma, liver cancer, non-small cell carcinoma of the lung, cancer of the small intestine, cancer of the esophagus, melanoma, Kaposi's sarcoma, cancer of the endocrine system, cancer of the thyroid gland, cancer of the parathyroid gland, cancer of the adrenal gland, bone cancer, pancreatic cancer, skin cancer, cancer of the head or neck, cutaneous or intraocular malignant melanoma, uterine cancer, brain stem glioma, pituitary adenoma, epidermoid cancer, carcinoma of the cervix squamous cell cancer, carcinoma of the fallopian tubes, carcinoma of the endometrium, carcinoma of the vagina, sarcoma of soft tissue, cancer of the urethra, carcinoma of the vulva, cancer of the penis, cancer of the bladder, cancer of the kidney or ureter, carcinoma of the renal pelvis, spinal axis tumor, neoplasm of the central nervous system (CNS), primary CNS lymphoma, tumor angiogenesis, metastatic lesions of said cancers, or combinations thereof. In another embodiment, the cancer is not melanoma.

In another embodiment, the subject providing sample (a) and/or sample (b):
(i) has not received a prior treatment for the cancer;
(ii) has received one or more previous treatments for the cancer; or
(iii) has minimal residual disease (MRD).

### Use of Artificial Environment (AE)

In one aspect, provided herein is the use of an Artificial Environment (AE) consisting of a plasma fraction, an erythrocyte fraction or a combination thereof, free from leucocytes, in the method of producing CAR-T cells one of the components of the ex vivo reaction mixture comprising a least one T cell, at least one cancer cell and a bispecific T cell engager antibody (BiTE).

In embodiments, provided herein is the use of an Artificial Environment (AE) consisting of a plasma fraction, an erythrocyte fraction or a combination thereof, free from leucocytes, as one of the components in any of the methods of the invention.

### Use of the Microenvironment or Native Environment of a patient sample

In another aspect, provided herein is the use of the whole sample from a patient (e.g. a bone marrow sample) that includes the Microenvironment or Native Environment (NE) of the sample. The NE or microenvironment is the environment in which the tumor exists, including surrounding blood vessels, immune cells, fibroblasts, stromal cells, the extracellular matrix (ECM), soluble factors (e.g. tumor derived exosomes, signaling molecules. growth factors, micro RNA, chemokines, cytokines and any soluble molecule derived from tumor or non-tumor cells), all of which affect tumor cell dynamics.

In embodiments, provided herein is the use of a whole sample that includes the Microenvironment or NE consisting of all components of a patient sample without separation or isolation of any parts of the patient sample, as one of the components in any of the methods of the invention. Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. All publications, patent applications, patents, and other references mentioned herein are incorporated by reference in their entirety. In the case of conflict, the present specification, including definitions, will control. In addition, the materials, methods, and examples are illustrative only and are not intended to be limiting.

Other features and advantages of the invention will be apparent from the following detailed description and claims.

### Brief Description of the Drawings

**Figure 1****.** Clinical correlation achieved by the PM Test for 1^{st} line CYT+IDA in AML.
**Figure 2****.** Survival index with Cytarabine concentration.
**Figure 3****.** Typical dose-response curve and Area Under the Curve (AUC).
**Figure 4****.** Example of treatments classification section of the report.
**Figure 5****.** Differences in residual error of model fitting and how it is graphically displayed in horizontal error bars.
**Figure 6****.** Case example of result details section showing individual drugs activity marker (AUC) and confidence interval on the right side and synergy parameter values (alpha) on the right chart also together with associated confidence intervals.
**Figure 7****.** Depicts an experimental design for using BiTE derived T-cells to generate an effective CAR-T in ALL patients (**Figure 7A**) and AML patients (**Figure 7B**) patients.
**Figure 8****.** The X axes represent the absolute number of activated CD25+ T Cells in both the CAR-T cells and activated T-Cells and the Y axes display the absolute number of TOM-1 B-Cells (**Figures 8A**, **8C** and **8E**) and the absolute number of patient's autologous B-Cells (**Figures 8B**, **8D** and **8F**). The ability of the engineered CAR-T Cells (dotted lines) and the activated autologous T-Cells (solid lines), to deplete the B cell population is shown at 3 time points, 6 hours, 24 hours and 48 hours.
**Figure 9****.** Fitted dose response curves for ICT, CART-ICT, and CART-PB, generated on 4 AML samples. Empty slots represent that these cell therapy constructs could not be generated.
**Figure 10****.** Fitted ex vivo dose response curves comparing the activity of the 3 different cell therapies (CART-PB, ICT, CART-ICT) that could be generated in each of the 4 AML samples (columns).
**Figure 11****.** Overlapped ex vivo dose response curves of all CART-PB, ICTs, and CART-ICTs cell therapy constructs of all 4 AML samples showing that interpatient variability is larger than activity differences among these constructs.
**Figure 12****.** Dose response curve of CART-NKG2D tumor-killing activity against a Melanoma sample, and control. Grey bars represent number of tumor cells per well, shown on left axis. Black bars represent number of CART cells per well, shown on right axis.
**Figure 13****.** Measurement of the efficacy and activity of CART-NKG2D cells in AML by the % of leukemic cells alive. Cryopreserved vials from 4 AML samples (columns) were incubated with CART-NKG2D at 3 Effector:Target (E:T) ratios (horizontal axis 0.5:1, 1:1, 5:1) and 4 incubation times (vertical panels; 1 h, 2 h, 4 h, 24 h).
**Figure 14****.** Fitting of dose response curves of tumor-killing by CART-NKG2D for each AML sample.
**Figure 15****.** Precision Medicine ex vivo Test for CART-NKG2D in AML samples. Left, overlap dose response curves at 24 h showing the direction towards sensitive vs resistant samples. Right quantitative ranking of activity of the Area Under the Curve (AUC) calculated for each sample.
**Figure 16****.** Time dependent kinetic effects of the tumor-killing activity of CART-NKG2D on AML samples.
**Figure 17****.** Activity and trogocytosis CART-CD19 on a B-ALL sample. (A) Cytotoxicity shown by number of tumor cells at dilutions of CART cells. (B) Trogocytotic CART cells high in CD5 and DID dye R4. (C) Forwards scatter vs Pulse with identifies most trogocytotic CART cells as doblets (right shifted cell population) than singlets (left shifted cell population). (D) Singlets in leukemic control.
**Figure 18****.** Trogocytosis of CART-NKG2D on an AML sample (up, R7), composed of singlets and few doblets (down).
**Figure 19****.** FACS sorting of trogocytotic CART-NKG2D cells on an AML sample.
**Figure 20****.** Activity of non-trogocytotic sorted DID- CART-NKG2D cells on the AML sample. Upper panel shows results at 12 h and lower panels at 36 h. Left columns show control and dose response depletion of tumor cells. Middle column shows the number of CART NKG2D+DID- cells. Right columns show the number of CART NKG2D+DID+.
**Figure 21****.** Activity of trogocytotic sorted DID+ CART-NKG2D cells on the AML sample. Upper panel shows results at 12 h and lower panels at 36 h. Left columns show control and dose response depletion of tumor cells. Middle column shows the number of CART NKG2D+DID- cells. Right columns show the number of CART NKG2D+DID+.
**Figure 22****.** Enhanced tumor-killing activity of trogocytotic (DID+, dotted line) vs non-trogocytotic (DID-, continuous line), shown as the absolute decrease of leukemic blasts between 12 to 36 h incubation, relative to the number of CART-NKG2D T cells.
**Figure 23****:** Measurement of activity of purified activated T cells in presence and absence of an immune checkpoint inhibitor. Blast cells from an AML sample were incubated with a CD3xCD123 BiTE alone (grey squares) or in combination with the anti-PD1 antibody Nivolumab (black circles). The blast cells were combined with either activated CD25+CD3+ T cells (**A**), CD4+CD25+ T cells (**B**) or CD8+CD25+ T cells (**C**) at various E:T (Effector:Target) ratios (x-axis). The percentage of survival (normalized) of the leukemic blast cells are displayed on the y-axis.
**Figure 24****:** A CLL PB sample that was resistant to Blinatumomab (CD3-CD19 BiTE), was used to assess the ability of an anti-PD1 antibody (Nivolumab) to increase the number of CD8 (panel A) and CD4 (panel B) activated T-cells, and the impact on the killing efficacy of those T cells against live tumor cells (panel C). For all graphs, the solid lines are Blinatumomab only and the dashed lines are Blinatumomab plus Nivolumab). The x-axis represents a dose-response of Blinatumomab with the dashed lines also having a constant concentration of Nivolumab.
**Figure 25****.** Novel approach for selection of immune check point to combine with a BITE treatment.
**Figure 26****.** PM Test to predict ICHK combinations with a BiTE. For AML. Left; expression levels of ICHKs in BiTE treated resistant tumor cells, and adding PD1, TIM3, or both ICHKs. Middle; dose response curves of BiTE and combinations with these ICHKs. Right; dose response curves of BiTE-activated T cells (CD25+ CD5+). Sample treated with CD3xCD123 BiTE requires PD1 + TIM3.
**Figure 27****.** PM Test cannot identify any BiTE-ICHK combination that reverses leukemic cell resistance.
**Figure 28****:** Adding all immune check point inhibitors to a CART-NKG2D on 2 AML samples (left and right panels) reverses partially resistance to CART, further decreasing tumor cells. Left panel 4 and 24 h. Right panel only 24 h.
**Figure 29****.** PM Test of combinations of a CART-NKG2D with ICHKs on a melanoma sample. Dilutions 1-4 are equivalent to 20X, 10X, 5X, 2,5X.
**Figure 30****.** Cytokine levels on supernatant of BiTE incubated AML samples versus the BiTE tumor-killing activity represented by their AUC, shows a non-linear relationship.
**Figure 31****.** Correlation between Effective E:T Ratio and supernatant levels for cytokines IL-13 and IL-2 for a CART-NKG2D on AML samples.
**Figure 32****:** PM Test Cytokine Storm: cytokine levels (columns) in supernatant of CART-NKG2D on 4 AML samples (lines), plotted versus the tumor-killing activity calculated as the % survival.
**Figure 33****:** PM Test Cytokine Storm: cytokine levels (columns) in supernatant of CART-NKG2D on a single melanoma sample, plotted versus the tumor-killing activity calculated as the % survival.
**Figure 34****:** Effect of Artificial Environment (AE) on the tumor-killing activity of CART-CD19 on an ALL sample. A significant difference exists between the median delta leukemic cells versus median number of CART cells with or without AE.
**Figure 35****.** Absolute number of activated T Cells (CD5+CD25+) overtime. The left panel represents the control wells with only PBS incubating with Artificial Environment (AE, grey) and without AE (black). The middle panel represents the Blinatumomab incubated activated T cells. The right panel shows the ratio of activated T cells incubating with Blinatumomab vs control PBS, the fold over of T cell activation induced by Blinatumomab.
**Figure 36****.** Absolute number of tumor cells over time. The left panel represent the control wells with only PBS incubating with Artificial Environment (AE, grey) and without AE (black). The middle panel represents the Blinatumomab incubated tumor cells. Right panel shows the ratio of tumor cells incubating with Blinatumumab vs control PBS, the fold over of T cell activation induced by Blinatumumab.
**Figure 37****.** Normalized and overlapped dose response curves showing the median fitting of 6 AML samples for a CD3xCD123 bispecific and incubation time. Three media conditions were studied: AE (light grey), Ficoll (medium grey), and Ficoll+IL15 (black).

### Detailed Description of the Invention

The present disclosure relates, at least in part, to a personalized medicine approach to generating and/or selecting immune effector cells that have enhanced cytotoxic activity toward undesired target cells, e.g., cancer cells. Featured herein, for example, is a method for producing immune effector cells (e.g., T cells, e.g., CTLs, e.g. CAR-Ts) that have enhanced cytotoxic activity toward target, e.g., cancer cells. In embodiments, the method comprises bringing immune effector cells (e.g., T cells, e.g., cytotoxic T lymphocytes (CTLs), e.g. CAR-Ts) in spatial proximity with target cells, e.g., cancer cells.

Without wishing to be bound by theory, it is believed that the spatial proximity of the immune effector cells with the target cells, e.g., cancer cells, increases the number of immune effector cells that undergo immune cell activation, and some of which undergo a process called trogocytosis, e.g., compared to the number of cells that would undergo trogocytosis in the absence of a bispecific T cell engager antibody (BiTE). Also, without wishing to be bound by theory, it is believed that T cells (e.g., CTLs) that have undergone trogocytosis (also referred to as trogocytoticT cells) have enhanced cytotoxic activity toward target cells, e.g., cancer cells. Trogocytotic T cells can comprise a number of memory T cells that include tumor-specific T cells and are poised and highly sensitized to kill the specific target cells (e.g., cancer cells) to which they are exposed during the method described herein.

It is also believed that a percentage of T cells in solid tumors and in hematological malignancies are enriched in tumor infiltrating lymphocytes (TILs), and/or cancer antigen-specific CTLs (i.e., CTLs that recognize antigens specific to cancer cells). It is believed that most if not all of the T cells present inside the mass of a solid tumor are immunosuppressed Tumor-Specific Antigen T cells called TILs (Tumor Infiltrated Lymphocytes). It is also believed that the % of TILs in a solid tumor is an important predictor of clinical response to immunotherapy treatments. These concepts have led to the extended use of the "Basal E:T Ratio", the basal ratio of effector to target cells in a solid tumor, as a key immuno-oncology variable. However, in hematological tissues with cancer cells, from hematological malignancies or hematological tissues of solid tumors, such as blood, bone marrow, spleen, lymph nodes, there are always many T cells present. Thus, the Basal E:T Ratio in these hematological tissues has a very different meaning than in solid tumors. In fact, it is believed that the population of immunosuppressed Tumor-Specific Antigen (TSA) T cells in these hematological tissues with cancer cells is very small. Hence, if the Basal E:T Ratios are calculated following the same approach as in solid tumors, the ratio of total T cells to cancer cells, and the % of TSA T cells is very low, these Basal E:T Ratios may grossly overestimate the number of T cells with the innate capacity to kill effectively cancer cells (TSA) but immunosuppressed. The "Effective E:T Ratio" discovered herein captures this same concept of the ratio of the number of effector T cells with capacity to kill cancer cells effectively, divided by the number of cancer cells; it is an objective measurement in the presence of a BiTE of the number of activated, CAR-T cells newly generated (the only ones that could kill cancer cells), and the number of cancer cells that have been killed, both relative to control conditions. The overwhelming use of the Basal E:T Ratio as a key variable in publications of bispecific antibodies incubated with samples of hematological malignancies indicates the lack of appreciation of the heterogeneity in T cells of hematological malignancies tissues.

It is believed that a higher percentage of these CTLs exist in microenvironments with a 3-dimensional structure, such as solid tumors, bone marrow, and lymph nodes, while a lower percentage of these CTLs may exist in more fluid environments, such as peripheral blood. The CTLs, e.g., cancer antigen-specific CTLs, may be a preferred starting material for generating CTLs having enhanced cancer killing activity, e.g., by incubating a sample (containing cancer cells and the CTLs) with a bispecific T cell engager antibody (BiTE). In some examples, the sample can be a microenvironment having a 3-dimensional structure, e.g., solid tumor, bone marrow, or lymph node. In other examples, the sample can be a more fluid microenvironment, such as peripheral blood.

Additionally, without wishing to be bound by theory, it is thought that some of the CTLs, e.g., cancer antigen-specific CTLs, are immunosuppressed by their microenvironment, e.g., the bone marrow or solid tumor. It is also believed that by bringing into direct contact a CTL (e.g., cancer antigen-specific CTLs) with a cancer cell *ex vivo,* a bispecific T cell engager antibody (BiTE) can promote the activation and/or proliferation of the CTL (e.g., cancer antigen-specific CTL). The activation of the CTL (e.g., cancer antigen-specific CTL) may release the CTL from its immunosuppressed state and induce its strong proliferation. This can result in a large number of CTLs in the mixture that specifically recognize a cancer antigen and kill with high efficacy the cancer cells having that specific antigen. In bringing together the cancer cell with the CTL, the bispecific T cell engager antibody (BiTE) may also facilitate the trogocytosis of the CTLs. Thus, it is believed that the trogocytotic T cells in the mixture tend to be those CTLs that have high efficacy of killing specific cancer cells. The population of CTLs having a high efficacy of killing specific cancer cells is also referred to herein as trogocytotic T cells. Advantageously, the use of a bispecific T cell engager antibody (BiTE) *ex vivo* can lead to the generation of such high killing efficacy CTLs even from a sample containing very few cancer antigen-specific CTLs.

Thus, the bispecific T cell engager antibody (BiTE) provides a more efficient method of generating immune effector cells (e.g., T cells) having enhanced target cell killing activity (and method for generating greater numbers of such cells) than previously available techniques, e.g., previously available ACTs. bispecific T cell engager antibody (BiTE) and trogocytosis and methods to measure their activity and recognize high efficacy T cells are described in greater detail below. Constructing CAR-T cells using these BiTE-activated T cells is likely to generate a better T cell therapy, combining the higher potency of the CAR construct directed toward one antigen, with the broader antigen recognition of these BiTE activated T cells enriched in tumor-specific antigen T cells.

In addition to methods of generating such cells, also provided herein are compositions, e.g., pharmaceutical compositions, comprising immune effector cells (e.g., T cells, e.g., CTLs) that have enhanced cytotoxic activity toward cancer cells (e.g., CAR-T cells, e.g., trogocytotic T cells).

Furthermore, without wishing to be bound by theory, it is believed that therapies comprising the immune effector cells (e.g., CAR-T cells) described herein are surprisingly effective in killing a variety of cancers, ranging from solid cancers to hematological cancers. This is unlike many previously available ACTs, such as isolated/expanded tumor-infiltrating lymphocytes, which tend to be effective primarily only in highly immunogenic cancers, e.g., melanomas. Thus, particularly surprising is the ability of the immune effector cells (e.g., CAR-T cells) described herein to kill and treat cancers in which there typically is a low/minimal immune response against the cancer cells (e.g., unlike melanomas, which are thought to have a higher mutation rate than other cancer types and may thus be more immunogenic).

In embodiments, the immune effector cells, e.g., CAR-T cells, e.g., trogocytotic T cells, described herein, the method of producing same, and the methods of use (e.g., as treatment) can have one or more of the following advantages. In some embodiments, the CAR-T cells described herein can target (and eliminate/reduce) multiple types of cancer cells. For example, the CAR-T cells described herein can be produced without having to identify specific antigens against which to direct the T cells. In embodiments, CAR-T cells described herein can be produced without pre-labeling of cancer cells, e.g., pre-labeling cancer cell membranes with a detectable marker or pre-labeling cancer cells with a specific antigen. In embodiments, the CAR-T cells described herein can be produced without pre-activating T cells with an antigen before exposure/incubation with cancer cells. In embodiments, the CAR-T cells described herein can be produced by incubating of bispecific T cell engager antibody (BiTE) with a blood sample (e.g., bone marrow, whole blood, or peripheral blood) from a subject without having to separate any cells from the blood sample. For example, the blood sample may contain both the target cells (e.g., cancer cells) and the immune effector cells (e.g., T cells, e.g., CTLs) starting material that is to be targeted to the target cells, such that separate preparations of the cancer cells and the starting immune effector cells are not required.

Another advantage of the methods and compositions herein includes a safety advantage of the activated tumor-antigen specific T cells, e.g., trogocytotic T cells or high activity cancer-killing T cells. Without wishing to be bound by theory, it is believed that the activated tumor-antigen specific T cells described herein, e.g., produced using a method described herein, preferentially recognize cancer cells expressing a specific cancer antigen and have reduced reactivity to other cells that do not express the specific cancer antigen, e.g., normal cells. This can confer a safety advantage to these activated tumor-antigen specific T cells, as they would preferentially kill the cancer cells. Without wishing to be bound by theory, it is thought that this specificity is due to the preferred and/or selective activation and proliferation in an *ex vivo* assay using a bispecific T cell engager antibody (BiTE) of activated tumor-antigen specific T cells from CTLs that are already specific for cancer antigen(s).

Also, provided herein are methods of selecting the appropriate immunotherapy for a subject, e.g., a patient. For example, provided herein are methods of screening for bispecific T cell engager antibodies (BiTE), e.g., bispecific T cell engager antibodies (BiTE) having optimal activity in generating immune effector cells (e.g., T cells, e.g., CTLs) that have enhanced cytotoxic activity toward cancer cells (e.g., CAR-T cells, e.g., trogocytotic T cells). In embodiments, the candidate bispecific T cell engager antibodies (BiTE) are new compounds/molecules not previously described, and these methods are used for drug discovery.

To generate BiTE-activated T cells to construct CAR-Ts, we follow the following order: First, incubation with only the BiTE may generate activated T cells with a high Effective E:T Ratio, that is activated T cells with a high killing activity. When this happens, the hypothesis is that these activated T cells are well enriched in tumor-specific T cells, and we can use them directly to generate CAR-Ts. When the BiTE-activated T cells are not high cancer killers, we add two alternative approaches: One is to add to the mixture of BiTE incubating with cancer cells and T cells a number of T cell activity enhancing agents, in particular immune check point inhibitors, and especially those targeting T cells. If adding these T cell activity enhancing agents we generate a high cancer killer activated T cells, then we can use them to generate CAR-Ts. If this is not the case, then we hypothesize that the subset of trogocytotic activated T cells within the group of BiTE-activated T cells would represent a subset of high cancer killer T cells. To isolate or enrich the trogocytotic T cells, we have 3 approaches:
a. We can detect them in the original BiTE mixture because they have acquired antibody fluorescent markers from the tumor cells, then we can use the markers combined with activated T cell markers to isolate/enrich them, generating activated T cells that can be used to generate CAR-Ts.
b. If they have not acquired tumor markers in the original BiTE mixture, then we need to isolate/enrich first all activated T cells, and then mix them with a new group of cancer cells frOm the patient, labeling first with a cell membrane tracer dye. Then the activated T cells that acquire the cancer cell tracker dye first, in a short time, we hypothesisze they represent the trogocytotic activated T cells that have higher cancer killing activity. These early trogocytotic T cells can be isolated/enriched to generate CAR-Ts.
c. We can first separate the samples tumor cells vs T cells, label tumor cells with a membrane cell dye, then mix them again, to incubate with the BiTE. We can then detect them in the original BiTE mixture because they have acquired membrane cell dyes from the tumor cells, then we can use these markers combined with activated T cell markers to isolate/enrich them, generating activated T cells that can be used to generate CAR-Ts.

Provided herein are also methods for selecting the optimal immune check point molecule for a cancer patient, leveraging similar ex vivo assays as shown above using BiTE-activated T cells.

There are more than 600 clinical trials currently ongoing using different immune check point inhibitors, that demonstrate the very high interest in this class of immunotherapy. Approvals of PD1 and PDL1 have generated tremendous interest and very good response rates across several cancers. Many of these trials are testing combinations of immune check point molecules with other drugs.

There is also high interest in identifying biomarkers to select patients suitable for immune check point therapies. For instance, the FDA has approved for the first time a new drug, the immune check point molecule PD-1, for all solid cancers with MSI-H or dMMR mutations, present in about 5-10% of all solid tumors.

While expression of immune check point molecules can be very important, Vivia has discovered a novel approach where the expression of these immune check point molecules should be measured not only in the patient samples at baseline, but comparing with the same patient sample after incubating with a BiTE, that activates T cell killing tumor cells, in the subset of resistant tumor cells, whenever present.

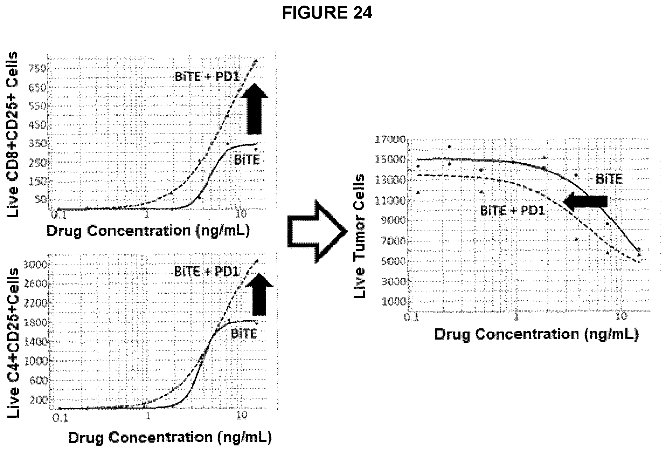

In patient samples of hematological malignancies, when incubating with a BiTE, if the activated T cells kill all tumor cells, then adding an immune check point molecule such as PD1 has no effect. However, in samples where the BiTE-activated T cells cannot kill all tumor cells, and a resistant subset remains, in these cases adding an immune check point molecule such as PD1 can increase activity (Figure 24). This suggests that at least part of the reason for the immuno-resistance of these tumor cells may be due to PD1 activation, and that adding this immune check point molecule could partially revert this resistance.

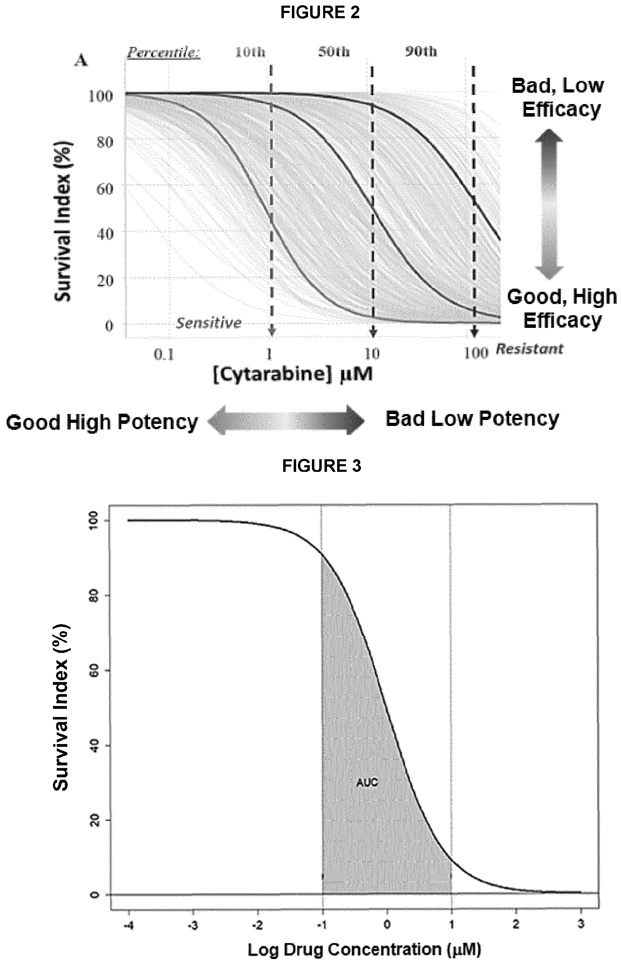

BiTE resistance may be due to expression of immune check point molecules. These ex vivo assays identify the subset of tumor cells resistant to activated T-cells. Hence, we can measure in these resistant immunosuppressed populations which immune check point proteins are expressed. An example is shown in Figure 3, where PDL1 expression was found in 4 samples that were resistant to BiTE in these assays. Selection of appropriate Immune checkpoint inhibitors (e.g. PDL1 for these samples) for each sample could improve BiTE activity. This can be tested in these samples measuring the activity of a BiTE in combination with ICHKs. Effective combinations could become a follow-up therapeutic option for patients that show resistance in clinical trials, for example, following a basket trial design.

We can combine this selective expression in our assay resistant cells, with our activity assay adding immune check point molecules and measuring whether they indeed revert immuno-resistance. Both methods are synergistic and reinforce each other. The common idea is that these immune check point molecules proteins can be expressed in many cells for different reasons, but only a few subsets of them are responsible for the immuno-resistance. This combined expression in resistant cells and functional activity testing can identify the right immune check point molecule for each patient.

Provided herein are also methods for identifying patients likely to suffer a Cytokine Release Syndrome (CRS) when treated with immunotherapies such as BiTEs or T cell therapies such as CAR-Ts. This method can help preventing patients from suffering CRS, by including those unlikely to suffer CRS, and suggest lower doses for those predicted to suffer CRS. These assays predicting CRS are leveraging similar ex vivo assays as shown above using BiTE-activated T cells.

We are measuring ex vivo cytokine profiling in the supernatant of our activity assays. Ex vivo supernatant cytokines have been evaluated and published, even by reputable agencies such as NIH (Vessillier *et al.,* 2015; Finco *et al.,* 2014; Eastwood *et al.,* 2013). Their conclusion is that ex vivo supernatant testing does not predict CRS. These publications measure only absolute cytokine levels, mostly when adding CARTs to tumor cells. However, nobody has associated ex vivo cytokine levels with ex vivo immnotherapy activity, while toxicity is normally associated with activity. Thus, we have measured cytokine levels in supernatants of our ex vivo assays and at the same time measure the activity of the immunotherapy agents (BiTEs), and studied the relationship between toxicity indicated by the cytokine supernatant levels and activity indicated by the pharmacological parameters.

The expectation that BiTE-activated T cells cancer-killing activity is associated with toxicity in terms of cytokine released has been validated in preliminary results. However, the non-linear relationship observed, if validated, may enable patient selection and dosage selection to prevent CRS.

The expectation that when the BiTE generates a high activity cancer-killer T cell, one with a high Effective E:T Ratio, there would be less cytokines released, has been validated in these preliminary experiments. Anti-inflammatory cytokines released may be responsible for lesser probability of CRS in the patient.

Similar results are expected for other immunotherapies that cause CRS such as CAR-Ts.

### Definitions

As used herein, the articles "a" and "an" refer to one or more than one, *e.g*., to at least one, of the grammatical object of the article. The use of the words "a" or "an" when used in conjunction with the term "comprising" herein may mean "one," but it is also consistent with the meaning of "one or more," "at least one," and "one or more than one."

As used herein, "about" and "approximately" generally mean an acceptable degree of error for the quantity measured given the nature or precision of the measurements. Exemplary degrees of error are within 20 percent (%), typically, within 10%, and more typically, within 5% of a given range of values.

The term "autologous" refers to any material derived from the same individual to whom it is later to be re-introduced into the individual.

The term "allogeneic" refers to any material derived from a different animal of the same species as the individual to whom the material is introduced.

The term "composition" for the purpose of present specification, the term composition includes "CAR-T cells," which term includes activated tumor antigen-specific T cells, including, but not limited to, effector memory T cells, cytotoxic T lymphocytes (CTLs), helper T cells, tumor infiltrating lymphocytes (TILs) and trogocytotic T cells, and pharmaceutical composition thereof.

An "effective amount" of the compound of interest is employed in treatment. The dosage of compounds used in accordance with the invention varies depending on the compound and the condition being treated for example the age, weight, and clinical condition of the recipient patient. Other factors include: the route of administration, the patient, the patient's medical history, the severity of the disease process, and the potency of the particular compound. The dose should be sufficient to ameliorate symptoms or signs of the disease treated without producing unacceptable toxicity to the patient. In general, an effective amount of the compound is that which provides either subjective relief of symptoms or an objectively identifiable improvement as noted by the clinician or other qualified observer.

A "bispecific T cell engager" or "bispecific antibody" or "BiTE" as used herein, refers to an agent that enhances trogocytosis of an immune effector cell, e.g., a T cell (e.g., CTL), by bringing an immune effector cell, e.g., a T cell, into proximity with a target cell, e.g., a cancer cell. In an embodiment, the BiTE binds (e.g., directly binds) to each of the immune effector cell and the target cell. In some embodiments, the BiTE is an antibody molecule, e.g., a bispecific antibody molecule that has a first binding specificity for the immune effector cell (e.g., T cell, e.g., CTL) and a second binding specificity for the target cell. Without wishing to be bound by theory, a BiTE can aid the sensitization and/or activation of a cytotoxic T cell (CTL), which in turn, is capable of recognizing and/or eliminating a tumor cell. In some embodiments, the BiTE increases a population of trogocytotic immune effector cells (e.g., T cells) by at least 0.5%, 1%, 5%, 10%, 25%, 30% or more, e.g., relative to the population of trogocytotic immune effector cells (e.g., T cells, e.g., CTLs) generated from a mixture of immune effector cells and cancer cells in the absence of the BiTE.

"Trogocytosis" as used herein refers to a process in which a portion of the cell membrane of a target cell (e.g., antigen presenting cell, e.g., cancer cell) is transferred to an immune effector cell (e.g., T cell, e.g., CTL), thereby forming a "trogocytotic" immune effector cell comprising a portion of the cell membrane from the target cell. In some embodiments, the portion of the cell membrane of the target cell comprises one or more target cell antigens. Thus, trogocytotic immune effector cells can comprise one or more target cell antigens on their cell surface. In other embodiments, the portion of the cell membrane of the target cell comprises membrane fluorescent dyes. Thus, trogocytotic immune effector cells aberrantly express cancer cell surface markers or membrane dyes previously used to stain cancer cells. Without wishing to be bound by theory, it is believed that immune effector cells (e.g., T cells, e.g., CTLs) that have undergone trogocytosis, e.g., captured one or more target cell antigens, are more effective at forming an immune response against (e.g., killing) the target cell, compared to immune effector cell that have not undergone trogocytosis.

"Immune effector cell," as that term is used herein, refers to a cell that is involved in an immune response, e.g., in the promotion of an immune effector response. Examples of immune effector cells include, but are not limited to, T cells, e.g., CD4+ and CD8+ T cells, alpha/beta T cells and gamma/delta T cells, B cells, natural killer (NK) cells, natural killer T (NKT) cells, and mast cells.

"Naive T cells," as used herein, refer to T cells that comprise antigen-inexperienced cells, e.g., that are precursors of memory cells. In some embodiments, naive T cells are younger T cells, i.e., that comprise a less differentiated phenotype. In some embodiments, naive T cells are characterized by expression of CD62L, CD27, CCR7, CD45RA, CD28, and CD127, and the absence of expression of CD57, CD95, CD122, KLRG-1, or CD45RO. In embodiments, naive T cells are characterized by long telomere length. For example, phenotypic markers associated with naive T cells are described, e.g., in Maus M (2014), incorporated by reference herein.

"Cytotoxic T lymphocytes" (CTLs) as used herein refer to T cells that have the ability to kill a target cell. In embodiments, CTLs express CD8 on their cell surface. Without wishing to be bound by theory, it is believed that CD8+ T cells become CTLs once they are activated by recognition of an antigen on a target cell. For example, CTL activation occurs when two steps occur: 1) an interaction between an antigen-bound MHC molecule on the target cell and a T cell receptor on the CTL is made; and 2) a costimulatory signal is made by engagement of costimulatory molecules on the T cell and the target cell. CTLs then recognize specific antigens on target cells and induce the destruction of these target cells, e.g., by cell lysis. In embodiments, CTLs target and kill cancer cells and cells that are infected, e.g., with a virus, or that are damaged in other ways. In embodiments, CD4+ T cells can also kill target cells, and thus, "CTL" as used herein can also refer to CD4+ T cells.

"Tumor infiltrating lymphocytes" (TILs) are used herein refer to lymphocytes that have migrated into a tumor. In embodiments, TILs can be cells at different stages of maturation or differentiation, e.g., TILs can include CTLs, Tregs, and/or effector memory T cells, among other types of lymphocytes. In embodiments, the TILs include CTLs that are cancer antigen-specific, i.e., they recognize specific cancer antigens. In embodiments, TILs have tumor killing activity. In embodiments, TILs may include a different composition or different populations of cells compared to lymphocytes isolated from a sample other than a tumor.

"Effector memory T cell" as used herein refers to T cells that respond at a fast timescale to the presence of antigen, e.g., by rapidly producing effector cytokines. For example, upon contact with an antigen, the effector memory T cell secretes a large amount of inflammatory cytokines. In embodiments, an effector memory T cell has the following cell surface phenotype: CD62L^{low}, CD44, TCR, CD3, IL-7R (CD127), IL-15R, and CCR7^{low}.

"Effective ratio" or "Effective E:T ratio" as used herein refers to the ratio between the activated T cells and the target cancer cells after exposure to a BiTE and/or an immunomodulatory agent. Effective E:T ratio is calculated using the number of activated T cells (E) and the number of target cancer cells (T) after exposure to a BiTE and/or an immunomodulatory agent. In other embodiments, Effective E:T ratio can be calculated for different concentrations of BiTE, e.g., at maximum concentration of BiTE, at a concentration of BiTE that generates a maximum peak in the number of activated or cytotoxic, activated T cells, or at the EC50 concentration of the respective dose response curves. In embodiments, the Effective E:T ratio can also be expressed as the Effective T:E ratio. As used herein, "Basal E:T ratio" is defined as the ratio between the total number of effector T cells, without specifying their subtype, versus total number of target cells. Thus Basal E:T ratio differs from the "Effective E:T ratio", as Basal E:T ratio refers to the ratio between the total number of T cells and the target cancer cells in the absence of, or before exposure to, a BiTE and/or an immunomodulatory agent.

"Regulatory T cells" (Tregs) as used herein refers to T cells generated in the thymus that mediate immunosuppression and tolerogenic responses, e.g., through contact-independent and contact-dependent mechanisms. Some Tregs are inducible Tregs, which are generated from naive T cells in the periphery. In embodiments, Tregs maintain tolerance to self-antigens and help to reduce autoimmunity. In embodiments, Tregs suppress and/or downregulate proliferation and induction of effectorT cells. In embodiments, Tregs express one or more of the following markers on the cell surface: αβ T cell receptor (TCR), CD3, CD4, CD25, CTLA4, and/or glucocorticoid-induced TNF receptor (GITR). In embodiments, Tregs secrete one or more of the following molecules: IL-10, TGFβ, and/or IL-35.

A "clone" as used herein refers to a population of cells that are derived from the same ancestor cell. In embodiments, the cells within a clone of cells share the same phenotype(s) and genotype(s).

"Antibody molecule" as used herein refers to a protein, e.g., an immunoglobulin chain or fragment thereof, comprising at least one immunoglobulin variable domain sequence. An antibody molecule encompasses antibodies (e.g., full-length antibodies) and antibody fragments. For example, a full-length antibody is an immunoglobulin (Ig) molecule (e.g., an IgG antibody) that is naturally occurring or formed by normal immunoglobulin gene fragment recombinatorial processes). In embodiments, an antibody molecule refers to an immunologically active, antigen-binding portion of an immunoglobulin molecule, such as an antibody fragment. An antibody fragment, e.g., functional fragment, is a portion of an antibody, e.g., Fab, Fab', F(ab')₂, F(ab)₂, variable fragment (Fv), domain antibody (dAb), or single chain variable fragment (scFv). A functional antibody fragment binds to the same antigen as that recognized by the intact (e.g., full-length) antibody. The terms "antibody fragment" or "functional fragment" also include isolated fragments consisting of the variable regions, such as the "Fv" fragments consisting of the variable regions of the heavy and light chains or recombinant single chain polypeptide molecules in which light and heavy variable regions are connected by a peptide linker ("scFv proteins"). In some embodiments, an antibody fragment does not include portions of antibodies without antigen binding activity, such as Fc fragments or single amino acid residues. Exemplary antibody molecules include full length antibodies and antibody fragments, e.g., dAb (domain antibody), single chain, Fab, Fab', and F(ab')₂ fragments, and single chain variable fragments (scFvs).

In embodiments, an antibody molecule is monospecific, e.g., it comprises binding specificity for a single epitope. In some embodiments, an antibody molecule is multispecific, e.g., it comprises a plurality of immunoglobulin variable domain sequences, where a first immunoglobulin variable domain sequence has binding specificity for a first epitope and a second immunoglobulin variable domain sequence has binding specificity for a second epitope. In some embodiments, an antibody molecule is a bispecific antibody molecule. "Bispecific antibody molecule" as used herein refers to an antibody molecule that has specificity for more than one (e.g., two, three, four, or more) epitope and/or antigen. A bispecific antibody molecule can encompass a variety of formats and is described in greater detail in the *Bispecific antibody molecules* section herein.

"Antigen" (Ag) as used herein refers to a molecule that can provoke an immune response, e.g., involving activation of certain immune cells and/or antibody generation. Any macromolecule, including almost all proteins or peptides, can be an antigen. Antigens can also be derived from genomic recombinant or DNA. For example, any DNA comprising a nucleotide sequence or a partial nucleotide sequence that encodes a protein capable of eliciting an immune response encodes an "antigen". In embodiments, an antigen does not need to be encoded solely by a full-length nucleotide sequence of a gene, nor does an antigen need to be encoded by a gene at all. In embodiments, an antigen can be synthesized or can be derived from a biological sample, e.g., a tissue sample, a tumor sample, a cell, or a fluid with other biological components.

The "antigen-binding site," or "binding portion" of an antibody molecule refers to the part of an antibody molecule, e.g., an immunoglobulin (Ig) molecule, that participates in antigen binding. In embodiments, the antigen binding site is formed by amino acid residues of the variable (V) regions of the heavy (H) and light (L) chains. Three highly divergent stretches within the variable regions of the heavy and light chains, referred to as hypervariable regions, are disposed between more conserved flanking stretches called "framework regions," (FRs). FRs are amino acid sequences that are naturally found between, and adjacent to, hypervariable regions in immunoglobulins. In embodiments, in an antibody molecule, the three hypervariable regions of a light chain and the three hypervariable regions of a heavy chain are disposed relative to each other in three-dimensional space to form an antigen-binding surface, which is complementary to the three-dimensional surface of a bound antigen. The three hypervariable regions of each of the heavy and light chains are referred to as "complementarity-determining regions", or "CDRs". The framework region and CDRs have been defined and described, e.g., in Kabat EA (1991) and Chothia C (1987). Each variable chain (e.g., variable heavy chain and variable light chain) is typically made up of three CDRs and four FRs, arranged from amino-terminus to carboxy-terminus in the amino acid order: FR1, CDR1, FR2, CDR2, FR3, CDR3, and FR4.

"Minimal residual disease (MRD)" as used herein refers to a small population of cells, e.g., diseased cells, e.g., cancerous cells, remaining in a patient during or after treatment, e.g., when the patient is in remission (i.e., with no signs or symptoms of disease). In embodiments, MRD can be a source of cells that causes relapse of the disease, e.g., cancer, in a patient. MRD can be detected using flow cytometry, protein, DNA, or RNA-based assays capable of measuring small numbers of diseased cells in patient samples, e.g., tissue samples.

"Cancer" as used herein can encompass all types of oncogenic processes and/or cancerous growths. In embodiments, cancer includes primary tumors as well as metastatic tissues or malignantly transformed cells, tissues, or organs. In embodiments, cancer encompasses all histopathologies and stages, e.g., stages of invasiveness/severity, of a cancer. In embodiments, cancer includes relapsed and/or resistant cancer. The terms "cancer" and "tumor" can be used interchangeably.

"Sample" or "tissue sample" refers to a biological sample obtained from a tissue or bodily fluid of a subject or patient. The source of the tissue sample can be solid tissue as from a fresh, frozen and/or preserved organ, tissue sample, biopsy, or aspirate; blood or any blood constituents (*e*.*g*., serum, plasma); bone marrow or any bone marrow constituents; bodily fluids such as urine, cerebral spinal fluid, whole blood, plasma and serum. The sample can include a non-cellular fraction (*e*.*g*., urine, plasma, serum, or other non-cellular body fluid). In other embodiments, the body fluid from which the sample is obtained from an individual comprises blood (*e*.*g*., whole blood). In an embodiment, the sample is a whole blood sample, a whole bone marrow sample, a whole peripheral blood sample, or a whole tumor sample obtained from the subject. In embodiments, a "whole" sample, e.g., when referring to a whole blood sample, whole bone marrow sample, or a whole peripheral blood sample, is a sample where substantially no components (e.g., cells) have been removed or isolated from the sample. In one embodiment, the sample, e.g., blood sample, is diluted (e.g., with a physiologically compatible buffer or media) prior to use in the remaining steps of the method. In other embodiments, a "whole" sample, e.g., a whole tissue sample or whole tumor sample, substantially maintains the microenvironment from the tissue of origin, e.g., substantially maintains the structure of the tumor or immune microenvironment. In another embodiment, the sample, e.g., tumor sample, is processed into smaller pieces (e.g., ground, chopped, blended, pulverized, etc.) and diluted (e.g., with a physiologically compatible buffer or media).

"Cell Surface Label" as used herein refers to an agent that interacts, e.g., specifically and/or non-specifically to, a cell surface component, e.g., a cell surface protein, a glycan, a cell membrane. In embodiments, the agent comprises a detectable signal that functions to label the cell surface or the cell itself. In embodiments, the detectable signal is a chemical molecule that emits fluorescence at a known wavelength, e.g., a fluorochrome. In one embodiment, a cell surface label is an antibody that selectively recognizes one or more cell surface targets, wherein the antibody is attached, e.g., chemically attached, to a fluorophore molecule, e.g., also referred to herein as a "fluorescently labeled antibody". In one embodiment, the cell surface label is another macromolecule that can recognize one or more cell surface targets, such as an aptamer. In another embodiment, the cell surface label is a cell tracker dye. In embodiments, a cell tracker dye is a molecule containing a fluorescent molecule, e.g., a fluorochrome, that can distribute or diffuse throughout the cell surface membrane in a non-specific manner. In one embodiment, a cell tracker dye can be amphiphilic, e.g., distributing to the membrane-water interface, lipophilic, or hydrophobic, e.g., covalently attached to lipids that reside in the membrane bilayer.

The term "immune checkpoint molecule" refers to molecules that can, in some cases, reduce the ability of immune cells, including a CAR-expressing cell to mount an immune effector response. Exemplary checkpoint molecules include but are not limited to PDL-1, PDL-2, B7-1, B7-2, 4-1BBL, Galectin, ICOSL, GITRL, MHCII, OX40L, CD155, B7-H3, PD1, CTLA-4, 4-1BB, TIM-3, ICOS, GITR, LAG-3, KIR, OX40, TIGIT, CD160, 2B4, CD80, CD86, B7-H4 (VTCN1), HVEM (TNFRSF14 or CD270), BTLA, KIR, MHC class I, MHC class II, GAL9, VISTA, BTLA, TIGIT, LAIR1, and A2aR. See, e.g., Pardoll DM (2012), incorporated herein by reference.

The term "Cytokine-Release Syndrome (CRS)" refers to a side effect of an immunotherapy. As part of their immune-related duties, T cells release cytokines, chemical messengers that help to stimulate and direct the immune response. In the case of CRS, there is a rapid and massive release of cytokines into the bloodstream, which can lead to dangerously high fevers and precipitous drops in blood pressure.

The term "Chimeric Antigen Receptors (CAR)" refers engineered synthetic receptors, which graft an arbitrary specificity onto an immune effector cell (T cell). Typically, these receptors are used to graft the specificity of a monoclonal antibody onto a T cell, with transfer of their coding sequence facilitated by retroviral vectors. The receptors are called chimeric because they are composed of parts from different sources. CAR are expressed on the surface of genetically engineered T cells (CAR-T cells).

The term "neoantigen" refers to a newly formed antigen that has not been previously recognized by the immune system. Neoantigens are often associated with tumor antigens and are found in oncogenic cells. Neoantigens can be formed when a protein undergoes further modification within a biochemical pathway such as glycosylation, phosphorylation or proteolysis. This can alter the structure of the protein, and produce new epitopes that are called neoantigenic determinants as they give rise to new antigenic determinants. Recognition requires separate, specific antibodies.

The term "Artificial Environment" (AE, also referred to as AE fraction) refers to fraction or mixture of fractions isolated from a peripheral blood, or bone marrow, or lymph node sample from a donor after density gradient centrifugation excluding leukocyte fraction (AE leukocyte-free). Residual leukocytes could still remain in the AE. AE can be only the plasma fraction, only the erythrocyte fraction, or a combination of the two fractions at any ratio (e.g. 1:1, 1 :2, 2:1, etc.).

The term "AE Leukocyte-free" refers to fraction or sample without leukocytes, or with a residual number of leukocytes, defined as less than 100 leukocytes per µl of AE.

Primary tumor cells - Refers to tumor cells taken directly from living tissue (e.g. bone marrow, peripheral blood, lymph nodes, spleen, or tumor biopsy), isolated and established for ex vivo growth. Primary tumor cells may have been previously extracted and cryopreserved and thawed before use, or may be recently extracted and used without cryopreservation.

Primary cell population - Refers to cells (non-diseased) taken directly from living tissue (e.g. bone marrow, peripheral blood, lymph nodes, spleen, or tumor biopsy) that are established for ex vivo growth.

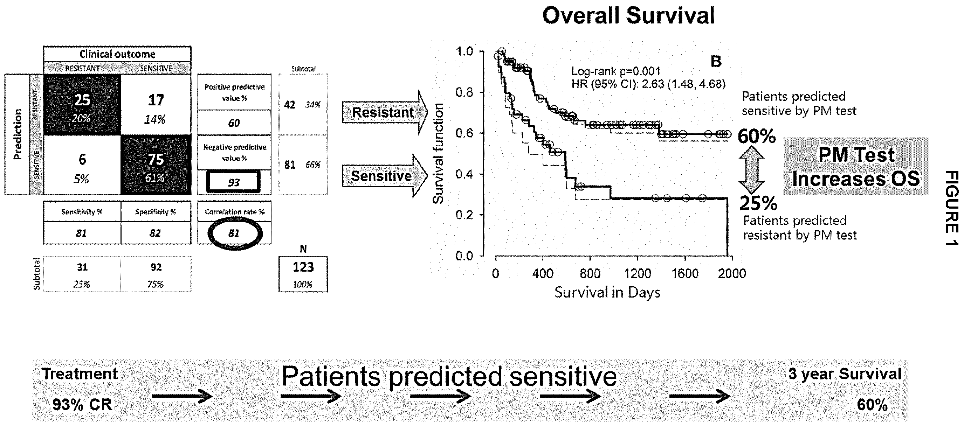

Erythrocyte fraction - AE comprising mainly erythrocytes. When a sample of peripheral blood, bone marrow or lymph node is separated into various component parts by density gradient centrifugation, this is the bottom fraction as see in Fig. 1. Residual leukocytes could still remain in this fraction, but at a concentration of less than 100 leukocytes/ µl AE.

Whole sample (e.g. whole peripheral blood, whole bone marrow or whole lymph node) - The use of the sample in its entirety. No components have been removed or isolated from the sample. As an example, lymphocytes isolated from a bone marrow sample are not considered whole sample.

The term "Native Environment (NE)" refers to the environment in which the tumor exists, including surrounding blood vessels, immune cells, fibroblasts, stromal cells, the extracellular matrix (ECM), soluble factors (e.g. tumor derived exosomes, signaling molecules. growth factors, micro RNA, chemokines, cytokines and any soluble molecule derived from tumor or non-tumor cells), all of which affect tumor cell dynamics. The terms "Native Environment (NE)" and "microenvironment" can be used interchangeably.

### Production of CAR-T Cells

Provided herein are in vitro methods of producing a genetically engineered T cell expressing Chimeric Antigen Receptors (CAR-T) or a CAR-T cell preparation. There are many CAR-T cell preparations already in clinical trial testing, 104 examples are shown in the Table 1.

**Table 1. 104 examples of CAR-T cell preparations already in clinical trial testing**

| **Rank** | **Title** | **Interventions** | **Sponsor/ Collaborators** | **URL** |
|---|---|---|---|---|
| 1 | Study Evaluating the Efficacy and Safety With CAR-T for Recurrent or Refractory Acute Non T Lymphocyte Leukemia | Biological: CD19-targeted CAR-T cells | Sinobioway Cell Therapy Co., Ltd.\|The Second Hospital of Anhui Medical University | https://ClinicalTrials .gov/show/NCT027 35291 |
| 2 | Treatment of Relapsed and/or Chemotherapy Refractory CD33 Positive Acute Myeloid Leukemia by CART-33 | Biological: CART33 cells\|Biological: anti-CD33 CART\|Biological: anti-CD33 CAR T cells | Chinese PLA General Hospital | https://ClinicalTrials .gov/show/NCT018 64902 |
| 3 | Phase I Study of CD19-CAR-T2 Cells for Patients With Chemotherapy Resistant or Refractory CD19+ Acute Leukemia | Biological: CD19-CAR-T2 Cells | Guangdong General Hospital\|Chinese Academy of Sciences\|Guangdo ng Zhaotai InVivo Biomedicine Company Limited | https://ClinicalTrials .gov/show/NCT028 22326 |
| 4 | A Phase 1 Study of CD22 CAR T-Cell Immunotherapy for CD22+ Leukemia | Biological: Patient-derived CD22-specific CAR T-cells also expressing an EGFRt | Seattle Children's Hospital | https://ClinicalTrials .gov/show/NCT032 44306 |
| 5 | CD19 CART Cells in Patients With Resistant or Refractory CD19+ Acute Lymphoblastic Leukemia | Biological: CD19 CAR T cells | Institute of Hematology & Blood Diseases Hospital\|Union Stem cell & gene engineering Co.LTD | https://ClinicalTrials .gov/show/NCT029 75687 |
| 6 | A Study to Assess CD19-targeted Immunotherapy T Cells in Patients With Relapsed or Refractory CD19+ B Cell Leukemia | Drug: anti-CD19-CAR-T cells | Shanghai GeneChem Co., Ltd. | https://ClinicalTrials .gov/show/NCT026 72501 |
| 7 | CAR-T Therapy for Central Nervous System B-cell Acute Lymphocytic Leukemia | Biological: CD19 CAR-T cells | Shanghai Unicar-Therapy Biomedicine Technology Co.,Ltd\|The First Affiliated Hospital of Soochow University | https://ClinicalTrials .gov/show/NCT030 64269 |
| 8 | Pilot Study of T-APCs Following CAR T Cell Immunotherapy for CD19+ Leukemia | Biological: T-cell Antigen Presenting Cells expressing truncated CD19 (T-APC) | Seattle Children's Hospital | https://ClinicalTrials .gov/show/NCT031 86118 |
| 9 | CD19 Chimeric Antigen Receptor (CAR)-Modified T Cell Therapy in Treating Patients With Acute Lymphocytic Leukemia | Genetic: Second generation CAR-T cells | Wuhan Sian Medical Technology Co., Ltd\|Wuhan Union Hospital, China | https://ClinicalTrials .gov/show/NCT029 65092 |
| 10 | Chimeric Antigen Receptor (CAR)-Modified T Cell Therapy in Treating Patients With Acute Lymphoblastic Leukemia | Biological: Third generation CAR-T cells | Affiliated Hospital to Academy of Military Medical Sciences\|Peking University | https://ClinicalTrials .gov/show/NCT021 86860 |
| 11 | Immunotherapy for High Risk/Relapsed CD19+ Acute Lymphoblastic Leukaemia Using CAR T-cells to Target CD19 | Biological: CD19CAT-41 BBZ CAR T-cells | University College, London | https://ClinicalTrials .gov/show/NCT029 35257 |
| 12 | The Safety and Efficacy of CART-19 Cells in B-cell Acute Lymphoblastic Leukemia (B-ALL). | Drug: Cyclophosphamide, Drug: Fludarabine\|Biologi cal: CART-19 cells | Henan Cancer Hospital\|The Beijing Pregene Science and Technology Company, Ltd. | https://ClinicalTrials .gov/show/NCT029 24753 |
| 13 | Phase I/IIA Study of CART19 Cells for Patients With Chemotherapy Resistant or Refractory CD19+ Leukemia and Lymphoma | Biological: CART-19 | University of Pennsylvania | https://ClinicalTrials .gov/show/NCT016 26495 |
| 14 | Autologous ROR1R-CAR-T Cells for Chronic Lymphocytic Leukemia (CLL) | Procedure: ROR1R-CAR-T Cell lnfusion\|Drug: Fludarabine\|Drug: Cyclophosphamide, Drug: Rituximab\|Drug: Bendamustine | M.D. Anderson Cancer Center\|CLL Global Research Foundation Alliance | https://ClinicalTrials .gov/show/NCT021 94374 |
| 15 | Allogeneic CART-19 for Elderly Relapsed/Refractor y CD19+ ALL | Biological: allogeneic CART-19 | The Affiliated Hospital of the Chinese Academy of Military Medical Sciences\|Chinese PLA General Hospital | https://ClinicalTrials .gov/show/NCT027 99550 |
| 16 | Pilot Trial Of Autologous T Cells Engineered To Express Anti-CD19 Chimeric Antigen Receptor (CART19)ln Combination With Ibrutinib In Patients With Relapsed Or Refractory CD19+ Chronic Lymphocytic Leukemia (CLL)Or Small Lymphocytic Lymphoma (SLL) | Biological: CART 19 | University of Pennsylvania | https://ClinicalTrials .gov/show/NCT026 40209 |
| 17 | CART19 in Patient With ALL | Biological: CART 19 | University of Pennsylvania | https://ClinicalTrials .gov/show/NCT029 35543 |
| 18 | Universal CD19-CART Treating ALL | Biological: universal CD19-CART | Shanghai Bioray Laboratory Inc.\|Second Xiangya Hospital of Central South University | https://ClinicalTrials .gov/show/NCT032 29876 |
| 19 | CART-19 Cells For MRD Positive CD19+ ALL | Biological: CART-19 | Fujian Medical University | https://ClinicalTrials .gov/show/NCT030 27739 |
| 20 | Study of Adoptive Cellular Therapy Using Autologous T Cells Transduced With Lentivirus to Express a CD33 Specific Chimeric Antigen Receptor in Patients With Relapsed or Refractory CD33-Positive Acute Myeloid Leukemia | Procedure: Leukapheresis\|Dru g: Fludarabine\|Drug: Cylophosphamide\| Biological: CD33-CAR-T Cell Infusion | M.D. Anderson Cancer Centerllntrexon Corporation IZiopha rm | https://ClinicalTrials .gov/show/NCT031 26864 |
| 21 | Efficacy of CART-19 Cell Therapy in B Cell Acute Lymphoblastic Leukemia | Biological: CART-19 | Beijing Sanwater Biological Technology Co., Ltd. | https://ClinicalTrials .gov/show/NCT028 10223 |
| 22 | CAR T in the Treatment of AML | Biological: Muc1/CD33/CD38/ CD56/CD117/CD12 3-specific gene-engineered T cells | Shenzhen Geno-Immune Medical Institute | https://ClinicalTrials .gov/show/NCT032 22674 |
| 23 | Pilot Study on the Infusion of ARI-0001 Cells in Patients With CD19+ Leukemia or Lymphoma Refractory to Therapy | Biological: Adult differentiated autologous T-cells | Sara V. Latorre\|Instituto de Salud Carlos III\|Institut d'Investigacions BiomÃ¨diques August Pi i Sunyer | https://ClinicalTrials .gov/show/NCT031 44583 |
| 24 | CD19 CAR-T Cells for Patients With Relapse and Refractory CD19+ B-ALL. | Drug: Cyclophosphamide\| Drug: Fludarabine\|Biologi cal: CD19 CAR-T | Henan Cancer Hospital\|The Pregene (ShenZhen) Biotechnology Company, Ltd. | https://ClinicalTrials .gov/show/NCT032 63208 |
| 25 | A Clinical Research of CAR T Cells Targeting CD19 Positive Malignant B-cell Derived Leukemia and Lymphoma | Biological: Chimeric Antigen Receptor Modified T cells Targeting CD19 | Southwest Hospital, China | https://ClinicalTrials .gov/show/NCT023 49698 |
| 26 | Humanized CD19 CAR-T Cells With CRS Suppression Technology for r/r CD19+ Acute Lymphoblastic Leukemia | Biological: Humanized CD19 CAR-T cells\|Biological: Humanized CD19 CAR-T cells with CRS suppression technology | Shanghai Unicar-Therapy Biomedicine Technology Co.,Ltd\|The First Affiliated Hospital of Soochow University | https://ClinicalTrials .gov/show/NCT032 75493 |
| 27 | A Pediatric and Young Adult Trial of Genetically Modified T Cells Directed Against CD19 for Relapsed/Refractor y CD19+ Leukemia | Biological: Patient Derived CD19 specific CAR T cells also expressing an EGFRt | Seattle Children's Hospital | https://ClinicalTrials .gov/show/NCT020 28455 |
| 28 | Study of the Tocilizumab Optimization Timing for CART19 Associated Cytokine Release Syndrome | Drug: Tocilizumab\|Biologi cal: CART 19 | University of Pennsylvania\|Childr en's Hospital of Philadelphia | https://ClinicalTrials .gov/show/NCT029 06371 |
| 29 | Donor-derived Anti-CD123-CART Cells for Recurred AML After Allo-HSCT | Biological: CD123CAR-41BB-CD3zeta-EGFRt-expressing T cells | Affiliated Hospital to Academy of Military Medical Sciences | https://ClinicalTrials .gov/show/NCT031 14670 |
| 30 | Humanized CAR-T Therapy for Treatment of B Cell Malignancy | Biological: CAR-T | Kai Lin Xu; Jun Nian Zheng\|iCarTAB BioMed Inc.\|Huaian first people's hospital\|Xuzhou Medical University | https://ClinicalTrials .gov/show/NCT027 82351 |
| 31 | Anti-CD19 CAR T Infusion Combined With Allogeneic Stem Cell Transplantation for B-cell Leukemia/Lympho ma | Biological: anti-CD19 CAR-T\|Drug: Fludarabine\|Drug: Cyclophosphamide | First Affiliated Hospital of Wenzhou Medical Univeristy | https://ClinicalTrials .gov/show/NCT031 10640 |
| 32 | A Phase I Trial of 4SCAR19 Cells in the Treatment of Relapsed and Refractory B Cell Leukemia | Genetic: prophylactic 4SCAR19 cells | The First People's Hospital of Yunnan\|Shenzhen Geno-Immune Medical Institute | https://ClinicalTrials .gov/show/NCT029 68472 |
| 33 | Pilot Study of Autologous Anti-CD22 Chimeric Antigen Receptor Redirected T Cells In Patients With Chemotherapy Resistant Or Refractory Acute Lymphoblastic Leukemia | Biological: CART22 cells | University of Pennsylvania | https://ClinicalTrials .gov/show/NCT025 88456 |
| 34 | Efficacy and Safety of PZ01 Treatment in Patients With r/r CD19+ B-cell Acute Lymphoblastic Leukemia/B Cell Lymphoma | Drug: PZ01 CAR-T cells | Pinze Lifetechnology Co. Ltd.\|Chinese Academy of Sciences\|Navy General Hospital, Beijing | https://ClinicalTrials .gov/show/NCT032 81551 |
| 35 | CART19 to Treat B-Cell Leukemia or Lymphoma That Are Resistant or Refractory to Chemotherapy | Biological: CART-19 | University of Pennsylvania | https://ClinicalTrials .gov/show/NCT010 29366 |
| 36 | CD22 Redirected Autologous T Cells for ALL | Biological: CART22 cells transduced with a lentiviral vector to express anti-CD22 scFv TCRz:41 BB | University of Pennsylvania\|Childr en's Hospital of Philadelphia | https://ClinicalTrials .gov/show/NCT026 50414 |
| 37 | CAR-T Cell Immunotherapy in CD19 Positive Relapsed or Refractory Leukemia and Lymphoma | Biological: PCAR-019 (anti-CD19 CAR-T cells) | PersonGen BioTherapeutics (Suzhou) Co., Ltd.\|The First People's Hospital of HefeilHefei Binhu Hospital\|Anhui Provincial Hospital | https://ClinicalTrials .gov/show/NCT028 19583 |
| 38 | CAR-T Cells Combined With Peptide Specific Dendritic Cell in Relapsed/Refractor y Leukemia | Biological: Chimeric antigen receptor T cells\|Biological: Eps8 peptide specific dendritic cell | Zhujiang Hospital\|Shenzhen Geno-Immune Medical Institute\|Sun Yat-Sen Memorial Hospital of Sun Yat-Sen University | https://ClinicalTrials .gov/show/NCT032 91444 |
| 39 | CD19 /22 CAR T Cells (AUTO3) for the Treatment of B Cell ALL | Biological: AUTO3 (CD19/22 CAR T cells | Autolus Limited | https://ClinicalTrials .gov/show/NCT032 89455 |
| 40 | Chimeric Antigen Receptor T Cells (CART) Therapy in Refractory/Relapse d B Cell Hematologic Malignancies | Biological: CD19CART | Innovative Cellular Therapeutics Co., Ltd. | https://ClinicalTrials .gov/show/NCT028 13837 |
| 41 | CARPALL: Immunotherapy With CD19 CAR T-cells for CD19+ Haematological Malignancies | Procedure: Leukapheresis\|Dru g: Lymphodepletion with fludarabine\|Drug: Lymphodepletion with cyclophosphamidel Biological: CD19 CAR T-cells | University College, London | https://ClinicalTrials .gov/show/NCT024 43831 |
| 42 | Allo CART-19 Protocol | Biological: CART-19 | University of Pennsylvania | https://ClinicalTrials .gov/show/NCT015 51043 |
| 43 | Administration of Autologous CAR-T CD19 Antigen With Inducible Safety Switch in Patients With Relapsed/Refractor y Acute Lymphoblastic Leukemia | Biological: iC9-CAR19 cells\|Drug: AP1903\|Drug: Cyclophosphamide, Drug: Fludarabine | UNC Lineberger Comprehensive Cancer Center | https://ClinicalTrials .gov/show/NCT030 16377 |
| 44 | Competitive Transfer of Î±CD19-TCRz-CD28 and Î±CD19-TCRz-CD137 CAR-T Cells for B-cell Leukemia/Lympho ma | Biological: anti-CD19 CAR-T\|Drug: Fludarabine\|Drug: Cyclophosphamide | The Second Affiliated Hospital of Henan University of Traditional Chinese Medicine\|Xinqiao Hospital of Chongqing\|Xuzhou Medical University | https://ClinicalTrials .gov/show/NCT026 85670 |
| 45 | CD19-targeting 3rd Generation CAR T Cells for Refractory B Cell Malignancy - a Phase I/IIa Trial. | Biological: Autologous 3rd generation CD19-targeting CAR T cells | Uppsala University\|Uppsala University Hospital\|Karolinska University Hospital\|AFA FÃ¶rsÃ¤kring AB\|Swedish Cancer Society | https://ClinicalTrials .gov/show/NCT021 32624 |
| 46 | A Clinical Research of CD123-Targeted CAR-T in Myeloid Malignancies | Biological: Anti-CD123-CAR-transduced T cells | Southwest Hospital, China | https://ClinicalTrials .gov/show/NCT029 37103 |
| 47 | CAR T Cells for Refractory B Cell Malignancy | Biological: Autologous CD19-targeting CAR T cells | Hebei Senlang Biotechnology Inc., Ltd.\|The Second Hospital of Hebei Medical University | https://ClinicalTrials .gov/show/NCT029 63038 |
| 48 | CD19-targeting, 3rd Generation CAR T Cells for Refractory B Cells Malignancy | Biological: CAR T cells | Uppsala University\|Uppsala University Hospital\|AFA fÃ¶rsÃ¤¤kringar | https://ClinicalTrials .gov/show/NCT030 68416 |
| 49 | A Study Evaluating UCART019 in Patients With Relapsed or Refractory CD19+ Leukemia and Lymphoma | Biological: UCART019 | Chinese PLA General Hospital | https://ClinicalTrials .gov/show/NCT031 66878 |
| 50 | Safety and Efficacy Evaluation of IM19 CAR-T Cells | Biological: IM19 CAR-T | Beijing Immunochina Medical Science & Technology Co., Ltd. | https://ClinicalTrials .gov/show/NCT031 42646 |
| 51 | Study Evaluating the Efficacy and Safety of JCAR015 in Adult B-cell Acute Lymphoblastic Leukemia (B-ALL) | Biological: JCAR015 (CD19-targeted CAR T cells) | Juno Therapeutics, Inc. | https://ClinicalTrials .gov/show/NCT025 35364 |
| 52 | Study of Redirected Autologous T Cells Engineered to Contain Anti-CD19 Attached to TCR and 4-1 BB Signaling Domains in Patients With Chemotherapy Resistant or Refractory Acute Lymphoblastic Leukemia | Biological: CART-19 | University of Pennsylvania | https://ClinicalTrials .gov/show/NCT020 30847 |
| 53 | CD19-CART Treatment for ALL | Biological: CD19 CART | Shanghai Bioray Laboratory Inc.\|Second Xiangya Hospital of Central South University | https://ClinicalTrials .gov/show/NCT032 32619 |
| 54 | Safety and Efficacy Evaluation of IM19 CAR-T Cells (IM19CAR-T) | Biological: IM19 CAR-T\|Drug: fludarabine and cyclophosphamide | Beijing Immunochina Medical Science & Technology Co., Ltd. | https://ClinicalTrials .gov/show/NCT031 73417 |
| 55 | Interleukin-2 Following 4SCAR19/22 T Cells Targeting Refractory and/or Recurrent B Cell Malignancies | Biological: 4SCAR19/22 T cells\|Drug: Interleukin-2 | Zhujiang HospitalShenzhen Geno-Immune Medical Institute | https://ClinicalTrials .gov/show/NCT030 98355 |
| 56 | A Clinical Research of CD20-Targeted CAR-T in B Cell Malignancies | Biological: Anti-CD20-CAR-transduced T cells | Southwest Hospital, China | https://ClinicalTrials .gov/show/NCT027 10149 |
| 57 | A Trial of "Armored" CAR T Cells Targeting CD19 For Patients With Relapsed CD19+ Hematologic Malignancies | Biological: EGFRt/19-28z/4-1BBL CART cells | Memorial Sloan Kettering Cancer Center\|Juno Therapeutics, Inc. | https://ClinicalTrials .gov/show/NCT030 85173 |
| 58 | A Clinical Research of CD22-Targeted CAR-T in B Cell Malignancies | Biological: Anti-CD22-CAR-transduced T cells | Southwest Hospital, China | https://ClinicalTrials .gov/show/NCT029 35153 |
| 59 | Anti-CD22 CAR-T Cell Therapy Targeting B Cell Malignancies | Biological: Anti-CD22-CAR-transduced T cells | Affiliated Hospital to Academy of Military Medical Sciences | https://ClinicalTrials .gov/show/NCT032 62298 |
| 60 | Treatment of Relapsed and/or Chemotherapy Refractory B-cell Malignancy by CART19 | Biological: anti-CD19-CAR vector-transduced T cells | Chinese PLA General Hospital | https://ClinicalTrials .gov/show/NCT018 64889 |
| 61 | a Clinical Research of Sequential CAR-T Bridging HSCT in the Treatment of Relapse/Refractory B-cell Malignancies | Biological: CD19 or CD20 CAR T cells briging HSCT | Southwest Hospital, China | https://ClinicalTrials .gov/show/NCT028 46584 |
| 62 | Study Evaluating the Efficacy and Safety of PCAR-019 in CD19 Positive Relapsed or Refractory Leukemia and Lymphoma | Biological: PCAR-019 (anti-CD19 CAR-T cells) | PersonGen BioTherapeutics (Suzhou) Co., Ltd.\|Anhui Provincial Hospital | https://ClinicalTrials .gov/show/NCT028 51589 |
| 63 | CAR-T Therapy in Relapsed or Refractory Haematopoietic and Lymphoid Malignancies | Biological: Autologous CAR-T | Hebei Senlang Biotechnology Inc., Ltd.\|Hebei Medical University Fourth Hospital | https://ClinicalTrials .gov/show/NCT031 21625 |
| 64 | Immunotherapy With Bispecific CAR-T Cells for B-Cell Lymphoma, ALL and CLL | Biological: anti-CD19 anti-CD20 Bispecific CAR-T | Beijing Doing Biomedical Co., Ltd. | https://ClinicalTrials .gov/show/NCT032 71515 |
| 65 | A Clinical Research of CD30-Targeted CAR-T in Lymphocyte Malignancies | Biological: Anti-CD30-CAR-transduced T cells | Southwest Hospital, China | https://ClinicalTrials .gov/show/NCT029 58410 |
| 66 | A Study of Anti-CD19 CAR-T Cell Immunotherapy for Refractory /Relapsed B Cell Malignancies | Combination Product: Drugs and anti-CD19 CAR transduced T cells | Second Affiliated Hospital of Guangzhou Medical University\|Shenzhe n Institute for Innovation and Translational Medicine\|Guangzh ou First People's Hospital\|First People's Hospital of Foshan\|Dongguan People's Hospital\|The First Affiliated Hospital of Guangdong Pharmaceutical University | https://ClinicalTrials .gov/show/NCT031 91773 |
| 67 | CD19 CAR and PD-1 Knockout Engineered T Cells for CD19 Positive Malignant B-cell Derived Leukemia and Lymphoma | Biological: CD19 CAR and PD-1 knock out engineered T-cells\|Biological: CD19 CAR T-cells | Third Military Medical University | https://ClinicalTrials .gov/show/NCT032 98828 |
| 68 | huJCAR014 CAR-T Cells in Treating Adult Patients With Relapsed or Refractory B-Cell Non-Hodgkin Lymphoma or Acute Lymphoblastic Leukemia | Biological: Autologous Anti-CD19CAR-4-1BB-CD3zeta-EGFRt-expressing CD4+/CD8+ T-lymphocytes (huJCAR014)\|Drug: Cyclophosphamide\| Drug: FludarabinelOther: Laboratory Biomarker Analysis\|Procedure LeukapheresislOth er: Pharmacological Study | Fred Hutchinson Cancer Research Center\|National Cancer Institute (NCI) | https://ClinicalTrials .gov/show/NCT031 03971 |
| 69 | CD19-directed CAR T Cells Therapy in Relapsed/Refractor y B Cell Malignancy | Biological: CD19-directed CAR-T cells | Shanghai Tongji Hospital, Tongji University School of Medicine | https://ClinicalTrials .gov/show/NCT025 37977 |
| 70 | Treatment of Relapsed and/or Chemotherapy Refractory B-cell Malignancy by Tandem CAR T Cells Targeting CD19 and CD22 | Biological: anti-CD19/22-CAR vector-transduced T cells | Chinese PLA General Hospital | https://ClinicalTrials .gov/show/NCT031 85494 |
| 71 | CD19/CD22 Chimeric Antigen Receptor T Cells and Chemotherapy in Treating Children or Young Adults With Recurrent or Refractory CD19 Positive B Acute Lymphoblastic Leukemia | Biological: Chimeric Antigen Receptor T-Cell Therapy\|Drug: Cyclophosphamide\| Drug: Fludarabine Phosphate\|Other: Laboratory Biomarker AnalysislOther: Questionnaire Administration | Stanford University\|National Cancer Institute (NCI) | https://ClinicalTrials .gov/show/NCT032 41940 |
| 72 | Cellular Immunotherapy in Treating Patients With High-Risk Acute Lymphoblastic Leukemia | Biological: Chimeric Antigen Receptor T-Cell Therapy\|Other: laboratory biomarker analysis | City of Hope Medical Center\|National Cancer Institute (NCI) | https://ClinicalTrials .gov/show/NCT021 46924 |
| 73 | Study of TBI-1501 for Relapsed or Refractory Acute Lymphoblastic Leukemia | Biological: TBI-1501 | Takara Bio Inc. | https://ClinicalTrials .gov/show/NCT031 55191 |
| 74 | CD19+ CAR T Cells for Lymphoid Malignancies | Drug: Fludarabine monophosphate\|Dr ug: Cyclophosphamide\| Procedure: T Cell Infusion | M.D. Anderson Cancer Center\|Ziopharm\|In trexon Corporation | https://ClinicalTrials .gov/show/NCT025 29813 |
| 75 | CD123 Redirected Autologous T Cells for AML | Biological: Autologous Anti-CD 123 CAR TCR/4-1 BB-expressing T-lymphocytes\|Drug: Cyclophosphamide | University of Pennsylvania | https://ClinicalTrials .gov/show/NCT026 23582 |
| 76 | Immunotherapy With CD19 CAR T-cells for B-Cell Lymphoma, ALL and CLL | Biological: Anti-CD19-CAR | Beijing Doing Biomedical Co., Ltd.\|First Hospital of Jilin University | https://ClinicalTrials .gov/show/NCT025 46739 |
| 77 | T-cells Expressing Anti-CD19 CAR in Pediatric and Young Adults With B-cell Malignancies | Biological: CD19 CAR T cells | Sheba Medical Center | https://ClinicalTrials .gov/show/NCT027 72198 |
| 78 | CD19/CD22 Chimeric Antigen Receptor T Cells and Chemotherapy in Treating Patients With Recurrent or Refractory CD19 Positive Diffuse Large B-Cell Lymphoma or B Acute Lymphoblastic Leukemia | Biological: Chimeric Antigen Receptor T-Cell Therapy\|Drug: Cyclophosphamide\| Drug: Fludarabine Phosphate\|Other: Laboratory Biomarker AnalysislOther: Questionnaire Administration | David Miklos\|Stanford University | https://ClinicalTrials .gov/show/NCT032 33854 |
| 79 | A Clinical Research of BCMA-Targeted CAR-T in B Cell Malignancies | Biological: Anti-BCMA-CAR-transduced T cells | Southwest Hospital, China | https://ClinicalTrials .gov/show/NCT029 54445 |
| 80 | Treatment of Relapsed and/or Chemotherapy Refractory B-cell Malignancy by Tandem CAR T Cells Targeting CD19 and CD20 | Biological: anti-CD19/20-CAR vector-transduced T cells | Chinese PLA General Hospital | https://ClinicalTrials .gov/show/NCT030 97770 |
| 81 | Allogeneic CART-33 for Relapsed/Refractor y CD33+ AML | Biological: allogeneic CART-33 | The Affiliated Hospital of the Chinese Academy of Military Medical Sciences\|Chinese PLA General Hospital | https://ClinicalTrials .gov/show/NCT027 99680 |
| 82 | Activated T-Cells Expressing 2nd or 3rd Generation CD19-Specific CAR, Advanced B-Cell NHL, ALL, and CLL (SAGAN) | Biological: CD19 CAR T Cells\|Drug: Fludarabine\|Drug: Cyclophosphamide | Baylor College of Medicine\|Center for Cell and Gene Therapy, Baylor College of Medicine\|Texas Children's Hospital\|The Methodist Hospital System | https://ClinicalTrials .gov/show/NCT018 53631 |
| 83 | Combination Transfer of Î±CD19-TCRz-41 BB and Î±CD22-TCRz-41BB CAR-T Cells for B-cell Hematologic Malignancy | Biological: Mixed CAR-T Transfer | Xuzhou Medical University | https://ClinicalTrials .gov/show/NCT029 03810 |
| 84 | CD19 Redirected Autologous T Cells | Biological: CART-19 | University of Pennsylvania | https://ClinicalTrials .gov/show/NCT017 47486 |
| 85 | Autologous T-Cells Expressing a Second Generation CAR for Treatment of T-Cell Malignancies Expressing CD5 Antigen | Genetic: CD5.CAR/28zeta CAR T cells\|Drug: Fludarabine\|Drug: Cytoxan | Baylor College of Medicine\|Center for Cell and Gene Therapy, Baylor College of Medicine\|The Methodist Hospital System\|Texas Children's Hospital | https://ClinicalTrials .gov/show/NCT030 81910 |
| 86 | Genetically Engineered Lymphocyte Therapy in Treating Patients With Lymphoma That is Resistant or Refractory to Chemotherapy | Biological: anti-CD20-CAR vector-transduced autologous T cells\|Other: genetically engineered lymphocyte therapy | Chinese PLA General Hospital | https://ClinicalTrials .gov/show/NCT017 35604 |
| 87 | Pilot Study of Redirected Autologous T Cells Engineered to Contain Humanized Anti-CD19 in Patients With Relapsed or Refractory CD19+ Leukemia and Lymphoma Previously Treated With Cell Therapy | Biological: huCART19 | University of Pennsylvania | https://ClinicalTrials .gov/show/NCT023 74333 |
| 88 | Safety Study of Chimeric Antigen Receptor Modified T-cells Targeting NKG2D-Ligands | Biological: CM-CS1 T-cell infusion | Celyad (formerly named Cardio3 BioSciences)\|Dana-Farber Cancer lnstitute\|National Heart, Lung, and Blood Institute (NHLBI) | https://ClinicalTrials .gov/show/NCT022 03825 |
| 89 | Treatment of Relapsed and/or Chemotherapy Refractory Advanced Malignancies by CART133 | Biological: anti-CD133-CAR vector-transduced T cells | Chinese PLA General Hospital | https://ClinicalTrials .gov/show/NCT025 41370 |
| 90 | Leukapheresis for CAR-Therapy Manufacturing | | National Cancer Institute (NCI)\|National Institutes of Health Clinical Center (CC) | https://ClinicalTrials .gov/show/NCT032 26704 |
| 91 | CD19 Chimeric Receptor Expressing T Lymphocytes In B-Cell Non Hodgkin's Lymphoma, ALL & CLL | Genetic: CD19CAR-28-zeta T cells\|Drug: Ipilimumab | Baylor College of Medicine\|Texas Children's Hospital\|The Methodist Hospital System\|Center for Cell and Gene Therapy, Baylor College of Medicine | https://ClinicalTrials .gov/show/NCT005 86391 |
| 92 | Clinical Study of Redirected Autologous T Cells With a Chimeric Antigen Receptor in Patients With Malignant Tumors | Genetic: CAR-CD19 T cell\|Genetic: CAR-BCMA T cell\|Genetic: CAR-GPC3 T cell\|Genetic: CAR-CLD18 T cell\|Drug: Fludarabine\|Drug: Cyclophosphamide | Kang YU\|Carsgen Therapeutics, Ltd.\|First Affiliated Hospital of Wenzhou Medical Univeristy | https://ClinicalTrials .gov/show/NCT033 02403 |
| 93 | Laboratory Treated T Cells in Treating Patients With Relapsed or Refractory Chronic Lymphocytic Leukemia, Non-Hodgkin Lymphoma, or Acute Lymphoblastic Leukemia | Biological: Autologous Anti-CD19CAR-4-1 BB-CD3zeta-EGFRt-expressing T Lymphocytes\|Other : Laboratory Biomarker Analysis | Fred Hutchinson Cancer Research Center\|National Cancer Institute (NCI) | https://ClinicalTrials .gov/show/NCT018 65617 |
| 94 | Immunotherapy After Chemotherapy in Treating Patients With Relapsed or Refractory B Cell Non-Hodgkin Lymphoma | Biological: Chimeric Antigen Receptor T-Cell Therapy\|Drug: Cyclophosphamide, Drug: FludarabinelOther: Laboratory Biomarker Analysis\|Procedure : Leukapheresis | Fred Hutchinson Cancer Research Center\|National Cancer Institute (NCI) | https://ClinicalTrials .gov/show/NCT032 77729 |
| 95 | CD19 CART Cells for B Cell Malignancies After Allogeneic Transplant | Biological: allogeneic cytomegalovirus-specific cytotoxic T lymphocytes | Fred Hutchinson Cancer Research Center\|National Cancer Institute (NCI) | https://ClinicalTrials .gov/show/NCT014 75058 |
| 96 | T Cells Expressing a Fully-human AntiCD19 Chimeric Antigen Receptor for Treating B-cell Malignancies | Biological: Anti-CD19-CAR T cells\|Drug: Cyclophosphamide, Drug: Fludarabine | National Cancer Institute (NCI)\|National Institutes of Health Clinical Center (CC) | https://ClinicalTrials .gov/show/NCT026 59943 |
| 97 | A Dose Escalation Phase I Study to Assess the Safety and Clinical Activity of Multiple Cancer Indications | Biological: NKR-2 cells | Celyad (formerly named Cardio3 BioSciences) | https://ClinicalTrials .gov/show/NCT030 18405 |
| 98 | Genetically Modified T-Cell Therapy in Treating Patients With Advanced ROR1 + Malignancies | Other: Laboratory Biomarker Analysis\|Biological: ROR1 CAR-specific Autologous T-Lymphocytes | Fred Hutchinson Cancer Research Center\|National Cancer Institute (NCI) | https://ClinicalTrials .gov/show/NCT027 06392 |
| 99 | Study Evaluating Safety and Efficacy of UCART123 in Patients With Acute Myeloid Leukemia | Biological: UCART123 | Cellectis S.A. | https://ClinicalTrials .gov/show/NCT031 90278 |
| 100 | Genetically Modified T-cell Immunotherapy in Treating Patients With Relapsed/Refractor y Acute Myeloid Leukemia and Persistent/Recurren t Blastic Plasmacytoid Dendritic Cell Neoplasm | Drug: cyclophosphamidel Biological: Autologous CD123CAR-CD28-CD3zeta-EGFRt-expressing T Lymphocytes\|Other : laboratory biomarker analysis\|Biological: Allogeneic CD123CAR-CD28-CD3zeta-EGFRt-expressing T-lymphocytes\|Drug: Fludarabine Phosphate | City of Hope Medical Center\|National Cancer Institute (NCI) | https://ClinicalTrials .gov/show/NCT021 59495 |
| 101 | A Phase I/II Multiple Center Trial of 4SCAR19 Cells in the Treatment of Relapsed and Refractory B Cell Malignancies | Genetic: Therapeutic 4SCAR19 cells | Shenzhen Geno-Immune Medical Institute | https://ClinicalTrials .gov/show/NCT030 50190 |
| 102 | Combination CAR-T Cell Therapy Targeting Hematological Malignancies | Biological: 4SCAR19 and 4SCAR22\|Biologica I: 4SCAR19 and 4SCAR38\|Biologica I: 4SCAR19 and 4SCAR20\|Biologica I: 4SCAR19 and 4SCAR123 | Shenzhen Geno-Immune Medical Institute | https://ClinicalTrials .gov/show/NCT031 25577 |
| 103 | CAR T Cell Receptor Immunotherapy for Patients With B-cell Lymphoma | Drug: Fludarabine\|Drug: Cyclophosphamide\| Biological: Anti-CD19-CAR PBL | National Cancer Institute (NCI)\|National Institutes of Health Clinical Center (CC) | https://ClinicalTrials .gov/show/NCT009 24326 |
| 104 | Study to Evaluate the Safety and Clinical Activity of UCART123 in Patients With BPDCN | Biological: UCART123 | Cellectis S.A. | https://ClinicalTrials .gov/show/NCT032 03369 |

Provided herein is an *in vitro* method of producing a genetically engineered T cell expressing Chimeric Antigen Receptors (a CAR-T cell) or a CAR-T cell preparation:
(a) providing a sample comprising at least one T cell from a subject having a cancer;
(b) providing a sample comprising at least one cancer cell;
(c) forming an *ex vivo* reaction mixture comprising the at least one T cell, the at least one cancer cell, and a bispecific T cell engager antibody (BiTE) under conditions and for a period of time sufficient to allow the at least one T cell to become activated and kill at least one cancer cell, thereby producing at least one activated T cell;
(d) selecting the activated T cell, wherein the activated T cell is defined by having an effective E:T ratio higher than 1:5 between the number of activated T cells (E) and the number of target cancer cells (T) after exposure to the bispecific T cell engager antibody (BiTE); and
(e) genetically engineering the activated T cell to produce Chimeric Antigen Receptors (CAR) on the surface of the activated T cell, thereby producing at least one CAR-T cell.

Provided herein is an *in vitro* method of producing a genetically engineered T cell expressing Chimeric Antigen Receptors (a CAR-T cell) or a CAR-T cell preparation:
(a) providing a sample comprising at least one T cell from a subject having a cancer;
(b) providing a sample comprising at least one cancer cell;
(c) forming an *ex vivo* reaction mixture comprising the at least one T cell, the at least one cancer cell, and a bispecific T cell engager antibody (BiTE) under conditions and for a period of time sufficient to allow the at least one T cell to acquire a surface marker from at least one cancer cell, thereby producing at least one activated T cell;
(d) selecting the activated T cell, wherein the activated T cell is defined by having acquired a cell surface marker from at least one cancer cell after exposure to the bispecific T cell engager antibody (BiTE); and
(e) isolating or enriching the activated T cells that have acquired a surface marker, using a fluorescently labeled molecule (e.g., antibody or fragment thereof) that binds to i) one or more cancer antigens ii) one or more markers of activated T cells, or both i) and ii); and
(f) genetically engineering the selected activated T cells to produce Chimeric Antigen Receptors (CAR) on the surface of the activated T cell, thereby producing at least one CAR-T cell.

Provided herein is an *in vitro* method of producing a genetically engineered T cell expressing Chimeric Antigen Receptors (a CAR-T cell) or a CAR-T cell preparation:
(a) providing a sample comprising at least one T cell from a subject having a cancer;
(b) providing a sample comprising at least one cancer cell;
(c) Isolating or enriching the cancer cells from the sample, adding a membrane dye or a cell tracker dye,
(d) forming an *ex vivo* reaction mixture comprising the at least one T cell, the at least one cancer cell, and a bispecific T cell engager antibody (BiTE) under conditions and for a period of time sufficient to allow the at least one T cell to acquire a surface marker from at least one cancer cell, thereby producing at least one activated T cell;
(e) selecting the activated T cell, wherein the activated T cell is defined by having acquired a cell surface marker from at least one cancer cell after exposure to the bispecific T cell engager antibody (BiTE); and
(f) isolating or enriching the activated T cells that have acquired a cancer surface marker, using the fluorescently membrane dye and one or more markers of activated T cells,; and
(g) genetically engineering the selected activated T cells to produce Chimeric Antigen Receptors (CAR) on the surface of the activated T cell, thereby producing at least one CAR-T cell.

Processes for genetic engineering T cells to produce Chimeric Antigen Receptors (CAR) on the surface of the T cell are available to the skilled person, e.g. in the documents Morgan et al., (2016), Dai et al., (2016) and Olbrich H et al., (2017).

In embodiments, the bispecific T cell engager antibody (BiTE) has a first element providing affinity for the T cell and a second element having affinity for the cancer cell, wherein the first element binds to a T cell and does not bind to a substantial number of cancer cells and wherein the second element binds to a cancer cell and does not bind to a substantial number of T cells.

In embodiments, the first element binding to T cell comprises one or more of the following cell receptors: CD8, CD3, CD4, α/β T cell receptor (α/β TCR), CD45RO, and/or CD45RA.

"CD" refers to cluster of differentiation (CD) cell surface molecules, that can be used as markers for the immunophenotyping of cells. They are used for the diagnosis and identification of hematological malignancies (e.g., leukemia, multiple myeloma, lymphoma) and of leukocytes. CD markers are also used to identify and diagnose solid tumors. "TCR" refers to T cell receptor.

"CD45RO" refers to a membrane glycoprotein. It is a splice variant of tyrosine phosphatase CD45, lacking the A, B, and C determinants. The CD45RO isoform is expressed on activated and memory T cells, some B cell subsets, activated monocytes/macrophages, and granulocytes.

In embodiments, the second element binds to one or more of the following cell receptors: CD20, CD28, CD30, CD33, CD52; EpCAM, CEA, gpA33, mucin, TAG-72, carbonic anhydrase IX, PSMA, folate binding protein; one or more of a ganglioside selected from: GD2, GD3, or GM2; Lewis-Y2, VEGF, VEGFR, αVβ3, α5β1, ErbB1/EGFR, ErbB2/HER2, ERbB3, c-MET, IGF1R, EphA3, TRAIL-R1, TRAIL-R2, RANKL, FAP, tenascin, CD123, CD19, and/or BCMA.

"EpCAM" refers to Epithelial cell adhesion molecule. Is a transmembrane glycoprotein mediating Ca²⁺-independent homotypic cell-cell adhesion in epithelia.

"CEA" refers to carcinoembryonic antigen. It encompasses a set of highly related glycoproteins involved in cell adhesion.

"gpA33" refers to cell surface A33 antigen. Is a protein that in humans is encoded by the GPA33 gene. The glycoprotein encoded by this gene is a cell surface antigen that is expressed in greater than 95% of human colon cancers.

"TAG-72" refers to tumor-associated glycoprotein 72. Is a glycoprotein found on the surface of many cancer cells, including ovary, breast, colon, lung, and pancreatic cancers. Is a tumor marker TAG-72 is also the target of the anti-cancer drugs anatumomab, mafenatox and minretumomab.

"PSMA" refers to prostate-specific membrane antigen, also known as glutamate carboxypeptidase II (GCPII), N-acetyl-L-aspartyl-L-glutamate peptidase I (NAALADase I), NAAG peptidase. Is an enzyme that in humans is encoded by the FOLH1 (folate hydrolase 1) gene.

"VEGF" refers to vascular endothelial growth factor, originally known as vascular permeability factor (VPF). Is a signal protein produced by cells that stimulates the formation of blood vessels. To be specific, VEGF is a sub-family of growth factors, the platelet-derived growth factor family of cystine-knot growth factors. They are important signaling proteins involved in both vasculogenesis (the de novo formation of the embryonic circulatory system) and angiogenesis (the growth of blood vessels from pre-existing vasculature).

"VEGFR" refers to receptors for vascular endothelial growth factor (VEGF).

"αVβ3" refers to a type of integrin that is a receptor for vitronectin. Is expressed by platelets and is a receptor for phagocytosis on macrophages or dendritic cells.

"α5β1" refers to an integrin that binds to matrix macromolecules and proteinases and thereby stimulates angiogenesis. It is the primary receptor for fibronectin.

ErbB1/EGFR refers to epidermal growth factor receptor (EGFR; ErbB-1; HER1 in humans). Is a transmembrane protein that is a receptor for members of the epidermal growth factor family (EGF family) of extracellular protein ligands.

"ERbB3" refers to receptor tyrosine-protein kinase, also known as HER3 (human epidermal growth factor receptor 3). Is a membrane bound protein and is a member of the epidermal growth factor receptor (EGFR/ERBB) family of receptor tyrosine kinases.

"c-MET" refers to tyrosine-protein kinase Met or hepatocyte growth factor receptor (HGFR). It possesses tyrosine kinase activity. Is a single pass tyrosine kinase receptor essential for embryonic development, organogenesis and wound healing.

"IGF1R" refers to insulin-like growth factor 1 (IGF-1) receptor. Is a protein found on the surface of human cells. It is a transmembrane receptor that is activated by a hormone called insulin-like growth factor 1 (IGF-1) and by a related hormone called IGF-2. It belongs to the large class of tyrosine kinase receptors.

"EphA3" refers to EPH receptor A3 (ephrin type-A receptor 3). It is a protein. It belongs to the ephrin receptor subfamily of the protein-tyrosine kinase family. EPH and EPH-related receptors have been implicated in mediating developmental events, particularly in the nervous system.

"TRAIL-R1" refers to death receptor DR4 (TRAIL-R1 receptor). TRAIL refers to TNF-related apoptosis-inducing ligand, is a protein functioning as a ligand that induces the process of cell death called apoptosis. TRAIL is a cytokine that is produced and secreted by most normal tissue cells, causes apoptosis primarily in tumor cells, by binding to certain death receptors. TRAIL and its receptors have been used as the targets of several anti-cancer therapeutics since the mid-1990s, such as Mapatumumab. TRAIL has also been designated CD253 (cluster of differentiation 253) and TNFSF10 (tumor necrosis factor (ligand) superfamily, member 10).

"TRAIL-R2" refers to death receptor DR5 (TRAIL-R2 receptor).

"RANKL" refers to receptor activator of nuclear factor kappa-B ligand (RANKL), also known as tumor necrosis factor ligand superfamily member 11 (TNFSF11), TNF-related activation-induced cytokine (TRANCE), osteoprotegerin ligand (OPGL), and osteoclast differentiation factor (ODF), is a protein that in humans is encoded by the TNFSF11 gene. It is known as a type II membrane protein and is a member of the tumor necrosis factor (TNF) superfamily. It has been identified to affect the immune system and control bone regeneration and remodeling. RANKL is an apoptosis regulator gene, a binding partner of osteoprotegerin (OPG), a ligand for the receptor RANK and controls cell proliferation by modifying protein levels of Id4, Id2 and cyclin D1.

"FAP" refers to fibroblast activation protein alpha. It is a melanoma membrane-bound gelatinase, protein. It is selectively expressed in reactive stromal fibroblasts of epithelial cancers, granulation tissue of healing wounds, and malignant cells of bone and soft tissue sarcomas. This protein is thought to be involved in the control of fibroblast growth or epithelial-mesenchymal interactions during development, tissue repair, and epithelial carcinogenesis.

"BCMA" refers to B-cell maturation antigen (or BCM), also known as tumor necrosis factor receptor superfamily member 17 (TNFRSF17). It is a member of the TNF-receptor superfamily. This receptor is preferentially expressed in mature B lymphocytes, and may be important for B cell development and autoimmune response.

In embodiments, the T cell engager antibody (BiTE) is selected from the group consisting of BsMAb CD19/CD3, BsMAb CD123/CD3, BsMAb CD3/CD28 and BsMAb EpCAM/CD3, BsMAb CD20/CD3, BsMAb CD22/CD3, BsMAb CD33/CD3, BsMAb BCMA/CD3.

In embodiments, the ex vivo reaction mixture further comprises one or multiple agents that enhance T cell activity.

In embodiments, the agents that enhance T cell activity are selected from one or more of a chemotherapy drug, a targeted anti-cancer therapy, an oncolytic drug, a cytotoxic agent, an immune-based therapy, a cytokine, an agonist of T cells (e.g., agonistic antibody or fragment thereof or an activator of a costimulatory molecule), an inhibitor of an inhibitory molecule (e.g., immune checkpoint inhibitor), an immunomodulatory agent, a vaccine, or a cellular immunotherapy.

In embodiments, the agents enhancing T cell activity is selected from an agonist of T cells (e.g., an agonistic antibody or fragment thereof or an activator of a costimulatory molecule), and/or an inhibitor of an immune checkpoint inhibitor.

In embodiments, the inhibitors of the immune checkpoint inhibitor is an inhibitor of one or more of: PDL-1, PDL-2, B7-1 (CD80), B7-2 (CD86), 4-1BBL, Galectin, ICOSL, GITRL, OX40L, CD155, B7-H3, PD1, CTLA-4, 4-1BB, TIM-3, ICOS, GITR, LAG-3, KIR, OX40, TIGIT, CD160, 2B4, B7-H4 (VTCN1), HVEM (TNFRSF14 or CD270), BTLA, KIR, MHC class I, MHC class II, GAL9, VISTA, LAIR1, and A2aR

In embodiments, the inhibitors of the immune checkpoint inhibitor comprise one or more of: ipilimumab, tremelimumab, MDX-1106, MK3475, CT-011, AMP-224, MDX-1105, IMP321, or MGA271.

In embodiments, the agents enhancing T cell activity comprises molecules (e.g. antibodies) constructed combining fragments of these molecules enhancing T cell activity, e.g. bispecific or multispecific antibody formats combining recognition arms of several immune checkpoint inhibitors, including but not limited to PD1-PDL1, PD1-PDL2, PD1-LAG3, PD1-TIM3.

In embodiments, the agonist of T cells comprises an antibody or fragment thereof to CD137, CD40, and/or glucocorticoid-induced TNF receptor (GITR).

In embodiments, the immunomodulatory agent comprises/is lenalidomide, ibrutinib or bortezomib.

In embodiments, the agent enhancing T cell activity enhances and/or restores the immunocompetence of T cells.

In embodiments, the immunomodulatory agent is an inhibitor of MDSCs and/or Treg cells.

In embodiments, the immunomodulatory agent activates an immune response to a tumor specific antigen, e.g., it is a vaccine (e.g., a vaccine against targets such as gp100, MUC1 or MAGEA3.

In embodiments, the immunomodulatory agent is a cytokine, e.g., a recombinant cytokine chosen from one or more of GM-CSF, IL-7, IL-12, IL-15, IL-18 or IL-21.

In embodiments, the immunomodulatory agent is a modulator of a component (e.g., enzyme or receptor) associated with amino acid catabolism, signalling of tumor-derived extracellular ATP, adenosine signalling, adenosine production, chemokine and chemokine receptor, recognition of foreign organisms, or kinase signalling activity.

In embodiments, the immunomodulatory agent is an inhibitor (e.g., small molecule inhibitor) of IDO, COX2, ARG1, ArG2, iNOS, or phosphodiesterase (e.g., PDE5); a TLR agonist, or a chemokine antagonist.

In another aspect, selecting the activated T cell in step (d) comprises
(a) isolating or enriching the trogocytotic T cell using a fluorescently labeled molecule (e.g., antibody or fragment thereof, or a cell tracker dye) that binds to i) one or more cancer antigens, or diffuses into the cancer cell membrane or ii) one or more markers of activated T cells, or both i) and ii); and
(b) genetically engineering the trogocytotic activated T cells to produce Chimeric Antigen Receptors (CAR) on the surface of the activated T cell, thereby producing at least one CAR-T cell.

In an embodiment, the selecting and/or enriching step (a) comprises using fluorescence activated cell sorting (FACS). In another embodiment, the selecting and/or enriching step (a) comprises using a bead (e.g., magnetic bead) coated with an antibody or fragment thereof that binds to i) one or more cancer antigens or ii) one or more markers of activated T cells, or both i) and ii). In another embodiment, the cancer-killing T cell preparation is enriched or purified and comprises trogocytotic cancer-killing T cells, e.g., at a concentration of at least 50% (e.g., at least 50%, 55%, 60%, 65%, 70%, 75%, 80%, 85%, 90%, 95%, 99%, or greater) of the total number of cells in the preparation.

In embodiments, the method comprises one, two or all of the following *in vitro* steps:
i) expanding the CAR-T cell from the methods of producing CAR-T cells;
ii) enriching for the CAR-T cell from the methods of producing CAR-T cells; or
iii) purifying the CAR-T cell from the method of producing CAR-T cells.

In embodiments, Chimeric Antigen Receptors recognize a neoantigen of a cancer cell.

In embodiments, the activated T cell is transfected to produce Chimeric Antigen Receptors (CAR) on the surface of said activated T cell. Various genetic methods are used to transfer a specific gene into human T lymphocytes, described in Morgan et al. 2016. There are described two types of methods including viral and nonviral. The advantages or drawbacks of each one are related to the expression levels, stability and their clinical safety. The more frequent viral approach to transduction on tumors includes Gamma Retrovirus vectors, Lentiviral Vectors and Alpha retroviral vectors, that present higher infection rate. The nonviral approach include transposons, and mRNA Electroporation that are easier to produce and have less clinical risk but with less efficacy.

In embodiments, the expansion of the CAR-T cell comprises increasing the number of CAR-T cells by to 2-fold to 10⁶-fold or more.

In embodiments, the selection of the activated T cell, is based on a parameter chosen from one or more of: increased cancer cell killing activity, reduced toxicity, reduced off-target effect, increased viability, increased proliferation, or Effective E:T ratio.

In embodiments, the selecting step (d) comprises using a fluorescently labeled compound that binds to i) one or more cancer antigens, or diffuses into the cancer cell membrane or ii) one or more markers of activated T cells, or both i) and ii); or comprises using a bead coated with an antibody or fragment thereof that binds to i) one or more cancer antigens or ii) one or more markers of activated T cells, or both i) and ii).

In embodiments, the CAR-T cell preparation comprises trogocytotic CAR-T cells at a concentration of at least 50% of the total number of cells in the CAR-T cell preparation.

In embodiments, the CAR-T cell or CAR-T cell preparation comprises one or more CD8+ T cells and/or one or more CD25+ T cells, and/or one or more CD8+/CD25+ T cells and/or one or more CD4+/CD25+ T cells, and or one or more cytotoxic T lymphocytes (CTLs) or one or more tumor infiltrating lymphocytes (TILs) and/or one or more trogocytotic T cells.

In embodiments, the CAR-T cell preparation comprises regulatory T cells (Tregs) at a concentration of less than 10% of the total number of cells in the CAR-T cell preparation; and/or naive T cells at a concentration of less than 10% of the total number of cells in the CAR-T cell preparation.

In embodiments, the method further comprises separating individual clones from the CAR-T cell preparation, wherein the separating step comprises clonal expansion of single cells by:
(i) separating the preparation of CAR-T cells into single cells and
(ii) expanding the single cells to generate one or more preparations of CAR-T cells.

In embodiments, the sample of step (a) and the sample of step (b) are from the same subject.

In embodiments, step (a) and step (b) comprise providing one sample comprising both the at least one cancer cell and the at least one T cell.

In embodiments, the sample (a) is selected from: whole blood, peripheral blood, bone marrow, lymph node, spleen, a primary tumor and a metastasis.

In embodiments, the sample (a) is derived from a tissue with a microenvironment, wherein substantially no components have been removed or isolated from the sample.

In embodiments, the subject is an adult or a pediatric subject.

In embodiments, the cancer of the sample (b) is a hematological cancer selected from: Hodgkin's lymphoma, Non-Hodgkin's lymphoma (B cell lymphoma, diffuse large B cell lymphoma, follicular lymphoma, mantle cell lymphoma, marginal zone B-cell lymphoma, Burkitt lymphoma, lymphoplasmacytic lymphoma, hairy cell leukemia), acute myeloid leukemia, chronic myeloid leukemia, myelodysplastic syndrome, multiple myeloma, chronic lymphocytic leukemia or acute lymphocytic leukemia.

In embodiments, the cancer is a solid cancer selected from: ovarian cancer, rectal cancer, stomach cancer, testicular cancer, cancer of the anal region, uterine cancer, colon cancer, rectal cancer, renal-cell carcinoma, liver cancer, non-small cell carcinoma of the lung, cancer of the small intestine, cancer of the esophagus, melanoma, Kaposi's sarcoma, cancer of the endocrine system, cancer of the thyroid gland, cancer of the parathyroid gland, cancer of the adrenal gland, bone cancer, pancreatic cancer, skin cancer, cancer of the head or neck, cutaneous or intraocular malignant melanoma, uterine cancer, brain stem glioma, pituitary adenoma, epidermoid cancer, carcinoma of the cervix squamous cell cancer, carcinoma of the fallopian tubes, carcinoma of the endometrium, carcinoma of the vagina, sarcoma of soft tissue, cancer of the urethra, carcinoma of the vulva, cancer of the penis, cancer of the bladder, cancer of the kidney or ureter, carcinoma of the renal pelvis, spinal axis tumor, neoplasm of the central nervous system (CNS), primary CNS lymphoma, tumor angiogenesis, metastatic lesions of said cancers, or combinations thereof.

In embodiments, the cancer is not melanoma.

In embodiments, the subject providing sample (a) and/or sample (b):
(i) has not received a prior treatment for the cancer;
(ii) has received one or more previous treatments for the cancer; or
(iii) has minimal residual disease (MRD).

In embodiments, the method further comprises repeating steps (a)-(e) using a sample of T cells and cancer cells different from the sample used in previous steps (a)-(e).

In embodiments, the CAR-T cells produced from each repeat of steps (a)-(e) is pooled to a form a mixture of CAR-T cells.

In embodiments, the method further comprises evaluating the activity of the CAR-T cell or CAR-T cell preparation.

In embodiments, evaluating comprises:
(a) providing a CAR-T cell or a CAR-T cell preparation thereof obtainable according to the method of claim 1;
(b) providing a sample of cancer cells, wherein the cancer cells are from the same subject;
(c) contacting the CAR-T cell or the CAR-T cell preparation thereof with the cancer cells for a period of time sufficient to allow the CAR-T cell to kill the cancer cells;
(d) determining the level of cancer cells after step (c), and optionally determining the level of CAR-T cells after step (c); and optionally,
(e) determining the ratio of either cancer cell to CAR-T cell, or CAR-T cell to cancer cell, from step (d).

In embodiments, step (c) additionally comprises adding a bispecific T cell engager antibody (BiTE) at increasing dosages.

In embodiments, the activity of the CAR-T cell is determined by dose response and/or pharmacodynamic parameters of CAR-T cells and cancer cells, selected from EC50, Emax, Effective E:T ratio, or kinetic parameters.

In embodiments, a decrease in the level or amount of cancer cells, relative to a reference level, is indicative of increased cell killing activity, or wherein a reduced change or no substantial change in the level or amount of cancer cells relative to a reference level, is indicative of decreased cell killing activity.

In embodiments, a high Effective E:T ratio indicates that the CAR-T cell or CAR-T cell preparation thereof is an effective killer of cancer cells, and wherein a low level of cancer cell relative to CAR-T cell, defined as a low ratio of cancer cell to CAR-T cell, is indicative of a poor CAR-T cell killing activity.

In embodiments, an Effective E:T ratio of 1:10 or higher is indicative of potent CAR-T cell killing activity and a ratio of 1:1, 1:3, or 1:5 of is indicative of poor CAR-T cell killing activity.

In embodiments, the level of cancer cells and/or CAR-T cells is determined at time 0 to 72 hours, or several days after step (c).

In embodiments, the method is performed using an automated fluorescence-based platform.

In embodiments, the method is performed using flow cytometry.

Provided herein are methods for producing (e.g., making/providing) immune effector cells (e.g., T cells, e.g., CTLs) that have enhanced cancer-killing activity (e.g., CAR-T cells).

In embodiments, the method involves providing a T cell and a cancer cell from a subject (e.g., the same subject for both the T cell and the cancer cell or a different subject for the T cell versus the cancer cell).

In embodiments, the T cell and cancer cell are provided in the form of a sample from a subject. The sample can be a blood sample, e.g., a whole blood, peripheral blood, or bone marrow sample. In other embodiments, the sample is from a solid tumor (e.g., sample resected from a primary tumor or a metastasis), a lymph node, or a spleen.

In embodiments, substantially no components (e.g., cells) have been removed or isolated from the sample. For example, the sample, e.g., blood sample, is diluted (e.g., with a physiologically compatible buffer or media) prior to use in the remaining steps of the method. In other examples, the sample, e.g., tumor sample, is processed into smaller pieces (e.g., ground, chopped, blended, pulverized, etc.) and diluted (e.g., with a physiologically compatible buffer or media) prior to use in the remaining steps of the method.

In other embodiments, the T cell and the cancer cell are provided in the different samples from a subject. For example, the T cell is provided in the form of a blood sample, e.g., a whole blood, peripheral blood, or bone marrow sample. In other examples, the T cell is provided in the form of a tumor sample (e.g., sample resected from a primary tumor or a metastasis), e.g., where the T cell comprises a tumor-infiltrating T cell. For example, the cancer cell is provided in the form of a blood sample, e.g., a whole blood, peripheral blood, or bone marrow sample, e.g., where the cancer cell comprises a circulating tumor cell (CTC). In other examples, the cancer cell is provided in the form of a sample from a solid tumor (e.g., sample resected from a primary tumor or a metastasis), a lymph node, or a spleen.

The method further involves forming an *ex vivo* reaction mixture with the T cell and the cancer cell, along with a bispecific T cell engager antibody (BiTE). Any BiTE described herein can be used in the method. BiTE are described in greater detail in the "Bispecific T cell engager antibody (BiTE)" section herein. In embodiments, the *ex vivo* reaction mixture is formed under conditions, such as for a period of time, sufficient to allow the T cell to acquire a cell surface marker from the cancer cell (e.g., to allow the T cell to undergo trogocytosis). The method thereby produces an activated T cell.

Without wishing to be bound by theory, it is believed that in both hematological and solid tumors, there can be different tissues affected with tumor cells in a subject. For example, in solid tumors, metastases can contain tumor cells that have different characteristics, e.g., expression patterns (e.g., different antigen expression patterns), from tumor cells within the primary tumor site. As such, the method can include using bispecific T cell engager antibody (BiTE) to activate cancer-specific CTLs within each tissue that is affected by cancer cells in a subject's body.

In embodiments, the sample is derived from a primary solid tumor from the subject, is derived from a metastasis from the subject, and/or is a blood (e.g., whole blood, bone marrow, or peripheral blood) or lymph sample from the subject.

In embodiments, a method of producing/generating CAR-T cells described herein is repeated using different samples from a given subject, where each repetition includes using a different sample of cancer cell, e.g., primary solid tumor, metastases, blood (e.g., whole blood, bone marrow, or peripheral blood), or lymph.

In embodiments, the method further comprises pooling the CAR-T cells generated using each of these different cancer cell samples.

Without wishing to be bound by theory, it is thought that such a method will generate CAR-T cells effective against the different kinds of cancer cells that may be present in different tissues of a given subject. Without wishing to be bound by theory, it is believed that such a method can advantageously kill cancer cells throughout a subject's body (e.g., both at a primary tumor and at metastases and perhaps also circulating in the blood) instead of only killing cancer cells at one site within the body.

Additionally, without wishing to be bound by theory, it is believed that CTCs (tumor cells found in peripheral blood of cancer patients, typically solid tumor cancer patients) may be responsible for metastasis and hence are a good target for killing. As such, the methods described herein include incubation of a bispecific T cell engager antibody (BiTE) *ex vivo* with a peripheral blood sample (containing CTCs and T cells), thereby bringing into proximity CTCs with their cognate cancer antigen-specific T cells in order to generate activated T cells. In other embodiments, e.g., in peripheral blood samples are not sufficiently enriched with cancer antigen-specific T cells, a sample from a 3-dimensional microenvironment (e.g., bone marrow, tumor, metastasis) likely enriched in cancer antigen-specific T cells is used instead of peripheral blood samples. In such cases, for example, a method can include incubating *ex vivo* a bispecific T cell engager antibody (BiTE) and an isolated CTC with a bone marrow, tumor, or metastasis sample (containing cancer antigen-specific T cells) from a subject. This *ex vivo* mixture enables the bispecific T cell engager antibody (BiTE) to bring into spatial proximity the CTCs with the cancer antigen-specific T cells that match those antigens on the CTCs, thereby activating the appropriate T cells to generate trogocytotic T cells. In embodiments, such a method can be repeated using each tissue of the subject affected by cancer, e.g., to maximize the matching of the CTCs with the appropriate cancer antigen-specific T cells.

### Method for testing cellular responsiveness of primary cell populations

Provided herein is an *ex vivo* method for testing cellular responsiveness of primary cell populations to a genetically engineered T cell expressing Chimeric Antigen Receptors (a CAR-T cell) that comprises:
i) submit a whole sample from a subject selected from: peripheral blood (PB), or bone marrow (BN), or lymph node (LN) to a separation process to isolate an Artificial Environment (AE) consisting in a plasma fraction, an erythrocyte fraction or a combination thereof, free from leucocytes,
ii) mix the leucocyte-free AE obtained in the previous step with a primary cell population,
iii) add to the mixture of step ii) at least one genetically engineered T cell expressing Chimeric Antigen Receptors (a CAR-T cell) to be tested, obtainable according to the methods for producing CAR-T cells,
iv) incubate the mixture obtained in step iii) during from 2 hours to 14 days to allow the a genetically engineered T cell expressing Chimeric Antigen Receptors (a CAR-T cell) tested to exert any activity it might have on the primary cell population,
v) assess the viability and/or proliferation of the primary cell population in the presence or absence of the genetically engineered T cell expressing Chimeric Antigen Receptors (a CAR-T cell) tested,
vi) produce comparative data on viability and/or on proliferation of the primary tumor cell population between the assessment made in presence and in absence of the genetically engineered T cell expressing Chimeric Antigen Receptors (a CAR-T cell) tested and relate the data obtained to values indicative of the genetically engineered T cell expressing Chimeric Antigen Receptors (a CAR-T cell) activity for reducing/increasing viability and/or proliferation of the primary cell population.

### Further Processing of CAR-T cells

In accordance with a method described herein, in embodiments, the CAR-T cells can be further selected, enriched, purified, and/or expanded.

In embodiments, the CAR-T cells described herein, e.g., produced by a method described herein, are selected, e.g., from a reaction mixture. For example, the reaction mixture contains a T cell, a cancer cell, and a bispecific T cell engager antibody (BiTE). In embodiments, the CAR-T cells described herein are purified away from the T cell(s) and/or the bispecific T cell engager antibody (BiTE). In embodiments, the CAR-T cells described herein are enriched from the mixture of cells (e.g., cancer cells and/or various types of T cells) in the reaction mixture.

In embodiments, the selection step, purification, and/or the enrichment step comprises using flow cytometry (e.g., fluorescence activated cell sorting (FACS)) or other separation methods such as beads (e.g., magnetic beads). For example, beads can be coated with an antibody or fragment thereof that binds to one or more cancer antigens and/or one or more markers of activated T cells (e.g., CTLs). In this way, cells that bind to such beads would be activated T cells (e.g., CTLs) that express one or more cancer antigens. These cells are likely trogocytotic T cells with enhanced cancer-killing activity. Likewise, in FACS, cells expressing both markers of activated T cells (e.g., CTLs) and markers of cancer cells (e.g., likely trogocytotic T cells) can be separated from other cell types. Negative or positive selection methods can be used for the selection step, purification, and/or the enrichment step.

In embodiments, the CAR-T cells described herein, e.g., produced by a method described herein, are expanded, e.g., to generate an amount of CAR-T cells for administration into a subject. In embodiments, a preparation of CAR-T cells described herein is expanded. In embodiments, the expansion is performed prior to selection, enrichment, or purification of certain T cell populations. In other embodiments, the expansion is performed after selection, enrichment, or purification of certain T cell populations.

Exemplary methods of expanding cells, e.g., CAR-T cells, includes those described in US 8,034,334, US 2012/0244133 and Montes M (2005), incorporated herein by reference. In embodiments, expansion of the CAR-T cells comprises increasing the number of CAR-T cells, e.g., in a preparation, e.g., by at least about 2-fold (e.g., at least about 3-, 4-, 5-, 6-, 7-, 8-, 9-, 10-, 15-, 20-, 50-, 100-, 1000-, 10⁴-, 10⁵-, 10⁶-fold, or more). In embodiments, the expansion is performed over the course of at least 2 days, e.g., at least 2, 3, 4, 5, 6, 7, 8, 9, 10, 11, 12, 13, 14, or more days. In some examples, the cells are expanded by culturing the cells in the presence of a cytokine such as IL-2 and/or IL-15, optionally with the addition of an agent that stimulates the T-cell receptor, e.g., an anti-CD3 antibody or fragment thereof.

In embodiments, the selection step, purification step, and/or expansion step comprises the sequential addition of a low, e.g., an insufficient, number of cancer cells. The methods described herein comprising incubating cancer cells, T cells, and a bispecific T cell engager antibody (BiTE) to generate a cytotoxic T cell can generate different clones of cytotoxic T cells. In embodiments, selection of the cytotoxic T cell clones that are the most efficient or most potent at killing cancer cells can be achieved by sequentially adding low, e.g., insufficient, amounts of cancer cells. In an embodiment, a low, or insufficient, number or amount of cancer cells that can be added to a reaction comprising CAR-T cells is 50% or less, e.g., 30%, 10%, 1%, 0.1%, or 0.01% or less of the number of activated T cells. In one embodiment, the low, or insufficient, number of cancer cells can be added to the mixture (e.g., comprising cancer cells, T cells, and/or a bispecific T cell engager antibody (BiTE)) at least one or more times (e.g., 2, 3, 4, 5, 6, 7, 8, 9 or 10, times). In one embodiment, the low, or insufficient, number of cancer cells is added every 6-48 hours (e.g., 6 hours, 12 hours, 24 hours, 36 hours, or 48 hours). In an embodiment, the low, or insufficient, number of cancer cells that are added are cancer cells from the patient. In an embodiment, the low, or insufficient, number of cancer cells that are added are not cancer cells from the patient. In an embodiment, the low, or insufficient, number of cancer cells that are added are cancer cells from a cancer cell line.

Without wishing to be bound by theory, it is believed that once the cancer cells in the mixture with the T cells and bispecific T cell engager antibody (BiTE) are eliminated, e.g., by the newly generated CAR-T cells, the CAR-T cells can become exhausted and decrease in number. However, without wishing to be bound by theory, when a low, e.g., insufficient, number of cancer cells is added to the generated CAR-T cells, a subset of the CAR-T cells will recognize and kill the newly added cancer cells. Recognition of the cancer cell can occur, for example, through selective recognition and binding of its TCR to the cancer antigen expressed in the cancer cell surface; and the T cell clone with the highest affinity, or fastest kₒₙ kinetic constant, to the cancer antigen can bind more strongly and/or faster to the newly added cancer cells, thereby resulting in elimination of the cancer cells and activation of proliferation of the CAR-T cell clone. Due to the direct competition between the CAR-T cells, the subset of the CAR-T cells that are more efficacious will be activated and will proliferate, while the remainder of the CAR-T cells that do not recognize the newly added cancer cells will continue the process of exhaustion and self-elimination, thereby leaving on the more efficacious CAR-T cells. Thus, without wishing to be bound by theory, the subset of CAR-T cells that kills the newly added cancer cells are believed to be the best killers, e.g., the most active or potent CAR-T cells. In embodiments, without wishing to be bound by theory, repeating this process of adding a low, e.g., insufficient, number of cancer cells is believed to impose an evolutionary selective pressure towards the most active and more efficacious or potent CAR-T cells to preferentially or selectively activate and proliferate. Accordingly, sequential addition of a low, e.g., insufficient, number of cancer cells is useful for the selection and enrichment of the most active and efficacious CAR-T cell clones.

In embodiments, the selected, purified, enriched, and/or expanded cells can form a preparation of CAR-T cells.

### Bispecific T cell engager antibody (BiTE)

### Bispecific antibody molecules

In embodiments, bispecific antibody molecules can comprise more than one antigen-binding site, where different sites are specific for different antigens. In embodiments, bispecific antibody molecules can bind more than one (e.g., two or more) epitopes on the same antigen. In embodiments, bispecific antibody molecules comprise an antigen-binding site specific for a target cell (e.g., cancer cell) and a different antigen-binding site specific for an immune effector cell (e.g., a T cell, e.g., CTL). Bispecific antibody molecules can be classified into five different structural groups: (i) bispecific immunoglobulin G (BsIgG); (ii) IgG appended with an additional antigen-binding moiety; (iii) bispecific antibody fragments; (iv) bispecific fusion proteins; and (v) bispecific antibody conjugates.
(i) BsIgG is a format that is monovalent for each antigen. Exemplary BsIgG formats include but are not limited to crossMab, DAF (two-in-one), DAF (four-in-one), DutaMab, DT-IgG, knobs-in-holes common LC, knobs-in-holes assembly, charge pair, Fab-arm exchange, SEEDbody, triomab, LUZ-Y, Fcab, κλ-body, orthogonal Fab. See Spiess (2015) at Figure 1. Exemplary BsIgGs include catumaxomab (Fresenius Biotech, Trion Pharma, Neopharm), which contains an anti-CD3 arm and an anti-EpCAM arm; and ertumaxomab (Neovii Biotech, Fresenius Biotech), which targets CD3 and HER2. In some embodiments, BslgG comprises heavy chains that are engineered for heterodimerization. For example, heavy chains can be engineered for heterodimerization using a "knobs-into-holes" strategy, a SEED platform, a common heavy chain (e.g., in κλ-bodies), and use of heterodimeric Fc regions. See Spiess C (2015). Strategies that have been used to avoid heavy chain pairing of homodimers in BsIgG include knobs-in-holes, duobody, azymetric, charge pair, HA-TF, SEEDbody, and differential protein A affinity. *See Id.* BsIgG can be produced by separate expression of the component antibodies in different host cells and subsequent purification/assembly into a BsIgG. BsIgG can also be produced by expression of the component antibodies in a single host cell. BsIgG can be purified using affinity chromatography, e.g., using protein A and sequential pH elution.
(ii) IgG appended with an additional antigen-binding moiety is another format of bispecific antibody molecules. For example, monospecific IgG can be engineered to have bispecificity by appending an additional antigen-binding unit onto the monospecific IgG, e.g., at the N- or C- terminus of either the heavy or light chain. Exemplary additional antigen-binding units include single domain antibodies (e.g., variable heavy chain or variable light chain), engineered protein scaffolds, and paired antibody variable domains (e.g., single chain variable fragments or variable fragments). S*ee Id*. Examples of appended IgG formats include dual variable domain IgG (DVD-Ig), IgG(H)-scFv, scFv-(H)IgG, IgG(L)-scFv, scFv-(L)IgG, IgG(L,H)-Fv, IgG(H)-V, V(H)-IgG, IgG(L)-V, V(L)-IgG, KIH IgG-scFab, 2scFv-lgG, IgG-2scFv, scFv4-Ig, zybody, and DVI-IgG (four-in-one). See Spiess C (2015), Figure 1. An example of an IgG-scFv is MM-141 (Merrimack Pharmaceuticals), which binds IGF-1R and HER3. Examples of DVD-Ig include ABT-981 (AbbVie), which binds IL-1α and IL-1β; and ABT-122 (AbbVie), which binds TNF and IL-17A.
(iii) Bispecific antibody fragments (BsAb) are a format of bispecific antibody molecules that lack some or all of the antibody constant domains. For example, some BsAb lack an Fc region. In embodiments, bispecific antibody fragments include heavy and light chain regions that are connected by a peptide linker that permits efficient expression of the BsAb in a single host cell. Exemplary bispecific antibody fragments include but are not limited to nanobody, nanobody-HAS, BiTE, Diabody, DART, TandAb, scDiabody, scDiabody-CH3, Diabody-CH3, triple body, miniantibody, minibody, TriBi minibody, scFv-CH3 KIH, Fab-scFv, scFv-CH-CL-scFv, F(ab')2, F(ab')2-scFv2, scFv-KIH, Fab-scFv-Fc, tetravalent HCAb, scDiabody-Fc, Diabody-Fc, tandem scFv-Fc, and intrabody. *See Id.* For example, the BiTE format comprises tandem scFvs, where the component scFvs bind to CD3 on T cells and a surface antigen on cancer cells. Exemplary BiTEs include blinatumomab (Amgen), which binds CD3 and CD19; solitomab (Amgen), which binds CD3 and EpCAM; MEDI 565 (Medlmmune, Amgen), which binds CD3 and CEA; and BAY2010112 (Bayer, Amgen), which binds CD3 and PSMA. Exemplary DARTs include MGD006 (Macrogenics), which binds CD3 and CD123; and MGD007 (Macrogenics), which binds CD3 and gpA33. Exemplary TandAbs include AFM11 (Affimed Therapeutics), which binds CD3 and CD19; and AFM13 (Affimed Therapeutics), which binds CD30 and CD16A. An example of a tandem scFv is rM28 (University Hospital of Tubingen), which binds CD28 and MAPG. Exemplary nanobodies include ozoralizumab (Ablynx), which binds TNF and HSA; ALX-0761 (Merck Serono, Ablynx), which binds IL-17A/F and HSA; ALX-0061 (AbbVie, Ablynx), which binds IL-6R and HSA; ALX-0141 (Ablynx, Eddingpharm), which binds RANKL and HSA. The component fragments of BsAb can be identified/selected using phage display. In some embodiments, the BiTE does not comprise blinatumomab.
(iv) Bispecific fusion proteins include antibody fragments linked to other proteins, e.g., to add additional specificity and/or functionality. An example of a bispecific fusion protein is an immTAC, which comprises an anti-CD3 scFv linked to an affinity-matured T-cell receptor that recognizes HLA-presented peptides. In embodiments, the dock-and-lock (DNL) method can be used to generate bispecific antibody molecules with higher valency. Also, fusions to albumin binding proteins or human serum albumin can be extend the serum half-life of antibody fragments. *See Id.*
   In embodiments, chemical conjugation, e.g., chemical conjugation of antibodies and/or antibody fragments, can be used to create BsAb molecules. *See Id.* An exemplary bispecific antibody conjugate includes the CovX-body format, in which a low molecular weight drug is conjugated site-specifically to a single reactive lysine in each Fab arm or an antibody or fragment thereof. In embodiments, the conjugation improves the serum half-life of the low molecular weight drug. An exemplary CovX-body is CVX-241 (NCT01004822), which comprises an antibody conjugated to two short peptides inhibiting either VEGF or Ang2. *See Id.*
(v) Bispecific antibody molecules can be produced by recombinant expression, e.g., of at least one or more component, in a host system. Exemplary host systems include eukaryotic cells (e.g., mammalian cells, e.g., CHO cells, or insect cells, e.g., SF9 or S2 cells) and prokaryotic cells (e.g., *E. coli*)*.* Bispecific antibody molecules can be produced by separate expression of the components in different host cells and subsequent purification/assembly. Alternatively, bispecific antibody molecules can be produced by expression of the components in a single host cell. Purification of bispecific antibody molecules can be performed by various methods such as affinity chromatography, e.g., using protein A and sequential pH elution. In other embodiments, affinity tags can be used for purification, e.g., histidine-containing tag, myc tag, or streptavidin tag.

In embodiments, a BiTE includes multispecific constructs with more than 2 recognition arms, a common development in the field of bispecific antibodies, and a natural extension of the same concept. In embodiments, multispecific constructs can add more recognition fragments of the same type, or include fragments with different recognition properties.

Bispecific antibodies can also be named DART, DutaFab, Duobodies, Biparatopic, Adaptir.

### Other Compositions and Methods of Enhancing T Cell Activity

In accordance with the compositions and methods described herein, in embodiments, other immunomodulatory agents can be used in addition to a bispecific T cell engager antibody (BiTE) to enhance T cell activity, e.g., trogocytosis. These immunomodulatory agents include, but are not limited to, immune checkpoint inhibitors, agonists of T cells, and other immunomodulatory drugs. Alternatively, other agents for enhancing T cell activity, e.g., trogocytosis, (e.g., use of immunomodulatory agents, such as immune checkpoint inhibitors, agonists of T cells, and other immunomodulatory drugs) can be used instead of a BiTE.

### Immune checkpoint inhibitors

In embodiments, methods described herein comprise use of an immune checkpoint inhibitor, e.g., in a reaction mixture with a cancer cell and an immune effector cell (e.g., T cell, e.g., CTL), e.g., in addition to or instead of a bispecific T cell engager antibody (BiTE). In embodiments, methods described herein comprise contacting a cancer cell and an immune effector cell (e.g., T cell, e.g., CTL) with an immune checkpoint inhibitor. The methods can also be used in a therapeutic protocol *in vivo.*

In embodiments, an immune checkpoint inhibitor inhibits a checkpoint molecule. Checkpoint molecules can, in some cases, reduce the ability of a CAR-expressing cell to mount an immune effector response. Exemplary checkpoint molecules include but are not limited to CTLA4, PD1, PD-L1, PD-L2, TIM3, LAG3, CD160, 2B4, CD80, CD86, B7-H3 (CD276), B7-H4 (VTCN1), HVEM (TNFRSF14 or CD270), BTLA, KIR, MHC class I, MHC class II, GAL9, VISTA, BTLA, TIGIT, LAIR1, and A2aR. See, e.g., Pardoll DM (2012), incorporated herein by reference.

In embodiments, the immune checkpoint inhibitor is a PD-1 inhibitor, e.g., an anti-PD-1 antibody such as Nivolumab, Pembrolizumab or Pidilizumab. Nivolumab (also called MDX- 1106, MDX-1106-04, ONO-4538, or BMS-936558) is a fully human IgG4 monoclonal antibody that specifically inhibits PD1. See, e.g., US 8,008,449 and WO2006/121168. Pembrolizumab (also called Lambrolizumab, MK-3475, MK03475, SCH-900475 or KEYTRUDA^{®}; Merck) is a humanized IgG4 monoclonal antibody that binds to PD-1. See, e.g., Hamid O (2013), US 8,354,509 and WO2009/114335. Pidilizumab (also called CT-011 or Cure Tech) is a humanized IgG1k monoclonal antibody that binds to PD1. See, e.g., WO2009/101611. In one embodiment, the inhibitor of PD-1 is an antibody molecule having a sequence substantially identical or similar thereto, e.g., a sequence at least 85%, 90%, 95% identical or higher to the sequence of Nivolumab, Pembrolizumab or Pidilizumab. Additional anti-PD1 antibodies, e.g., AMP 514 (Amplimmune), are described, e.g., in US 8,609,089, US 2010/028330, and/or US 2012/0114649.

In some embodiments, the PD-1 inhibitor is an immunoadhesin, e.g., an immunoadhesin comprising an extracellular/PD-1 binding portion of a PD-1 ligand (e.g., PD-L1 or PD-L2) that is fused to a constant region (e.g., an Fc region of an immunoglobulin). In embodiments, the PD-1 inhibitor is AMP-224 (B7-DClg, e.g., described in WO2011/066342 and WO2010/027827), a PD-L2 Fc fusion soluble receptor that blocks the interaction between B7-H1 and PD-1.

In embodiments, the immune checkpoint inhibitor is a PD-L1 inhibitor, e.g., an antibody molecule. In some embodiments, the PD-L1 inhibitor is YW243.55.S70, MPDL3280A, MEDI-4736, MSB-0010718C, or MDX-1105. In some embodiments, the anti-PD-L1 antibody is MSB0010718C (also called A09-246-2; Merck Serono), which is a monoclonal antibody that binds to PD-L1. Exemplary humanized anti-PD-L1 antibodies are described, e.g., in WO2013/079174. In one embodiment, the PD-L1 inhibitor is an anti-PD-L1 antibody, e.g., YW243.55.S70. The YW243.55.S70 antibody is described, e.g., in WO 2010/077634. In one embodiment, the PD-L1 inhibitor is MDX-1105 (also called BMS-936559), which is described, e.g., in WO2007/005874. In one embodiment, the PD-L1 inhibitor is MDPL3280A (Genentech / Roche), which is a human Fc-optimized IgG1 monoclonal antibody against PD-L1. See, e.g., US 7,943,743 and US 2012/0039906. In one embodiment, the inhibitor of PD-L1 is an antibody molecule having a sequence substantially identical or similar thereto, e.g., a sequence at least 85%, 90%, 95% identical or higher to the sequence of YW243.55.S70, MPDL3280A, MEDI-4736, MSB-0010718C, or MDX-1105.

In embodiments, the immune checkpoint inhibitor is a PD-L2 inhibitor, e.g., AMP-224 (which is a PD-L2 Fc fusion soluble receptor that blocks the interaction between PD1 and B7-H1. See, e.g., WO2010/027827 and WO2011/066342.

In one embodiment, the immune checkpoint inhibitor is a LAG-3 inhibitor, e.g., an anti-LAG-3 antibody molecule. In embodiments, the anti-LAG-3 antibody is BMS-986016 (also called BMS986016; Bristol-Myers Squibb). BMS-986016 and other humanized anti-LAG-3 antibodies are described, e.g., in US 2011/0150892, WO2010/019570, and WO2014/008218.

In embodiments, the immune checkpoint inhibitor is a TIM-3 inhibitor, e.g., anti-TIM3 antibody molecule, e.g., described in US 8,552,156, WO 2011/155607, EP2581113 and US 2014/044728.

In embodiments, the immune checkpoint inhibitor is a CTLA-4 inhibitor, e.g., anti-CTLA-4 antibody molecule. Exemplary anti-CTLA4 antibodies include Tremelimumab (lgG2 monoclonal antibody from Pfizer, formerly known as ticilimumab, CP-675,206); and Ipilimumab (also called MDX-010, CAS No. 477202-00-9). Other exemplary anti-CTLA-4 antibodies are described, e.g., in US 5,811,097.

### Agonists of a T cell (e.g., Agonistic antibody)

In embodiments, compositions and methods described herein comprise use of an agonist of T cells (e.g., agonistic antibody), e.g., in a reaction mixture with a cancer cell and an immune effector cell (e.g., T cell, e.g., CTL), e.g., in addition to or instead of a BiTE. In embodiments, methods described herein comprise contacting a cancer cell and an immune effector cell (e.g., T cell, e.g., CTL) with an agonist of T cells (e.g., agonistic antibody).

In embodiments, the agonist of T cells is an agonistic antibody or fragment thereof or an activator/agonist of a costimulatory molecule. In embodiments, the agonist of T cells comprises or is a costimulatory molecule. A costimulatory molecule is a cell surface molecule required for an efficient response of a lymphocyte, e.g., T cell, to an antigen. In embodiments, a costimulatory molecule is a molecule other than an antigen receptor or its ligands. Without wishing to be bound by theory, costimulation is believed to enhance expansion, survival, and effector function of T cells (e.g., enhance T cell persistence and/or anti-cancer activity. See, e.g., Song DJ (2012). Exemplary costimulatory molecules include but are not limited to CD28, ICOS (CD278), BTLA, LIGHT, HVEM (LIGHTR), CD160 (BY55), OX40, CD27, CD2, CD7, CD40, CD30, 4-1BB (CD137), ICAM-1, B7-1, a toll-like receptor, LFA-1 (CD11a/CD18), GITR, BAFFR, B7-H3, a signalling lymphocytic activation molecules (SLAM protein), SLAMF7, SLAM (SLAMF1, CD150, IPO-3), SLAMF4 (CD244, 2B4), an integrin, IL2R beta, ITGA4, a MHC class I molecule, a TNF receptor, CD49D, CD49f, LFA-1, CD29, CD18, TNFR2, CD84, RANKL, CD229, CD69, CD100 (SEMA4D), and SLAMF6 (NTB-A, Ly108).

In some embodiments, the agonist of T cells is an agonistic antibody or fragment thereof to CD137, GITR, or CD40.

Exemplary agonistic antibodies are described, e.g., in Scott AM (2012), incorporated herein by reference.

### Other immunomodulatory drugs

In embodiments, compositions and methods described herein comprise an immunomodulatory drug, e.g., lenalidomide, e.g., in a reaction mixture with a cancer cell and an immune effector cell (e.g., T cell, e.g., CTL), e.g., in addition to or instead of a bispecific T cell engager antibody (BiTE). In embodiments, methods described herein comprise contacting a cancer cell and an immune effector cell (e.g., T cell, e.g., CTL) with an agonist of T cells (e.g., agonistic antibody).

In one embodiment, the immunomodulatory agent is an inhibitor of MDSCs and/or Treg cells. Without wishing to be bound by theory, MDSCs and regulatory T (Treg) cells are important components of the immune suppressive tumor microenvironment. Experimental evidence has revealed that MDSCs can modulate the development and induction of Treg cells. For example, MDSCs can suppress T cell effector functions in various ways. Several factors can modulate the expression levels of Arginine, NADPH oxidase and NOS in MDSC subsets, with the final effect on the microenvironment including depletion of I-arginine, release of RNS and ROS (with ONOO-and H2O2 being the most prevalent molecules, respectively) or unopposed production of high NO levels. Moreover, I-cysteine can be sequestered by MDSCs. All of these molecules influence the intracellular signaling pathways that control T cell proliferation after antigen stimulation. MDSC-mediated immune suppression can also be associated with the expansion of Treg cell populations, inhibition of the T-cell proliferation and promotion of T-cell apoptosis.

In one embodiment, the immunomodulatory agent is lenalidomide.

In other embodiments, the immunomodulatory agent activates an immune response to a tumor specific antigen, e.g., it is a vaccine (e.g., a vaccine against targets such as gp100, MUC1 or MAGEA3.

In other embodiments, the immunomodulatory agent is a cytokine, e.g., a recombinant cytokine chosen from one or more of GM-CSF, IL-7, IL-12, IL-15, IL-18 or IL-21.

In other embodiments, the immunomodulatory agent is an autologous T cell, e.g., a tumor-targeted extracellular and intracellular tumor-specific antigen (e.g., a CAR-T cell or a TCR T cell).

In yet other embodiments, the immunomodulatory agent is a modulator of a component (e.g., enzyme or receptor) associated with amino acid catabolism, signalling of tumor-derived extracellular ATP, adenosine signalling, adenosine production, chemokine and chemokine receptor, recognition of foreign organisms, or kinase signalling activity. Exemplary agents include an inhibitor (e.g., small molecule inhibitor) of IDO, COX2, ARG1, ArG2, iNOS, or phosphodiesterase (e.g., PDE5); a TLR agonist, or a chemokine antagonist. Exemplary IDO inhibitors include INCB24360, 1-Methyl tryptophan inhibitor, and NLG919. Exemplary ARG1/ARG2 inhibitors include Compound 9, NCX-4016, and AT38. Exemplary PDE5 inhibitors include Tadalafil. Exemplary agents that modulate tumor extracellular ATP include agonist or antagonist of P2X7, and antagonist of P2Y₁₁. Exemplary agents that modulate adenosine signalling include antagonists of A_{2A} receptor (e.g., SCH58261 and SCH420814), and antagonists of A_{2B} receptor (e.g., PSB1115). Modulators of chemokines and chemokine receptors, such as CXCR1, CXCR2, CXCR4, CCR2 and CCR5 include, but are not limited to, CXCR2-specific antibodies, Plerixafor, PF-4136309 and Maraviroc. Modulators of TLRs such as TLR4 (e.g., OM-174, a TLR4 agonist), TLR7 (e.g., Imiquimod, 852A, a TLR7/8 agonist), TLR8 (e.g., VTX-2337, a TLR8 agonist) and TLR9 (e.g., IMO-2055, a TLR9 agonist). Exemplary kinase inhibitors include, but are not limited to, inhibitors of ALK, BRAF, RON, CSF1, PI3K-delta and PI3K-gamma.

Additional examples of immunomodulatory agents are further described in, e.g., Adams JL (2015) and Serafini P (2008), incorporated here by reference.

### Evaluation of CAR-T Cells

In accordance with a method described herein, in embodiments, CAR-T cells (e.g., preparations of CAR-T cells, e.g., selected, purified, enriched, and/or expanded cells) can be characterized or evaluated in a number of ways.

For example, the CAR-T cells can be characterized for expression of various cancer cell and/or effector T cell markers, e.g., with panels of antibodies, e.g., monoclonal antibodies. In embodiments, the cells are characterized for expression of markers such as PD-1 and TIM-3, among other immune checkpoint molecules (e.g., immune checkpoint molecules described herein). In embodiments, the presence of expression of PD-1 and/or TIM-3 on the cells can indicate that the CAR-T cells are more tumor immunoreactive.

In embodiments, the CAR-T cells can be evaluated for their reactivity to cancer cells, e.g., *in vitro* or ex *vivo.* Without wishing to be bound by theory, it is believed that reactivity to cancer cells, e.g., *in vitro* or *ex vivo,* is a measure of how effective the CAR-T cells will be at killing cancer cells *in vivo.* In embodiments, reactivity to cancer cells can be assessed by contacting the CAR-T cells with cancer cells, e.g., cancer-derived cell lines or primary cancer samples.

In embodiments, the primary cancer samples include cells isolated from a hematological malignancy in a subject, e.g., isolated from a blood sample (e.g., peripheral blood or bone marrow) of a subject having a hematological malignancy. For example, the CAR-T cells and the cancer cells are contacted by co-culturing, e.g., at a predetermined T cell:cancer cell ratio. Exemplary T cell:cancer cell ratios include about 1:4 to 1:100 (e.g., 1:4, 1:5, 1:6, 1:7, 1:8, 1:9, 1:10, 1:15, 1:20, 1:25, 1:30, 1:35, 1:40, 1:45, 1:50, 1:75, 1 :100, or higher). In embodiments, the reactivity can be assessed a period of time of 1-36 hours after co-culture (e.g., 1-36 hours, 1-6 hours, 6-12 hours, 12-24 hours, or 24-36 hours). Reactivity can be assessed by quantifying the amount of interferon-gamma released by the cells and/or the percentage of T cells that express 4-1BB. In embodiments, a higher level of interferon-gamma and/or 4-1BB compared to a control level indicates that the CAR-T cells are reactive to the cancer cells. Alternatively, markers for specific tumor lineages can be evaluated, including but not limited to, CD107a, granzyme B, perforin, and other specific lineage tumours markers.

In other embodiments, reactivity is assessed by first labeling the cancer cells with a marker (e.g., radioactive marker, e.g., ⁵¹Cr, a fluorescent marker, or other molecule) prior to co-culture with the CAR-T cells. After co-culture of labeled cancer cells with CAR-T cells, the amount of the marker released into the media (e.g., an indicator of extent of cancer cell lysis) is a measure of the extent of cancer cell death. The amount of radioactive marker, e.g., ⁵¹Cr, can be quantified by using any method to detect and quantify radioactivity. The amount of a fluorescent marker can be quantified using any method to detect and quantify fluorescence. The amount of a marker, e.g., a fluorescent marker or other molecule, can also be quantified using an antibody-based assay (e.g., ELISA). In embodiments, a higher level of the release marker from the cancer cells compared to a control level indicates that the CAR-T cells are reactive to the cancer cells.

In embodiments, the control level can be a level of interferon-gamma and/or 4-1 BB, and/or marker generated in a similar assay in the absence of a CAR-T cell, in the absence of cancer cells, or in the absence of labeled cancer cells.

Also, provided herein are methods for measuring the trogocytosis of the CAR-T cells using cell surface labeling. Measuring the trogocytosis of the CAR-T cells can be useful in the selection, characterization, and evaluation of the CAR-T cells produced by any of the methods described herein. Assays for measuring trogocytosis can also be useful in the screening assays.

In embodiments, trogocytosis is assessed by 1) contacting a cancer cell with a cell surface label, e.g., a fluorescently-labelled antibody or fragment thereof, or a cell tracker dye, thereby labeling the cancer cell; 2) contacting a T cell with the labelled cancer cell; and 3) measuring the trogocytosis by determining the T cells that have incorporated the cell surface label from the cancer cell. In one embodiment, the cell surface label is a fluorescently labeled antibody or fragment thereof that specifically binds to a target antigen, e.g., a cancer cell surface marker. In one embodiment, the cell surface label is a cell tracker dye that non-specifically diffuses throughout and/or distributes within the cell membrane.

In embodiments, when trogocytosis occurs, there is extensive contact of the cell membrane surface between a CAR-T cell and a target cancer cell in the immune synapse created between the cells, prior to the T cell inserting its toxic factors into the cancer. This contact involves deep overlap of the respective cell membranes involving patches of membrane across both cells. In the trogocytosis, the T cells take up some of these membrane patches, along with any cell surface labels, e.g., fluorescently-labelled antibodies or cell tracker dyes, present in these membrane patches.

An advantage to using fluorescently-labelled antibodies includes the identification and tracking of trogocytotic T cells that have incorporated a specific cell surface marker from a cancer cell. However, without wishing to be bound by theory, in some embodiments, use of fluorescently-labelled antibodies may not be able to detect trogocytosis. In embodiments, the number of cancer cell fluorescent antibodies taken up in the T cell can depend on their relative numbers on the membrane patches of the immune synapse. For example, in some embodiments, the density or number of cell surface markers on the target cell is too low for detection of any trogocytosis that may occur, e.g., there is not enough labelled antibody that recognizes a target for a detectable signal or not enough labelled antibody that is incorporated into the T cell after a trogocytotic event to be detected. In another example, in an embodiment, the fluorochromes linked to the antibody do not emit sufficient detectable signal, and thus, cannot be detected in a trogocytotic event where a small fraction of the labelled antibody targets is taken up by the CAR-T cell. In another example, in an embodiment, a fluorescently-labelled antibody bound to a cell surface marker may become internalized, which can result in substantially lowering the fluorescence signal to below the detection limit.

Cell tracker dyes include lipophilic or amphiphilic fluorochromes that do not stay in the aqueous medium, but rather distribute throughout the hydrophobic surface membrane of the cells. Thus, in contrast to fluorescently-labelled antibodies, in embodiments where cell tracker dyes are used, any patches of membrane of the cancer cell taken by the T cells will carry the fluorescent molecules and can be detectable. In embodiments where high doses of cell tracker dyes are used, the number of fluorescent molecules that is incorporated into the cell membrane of the T cells by trogocytosis can be higher, e.g., substantially higher, than the number of fluorescently labelled antibodies to specific cancer cell targets.

Due to the non-specific nature of the cell tracker dye in labeling cell membranes, use of cell tracker dyes in samples that contain both cancer cells and T cells (e.g., in whole samples as used herein) can cause the labelling of both the cancer cells and the T cells, and therefore, can prevent accurate measurement of trogocytotic events. Thus, in such embodiments, the cell tracker dye is added selectively only to the cancer cells, e.g., to the cancer cells in the absence of T cells or CAR-T cells. In embodiments where cell tracker dyes are used and where the samples contain both cancer cells and T cells, the cell tracker dye is not added to the sample directly. In such embodiments, the cancer cells, T cells, and a bispecific T cell engager antibody (BiTE) are provided under the conditions described herein to generate CAR-T cells. In an embodiment, the bispecific T cell engager antibody (BiTE) can be washed away from the cancer cells and T cells, e.g., CAR-T cells. In an embodiment, after the generated CAR-T cells kill all or almost all of the cancer cells, a labelled cancer cell, or a population of labelled cancer cells, can be added to the newly generated CAR-T cells. In embodiments, the labelled cancer cell or a population of labelled cancer cells can be cancer cells from the patient (e.g., directly from the patient or from a cryopreserved and thawed sample) or from a cancer cell line that has been labelled with cell tracker dye. In embodiments, without wishing to be bound by theory, addition of the labelled cancer cells with the generated CAR-T cells can reactivate the CAR-T cells and can induce proliferation, and accordingly, trogocytosis can be measured by detecting the signal emitted from the cell tracker dye.

Also, provided herein are methods of selecting the most effective CAR-T cells, e.g., trogocytotic T cells, e.g., for a specific patient.

In embodiments, the methods described herein comprise evaluating the CAR-T cell or preparation thereof for its likelihood to be efficacious *in vivo,* e.g., as an adoptive cell therapy. In embodiments, the methods comprise determining one or more of the following parameters: increased cancer cell killing activity, reduced toxicity, reduced off-target effect, increased viability, or increased proliferation. Without wishing to be bound by theory, it is believed that CAR-T cells (or preparations thereof) that have increased cancer cell killing activity, reduced toxicity, reduced off-target effect, increased viability, and/or increased proliferation are more likely to be efficacious *in vivo,* e.g., as an adoptive cell therapy.

The reduced toxicity of the CAR-T cell or preparation thereof are cells which kill significantly less non-pathological cells, i.e. they kill more selectively. This can be measured by labeling non-pathological cells and showing more selective cancer cell killing when compared to a reference, wherein said reference can be either different patient samples for the same cancer type, or different cell subsets (e.g. clones) within the same patient sample (e.g. trogocytotic).

The most common toxicity observed in cellular therapies is called Cytokine Storm, also known as Cytokine-Release Syndrome, cytokine cascade and hypercytokinemia. It is a potentially fatal immune reaction that arises when the cytokines released by BiTE-activated T cells or CAR-T cells in the process of killing by cell lysis cancer cells are released outside the cells, resulting in highly elevated levels of various cytokines. In embodiments, the BiTE-activated T cells or CAR-T cell or preparation thereof comprises cells having reduced toxicity because they generate less cytokines in the supernatant and/or intracellularly. In embodiments, the BiTE-activated T cells or CAR-T cell or preparation thereof comprises cells having both and simultaneously higher cancer-killing activity and reduced toxicity, because they generate less cytokines in the supernatant and/or intracellularly per unit of cancer cell killing, that is once the types and/or levels of cytokines released is normalized by the quantitative estimation of cancer cell killing activity such as Effective E:T Ratios, basal E:T ratios, EC50, Emax, kinetics, or a combination of these factors.

In embodiments, methods described herein further comprise determining the cancer-killing activity of a CAR-T cell (e.g., selected, enriched, purified, and/or expanded) CAR-T cell or preparation thereof. Cancer-killing activity can be determined by methods such as those comprising the following:
(a) contacting the CAR-T cells (or preparation thereof) with target cells that are derived from a cancer under conditions (e.g., for a period of time) sufficient to allow the CAR-T cells to kill the target cells; and
(b) determining the number of target cells after step (a).

In embodiments, a decrease in the number of target cells after the contacting step compared to the number of target cells before the contacting step indicates that the CAR-T cells are effective in killing cancer cells.

In embodiments, the activity of the CAR-T cells is tested against cancer cells from the same patient as those from which the T cells were isolated. In a further embodiment, the activity of the CAR-T cells is tested against cancer cells from a different patient (i.e., patient other than the one from which the T cells were isolated), e.g., that has the same type of cancer as the patient from which the T cells were isolated. In embodiments, the activity of the CAR-T cells is tested against cells lines derived from the same type of cancer as that in the patient from which the T cells were isolated.

In embodiments, the method can further comprise determining the number of CAR-T cells after step (a). In embodiments, an increase in the number of CAR-T cells compared to the number of CAR-T cells before the contacting step indicates that the CAR-T cells have increased viability and/or proliferation and may be more effective in killing cancer cells.

In embodiments, the evaluation and/or determination steps can be performed before and/or after a selection, enrichment, purification, or expansion step described herein. In embodiments, the CAR-T cells (or preparations thereof) that are determined to be more likely to be efficacious *in vivo* are expanded. In embodiments, additional expansion of the CAR-T cells can be achieved by contacting the CAR-T cells with cancer cells, e.g. cancer-derived cell lines or primary cancer samples. In one embodiment, a low, or insufficient, number of cancer cells are added to the CAR-T cells as described herein. In one embodiment, the cancer cells are added to the CAR-T cells one or more times, e.g., several times, e.g., sequentially. In one embodiment, each addition of the cancer cells, e.g., a low number of cancer cells, is performed when all or some portion of the cancer cells used in that contacting step are eliminated, e.g., killed. In embodiments, without wishing to be bound by theory, each addition of the cancer cells induces further expansion of the CAR-T cells and/or selective expansion of a particular CAR-T cell clone.

In some embodiments, CAR-T cells described herein (or preparations thereof), e.g., produced by methods described herein, contain more than one clone of a T cell. In embodiments, some clones may have higher cancer-killing activity than others.

In embodiments, clones of CAR-T cells containing the greatest activity (e.g., in killing cancer cells) are selected. In embodiments, clones can be separated by using limiting dilution or flow cytometry methods, e.g., to separate single cells from each other, e.g., plated into separate wells. In embodiments, the single cells are expanded to produce populations of each clone. Each clone can be evaluated for its likelihood to be efficacious *in vivo,* e.g., by determining cancer-killing activity and optionally, other parameters described herein. In embodiments, those clones exhibiting highest cancer-killing activity are further expanded and/or stored. In embodiments, the most active CAR-T cell clones are selected by adding a low, e.g., insufficient, number of cancer cells, e.g., from the same patient or a cancer cell line, sequentially, e.g., each time the existing cancer cells are eliminated. In embodiments, the most active T cell clone is among the first T cells that recognize, e.g., bind, and eliminate the few cancer cells, there by preferentially inducing the proliferation of the most active T cell clone. Accordingly, without wishing to be bound by theory, the aforementioned method is believed to enrich for the most active CAR-T cells of the T cell pool over time. Optionally, multiple clones exhibiting high cancer-killing activity are pooled together and, e.g., further expanded and/or stored.

In embodiments, CAR-T cells described herein (or preparations thereof) contain a single clone of a T cell.

In embodiments, CAR-T cells described herein are prepared according to Good Manufacturing Practice (GMP). For example, the *ex vivo* reaction mixture described herein, e.g., used in the production of CAR-T cells, is prepared according Good Manufacturing Practice (GMP). In embodiments, the CAR-T cells are prepared using an automated flow cytometry platform embedded in a GMP system or facility. In embodiments, the T cell, the cancer cell, or both, used in the *ex vivo* reaction mixture, are obtained from a hospital or a health care provider. In embodiments, the expansion, selection, purification, and/or enrichment of the CAR-T cells is performed according to Good Manufacturing Practice (GMP). In embodiments, methods described herein further comprise sending the CAR-T cell (e.g., produced by GMP) to a hospital or a health care provider.

### Pharmaceutical Compositions and Methods of Treatment

Provided herein is a composition comprising a CAR-T cell or CAR-T cell preparation thereof obtainable according to the method of producing a CAR-T cell.

In embodiments, the CAR-T cell: (i) has cytotoxic activity toward a cancer cell, and (ii) comprises at least 100 copies of the cancer cell surface marker; and comprises a detectable amount of a bispecific T cell engager antibody (BiTE).

In embodiments, the CAR-T cell is a cytotoxic T lymphocyte or a helper T cell selected from a CD8+ T cell or a CD4+ T cell.

In embodiments, the composition comprises cancer cells at a concentration of less than 30% the total number of cells in the composition or preparation, and comprises Tregs at a concentration of less than 30% of the total number of cells in the composition or preparation, and comprises naive T cells at a concentration of less than 30% of the total number of cells in the composition or preparation, and comprises red blood cells at a concentration of less than 30% of the total number of cells in the composition or preparation, and/or comprises non-immune cells at a concentration of less than 30% of the total number of cells in the composition or preparation.

In embodiments, the composition comprises CAR-T cells at a concentration of at least 30% of the total number of cells in the composition or preparation.

Also provided herein is a pharmaceutical composition comprising the composition and a pharmaceutically acceptable carrier.

Further provided herein is a pharmaceutical composition for use in Adoptive Cancer Therapy for treating a subject, wherein the subject is the same subject as that of step (a) and/or wherein the subject is the same subject as that of step (b) and/or wherein the subject is different from the subject as that as step (a) or (b).

In embodiments, the pharmaceutical composition is for use in Adoptive Cancer Therapy for treating a subject suffering (i) an hematological cancer selected from: Hodgkin's lymphoma, Non-Hodgkin's lymphoma (B cell lymphoma, diffuse large B cell lymphoma, follicular lymphoma, mantle cell lymphoma, marginal zone B-cell lymphoma, Burkitt lymphoma, lymphoplasmacytic lymphoma, hairy cell leukemia), acute myeloid leukemia, chronic myeloid leukemia, myelodysplastic syndrome, multiple myeloma, chronic lymphocytic leukemia or acute lymphocytic leukemia, or (ii) a solid cancer selected from: ovarian cancer, rectal cancer, stomach cancer, testicular cancer, cancer of the anal region, uterine cancer, colon cancer, rectal cancer, renal-cell carcinoma, liver cancer, non-small cell carcinoma of the lung, cancer of the small intestine, cancer of the esophagus, melanoma, Kaposi's sarcoma, cancer of the endocrine system, cancer of the thyroid gland, cancer of the parathyroid gland, cancer of the adrenal gland, bone cancer, pancreatic cancer, skin cancer, cancer of the head or neck, cutaneous or intraocular malignant melanoma, uterine cancer, brain stem glioma, pituitary adenoma, epidermoid cancer, carcinoma of the cervix squamous cell cancer, carcinoma of the fallopian tubes, carcinoma of the endometrium, carcinoma of the vagina, sarcoma of soft tissue, cancer of the urethra, carcinoma of the vulva, cancer of the penis, cancer of the bladder, cancer of the kidney or ureter, carcinoma of the renal pelvis, spinal axis tumor, neoplasm of the central nervous system (CNS), primary CNS lymphoma, tumor angiogenesis, metastatic lesions of said cancers, or combinations thereof.

Also provided herein is a method for treating a subject having cancer comprising providing a CAR-T cell or a CAR-T cell preparation thereof obtainable according to the method of producing a CAR-T cell or the composition, and administering an effective amount of the CAR-T cell, the CAR-T cell preparation or composition to the subject.

In embodiments, the method comprises:
(a) providing a sample from the subject, wherein the sample comprises a T cell and a cancer cell;
(b) contacting the sample *ex vivo* with a bispecific T cell engager antibody (BiTE) for a period of time;
(c) selecting the activated T cell; and
(d) genetically engineering the activated T cell to produce Chimeric Antigen Receptors (CAR) on the surface of the activated T cell, thereby producing at least one CAR-T cell; and
(e) administering an effective amount of the CAR-T cells to the subject.

In an embodiment, selecting the activated T cell in step (c) comprises
(a) isolating or enriching the trogocytotic T cell using a fluorescently labeled molecule (e.g., antibody or fragment thereof, or a cell tracker dye) that binds to i) one or more cancer antigens, or diffuses into the cancer cell membrane or ii) one or more markers of activated T cells, or both i) and ii); and
(b) genetically engineering the trogocytotic activated T cells to produce Chimeric Antigen Receptors (CAR) on the surface of the activated T cell, thereby producing at least one CAR-T cell.

In an embodiment, the selecting and/or enriching step (a) comprises using fluorescence activated cell sorting (FACS). In another embodiment, the selecting and/or enriching step (a) comprises using a bead (e.g., magnetic bead) coated with an antibody or fragment thereof that binds to i) one or more cancer antigens or ii) one or more markers of activated T cells, or both i) and ii). In another embodiment, the cancer-killing T cell preparation is enriched or purified and comprises trogocytotic cancer-killing T cells, e.g., at a concentration of at least 50% (e.g., at least 50%, 55%, 60%, 65%, 70%, 75%, 80%, 85%, 90%, 95%, 99%, or greater) of the total number of cells in the preparation.

In embodiments, the method further comprises administering a second therapeutic agent or procedure.

In embodiments, the second therapeutic agent or procedure is chosen from one or more of: chemotherapy, a targeted anti-cancer therapy, an oncolytic drug, a cytotoxic agent, an immune-based therapy, a cytokine, a surgical procedure, a radiation procedure, an agonist of T cells (agonistic antibody or fragment thereof or an activator of a costimulatory molecule), an inhibitor of an inhibitory molecule (immune checkpoint inhibitor), an immunomodulatory agent, a vaccine, or a cellular immunotherapy.

Pharmaceutical compositions disclosed herein can comprise a CAR-T cell, includes activated tumor antigen-specific T cells, including, but not limited to, effector memory T cells, cytotoxic T lymphocytes (CTLs), helper T cells, tumor infiltrating lymphocytes (TILs) and trogocytotic T cells or preparation thereof, as described herein, in combination with one or more physiologically or pharmaceutically acceptable carriers, diluents, or excipients. For example, the pharmaceutical composition can comprise buffers (e.g., neutral buffered saline, phosphate buffered saline; polypeptides/amino acids (e.g., glycine); anticoagulants (e.g. Heparin); proteins; antioxidants; carbohydrates (e.g., glucose, mannose, sucrose or dextran, mannitol); adjuvants (e.g., aluminium hydroxide); chelating agents (e.g., EDTA or glutathione); and/or preservatives. In embodiments, the pharmaceutical composition is substantially free of a contaminant, such as mycoplasma, endotoxin, lentivirus or components thereof, magnetic beads, bacteria, fungi, bovine serum albumin, bovine serum, and/or plasmid or vector components. In embodiments, the pharmaceutical composition comprises CAR-T cells that are prepared according to Good Manufacturing Practice (GMP).

In embodiments, the pharmaceutical composition is a purified preparation. For example, the pharmaceutical composition is substantially free of, e.g., there are no detectable levels of a contaminant. In embodiments, the contaminant comprises endotoxin, mycoplasma, p24, VSV-G nucleic acid, HIV gag, replication competent lentivirus (RCL), residual BiTE, antibodies, bovine serum albumin, bovine serum, pooled human serum, culture media components, vector packaging cell or plasmid components, a bacterium or fungus. In one embodiment, the bacterium is at least one selected from the group consisting of *Alcaligenes faecalis, Candida albicans*, *Escherichia coli*, *Haemophilus influenza*, *Neisseria meningitides*, *Pseudomonas aeruginosa*, *Staphylococcus aureus*, *Streptococcus pneumonia*, and *Streptococcus pyogenes* group A.

In certain embodiments, the pharmaceutical composition comprises a detectable (e.g., trace) amount of a bispecific T cell engager antibody (BiTE), e.g., a bispecific T cell engager antibody (BiTE) described herein. In embodiments, the BiTE is present at a concentration of less than 10% by weight, e.g., less than 10%, 9%, 8%, 7%, 6%, 5%, 4%, 3%, 2%, 1%, 0.5%, 0.1%, 0.05%, 0.01%, or less by weight (e.g., but no less than 0.0001% by weight).

In certain embodiments, the pharmaceutical composition comprises a detectable (e.g., trace) amount of a cell surface label, e.g., a fluorescent cell surface label, such as an antibody cell surface label or a cell tracker dye as described herein. In embodiments, the cell surface label, e.g., fluorescent cell surface label, is present at a concentration of less than 10% by weight, e.g., less than 10%, 9%, 8%, 7%, 6%, 5%, 4%, 3%, 2%, 1%, 0.5%, 0.1%, 0.05%, 0.01%, or less by weight (e.g., but no less than 0.0001% by weight).

In embodiments, the pharmaceutical composition comprises a CAR-T cell (or preparation thereof) prepared using a method described herein.

Methods described herein include treating a cancer in a subject by using a CAR-T cell (or preparation thereof) described herein, e.g., using a pharmaceutical composition described herein. Also provided are methods for reducing or ameliorating a symptom of a cancer in a subject as well as methods for inhibiting the growth of a cancer and/or killing one or more cancer cells. In embodiments, the methods described herein decrease the size of a tumor and/or decrease the number of cancer cells in a subject administered with a CAR-T cell described herein or a pharmaceutical composition described herein.

In embodiments, the cancer is a hematological cancer. In embodiments, the hematological cancer is a leukemia or a lymphoma. Exemplary hematological cancers include but are not limited to a Hodgkin's lymphoma, Non-Hodgkin's lymphoma (e.g., B cell lymphoma, diffuse large B cell lymphoma, follicular lymphoma, chronic lymphocytic leukemia, mantle cell lymphoma, marginal zone B-cell lymphoma, Burkitt lymphoma, lymphoplasmacytic lymphoma, hairy cell leukemia), acute myeloid leukemia, chronic myeloid leukemia, myelodysplastic syndrome, multiple myeloma, or acute lymphocytic leukemia. In embodiments, the cancer is other than acute myeloid leukemia (AML).

In embodiments, the cancer is a solid cancer. Exemplary solid cancers include but are not limited to ovarian cancer, rectal cancer, stomach cancer, testicular cancer, cancer of the anal region, uterine cancer, colon cancer, rectal cancer, renal-cell carcinoma, liver cancer, non-small cell carcinoma of the lung, cancer of the small intestine, cancer of the esophagus, melanoma, Kaposi's sarcoma, cancer of the endocrine system, cancer of the thyroid gland, cancer of the parathyroid gland, cancer of the adrenal gland, bone cancer, pancreatic cancer, skin cancer, cancer of the head or neck, cutaneous or intraocular malignant melanoma, uterine cancer, brain stem glioma, pituitary adenoma, epidermoid cancer, carcinoma of the cervix squamous cell cancer, carcinoma of the fallopian tubes, carcinoma of the endometrium, carcinoma of the vagina, sarcoma of soft tissue, cancer of the urethra, carcinoma of the vulva, cancer of the penis, cancer of the bladder, cancer of the kidney or ureter, carcinoma of the renal pelvis, spinal axis tumor, neoplasm of the central nervous system (CNS), primary CNS lymphoma, tumor angiogenesis, metastatic lesions of said cancers, or combinations thereof.

In embodiments, the cancer is not a melanoma.

In embodiments, the subject to be treated by CAR-T cells is the same as the subject from which T cells and/or cancer cells were isolated for the production of the CAR-T cells. In embodiments, the subject to be treated by CAR-T cells is different from the subject from which T cells and/or cancer cells were isolated for the production of the CAR-T cells. In embodiments, both subjects have or have had the same type of cancer.

In embodiments, the CAR-T cells (or pharmaceutical composition) are administered in a manner appropriate to the disease to be treated or prevented. The quantity and frequency of administration will be determined by such factors as the condition of the patient, and the type and severity of the patient's disease. Appropriate dosages may be determined by clinical trials. For example, when "an effective amount" or "a therapeutic amount" is indicated, the precise amount of the pharmaceutical composition (or CAR-T cells) to be administered can be determined by a physician with consideration of individual differences in tumor size, extent of infection or metastasis, age, weight, and condition of the subject. In embodiments, the pharmaceutical composition described herein can be administered at a dosage of 10⁴ to 10⁹cells/kg body weight, e.g., 10⁵ to 10⁶ cells/kg body weight, including all integer values within those ranges. In embodiments, the pharmaceutical composition described herein can be administered multiple times at these dosages. In embodiments, the pharmaceutical composition described herein can be administered using infusion techniques described in immunotherapy (see, *e*.*g*., Rosenberg SA (1988)).

In embodiments, the CAR-T cells (or preparations thereof) or pharmaceutical composition is administered to the subject parenterally. In embodiments, the cells are administered to the subject intravenously, subcutaneously, intratumorally, intranodally, intramuscularly, intradermally, or intraperitoneally. In embodiments, the cells are administered, e.g., injected, directly into a tumor or lymph node. In embodiments, the cells are administered as an infusion (e.g., as described in Rosenberg SA (1988)) or an intravenous push. In embodiments, the cells are administered as an injectable depot formulation.

In embodiments, a single dose of CAR-T cells (or pharmaceutical composition) is administered to a subject. In embodiments, multiple doses of CAR-T cells are administered to a subject. In embodiments, the time period between each dose is at least 12 hours, e.g., at least 12, 24, 36, 48, 72, 96 h or more, or at least 1, 2, 3, 4, 5, 6, 7 days or more, or at least 1, 2, 3, 4 weeks or more.

In embodiments, a single dose comprises 10³ to 10¹¹ CAR-T cells (e.g., 10³ to 10⁴, 10⁴ to 10⁵, 10⁵ to 10⁶, 10⁶ to 10⁷, 10⁷ to 10⁸, 10⁸ to 10⁹, 10⁹ to 10¹⁰, or 10¹⁰ to 10¹¹ CAR-T cells).

In embodiments, each dose of a multiple dose regimen comprises 10³ to 10¹¹ CAR-T cells (e.g., 10³ to 10⁴, 10⁴ to 10⁵, 10⁵ to 10⁶,10⁶ to 10⁷, 10⁷ to 10⁸, 10⁸ to 10⁹, 10⁹ to 10¹⁰, or 10¹⁰ to 10¹¹ CAR-T cells).

In embodiments, the CAR-T cells or preparations thereof (or pharmaceutical composition) decrease the number of or percentage of cancer cells in a subject, e.g., by at least 25%, at least 30%, at least 40%, at least 50%, at least 65%, at least 75%, at least 85%, at least 95%, or at least 99% relative to a negative control.

In embodiments, the subject is a mammal. In embodiments, the subject is a human, monkey, pig, dog, cat, cow, sheep, goat, rabbit, rat, or mouse. In embodiments, the subject is a human. In embodiments, the subject is a pediatric subject, e.g., less than 18 years of age, e.g., less than 17, 16, 15, 14, 13, 12, 11, 10, 9, 8, 7, 6, 5, 4, 3, 2, 1 or less years of age. In embodiments, the subject is an adult, e.g., at least 18 years of age, e.g., at least 19, 20, 21, 22, 23, 24, 25, 25-30, 30-35, 35-40, 40-50, 50-60, 60-70, 70-80, or 80-90 years of age.

### Combination Therapies

In accordance with a method described herein, in some embodiments, an effector T cell described herein, e.g., effector T cell population described herein (e.g., trogocytotic T cell) can be used in combination with a second therapeutic agent or procedure.

In embodiments, the effector T cell and the second therapeutic agent or procedure are administered/performed after a subject has been diagnosed with a cancer, e.g., before the cancer has been eliminated from the subject. In embodiments, the effector T cell and the second therapeutic agent or procedure are administered/performed simultaneously or concurrently. For example, the delivery of one treatment is still occurring when the delivery of the second commences, e.g., there is an overlap in administration of the treatments. In other embodiments, the effector T cell and the second therapeutic agent or procedure are administered/performed sequentially. For example, the delivery of one treatment ceases before the delivery of the other treatment begins.

In embodiments, combination therapy leads to more effective treatment, e.g., more effective killing of cancer cells. In embodiments, the combination of the first and second treatment is more effective (e.g., leads to a greater reduction in symptoms and/or cancer cells) than the first or second treatment alone. In embodiments, the combination therapy permits use of a lower dose of the first or the second treatment compared to the dose of the first or second treatment normally required to achieve similar effects when administered as a monotherapy. In embodiments, the combination therapy has a partially additive effect, wholly additive effect, or greater than additive effect.

In embodiments, the second therapeutic agent or procedure includes a therapy described in the "Other methods of enhancing T cell activity" section herein. In embodiments, the second therapeutic agent includes an immune checkpoint inhibitor (e.g., an immune checkpoint inhibitor described herein), an agonist of a T cell (e.g., an agonist of a T cell described herein), and/or another immunomodulatory drug (e.g., lenalidomide) as described herein.

### Method of identifying subjects susceptible to immune checkpoint immunotherapy treatment

Provided herein is an *in vitro* method of identifying subjects susceptible to immune checkpoint immunotherapy treatment, comprising:
(a) providing a sample comprising at least one T cell from a subject having a cancer;
(b) providing a sample comprising at least one cancer cell, e.g., from the subject;
(c) forming an *ex vivo* reaction mixture comprising the at least one T cell, the at least one cancer cell, and a bispecific T cell engager antibody (BiTE), under conditions (e.g., for a period of time) sufficient to allow the T cell to kill cancer cells, thereby producing the cancer-killing T cell;
(d) determining the pharmacological activity of the cancer-killing T cells obtained in step (c) by dose response and/or pharmacodynamic parameters of cancer-killing T cells and tumor cells, selected from EC50, Emax, AUC, Effective E:T Ratios, Basal E:T Ratios, or kinetic parameters;
(e) determining the pharmacological activity of the cancer-killing T cells repeating steps (c) and (d) by dose response or evaluating a single high saturating dose in combination with immune check point inhibitors, individually, or in combinations, or bispecific or multispecific antibody constructs combining immune checkpoint inhibitors, including the combination of all immune checkpoint inhibitors;
(f) determining the expression levels of immune checkpoint molecules in both the tumor cells and T cells in the reaction mixture of step (c), comparing basal levels with levels after incubation;
(g) identifying subjects susceptible to immune checkpoint immunotherapy treatment, whereby the bispecific T cell engager antibody (BiTE) incubation is only a reagent to activate T cells, by assessment of either of the following 2 criteria or a combination of them:
   i. step (d) reveals a resistant tumor cell population in the samples from the subject (i.e. incubation with the bispecific T cell engager antibody (BiTE) does not kill all tumor cells), and addition of one or more immune checkpoint inhibitors in (e) reverts resistance of tumor cell population;
   ii. step (f) reveals an increase in the expression level of an immune checkpoint molecule in either the tumor cells and/or T cells in the reaction mixture of step (c) after incubation, relative to basal levels prior incubation,
and wherein observance of both (i) and (ii) is indicative of a subject more susceptible to immune checkpoint immunotherapy treatment.

Provided herein is an *in vitro* method of identifying subjects susceptible to immune checkpoint immunotherapy treatment, comprising:
(a) providing a sample comprising at least one T cell from a subject having a cancer;
(b) providing a sample comprising at least one cancer cell, e.g., from the subject;
(c) forming an *ex vivo* reaction mixture comprising the at least one T cell, the at least one cancer cell, and a bispecific T cell engager antibody (BiTE), under conditions (e.g., for a period of time) sufficient to allow the T cell to kill cancer cells, thereby producing the cancer-killing T cell
(d) Isolating the activated T cells, by FACS or magnetic-beads or other methods, adding them to a cancer cell, e.g., from the subject, forming an *ex vivo* reaction mixture comprising under conditions (e.g., for a period of time) sufficient to allow the activated T cells to kill cancer cells; and;
(e) determining the pharmacological activity of the cancer-killing T cells obtained in step (d) by dose response and/or pharmacodynamic parameters of cancer-killing T cells and tumor cells, selected from EC50, Emax, AUC, Effective E:T Ratios, Basal E:T Ratios, or kinetic parameters and;
(f) determining the pharmacological activity of the cancer-killing T cells repeating steps (d) and (e) by dose response or evaluating a single high saturating dose in combination with immune check point inhibitors, individually, or in combinations, or bispecific or multispecific antibody constructs combining immune checkpoint inhibitors, including the combination of all immune checkpoint inhibitors;
(g) determining the expression levels of immune checkpoint molecules in both the tumor cells and T cells in the reaction mixture of step (d), comparing basal levels with levels after incubation;
(h) identifying subjects susceptible to immune checkpoint immunotherapy treatment, whereby the bispecific T cell engager antibody (BiTE) incubation is only a reagent to activate T cells, by assessment of either of the following 2 criteria or a combination of them:
   i. step (e) reveals a resistant tumor cell population in the samples from the subject (i.e. incubation with BiTE-activated isolated T cells does not kill all tumor cells), and addition of one or more immune checkpoint inhibitors in (f) reverts resistance of tumor cell population;
   ii. step (g) reveals an increase in the expression level of an immune checkpoint molecule in either the tumor cells and/or T cells in the reaction mixture of step (d) after incubation, relative to basal levels prior incubation,
and wherein observance of both (i) and (ii) is indicative of a subject more susceptible to immune checkpoint immunotherapy treatment.

Provided herein is an *in vitro* method of identifying subjects susceptible to immune checkpoint immunotherapy treatment to be combined with a bispecific T cell engager antibody (BiTE) immunotherapy, for decreasing resistance of said subject to said BiTE immunotherapy, comprising:
(a) providing a sample comprising at least one T cell from a subject having a cancer;
(b) providing a sample comprising at least one cancer cell, e.g., from the subject;
(c) forming an *ex vivo* reaction mixture comprising the at least one T cell, the at least one cancer cell, and the the bispecific T cell engager antibody (BiTE), being identical to BiTE of the immunotherapy, e.g., under conditions (e.g., for a period of time) sufficient to allow the T cell to kill cancer cells, thereby producing the cancer-killing T cell;
(d) determining the pharmacological activity of the cancer-killing T cells obtained in step (c) by dose response and/or pharmacodynamic parameters of cancer-killing T cells and tumor cells, selected from EC50, Emax, AUC, Effective E:T Ratios, Basal E:T Ratios, or kinetic parameters;
(e) determining the pharmacological activity of cancer-killing T cells obtained in step (c) by by dose response or evaluating a single high saturating dose in combination with immune check point inhibitors, individually, or in combinations, or bispecific or multispecific antibody constructs combining immune check point inhibitors, including the combination of all immune check point inhibitors;
(f) determining the expression levels of immune checkpoint molecules in both the tumor cells and T cells in the reaction mixture of step (c), comparing basal levels with levels after incubation,
(g) identifying subjects susceptible to immune checkpoint immunotherapy treatment to be combined with a bispecific T cell engager antibody (BiTE) immunotherapy, by assessment of either of the following 2 criteria or a combination of them:
   i. step (d) reveals a resistant tumor cell population in the samples from the subject (i.e. incubation with the bispecific T cell engager antibody (BiTE) does not kill all tumor cells), and addition of one or more immune checkpoint inhibitors in (e) reverts resistance of tumor cell population;
   ii. step (f) reveals an increase in the expression level of an immune checkpoint molecule in either the tumor cells and/or T cells in the reaction mixture of step (c) after incubation, relative to basal levels prior incubation;
and wherein observance of both (i) and (ii) is indicative of a subject more susceptible to immune checkpoint immunotherapy treatment to be combined with a bispecific T cell engager antibody (BiTE) immunotherapy.

Provided herein is an *in vitro* method of identifying subjects susceptible to immune checkpoint immunotherapy treatment to be combined with a bispecific T cell engager antibody (BiTE) immunotherapy, for decreasing resistance of said subject to said BiTE immunotherapy, comprising:
(a) providing a sample comprising at least one T cell from a subject having a cancer;
(b) providing a sample comprising at least one cancer cell, e.g., from the subject;
(c) forming an *ex vivo* reaction mixture comprising the at least one T cell, the at least one cancer cell, and the bispecific T cell engager antibody (BiTE), being identical to the BiTE of the immunotherapy, e.g., under conditions (e.g., for a period of time) sufficient to allow the T cell to kill cancer cells, thereby producing the cancer-killing T cell;
(d) Isolating the activated T cells, by FACS or magnetic-beads or other methods, adding them to a cancer cell, e.g., from the subject, forming an *ex vivo* reaction mixture comprising under conditions (e.g., for a period of time) sufficient to allow the activated T cells to kill cancer cells; and;
(e) determining the pharmacological activity of the cancer-killing T cells obtained in step (d) by dose response and/or pharmacodynamic parameters of cancer-killing T cells and tumor cells, selected from EC50, Emax, AUC, Effective E:T Ratios, Basal E:T Ratios, or kinetic parameters and;
(f) determining the pharmacological activity of the cancer-killing T cells repeating steps (d) and (e) by dose response or evaluating a single high saturating dose in combination with immune check point inhibitors, individually, or in combinations, or bispecific or multispecific antibody constructs combining immune checkpoint inhibitors, including the combination of all immune checkpoint inhibitors;
(g) determining the expression levels of immune checkpoint molecules in both the tumor cells and T cells in the reaction mixture of step (d), comparing basal levels with levels after incubation;
(h) identifying subjects susceptible to immune checkpoint immunotherapy treatment, in combination with the BiTE, by assessment of either of the following 2 criteria or a combination of them:
   i. step (e) reveals a resistant tumor cell population in the samples from the subject (i.e. incubation with BiTE-activated isolated T cells does not kill all tumor cells), and addition of one or more immune checkpoint inhibitors in (f) reverts resistance of tumor cell population;
   ii. step (g) reveals an increase in the expression level of an immune checkpoint molecule in either the tumor cells and/or T cells in the reaction mixture of step (d) after incubation, relative to basal levels prior incubation,
and wherein observance of both (i) and (ii) is indicative of a subject more susceptible to immune checkpoint immunotherapy treatment for decreasing resistance of said subject to said BiTE immunotherapy.

Provided herein is an *in vitro* method of identifying subjects susceptible to immune checkpoint immunotherapy treatment to be combined with a cellular immunotherapy such a CAR-T to treat a subject, for decreasing resistance of said subject to said cellular immunotherapy, comprising:
(a) providing a sample comprising at least one T cell selected from the group consisting of a tumor infiltrated lymphocyte (TIL), marrow infiltrated lymphocyte (MIL), a genetically engineered T cell, a CAR-T cell, or an activated T cell obtainable according to step (c) of the method of claim 1 or claim 2, or step (d) of the method of claim 3 and a genetically engineered T cell expressing Chimeric Antigen Receptors obtainable according to step (e) of the method of claim 1, step (f) of the method of claim 2, or step (g) of the method of claim 3, from a subject having a cancer;
(b) providing a cancer cell, e.g., from the subject;
(c) forming an *ex vivo* reaction mixture comprising (a) and (b), under conditions (e.g., for a period of time) sufficient to allow the T cells to kill cancer cells, thereby producing the cancer-killing T cell; and
(d) determining the pharmacological activity of cancer-killing T cells obtained in step (c) by dose response and/or pharmacodynamic parameters of cancer-killing T cells and tumor cells, selected from EC50, Emax, AUC, Effective E:T Ratios, Basal E:T Ratios, or kinetic parameters;
(e) determining the pharmacological activity of cancer-killing T cells obtained in step (c) by dose response or evaluating a single high saturating dose in combination with immune check point inhibitors, individually, or in combinations, or bispecific or multispecific antibody constructs combining immune check point inhibitors, including the combination of all immune check point inhibitors, either by full dose responses or evaluating a single high saturating dose.
(f) determining the expression levels of immune checkpoint molecules in both the tumor cells and T cells in the reaction mixture of step (c), comparing basal levels with levels after incubation,
(g) identifying subjects susceptible to immune checkpoint immunotherapy treatment in combination with the cellular therapy, by assessment of either of the following 2 criteria or a combination of them:
   i. step (d) reveals a resistant tumor cell population in the samples from the subject (i.e. incubation with T cell therapy does not kill all tumor cells), and addition of one or more immuno checkpoint inhibitors in (e) reverts resistance of tumor cell population;
   ii. step (f) reveals an increase in the expression level of an immune checkpoint molecule in either the tumor cells and/or T cells in the reaction mixture of step (c) after incubation, relative to basal levels prior incubation,
and wherein observance of both (i) and (ii) is indicative of a subject more susceptible to immune checkpoint immunotherapy treatment to be combined with a cellular immunotherapy.

Also provided herein is an *in vitro* method of identifying subjects susceptible to immune checkpoint immunotherapy treatment to be combined with a cellular immunotherapy such a CAR-T to treat a subject, for decreasing resistance of said subject to said cellular immunotherapy, comprising:
(a) providing a sample comprising at least one T cell selected from the group consisting of a tumor infiltrated lymphocyte (TIL), marrow infiltrated lymphocyte (MIL), a genetically engineered T cell, a CAR-T cell, or an activated T cell obtainable according to step (c) of the method of claim 1 and a genetically engineered T cell expressing Chimeric Antigen Receptors obtainable according to step (e) of the method of claim 1 from a subject having a cancer;
(b) providing a cancer cell, e.g., from the subject;
(c) forming an *ex vivo* reaction mixture comprising (a) and (b), under conditions (e.g., for a period of time) sufficient to allow the T cells to kill cancer cells, thereby producing the cancer-killing T cell; and
(d) determining the pharmacological activity of cancer-killing T cells obtained in step (c) by dose response and/or pharmacodynamic parameters of cancer-killing T cells and tumor cells, selected from EC50, Emax, AUC, Effective E:T Ratios, or kinetic parameters;
(e) determining the pharmacological activity of cancer-killing T cells obtained in step (c) by dose response or evaluating a single high saturating dose in combination with immune check point inhibitors, individually, or in combinations, or bispecific or multispecific antibody constructs combining immune check point inhibitors, including the combination of all immune check point inhibitors, either by full dose responses or evaluating a single high saturating dose.
(f) determining the expression levels of immune checkpoint molecules in both the tumor cells and T cells in the reaction mixture of step (c), comparing basal levels with levels after incubation,
(g) identifying subjects susceptible to immune checkpoint immunotherapy treatment in combination with the cellular therapy, by assessment of either of the following 2 criteria or a combination of them:
   i. step (d) reveals a resistant tumor cell population in the samples from the subject (i.e. incubation with T cell therapy does not kill all tumor cells), and addition of an immuno checkpoint inhibitor in (e) reverts resistance of tumor cell population;
   ii. step (f) reveals an increase in the expression level of an immune checkpoint molecule in either the tumor cells and/or T cells in the reaction mixture of step (c) after incubation, relative to basal levels prior incubation,
and wherein observance of both (i) and (ii) is indicative of a subject more susceptible to immune checkpoint immunotherapy treatment to be combined with a cellular immunotherapy.

In an embodiment, the immune check point molecules are added either from the beginning of the incubation or sequentially after a certain amount of time sufficient for the T cells to become activated killing tumor cells.

In an embodiment, different incubation times are evaluated, and any single incubation time can be used to identify subjects susceptible to immune check point immunotherapy, alone or in combination with other drugs.

In embodiments, the immune checkpoint molecule is selected from the group consisting of PDL-1, PDL-2, B7-1 (CD80), B7-2 (CD86), 4-1BBL, Galectin, ICOSL, GITRL, OX40L, CD155, B7-H3, PD1, CTLA-4, 4-1BB, TIM-3, ICOS, GITR, LAG-3, KIR, OX40, TIGIT, CD160, 2B4, B7-H4 (VTCN1), HVEM (TNFRSF14 or CD270), BTLA, KIR, MHC class I, MHC class II, GAL9, VISTA, LAIR1, and A2aR

"PD-1" refers to programmed cell death protein 1, also known as CD279 (cluster of differentiation 279). Is a cell surface receptor that plays an important role in down-regulating the immune system and promoting self tolerance by suppressing T cell inflammatory activity. PD-1 is an immune checkpoint and guards against autoimmunity through a dual mechanism of promoting apoptosis (programmed cell death) in antigen specific T-cells in lymph nodes while simultaneously reducing apoptosis in regulatory T cells (anti-inflammatory, suppressive T cells).

"PDL-1" refers to programmed cell death-ligand 1, also known as cluster of differentiation 274 (CD274) or B7 homolog 1 (B7-H1). It is a transmembrane protein that play a major role in suppressing the immune system during particular events such as pregnancy, tissue allografts, autoimmune disease and other disease states such as hepatitis.

"PDL-2" refers to programmed cell death-ligand 2 (also known as B7-DC or CD273 (cluster of differentiation 273).

"B7-1" refers to cluster of differentiation 80 (also CD80) and is a protein found on dendritic cells, activated B cells and monocytes that provides a costimulatory signal necessary for T cell activation and survival. It is the ligand for two different proteins on the T cell surface: CD28 (for autoregulation and intercellular association) and CTLA-4 (for attenuation of regulation and cellular disassociation). CD80 works in tandem with CD86 to prime T cells.

"B7-2" refers to cluster of differentiation 86 (also known as CD86) and is a protein expressed on antigen-presenting cells that provides costimulatory signals necessary for T cell activation and survival. It is the ligand for two different proteins on the T cell surface: CD28 (for autoregulation and intercellular association) and CTLA-4 (for attenuation of regulation and cellular disassociation). CD86 works in tandem with CD80 to prime T cells.

"4-1BB" refers to a type 2 transmembrane glycoprotein belonging to the TNF superfamily, expressed on activated T Lymphocytes.

"4-1BBL" refers to 4-1BB ligand.

"ICOS" refers to Inducible T-cell costimulator. It is also known as CD278 and is a CD28-superfamily costimulatory molecule that is expressed on activated T cells. It is thought to be important for Th2 cells in particular.

"ICOSL" refers to ICOS ligand. It is a protein and it has also been designated as CD275 (cluster of differentiation 275).

"GITR" refers to glucocorticoid-induced TNFR-related protein, also known as tumor necrosis factor receptor superfamily member 18 (TNFRSF18) activation-inducible TNFR family receptor (AITR). GITR is currently of interest in immunotherapy as a co-stimulatory immune checkpoint molecule.

"GITRL" refers to GITR ligand.

"OX40" refers to tumor necrosis factor receptor superfamily, member 4 (TNFRSF4), also known as CD134. Is a member of the TNFR-superfamily of receptors which is not constitutively expressed on resting naive T cells, unlike CD28.

"OX40L" refers to OX40 ligand.

"B7-H3" refers to CD276 (cluster of differentiation 276).

"CTLA-4" refers to cytotoxic T-lymphocyte-associated protein 4, also known as CD152 (cluster of differentiation 152). Is a protein receptor that downregulates immune responses. Is constitutively expressed in regulatory T cells but only upregulated in conventional T cells after activation. It acts as an "off" switch when bound to CD80 or CD86 on the surface of antigen-presenting cells.

"TIM-3" refers to T-cell immunoglobulin and mucin-domain containing-3, also known as hepatitis A virus cellular receptor 2 (HAVCR2).

"LAG-3" refers to lymphocyte-activation gene 3. It is also known as CD223 (cluster of differentiation 223). It is a cell surface molecule with diverse biologic effects on T cell function.

In embodiments, the immune checkpoint molecule is PD-1.

In embodiments, the method is performed using an automated fluorescence based platform.

In embodiments, the method is performed using flow cytometry.

In embodiments, the bispecific T cell engager antibody (BiTE) has a first element providing affinity for the T cell and a second element having affinity for the cancer cell, wherein the first element binds to a T cell and does not bind to a substantial number of cancer cells and wherein the second element binds to a cancer cell and does not bind to a substantial number of T cells.

In embodiments, the first element binding to T cell comprises one or more of the following cell receptors: CD8, CD3, CD4, α/β T cell receptor (α/β TCR), CD45RO, and/or CD45RA.

In embodiments, the second element binds to one or more of the following cell receptors: CD20, CD28, CD30, CD33, CD52; EpCAM, CEA, gpA33, mucin, TAG-72, carbonic anhydrase IX, PSMA, folate binding protein; ganglioside selected from: GD2, GD3, or GM2; Lewis-Y2, VEGF, VEGFR, αVβ3, α5β1, ErbB1/EGFR, ErbB2/HER2, ERbB3, c-MET, IGF1R, EphA3, TRAIL-R1, TRAIL-R2, RANKL, FAP, tenascin, CD123, CD19, and/or BCMA.

In embodiments, the T cell engager antibody (BiTE) is selected from the group consisting of BsMAb CD19/CD3, BsMAb CD123/CD3, BsMAb CD3/CD28 and BsMAb EpCAM/CD3.

In embodiments, Chimeric Antigen Receptors recognize a neoantigen of a cancer cell.

In embodiments, the sample of step (a) and the sample of step (b) are from the same subject.

In embodiments, step (a) and step (b) comprise providing one sample comprising both the cancer cell and the T cell.

In embodiments, the sample (a) is selected from: whole blood, peripheral blood, bone marrow, lymph node, spleen, a primary tumor and a metastasis.

In embodiments, the sample (a) is derived from a tissue with a microenvironment, wherein substantially no components have been removed or isolated from the sample.

In embodiments, the subject is an adult or a pediatric subject.

In embodiments, the cancer of sample (b) is a hematological cancer selected from: Hodgkin's lymphoma, Non-Hodgkin's lymphoma (B cell lymphoma, diffuse large B cell lymphoma, follicular lymphoma, mantle cell lymphoma, marginal zone B-cell lymphoma, Burkitt lymphoma, lymphoplasmacytic lymphoma, hairy cell leukemia), acute myeloid leukemia, chronic myeloid leukemia, myelodysplastic syndrome, multiple myeloma, chronic lymphocytic leukemia or acute lymphocytic leukemia.

In embodiments, the cancer is a solid cancer selected from: ovarian cancer, rectal cancer, stomach cancer, testicular cancer, cancer of the anal region, uterine cancer, colon cancer, rectal cancer, renal-cell carcinoma, liver cancer, non-small cell carcinoma of the lung, cancer of the small intestine, cancer of the esophagus, melanoma, Kaposi's sarcoma, cancer of the endocrine system, cancer of the thyroid gland, cancer of the parathyroid gland, cancer of the adrenal gland, bone cancer, pancreatic cancer, skin cancer, cancer of the head or neck, cutaneous or intraocular malignant melanoma, uterine cancer, brain stem glioma, pituitary adenoma, epidermoid cancer, carcinoma of the cervix squamous cell cancer, carcinoma of the fallopian tubes, carcinoma of the endometrium, carcinoma of the vagina, sarcoma of soft tissue, cancer of the urethra, carcinoma of the vulva, cancer of the penis, cancer of the bladder, cancer of the kidney or ureter, carcinoma of the renal pelvis, spinal axis tumor, neoplasm of the central nervous system (CNS), primary CNS lymphoma, tumor angiogenesis, metastatic lesions of said cancers, or combinations thereof.

In embodiments, the cancer is not melanoma.

In embodiments, the subject providing sample (a) and/or sample (b):
(i) has not received a prior treatment for the cancer;
(ii) has received one or more previous treatments for the cancer; or
(iii) has minimal residual disease (MRD).

### Combination therapies with immune checkpoint inhibitors

Provided herein is a method for treating a subject having cancer comprising providing a bispecific T cell engager antibody (BiTE) or a T cell selected from the group consisting of a tumor infiltrated lymphocyte (TIL), a genetically engineered T cell, a CAR-T cell, an activated T cell obtainable according to the step (c) of the method of claim 1 and a genetically engineered T cell expressing Chimeric Antigen Receptors obtainable according to step (e) of the method of producing a CAR-T cell, in combination with an inhibitor of at least one immune checkpoint molecule selected in the method of identifying immune checkpoint molecules as target for decreasing resistance to a cancer therapy.

In embodiments, the inhibitor of at least one immune checkpoint molecule is selected from the group consisting of Nivolumab, Pembrolizumab and Pidilizumab.

In embodiments, the inhibitor of at least one immune checkpoint molecule is Nivolumab.

In embodiments, the method further comprises administering a third therapeutic agent or procedure.

In embodiments, the third therapeutic agent or procedure is chosen from one or more of: chemotherapy, a targeted anti-cancer therapy, an oncolytic drug, a cytotoxic agent, an immune-based therapy, a cytokine, a surgical procedure, a radiation procedure, an agonist of T cells (agonistic antibody or fragment thereof or an activator of a costimulatory molecule), an inhibitor of an inhibitory molecule (immune checkpoint inhibitor), an immunomodulatory agent, a vaccine, or a cellular immunotherapy.

### Method of evaluating susceptibility to Cytokine-Release Syndrome (CRS)

Provided herein is an *in vitro* method of evaluating susceptibility of a subject to develop Cytokine-Release Syndrome (CRS) to a bispecific T cell engager antibody (BiTE) immunotherapy treatment, comprising:
(a) providing a sample comprising at least one T cell from a subject having a cancer;
(b) providing a sample comprising at least one cancer cell, e.g., from the subject;
(c) forming an *ex vivo* reaction mixture comprising the at least one T cell, the at least one cancer cell, and the bispecific T cell engager antibody (BiTE), being identical to BiTE of the immunotherapy treatment, e.g., under conditions (e.g., for a period of time) sufficient to allow the T cell to kill cancer cells, thereby producing the cancer-killing T cell; and
(d) determining the pharmacological activity of the cancer-killing T cells obtained in step (c) by dose response and/or pharmacodynamic parameters of cancer-killing T cells and tumor cells, selected from EC50, Emax, AUC, Effective E:T Ratios, Basal E:T Ratios, or kinetic parameters;
(e) determining the expression levels of multiple cytokines in the *ex vivo* reaction mixture, in supernatant and/or intracellular compartments, at basal and several time points; and
(f) evaluating susceptibility of a subject to develop Cytokine-Release Syndrome, by analyzing the results of (e) cytokine levels as a function of (d) cancer-killing activity, wherein a high expression value of anti-inflammatory cytokines in the sample, relative to (i.e. as a function of) its relative cancer-killing activity compared with other patient samples, is indicative of less susceptibility to develop Cytokine-Release Syndrome or wherein a low expression value of pro-inflammatory cytokines in the sample, relative to (i.e. as a function of) its relative cancer-killing activity compared with other patient samples, is indicative of less susceptibility to develop Cytokine-Release Syndrome.

Also provided herein is an *in vitro* method of evaluating susceptibility of a subject to develop Cytokine-Release Syndrome (CRS) to a Cellular therapy such as a CAR-T therapy, comprising:
(a) providing a sample comprising at least one T cell selected from the group consisting of a tumor infiltrated lymphocyte (TIL), marrow infiltrated lymphocyte (MIL), a genetically engineered T cell, a CAR-T cell, or an activated T cell obtainable according to the methods of producing CAR-T cells and a genetically engineered T cell expressing Chimeric Antigen Receptors obtainable according to the methods of producing CAR-T cells;
(b) providing a sample comprising at least one cancer cell from a subject having a cancer;
(c) forming an *ex vivo* reaction mixture comprising the sample of step (a) and the sample of step (b); e.g., under conditions (e.g., for a period of time) sufficient to allow said T cells to kill cancer cells; and
(d) determining the pharmacological activity of the cancer-killing T cells obtained in step (c) by dose response and/or pharmacodynamic parameters of cancer-killing T cells and tumor cells, selected from EC50, Emax, AUC, E:T Ratios, , or kinetic parameters;
(e) determining the expression levels of multiple cytokines in the *ex vivo* reaction mixture, in supernatant and/or intracellular compartments, at basal and several time points; and
(f) evaluating susceptibility of a subject to develop Cytokine-Release Syndrome, by analyzing the results of (e) cytokine levels as a function of (d) cancer-killing activity, wherein a high expression value of anti-inflammatory cytokines in the sample, relative to (i.e. as a function of) its relative cancer-killing activity compared with other patient samples, is indicative of less susceptibility to develop Cytokine-Release Syndrome or wherein a low expression value of pro-inflammatory cytokines in the sample, relative to (i.e. as a function of) its relative cancer-killing activity compared with other patient samples, is indicative of less susceptibility to develop Cytokine-Release Syndrome.

In embodiments, the treatment evaluated for susceptibility of a subject to develop Cytokine-Release Syndrome (CRS) is a combination among BiTEs, Cellular Therapies, and other immunotherapies or other non-immuno therapies.

In embodiments, the cytokine is selected from the group consisting of IL-1a, IL1β, IL-1 Ra, IL-2, IL-3, IL-4, IL-5, IL6, IL-7, IL-8, IL-9, IL-10, IL-12, IL12p70, IL-13, IL-15, IL-16, IL-17A, IL-17F, IL-18, IL-22, IP10, IFN-y, TNF-α.

In embodiments, the pharmacological parameter is Area Under the Curve (AUC) and levels of cytokine for IL-10 and/or INF-□, and their relationship is non-linear enabling selection of subjects with high cancer cell killing activity and moderate cytokine release.

In embodiments, the pharmacological parameter is Area Under the Curve (AUC) and levels of cytokine for IL-10 and/or INF-□, and their relationship is non-linear enabling selection of lower doses for subjects predicted with high cancer cell killing activity and high cytokine release, whereby such lower doses decrease the probability of suffering Cytokine Release Symdrome.

In embodiments, the pharmacological parameter is high Effective E:T Ratio coinciding with high levels of cytokine IL-13, an anti-inflammatory cytokine, indicative of high cancer-killing activity and low probability of cytokine release syndrome.

In embodiments, sequential time measurements identify dependent processes, such as cytokines induced by other cytokines, or short time vs longer time cytokine level variations, where any of these parameters (e.g. shorter time cytokines) may have higher clinical prediction capacity.

In embodiments, the method is performed using an automated fluorescence-based platform.

In embodiments, the method is performed using flow cytometry.

In embodiments, the bispecific T cell engager antibody (BiTE) has a first element providing affinity for the T cell and a second element having affinity for the cancer cell, wherein the first element binds to a T cell and does not bind to a substantial number of cancer cells and wherein the second element binds to a cancer cell and does not bind to a substantial number of T cells.

In embodiments, the first element binding to T cell comprises one or more of the following cell receptors: CD8, CD3, CD4, α/β T cell receptor (TCR), CD45RO, and/or CD45RA.

In embodiments, the second element binds to one or more of the following cell receptors: CD20, CD28, CD30, CD33, CD52; EpCAM, CEA, gpA33, mucin, TAG-72, carbonic anhydrase IX, PSMA, folate binding protein; one or more of a ganglioside selected from: GD2, GD3, or GM2; Lewis-Y2, VEGF, VEGFR, αVβ3, α5β1, ErbB1/EGFR, ErbB2/HER2, ERbB3, c-MET, IGF1R, EphA3, TRAIL-R1, TRAIL-R2, RANKL, FAP, tenascin, CD123, CD19, and/or BCMA.

In embodiments, the T cell engager antibody (BiTE) is selected from the group consisting of BsMAb CD19/CD3, BsMAb CD123/CD3, CD3/CD28 and EpCAM/CD3.

In embodiments, Chimeric Antigen Receptors recognize a neoantigen of a cancer cell.

In embodiments, the sample of step (a) and the sample of step (b) are from the same subject.

In embodiments, step (a) and step (b) comprise providing one sample comprising both the cancer cell and the T cell.

In embodiments, the sample (a) is derived from a tissue with a microenvironment, wherein substantially no components have been removed or isolated from the sample, selected from: whole blood, peripheral blood, bone marrow, lymph node, a biopsy of a primary tumor, or a biopsy of a metastasis or spleen.

In embodiments, the subject is an adult or a pediatric subject.

In embodiments, the cancer of sample (b) is a hematological cancer selected from: Hodgkin's lymphoma, Non-Hodgkin's lymphoma (B cell lymphoma, diffuse large B cell lymphoma, follicular lymphoma, chronic lymphocytic leukemia, mantle cell lymphoma, marginal zone B-cell lymphoma, Burkitt lymphoma, lymphoplasmacytic lymphoma, hairy cell leukemia), acute myeloid leukemia, chronic myeloid leukemia, myelodysplastic syndrome, multiple myeloma, or acute lymphocytic leukemia.

In embodiments, the cancer is a solid cancer selected from: ovarian cancer, rectal cancer, stomach cancer, testicular cancer, cancer of the anal region, uterine cancer, colon cancer, rectal cancer, renal-cell carcinoma, liver cancer, non-small cell carcinoma of the lung, cancer of the small intestine, cancer of the esophagus, melanoma, Kaposi's sarcoma, cancer of the endocrine system, cancer of the thyroid gland, cancer of the parathyroid gland, cancer of the adrenal gland, bone cancer, pancreatic cancer, skin cancer, cancer of the head or neck, cutaneous or intraocular malignant melanoma, uterine cancer, brain stem glioma, pituitary adenoma, epidermoid cancer, carcinoma of the cervix squamous cell cancer, carcinoma of the fallopian tubes, carcinoma of the endometrium, carcinoma of the vagina, sarcoma of soft tissue, cancer of the urethra, carcinoma of the vulva, cancer of the penis, cancer of the bladder, cancer of the kidney or ureter, carcinoma of the renal pelvis, spinal axis tumor, neoplasm of the central nervous system (CNS), primary CNS lymphoma, tumor angiogenesis, metastatic lesions of said cancers, or combinations thereof.

In embodiments, the cancer is not melanoma.

In embodiments, the subject providing sample (a) and/or sample (b):
(i) has not received a prior treatment for the cancer;
(ii) has received one or more previous treatments for the cancer; or
(iii) has minimal residual disease (MRD).

### Screening Assays for New bispecific T cell engager antibodies (BiTE) and Immunomodulators

Provided herein are methods of screening for candidate bispecific T cell engager antibodies (BiTE) and/or candidate immunomodulators. For example, the methods involve evaluating the efficacy, e.g., *ex vivo* efficacy, of bispecific T cell engager antibodies (BiTE). Methods herein include screening of multiple bispecific T cell engager antibodies (BiTE) and/or immunomodulator candidates and/or their combinations in order to identify the most effective set of bispecific T cell engager antibodies (BiTE) and/or immunomodulators for a specific tumor/cancer type of a specific patient.

In embodiments, the methods comprise a cell-based assay and can involve an automated sample preparation and automated evaluation, e.g., by flow cytometry, e.g., using the ExviTech^{®} platform. See, e.g., US 8,703,491, US 2013/0109101A1, US 2010/0298255A1, US 8,313,948 and Bennett TA (2014), incorporated herein by reference. For example, use of an automated platform, e.g., automated flow cytometry platform, can enable the evaluation of hundreds or thousands of different bispecific T cell engager antibodies (BiTE) and/or immunomodulators, and this evaluation can be made *ex vivo.* The use of flow cytometry methods permits the evaluation of individual cells and also the sorting of specific cell populations. Immune cells can be stained with antibodies that bind to cell type specific cell surface markers. Target cancer cells can be stained with cell surface labels, e.g., antibodies that bind to cell type-specific cell surface markers or cell tracker dyes that distribute in the target cell membrane. Cells can also be stained for molecules present in the interior of a cell, allowing for the characterization of cells by their production of proteins, e.g., interleukins or interferons.

In embodiments, candidate bispecific T cell engager antibodies (BiTE) and/or immunomodulators can be screened using an automated flow cytometry platform, such as the ExviTech^{®} platform. *See Id.* This platform permits the determination of the cancer-killing (e.g. trogocytotic) potential of hundreds or thousands of bispecific T cell engager antibodies (BiTE) and/or immunomodulators. The cancer-killing potential of bispecific T cell engager antibodies (BiTE) and/or immunomodulators can also be measured by ratios of target cancer cells to CAR-T cells as described herein. The platform also allows for the screening of many combinations of the bispecific T cell engager antibodies (BiTE) and/or immunomodulators.

In embodiments, the screening method comprises incubating one or more candidate bispecific T cell engager antibodies (BiTE) and/or immunomodulators with cancer cells and T cells. In embodiments, the method comprises incubating one or more candidate bispecific T cell engager antibodies (BiTE) and/or immunomodulators with a sample, e.g., blood sample, where the blood sample contains both cancer cells and T cells. In embodiments, the method comprises incubating one or more candidate bispecific T cell engager antibodies (BiTE) and/or immunomodulators with a tumor sample, where the tumor sample contains both cancer cells and T cells. In other embodiments, the cancer cells and T cells are from different samples, e.g., the cancer cells are from a tumor sample and the T cells are from a blood sample.

In embodiments, the sample comprises a blood sample, e.g., whole blood sample, peripheral blood, or bone marrow. In another embodiment, the sample is obtained from a lymph node or a spleen. In embodiments, the sample is obtained from any other tissue that is involved in a malignancy, e.g., hematological malignancy or solid cancer. In embodiments, samples are used in the method described herein soon after they are obtained. Alternatively, samples may be treated with a chemical to avoid coagulation and analyzed at a later time point. In one embodiment, a blood sample is treated with heparin to avoid coagulation. In another embodiment, a blood sample is treated with EDTA to avoid coagulation. In another embodiment, a blood sample is treated with an anticoagulant, including but not limited to a thrombin inhibitor, to avoid coagulation. In embodiments, the sample is used without purification or separation steps, e.g., so that the cellular environment is more similar to the *in vivo* environment.

In embodiments, the incubation time is sufficient for the T cell to acquire a cell surface marker from the cancer cell, e.g., to undergo trogocytosis to form a trogocytotic T cell, e.g., that kills the cancer cell. In embodiments, the incubation time is sufficient for the bispecific T cell engager antibodies (BiTE) and/or immunomodulators to induce a significantly higher Effective E:T ratio between eliminated cancer cells and CD8 and/or CD4 activated T cells. In embodiments, the incubation time is at least 12 hours (e.g., at least 12, 24, 36, 48, 72, 96, 120 h, or more). In embodiments, a second or subsequent sets of cancer cells are added after the first reaction mixture of cancer cells, T cells and a bispecific T cell engager antibody (BiTE) generates CAR-T cells *ex vivo,* and in these cases the incubation time is shorter, e.g., at least 1 hour (e.g. at least 2, 3, 4, 5, 6, 7, 8, 9, 10, 11, 12, 24 h, or more).

Hundreds or thousands of candidate bispecific T cell engager antibodies (BiTE) and/or immunomodulators can be evaluated or screened. The methods described herein are capable of analyzing large numbers of candidate bispecific T cell engager antibodies (BiTE) and/or immunomodulators (e.g., combinations of candidate bispecific T cell engager antibodies (BiTE) and/or immunomodulators) at various concentrations in the form of aliquots to assess a large number of variables for a personalized medicine regimen (e.g., for personalized production of and use of CAR-T cells). In one embodiment, the method analyzes about 5-500 aliquots (e.g., 5, 10, 15, 20, 25, 30, 35, 40, 45, 50, 100, 200, 500, or more) (optionally per candidate bispecific T cell engager antibodies (BiTE) and/or immunomodulator), or a range defined by any two of the preceding values. In another embodiment, the method analyzes about 96 or more aliquots. Additionally, the number of candidate bispecific T cell engager antibodies (BiTE) and/or immunomodulators can vary along with the number of aliquots. In one embodiment, both the number of aliquots and the number of different candidate bispecific T cell engager antibodies (BiTE) and/or immunomodulators are each greater than about 5 (e.g., 5, 10, 15, 20, 25, 30, 35, or 40), or a range defined by any two of the preceding values. In another embodiment, both the number of aliquots and the number of different candidate bispecific T cell engager antibodies (BiTE) and/or immunomodulators are each greater than about 50. In another embodiment, both the number of aliquots and the number of different candidate bispecific T cell engager antibodies (BiTE) and/or immunomodulators are each greater than about 96. In another embodiment, the active ingredients of approved drugs known in the art can be screened in the assays described herein to identify potential bispecific T cell engager antibodies (BiTE) and/or immunomodulators. Such approved drugs are safe in humans and can be used to generate the CAR-T cells for administration to a patient. In embodiments, the screening method comprises identifying (e.g., and quantifying) the target cell (e.g., cancer cell) population. Alternatively, or in addition, the screening method comprises identifying the effector cell population (e.g., trogocytotic T cell population). Cell populations can be identified by using antibodies, e.g., monoclonal antibodies, directed toward specific cell surface or intracellular markers, e.g., that are conjugate to detection labels, such as fluorescent tags. In embodiments, cell surface markers include cluster of differentiation (CD) markers, which are used for the identification of hematological malignancies (e.g., leukemia, multiple myeloma, lymphoma) and of leukocytes. CD markers are also used to identify and diagnose solid tumors. Flow cytometry can be used for detection and quantification of cell populations. Cell surface labels, e.g., cell tracker dyes, can also be used to label the surface membrane of cancer cells to measure trogocytosis by CAR-T cells. Immunohistochemistry can also be used to detect certain cell markers, e.g., to identify cell populations.

In embodiments, the effect of a candidate bispecific T cell engager antibody (BiTE) and/or immunomodulator on the population(s) and subpopulation(s) of effector cells (e.g., T cells) is determined. For example, the sample may contain various types of T cells before incubation with the bispecific T cell engager antibody (BiTE) and/or the immunomodulator and may contain different combinations of or different levels of various T cell types after incubation with the bispecific T cell engager antibody (BiTE) and/or immunomodulator. In embodiments, incubation with the bispecific T cell engager antibody (BiTE) and/or immunomodulator can lead to formation of and/or increase in the numbers of trogocytotic T cells. In embodiments, the percentage of T cells that become trogocytotic (and express markers from both effector T cells and cancer cells) after incubation with the bispecific T cell engager antibody (BiTE) and/or immunomodulator is measured.

In embodiments, the effect of a candidate bispecific T cell engager antibody (BiTE) and/or immunomodulator on the target cell (e.g., cancer cell) population is measured. The measurement can involve measuring cell depletion, e.g., quantifying the cell counts in the well(s) containing bispecific T cell engager antibody (BiTE) or immunomodulator to the well(s) containing a negative control.

In embodiments, the effect of a candidate bispecific T cell engager antibody (BiTE) and/or immunomodulator on the effector cell (e.g., trogocytotic T cell) population is measured. The measurement can involve cell proliferation analysis, e.g., comparing the cell counts in the well(s) containing bispecific T cell engager antibody (BiTE) and/or immunomodulator to the well(s) containing a negative control.

In embodiments, candidate bispecific T cell engager antibody (BiTE) and/or immunomodulators that lead to (i) depletion of target (e.g., cancer) cells; (ii) formation of or increase in levels of trogocytotic T cells (e.g., that contain markers from cancer cells and markers from effector T cells, e.g., CTLs), and/or (iii) proliferation of effector T cells (e.g., CTLs) are identified as effective bispecific T cell engager antibodies (BiTE) and/or immunomodulators, e.g., effective in generating T cells with enhanced cancer-killing activity.

In embodiments, candidate bispecific T cell engager antibodies (BiTE) and/or immunomodulators are evaluated in comparison with a reference, e.g., a bispecific T cell engager antibodies (BiTE) and/or immunomodulator described herein. In embodiments, a candidate bispecific T cell engager antibody (BiTE) that leads to a similar or greater depletion of target (e.g., cancer) cells compared to the reference is identified as an effective bispecific T cell engager antibody (BiTE) and/or immunomodulator. In embodiments, a candidate bispecific T cell engager antibody (BiTE) that leads to a similar or greater formation of or increase in levels of trogocytotic T cells (e.g., that contain markers from cancer cells and markers from effector T cells, e.g., CTLs) is identified as an effective bispecific T cell engager antibody (BiTE) and/or immunomodulator. In embodiments, a candidate bispecific T cell engager antibody (BiTE) and/or immunomodulator that leads to a similar or greater extent of proliferation of the CAR-T cells is identified as effective bispecific T cell engager antibody (BiTE) and/or immunomodulator.

In embodiments, the activity of a candidate bispecific T cell engager antibody (BiTE) and/or immunomodulator, and the T cells generated, is determined using an ex *vivo*/*in vitro* assay to measure dose response curves, whose mathematical fitting enable quantitative parameters to estimate the activity, selected from at least one from EC50, Effective E:T ratio, basal E:T ratios, Emax or kinetics.
- EC50 of the T cell proliferation determines the concentration of bispecific T cell engager antibody (BiTE) which generates 50% of the activated CAR-T cells in a sample. The EC50 of T cell activation is similar to the EC50 of cancer cell depletion.
- The Effective E:T ratio represents the activity of the CAR-T cells generated (Effective T cells) on the cancer cells (target cells). High Effective E:T Ratios predict sensitive patients to the CAR-T cells as autologous cell therapy, and low Effective E:T Ratios predict resistant patients to these CAR-T cells as autologous cell therapy.
- Emax of the dose response curves of the cancer cells determines the percentage (%) of cancer cells alive at high doses of the bispecific T cell engager antibody (BiTE) at a given incubation time. Longer incubations allow CAR-T cells to kill more cancer cells. The activated CAR-T cells need to kill 100% of cancer cells for these T cells to be a suitable monotherapy treatment for a patient. When killing cancer cells is significantly lower than 100%, and this does not improve at longer incubation times, those cancer cells alive are resistant and can be clinically informative to determine a treatment. In order to revert the resistant phenotype, addition of additional immunomodulatory agents, such as immune check point inhibitors, may overcome this immunosuppression and thus overcome this resistance.
   1. Adding *ex vivo* additional immunomodulatory agents to relieve immunosuppression is especially meaningful for CAR-T cells generated for subsequent cellular therapy. The reason is that this enables the combination of multiple immunotherapy agents, immunomodulatory agents, including multiple mechanism of action, up to 5, 10, or 20 agents, contrary to the combined treatments described in the prior art, which only allow the combination of 2-3 immunotherapy drugs administered simultaneously to the subject *in vivo* due to the toxicity restrictions which limit poly-immunotherapy. Screening assays as presented herein that measure *ex vivo*/*in vitro* toxicity of CAR-T cells, alone or normalized by their cancer cell killing activity, may be suitable to identify the optimal combination of immunotherapies incubated *ex vivo*/*in vitro* that limit their combined toxicity while enhancing their combined activity. Hence, CAR-T cells generated ex *vivo* for cellular therapy can exploit the advantages of poly-immunotherapy.
   2. Adding immunomodulatory agents to the CAR-T cells *in vivo,* as additional immuno-therapeutic treatment, resulting in a combination treatment. Screening assays as presented herein that measure ex *vivo*/*in vitro* toxicity of CAR-T cells, alone or normalized by their cancer cell killing activity, may be suitable to predict the optimal combination of immunotherapies to treat a patient by limiting their combined toxicity while enhancing their combined activity.
- The time-dependent kinetics of the CAR-T cell activity. The efficiency of the T cell activation and cancer cell depletion is different in each subject for the same bispecific T cell engager antibody (BiTE). CAR-T cells which kill cancer cells faster are likely to be more efficacious in cellular therapies. Faster activity is correlated with faster effects against the cancer cells in a patient, a positive outcome that reflects higher sensitivity. Faster activity means also higher affinity towards cancer cells when the CAR-T cells are CD8+ Tumor-Specific Antigen T cells that recognize cancer cells through a MHC-I mechanism.

In embodiments, the CAR-T cell preparation comprises cells having less toxicity *ex vivo*/*in vitro* because they kill significantly less non-pathological cells, i.e. they kill more selectively. This can be measured by labeling non-pathological cells and showing more selective cancer cell killing when compared to a reference, wherein said reference can be either different patient samples for the same cancer type, or different cell subsets (e.g. clones) within the same patient sample (e.g. trogocytotic).

The most common toxicity observed in cellular therapies is called Cytokine Storm, also known as Cytokine-Release Syndrome, cytokine cascade and hypercytokinemia. It is a potentially fatal immune reaction that arises when the cytokines released by CAR-T cells in the process of killing by cell lysis cancer cells are released outside the cells, resulting in highly elevated levels of various cytokines. In embodiments, the CAR-T cell preparation comprises cells having less toxicity *ex vivo*/*in vitro* because they generate less cytokines in the supernatant and/or intracellularly. In embodiments, the CAR-T cell preparation comprises cells having both and simultaneously higher cancer-killing activity and less toxicity *ex vivo*/*in vitro,* because they generate less cytokines in the supernatant and/or intracellularly per unit of cancer cell killing, that is once the types and/or levels of cytokines released is normalized by the quantitative estimation of cancer cell killing activity such as Effective E:T Ratios, basal E:T ratios, EC50, Emax, kinetics, or a combination of these factors.

In embodiments, the efficacy, e.g., potency, activity, of a candidate bispecific T cell engager antibody (BiTE) and/or immunomodulator, is determined using an ex *vivo*/*in vitro* assay using different ratios of CAR-T cell:target cell, e.g., where a target cell can be a cancer cell. In some embodiments, the assay involves providing a CAR-T cell or a preparation thereof, e.g., produced according to a method described herein. In embodiments, the assay further involves a step (a) forming a plurality of *ex vivo* reaction mixtures comprising a candidate bispecific T cell engager antibody (BiTE)(s) and/or immunomodulator(s), a target cell (e.g., cancer cell), and the CAR-T cell or preparation thereof under conditions (e.g., for a period of time and for certain concentrations of the candidate bispecific T cell engager antibody (BiTE) and/or immunomodulatory agent) sufficient to allow the CAR-T cells to kill the target cells. In embodiments, the *ex vivo* reaction mixtures comprise a plurality of target cell to T cell ratios. The assay can also involve a step (b) for each target cell to T cell ratio, determining the number of target cells after step (a), and optionally determining the number of CAR-T cells after step (a). In embodiments, the assay further comprises a step (c) correlating the target cell to T cell ratio from step (a) with the number of target cells in step (b). In embodiments, a high target cell to T cell ratio from step (a) (e.g., higher ratio than a reference ratio) that results in fewer target cells after step (a) indicates that the candidate bispecific T cell engager antibody (BiTE) and/or immunomodulator is an effective bispecific T cell engager antibody (BiTE) and/or immunomodulator (e.g., a potent bispecific T cell engager antibody (BiTE) and/or immunomodulator) for use in producing a CAR-T cell from the subject.

In embodiments, the reference ratio is a predetermined ratio, e.g., about 1:3 to 1:10, e.g., about 1:3, 1:4, 1:5, 1:6, 1:7, 1:8, 1:9, or 1:10. In embodiments, the high target cell to T cell ratio from step (b) is about 1:4 to 1:100 (e.g., 1:4, 1:5, 1:6, 1:7, 1:8, 1:9, 1:10, 1:15, 1:20, 1:25, 1:30, 1:35, 1:40, 1:45, 1:50, 1:75, 1:100, or higher).

In embodiments, fewer target cells after step (a) is indicated by a lower number of target cells in step (b) compared to a control value (e.g., lower by at least 1.5-fold, e.g., at least 2-, 3-, 4-, 6-, 8-, 10-, 25-, 50-, 100-, 150-, 200-, 500-, 1000-, or more). In embodiments, the control value is the number of target cells before the formation of the *ex vivo* mixtures, or the number of target cells in the *ex vivo* mixtures after a period of time insufficient to allow the CAR-T cells to kill the target cells. In embodiments, the control value is the number of target cells in the *ex vivo* mixtures incubated for the same period of time without a bispecific T cell engager antibody (BiTE), or with a null control of the bispecific T cell engager antibody (BiTE) that contains only the T cell interacting arm (e.g. CD3).

In embodiments, the CAR-T cell or preparation thereof comprises a T cell, e.g., CTL, that is CD8+ and CD25+ and/or a T cell that is CD4+ and CD25+.

In embodiments, the method (e.g., step (b) of the method is performed in an automated platform, e.g., an automated flow cytometry platform described herein, e.g., the ExviTech^{®} platform described herein.

In embodiments, effective candidate bispecific T cell engager antibodies (BiTE) and/or immunomodulators are used in a method described herein, e.g., method of producing CAR-T cells described herein, method of treatment described herein, method of evaluating cancer treatments described herein, and/or method of identifying patients responsive to CAR-T cells described herein.

### Evaluation Assays for Cancer Treatments

Provided herein are methods of evaluating whether or not a patient will respond to a certain cancer treatment. In embodiments, the methods of evaluating include methods of screening for cancer treatments that would likely be effective in a particular patient.

In embodiments, a number of types of cancer treatments (e.g. a chemotherapy, a targeted anti-cancer therapy, an oncolytic drug, a cytotoxic agent, an immune-based therapy, a cytokine, an agonist of T cells (e.g., agonistic antibody or fragment thereof or an activator of a costimulatory molecule), an inhibitor of an inhibitory molecule (e.g., immune checkpoint inhibitor), an immunomodulatory agent, a vaccine, or a cellular immunotherapy) can be evaluated. In embodiments, a cancer treatment to be evaluated includes but is not limited to an immune checkpoint inhibitor, e.g., an inhibitor of one or more of: CTLA4, PD1, PDL1, PDL2, B7-H3, B7-H4, TIM3, LAG3, BTLA, CD80, CD86, or HVEM. Exemplary immune checkpoint inhibitors include ipilimumab, tremelimumab, MDX-1106, MK3475, CT-011, AMP-224, MDX-1105, IMP321, or MGA271. In embodiments, a cancer treatment to be evaluated includes an agonist of T cells, e.g., an antibody or fragment thereof to CD137, CD40, and/or glucocorticoid-induced TNF receptor (GITR). In embodiments, a cancer treatment to be evaluated includes an immunomodulatory agent such as lenolidomide. Any of the immunomodulatory agents described herein can be evaluated.

In embodiments, the method of evaluating comprises: (a) providing a T cell from a subject having a cancer (e.g., a hematological cancer or a solid cancer); (b) providing a cancer cell, e.g., from the subject; (c) forming an *ex vivo* reaction mixture comprising the T cell, the cancer cell, and a bispecific T cell engager antibody (BiTE), e.g., under conditions (e.g., for a period of time) sufficient to allow the T cell to acquire a cell surface marker from the cancer cell; and (d) contacting the *ex vivo* reaction mixture with a candidate cancer treatment or combination of cancer treatments. The method further comprises determining one or more parameters indicating effectiveness of the candidate cancer treatment(s) in killing cancer cells in the particular patient.

Exemplary bispecific T cell engager antibodies (BiTE) are described in detail in the "Bispecific T cell engager antibody (BiTE)" section herein.

In embodiments, the T cell and the cancer cell are from the same sample, e.g., from the patient to be evaluated (for responsiveness to cancer treatment). For example, a blood sample (e.g., comprising both the cancer cell and the T cell) from the patient to be evaluated is provided as the sample. Alternatively, a tumor sample (e.g., comprising both the cancer cell and the T cell, e.g., tumor infiltrating T cell) from the patient to be evaluated is provided. In embodiments, the method does not comprise removing any components (e.g., cell components) from the sample, e.g., blood sample or the tumor sample, before forming the *ex vivo* reaction mixture. In embodiments, the blood sample or tumor sample can be a freshly isolated sample or a frozen and thawed sample.

In embodiments, the sample comprises a blood sample, e.g., whole blood sample, peripheral blood, or bone marrow. In another embodiment, the sample is obtained from a lymph node or a spleen. In embodiments, the sample is obtained from any other tissue that is involved in a malignancy, e.g., hematological malignancy or solid cancer. In embodiments, samples are used in the method described herein soon after they are obtained. Alternatively, samples may be treated with a chemical to avoid coagulation and analyzed at a later time point. In one embodiment, a blood sample is treated with heparin to avoid coagulation. In another embodiment, a blood sample is treated with EDTA to avoid coagulation. In another embodiment, a blood sample is treated with an anticoagulant, including but not limited to a thrombin inhibitor, to avoid coagulation. In embodiments, the sample is used without purification or separation steps, e.g., so that the cellular environment is more similar to *the in vivo* environment.

In embodiments, the reaction mixture is carried out in a container, e.g., a well of a multi-well dish or plate (e.g., a microplate, e.g., comprising 6, 12, 24, 48, or 96 wells), or an assay tube.

In embodiments, the method (e.g., by including the bispecific T cell engager antibody (BiTE)) generates a population of trogocytotic T cells that have enhanced cancer-killing activity. Without wishing to be bound by theory, it is believed that by generating this population of enhanced CAR-T cells, the assay is more sensitive in detecting effects of cancer treatments than in other types of assays not containing such CAR-T cells.

In embodiments, the method of evaluating can be performed in a high throughput matter, e.g., can involve screening for cancer treatments that would likely be effective in a particular patient. In embodiments, screening methods comprise a cell-based assay and can involve an automated sample preparation and automated evaluation, e.g., by flow cytometry, e.g., using the ExviTech^{®} platform. For example, use of an automated platform, e.g., automated flow cytometry platform, can enable the evaluation of hundreds or thousands of different cancer treatments, and this evaluation can be made *ex vivo.* In embodiments, candidate cancer treatments can be screened using an automated flow cytometry platform, such as the ExviTech platform. The platform also allows for the screening of many combinations of the cancer treatments.

Hundreds or thousands of candidate cancer treatments can be sampled. The methods described herein are capable of analyzing large numbers of candidate cancer treatments (e.g., combinations of candidate cancer treatments) at various concentrations in the form of aliquots to assess a large number of variables. In one embodiment, the method analyzes about 5 - 500 aliquots (e.g., 5, 10, 15, 20, 25, 30, 35, 40, 45, 50, 100, 200, 500, or more) (optionally per cancer treatment), or a range defined by any two of the preceding values. In another embodiment, the method analyzes about 96 or more aliquots. Additionally, the number of cancer treatments can vary along with the number of aliquots. In one embodiment, both the number of aliquots and the number of different candidate cancer treatments are each greater than about 5 - 40 (e.g., 5, 10, 15, 20, 25, 30, 35, or 40), or a range defined by any two of the preceding values. In another embodiment, both the number of aliquots and the number of different candidate cancer treatments are each greater than about 50. In another embodiment, both the number of aliquots and the number of different candidate cancer treatments are each greater than about 96.

The effects of candidate cancer treatments on cancer cells from the patient to be evaluated can be determined by the extent of CAR-T that occurs in the presence of the candidate treatment compared to in the absence of the candidate treatment. The extent of cancer-killing can be determined using methods described herein. In embodiments, cancer-killing activity is determined by measuring the Effective E:T ratio between target cancer cells eliminated and activated T cells (CAR-T cells), as described herein and referred to as Effective E:T ratio. For example, cancer cells can be identified by detection of cancer-specific cell markers, e.g., by using flow cytometry, and then quantified.

In embodiments, a candidate cancer treatment that leads to a greater extent of cancer-killing (e.g., lower numbers of cancer cells after treatment than before, or lower numbers of cancer cells compared to samples containing a negative control treatment) (e.g., lower numbers of cancer cells by at least 10% (e.g., 10%, 20%, 30%, 40%, 50%, 60%, 70%, 80%, 90%, 1.5-fold, 2-fold, 5-fold, 10-fold, 20-fold, 50-fold, 100-fold, or more) in the assay indicates that the patient is likely to be sensitive to or responsive to the cancer treatment, i.e., the cancer treatment is likely to effectively kill cancer cells and/or reduce tumor burden in the patient.

In embodiments, a candidate cancer treatment that leads to a greater extent of cancer-killing (e.g., lower numbers of cancer cells after treatment than before, or lower numbers of cancer cells compared to samples containing a negative control treatment) (e.g., lower numbers of cancer cells by at least 10% (e.g., 10%, 20%, 30%, 40%, 50%, 60%, 70%, 80%, 90%, 1.5-fold, 2-fold, 5-fold, 10-fold, 20-fold, 50-fold, 100-fold, or more) in the assay indicates that the cancer treatment(s) are likely to be effective in treating the cancer in the patient.

In embodiments, the method further comprises preparing and/or providing a report of the responsiveness of the patient to various candidate cancer treatments. In embodiments, the report is provided to a patient or to another person or entity, e.g., a caregiver, *e.g.,* a physician, *e.g.,* an oncologist, a hospital, clinic, third-party payor, insurance company or government office. In another embodiment, the report is provided to a party responsible for interpreting or determining the effect of the candidate cancer treatment on cancer cells (e.g., extent of cancer-killing).

In embodiments, the report can be in an electronic, web-based, or paper form. The report can include an output from the method, e.g., the identification of cancer cells, the quantification of cancer cells, and the extent of cancer cell killing corresponding to each cancer treatment or combination of cancer treatments.

In one embodiment, a report is generated, such as in paper or electronic form, which identifies the extent of cancer cell death and the associated cancer treatment that led to the effect.

Such information can include information on potential or suggested cancer treatments. The report can include information on the likely effectiveness of a cancer treatment, the acceptability of a cancer treatment, or the advisability of applying the cancer treatment to the patient. For example, the report can include information, or a recommendation on, the administration of a cancer treatment, e.g., the administration at a preselected dosage or in a preselected treatment regimen, e.g., in combination with other drugs, to the patient. In an embodiment, not all candidate cancer treatments tested in the method are identified in the report. For example, the report can be limited to cancer treatments likely to be effective in the patient. In other examples, the report can omit cancer treatment unlikely to be effective in the patient. The report can be delivered, e.g., to an entity described herein, within 3 - 21 days (e.g., 3, 4, 5, 6, 7, 14, or 21 days) from receipt of the sample by the entity practicing the method.

### Methods using 3D cell culture constructs

Methods for activating T cells or for evaluating activated T cells or CAR-Ts are performed by assay systems comprised of *ex vivo* 3D cell culture constructs built to mimic the microenvironment architecture of solid tumors. This is achieved for example by culturing primary tissues or established cell lines within spheroids, extracellular matrix gels, synthetic scaffolds, rotary cell culture systems, or on low/non-adherent culture plastics. Examples of *ex vivo* 3D systems are further described in, e.g., Costa EC et al., (2017), Benien P et al., (2014), Fennema E et al., (2013) and Nam KH et al., (2015), incorporated here by reference. In embodiments, provided herein is the use of any referenced ex vivo 3D system as one of the components in any of the methods of the invention.

In embodiments, when the method is applied to samples of solid tumor is performed using 3D cell culture constructs built to mimic the microenvironment architecture of solid tumors, selected from: spheroids, extracellular matrix gels, synthetic scaffolds, rotary cell culture systems, or on low/non-adherent culture plastics.

### Use of Artificial Enviroment (AE)

Provided herein is the use of an Artificial Environment (AE) consisting in a plasma fraction, an erythrocyte fraction or a combination thereof, free from leucocytes, in the method of producing CAR-T cells one of the components of the ex vivo reaction mixture comprising a least one T cell, at least one cancer cell and a bispecific T cell engager antibody (BiTE).

In embodiments, provided herein is the use of an Artificial Environment (AE) consisting in a plasma fraction, an erythrocyte fraction or a combination thereof, free from leucocytes, as one of the components in any of the methods of the invention.

The invention also refers to the following embodiments:
A1. An *in vitro* method of producing a genetically engineered T cell expressing Chimeric Antigen Receptors (a CAR-T cell) or a CAR-T cell preparation:
   (a) providing a sample comprising at least one T cell from a subject having a cancer;
   (b) providing a sample comprising at least one cancer cell;
   (c) forming an *ex vivo* reaction mixture comprising the at least one T cell, the at least one cancer cell, and a bispecific T cell engager antibody (BiTE) under conditions and for a period of time sufficient to allow the at least one T cell to become activated and kill at least one cancer cell, thereby producing at least one activated T cell;
   (d) selecting the activated T cell, wherein the activated T cell is defined by having an effective E:T ratio higher than 1:5 between the number of activated T cells (E) and the number of target cancer cells (T) after exposure to the bispecific T cell engager antibody (BiTE); and
   (e) genetically engineering the activated T cell to produce Chimeric Antigen Receptors (CAR) on the surface of the activated T cell, thereby producing at least one CAR-T cell.
A2. An *in vitro* method of producing a genetically engineered T cell expressing Chimeric Antigen Receptors (a CAR-T cell) or a CAR-T cell preparation:
   (a) providing a sample comprising at least one T cell from a subject having a cancer;
   (b) providing a sample comprising at least one cancer cell;
   (c) forming an *ex vivo* reaction mixture comprising the at least one T cell, the at least one cancer cell, and a bispecific T cell engager antibody (BiTE) under conditions and for a period of time sufficient to allow the at least one T cell to acquire a surface marker from at least one cancer cell, thereby producing at least one activated T cell;
   (d) selecting the activated T cell, wherein the activated T cell is defined by having acquired a cell surface marker from at least one cancer cell after exposure to the bispecific T cell engager antibody (BiTE); and
   (e) isolating or enriching the activated T cells that have acquired a surface marker, using a fluorescently labeled molecule (e.g., antibody or fragment thereof) that binds to i) one or more cancer antigens ii) one or more markers of activated T cells, or both i) and ii); and
   (f) genetically engineering the selected activated T cells to produce Chimeric Antigen Receptors (CAR) on the surface of the activated T cell, thereby producing at least one CAR-T cell.
A3. An *in vitro* method of producing a genetically engineered T cell expressing Chimeric Antigen Receptors (a CAR-T cell) or a CAR-T cell preparation:
   (a) providing a sample comprising at least one T cell from a subject having a cancer;
   (b) providing a sample comprising at least one cancer cell;
   (c) Isolating or enriching the cancer cells from the sample, adding a membrane dye or a cell tracker dye,
   (d) forming an *ex vivo* reaction mixture comprising the at least one T cell, the at least one cancer cell, and a bispecific T cell engager antibody (BiTE) under conditions and for a period of time sufficient to allow the at least one T cell to acquire a surface marker from at least one cancer cell, thereby producing at least one activated T cell;
   (e) selecting the activated T cell, wherein the activated T cell is defined by having acquired a cell surface marker from at least one cancer cell after exposure to the bispecific T cell engager antibody (BiTE); and
   (f) isolating or enriching the activated T cells that have acquired a cancer surface marker, using the fluorescently membrane dye and one or more markers of activated T cells; and
   (g) genetically engineering the selected activated T cells to produce Chimeric Antigen Receptors (CAR) on the surface of the activated T cell, thereby producing at least one CAR-T cell.
A4. The method of A1 to A3, wherein the selecting step (d) of A1 or A2 or step (e) of A3 and/or the enriching step (e) of A2 or step (f) of A3 comprises using fluorescence activated cell sorting (FACS).
A5. The method of A1 to A3, wherein the selecting step of A1 orA2 orstep (e) of A3 and/or the enriching step (e) of A2 or step (f) of A3 comprises using a bead (e.g., magnetic bead) coated with an antibody or fragment thereof that binds to i) one or more cancer antigens or ii) one or more markers of activated T cells, or both i) and ii).
A6. The method of any of A2 to A5, wherein the cancer-killing T cell preparation is enriched or purified and comprises cancer-killing T cells with cancer surface markers, e.g., at a concentration of at least 50% (e.g., at least 50%, 55%, 60%, 65%, 70%, 75%, 80%, 85%, 90%, 95%, 99%, or greater) of the total number of cells in the preparation.
A7. The method of any of A1 to A3, wherein the bispecific T cell engager antibody (BiTE) has a first element providing affinity for the T cell and a second element having affinity for the cancer cell, wherein the first element binds to a T cell and does not bind to a substantial number of cancer cells and wherein the second element binds to a cancer cell and does not bind to a substantial number of T cells.
A8. The method of A7, wherein the first element binding to T cell comprises one or more of the following cell receptors: CD8, CD3, CD4, α/β T cell receptor (α/β TCR), CD45RO, and/or CD45RA.
A9. The method of any of A7 or A8, wherein the second element binds to one or more of the following cell receptors: CD20, CD28, CD30, CD33, CD52; EpCAM, CEA, gpA33, mucin, TAG-72, carbonic anhydrase IX, PSMA, folate binding protein; one or more of a ganglioside selected from: GD2, GD3, or GM2; Lewis-Y2, VEGF, VEGFR, αVβ3, α5β1, ErbB1/EGFR, ErbB2/HER2, ERbB3, c-MET, IGF1R, EphA3, TRAIL-R1, TRAIL-R2, RANKL, FAP, tenascin, CD123, CD19, and/or BCMA.
A10. The method of any of A1 to A9, wherein the T cell engager antibody (BiTE) is selected from the group consisting of BsMAb CD19/CD3, BsMAb CD20/CD3, BsMAb CD22/CD3, BsMAb CD123/CD3, BsMAb CD33/CD3 BsMAb CD3/CD28, BsMAb BCMA/CD3, and BsMAb EpCAM/CD3.
A11. The method of any of A1 to A10, wherein the ex vivo reaction mixture further comprises one or multiple agents that enhance T cell activity.
A12. The method of A11, wherein the agents that enhance T cell activity are selected from one or more of a chemotherapy drug, a targeted anti-cancer therapy, an oncolytic drug, a cytotoxic agent, an immune-based therapy, a cytokine, an agonist of T cells (e.g., agonistic antibody or fragment thereof or an activator of a costimulatory molecule), an inhibitor of an inhibitory molecule (e.g., immune checkpoint inhibitor), an immunomodulatory agent, a vaccine, or a cellular immunotherapy.
A13. The method of A12, wherein the agents enhancing T cell activity is selected from an agonist of T cells (e.g., an agonistic antibody or fragment thereof or an activator of a costimulatory molecule), and/or an inhibitor of an immune checkpoint inhibitor.
A14. The method of A13, wherein the inhibitors of the immune checkpoint inhibitor is an inhibitor of one or more of: PDL-1, PDL-2, B7-1 (CD80), B7-2 (CD86), 4-1BBL, Galectin, ICOSL, GITRL, OX40L, CD155, B7-H3, PD1, CTLA-4, 4-1BB, TIM-3, ICOS, GITR, LAG-3, KIR, OX40, TIGIT, CD160, 2B4, B7-H4 (VTCN1), HVEM (TNFRSF14 or CD270), BTLA, KIR, MHC class I, MHC class II, GAL9, VISTA, LAIR1, and A2aR
A15. The method of any of A13 or A14, wherein the inhibitors of the immune checkpoint inhibitor comprises one or more of: ipilimumab, tremelimumab, MDX-1106, MK3475, CT-011, AMP-224, MDX-1105, IMP321, or MGA271.
A16. The method of any of A13 to A15, wherein the agents enhancing T cell activity comprises molecules (e.g. antibodies) constructed combining fragments of these molecules enhancing T cell activity, e.g. bispecific or multispecific antibody formats combining recognition arms of several immune checkpoint inhibitors, including but not limited to PD1-PDL1, PD1-PDL2, PD1-LAG3, PD1-TIM3.
A17. The method of A12, wherein the agonist of T cells comprises an antibody or fragment thereof to CD137, CD40, and/or glucocorticoid-induced TNF receptor (GITR).
A18. The method of A12, wherein the immunomodulatory agent comprises/is lenalidomide, ibrutinib or bortezomib.
A19. The method of A11, wherein the agent enhancing T cell activity enhances and/or restores the immunocompetence of T cells.
A20. The method of A12, wherein the immunomodulatory agent is an inhibitor of MDSCs and/or Treg cells.
A21. The method of A12, wherein the immunomodulatory agent activates an immune response to a tumor specific antigen, e.g., it is a vaccine (e.g., a vaccine against targets such as gp100, MUC1 or MAGEA3.
A22. The method of A12, wherein the immunomodulatory agent is a cytokine, e.g., a recombinant cytokine chosen from one or more of GM-CSF, IL-7, IL-12, IL-15, IL-18 or IL-21.
A23. The method of A12, wherein the immunomodulatory agent is a modulator of a component (e.g., enzyme or receptor) associated with amino acid catabolism, signalling of tumor-derived extracellular ATP, adenosine signalling, adenosine production, chemokine and chemokine receptor, recognition of foreign organisms, or kinase signalling activity.
A24. The method of A12, wherein the immunomodulatory agent is an inhibitor (e.g., small molecule inhibitor) of IDO, COX2, ARG1, ArG2, iNOS, or phosphodiesterase (e.g., PDE5); a TLR agonist, or a chemokine antagonist.
A25. The method of any of A1 to A24, comprising one, two or all of the following *in vitro* steps:
   i) expanding the CAR-T cell from step (e) of A1, step (f) of A2 or step (g) of A3;
   ii) enriching for the CAR-T cell from step (e) of A1, step (f) of A2 or step (g) of A3; or
   iii) purifying the CAR-T cell from step (e) of A1, step (f) of A2 or step (e) of A3.
A26. The method of any of A1 to A25, wherein Chimeric Antigen Receptors recognize a neoantigen of a cancer cell.
A27. The method of any of A1 to A26, wherein the activated T cell is transfected to produce Chimeric Antigen Receptors (CAR) on the surface of said activated T cell.
A28. The method of any of A1 to A27, wherein the expansion of the CAR-T cell comprises increasing the number of CAR-T cells by to 2-fold to 10⁶-fold or more.
A29. The method of any of A1 to A28, wherein the selection of the activated T cell, is based on a parameter chosen from one or more of: increased cancer cell killing activity, reduced toxicity, reduced off-target effect, increased viability, increased proliferation, or Effective E:T ratio.
A30. The method of any of A1 to A29, wherein the selecting step (d) of A1, step (d) of A2 or step (e) of A3 comprises using a fluorescently labeled compound that binds to i) one or more cancer antigens, or diffuses into the cancer cell membrane or ii) one or more markers of activated T cells, or both i) and ii); or comprises using a bead coated with an antibody or fragment thereof that binds to i) one or more cancer antigens or ii) one or more markers of activated T cells, or both i) and ii).
A31. The method of any of A1 to A30, wherein the CAR-T cell preparation comprises trogocytotic CAR-T cells at a concentration of at least 50% of the total number of cells in the CAR-T cell preparation.
A32. The method of any of A1 to A31, wherein the CAR-T cell or CAR-T cell preparation comprises one or more CD8+ T cells and/or one or more CD25+ T cells, and/or one or more CD8+/CD25+ T cells and/or one or more CD4+/CD25+ T cells, and or one or more cytotoxic T lymphocytes (CTLs) or one or more tumor infiltrating lymphocytes (TILs) or marrow infiltrated lymphocytes (MILs) and/or one or more trogocytotic T cells.
A33. The method of any of A1 to A32, wherein the CAR-T cell preparation comprises regulatory T cells (Tregs) at a concentration of less than 10% of the total number of cells in the CAR-T cell preparation; and/or naive T cells at a concentration of less than 10% of the total number of cells in the CAR-T cell preparation.
A34. The method of any of A1 to A33, further comprising separating individual clones from the CAR-T cell preparation, wherein the separating step comprises clonal expansion of single cells by:
   (i) separating the preparation of CAR-T cells into single cells and
   (ii) expanding the single cells to generate one or more preparations of CAR-T cells.
A35. The method of any of A1 to A34, wherein the sample of step (a) and the sample of step (b) of A1, A2 or A3 are from the same subject.
A36. The method of any of A1 to A35, wherein step (a) and step (b) of A1, A2 or A3 comprise providing one sample comprising both the at least one cancer cell and the at least one T cell.
A37. The method of any of A1 to A36, wherein the sample of step (a) of A1, A2 or A3 is selected from: whole blood, peripheral blood, bone marrow, lymph node, spleen, a primary tumor and a metastasis.
A38. The method of any of A1 to A37, wherein the sample of step (a) of A1, A2 or A3 is derived from a tissue with a microenvironment, wherein substantially no components have been removed or isolated from the sample.
A39. The method of any of A1 to A38, wherein the subject is an adult or a pediatric subject.
A40. The method of any of A1 to A39, wherein the cancer of the sample of step (b) of A1, A2 or A3 is a hematological cancer selected from: Hodgkin's lymphoma, Non-Hodgkin's lymphoma (B cell lymphoma, diffuse large B cell lymphoma, follicular lymphoma, mantle cell lymphoma, marginal zone B-cell lymphoma, Burkitt lymphoma, lymphoplasmacytic lymphoma, hairy cell leukemia), acute myeloid leukemia, chronic myeloid leukemia, myelodysplastic syndrome, multiple myeloma, chronic lymphocytic leukemia or acute lymphocytic leukemia.
A41. The method of any of A1 to A39, wherein the cancer is a solid cancer selected from: ovarian cancer, rectal cancer, stomach cancer, testicular cancer, cancer of the anal region, uterine cancer, colon cancer, rectal cancer, renal-cell carcinoma, liver cancer, non-small cell carcinoma of the lung, cancer of the small intestine, cancer of the esophagus, melanoma, Kaposi's sarcoma, cancer of the endocrine system, cancer of the thyroid gland, cancer of the parathyroid gland, cancer of the adrenal gland, bone cancer, pancreatic cancer, skin cancer, cancer of the head or neck, cutaneous or intraocular malignant melanoma, uterine cancer, brain stem glioma, pituitary adenoma, epidermoid cancer, carcinoma of the cervix squamous cell cancer, carcinoma of the fallopian tubes, carcinoma of the endometrium, carcinoma of the vagina, sarcoma of soft tissue, cancer of the urethra, carcinoma of the vulva, cancer of the penis, cancer of the bladder, cancer of the kidney or ureter, carcinoma of the renal pelvis, spinal axis tumor, neoplasm of the central nervous system (CNS), primary CNS lymphoma, tumor angiogenesis, metastatic lesions of said cancers, or combinations thereof.
A42. The method of any of A1 to A41, wherein the cancer is not melanoma.
A43. The method of any of A1 to A42, wherein the subject providing the sample of step (a) and/or the sample of step (b) of A1, A2 or A3:
   (i) has not received a prior treatment for the cancer;
   (ii) has received one or more previous treatments for the cancer; or
   (iii) has minimal residual disease (MRD).
A44. The method of any of A1 to A43, further comprising repeating steps (a)-(e) of A1, steps (a)-(f) of A2, or steps (a)-(g) of A3, using a sample of T cells and cancer cells different from the sample used in previous steps (a)-(e) of A1, steps (a)-(f) of A2, or steps (a)-(g) of A3, respectively.
A45. The method of any of A1 to A44, wherein the CAR-T cells produced from each repeat of steps (a)-(e) of A1, steps (a)-(f) of A2, or steps (a)-(g) of A3, respectively, is pooled to form a mixture of CAR-T cells.
A46. The method of any of A1 to A45, further comprising evaluating the activity of the CAR-T cell or CAR-T cell preparation.
A47. The method of A46, wherein evaluating comprises:
   (a) providing a CAR-T cell or a CAR-T cell preparation thereof obtainable according to the method of A1, A2, or A3;
   (b) providing a sample of cancer cells, wherein the cancer cells are from the same subject;
   (c) contacting the CAR-T cell or the CAR-T cell preparation thereof with the cancer cells for a period of time sufficient to allow the CAR-T cell to kill the cancer cells;
   (d) determining the level of cancer cells after step (c), and optionally determining the level of CAR-T cells after step (c); and optionally,
   (e) determining the ratio of either cancer cell to CAR-T cell, or CAR-T cell to cancer cell, from step (d).
A48. The method of any of A1 to A47, wherein step (c) of A1, step(c) of A2 or step (d) of A3 additionally comprises adding a bispecific T cell engager antibody (BiTE) at increasing dosages.
A49. The method of any of A1 to A48, wherein a decrease in the level or amount of cancer cells, relative to a reference level, is indicative of increased cell killing activity, or wherein a reduced change or no substantial change in the level or amount of cancer cells relative to a reference level, is indicative of decreased cell killing activity.
A50. The method of any of A1 to A49, wherein the Effective E:T ratio is 1:10 or higher.
A51. The method of any of A1 to A50, wherein the Effective E:T ratio is 1:20 or higher.
A52. The method of any of A1 to A51, wherein the level of cancer cells and/or CAR-T cells is determined at time 0 to 48 hours.
A53. The method of any of A1 to A52, wherein the method is performed using an automated fluorescence based platform.
A54. The method of any of A1 to A53, wherein the method is performed using flow cytometry.
A55. A composition comprising a CAR-T cell or CAR-T cell preparation thereof obtainable according to the method of any of A1 to A54.
A56. The composition according to A55, wherein the CAR-T cell requires (i) and at least one of (ii), (iii), or (iv): (i) has cytotoxic activity toward a cancer cell, and (ii) comprises at least 100 copies of the cancer cell surface marker, including a membrane cell marker on the cancer cell; and/or (iii) comprises a detectable amount of a bispecific T cell engager antibody (BiTE); and/or (iv) comprises a detectable amount of agents enhancing T cell activity such as e.g. immune check point inhibitors.
A57. The composition of any of A55 or A56, wherein the CAR-T cell is a cytotoxic T lymphocyte or a helper T cell selected from a CD8+ T cell or a CD4+ T cell.
A58. The composition of any of A55 to A57, wherein the composition comprises cancer cells at a concentration of less than 30% the total number of cells in the composition or preparation, and comprises Tregs at a concentration of less than 30% of the total number of cells in the composition or preparation, and comprises naive T cells at a concentration of less than 30% of the total number of cells in the composition or preparation, and comprises red blood cells at a concentration of less than 30% of the total number of cells in the composition or preparation, and/or comprises non-immune cells at a concentration of less than 30% of the total number of cells in the composition or preparation.
A59. The composition of any of A55 to A58, comprising CAR-T cells at a concentration of at least 30% of the total number of cells in the composition or preparation.
A60. A pharmaceutical composition comprising the composition of any of A55 to A59 and a pharmaceutically acceptable carrier.
A61. The pharmaceutical composition according to A60 for use in Adoptive Cancer Therapy for treating a subject, wherein the subject is the same subject as that of step (a) of A1, A2 or A3, and/or wherein the subject is the same subject as that of step (b) of A1, A2 or A3, and/or wherein the subject is different from the subject as that as step (a) or (b) of A1, A2 or A3.
A62. The pharmaceutical composition for use according to A61 in Adoptive Cancer Therapy for treating a subject suffering (i) an hematological cancer selected from: Hodgkin's lymphoma, Non-Hodgkin's lymphoma (B cell lymphoma, diffuse large B cell lymphoma, follicular lymphoma, mantle cell lymphoma, marginal zone B-cell lymphoma, Burkitt lymphoma, lymphoplasmacytic lymphoma, hairy cell leukemia), acute myeloid leukemia, chronic myeloid leukemia, myelodysplastic syndrome, multiple myeloma, chronic lymphocytic leukemia or acute lymphocytic leukemia, or (ii) a solid cancer selected from: ovarian cancer, rectal cancer, stomach cancer, testicular cancer, cancer of the anal region, uterine cancer, colon cancer, rectal cancer, renal-cell carcinoma, liver cancer, non-small cell carcinoma of the lung, cancer of the small intestine, cancer of the esophagus, melanoma, Kaposi's sarcoma, cancer of the endocrine system, cancer of the thyroid gland, cancer of the parathyroid gland, cancer of the adrenal gland, bone cancer, pancreatic cancer, skin cancer, cancer of the head or neck, cutaneous or intraocular malignant melanoma, uterine cancer, brain stem glioma, pituitary adenoma, epidermoid cancer, carcinoma of the cervix squamous cell cancer, carcinoma of the fallopian tubes, carcinoma of the endometrium, carcinoma of the vagina, sarcoma of soft tissue, cancer of the urethra, carcinoma of the vulva, cancer of the penis, cancer of the bladder, cancer of the kidney or ureter, carcinoma of the renal pelvis, spinal axis tumor, neoplasm of the central nervous system (CNS), primary CNS lymphoma, tumor angiogenesis, metastatic lesions of said cancers, or combinations thereof.
A63. A method for treating a subject having cancer comprising providing a CAR-T cell or a CAR-T cell preparation thereof obtainable according to the method of any of A1 to A54 or the composition of any of A55 to A59, and administering an effective amount of the CAR-T cell, the CAR-T cell preparation or composition to the subject.
A64. The method of A63, comprising:
   (a) providing a sample from the subject, wherein the sample comprises a T cell and a cancer cell;
   (b) contacting the sample *ex vivo* with a bispecific T cell engager antibody (BiTE) for a period of time;
   (c) selecting the activated T cell, wherein the activated T cell is defined by the subset of activated T cells having acquired a cell surface marker from at least one cancer cell, or by the full set of activated T cells having an effective E:T ratio higher than 1:5 between the number of activated T cells generated (E) and the number of target cancer cells killed (T) after exposure to the bispecific T cell engager antibody (BiTE);
   (d) genetically engineering the activated T cell to produce Chimeric Antigen Receptors (CAR) on the surface of the activated T cell, thereby producing at least one CAR-T cell; and
   (e) administering an effective amount of the CAR-T cells to the subject.
A65. The method of any of A63 or A64, further comprising administering to the subject a second therapeutic agent or procedure.
A66. The method of A65, wherein the second therapeutic agent or procedure is chosen from one or more of: chemotherapy, a targeted anti-cancer therapy, an oncolytic drug, a cytotoxic agent, an immune-based therapy such as immune check point inhibitors, a cytokine, a surgical procedure, a radiation procedure, an agonist of T cells (agonistic antibody or fragment thereof or an activator of a costimulatory molecule), an inhibitor of an inhibitory molecule (immune checkpoint inhibitor), an immunomodulatory agent, a vaccine, or a cellular immunotherapy.
A67. An *ex vivo* method for testing cellular responsiveness of primary cell populations to a genetically engineered T cell expressing Chimeric Antigen Receptors (a CAR-T cell) that comprises:
   i) submit a whole sample from a subject selected from: peripheral blood (PB), or bone marrow (BN), or lymph node (LN) to a separation process to isolate an Artificial Environment (AE) consisting in a plasma fraction, an erythrocyte fraction or a combination thereof, free from leucocytes,
   ii) mix the leucocyte-free AE obtained in the previous step with a primary cell population,
   iii) add to the mixture of step ii) at least one genetically engineered T cell expressing Chimeric Antigen Receptors (a CAR-T cell) to be tested, obtainable according to step (e) of A1, step (f) of A2, or step (g) of A3,
   iv) incubate the mixture obtained in step iii) during from 2 hours to 14 days to allow the a genetically engineered T cell expressing Chimeric Antigen Receptors (a CAR-T cell) tested to exert any activity it might have on the primary cell population,
   v) assess the viability and/or proliferation of the primary cell population in the presence or absence of the genetically engineered T cell expressing Chimeric Antigen Receptors (a CAR-T cell) tested,
   vi) produce comparative data on viability and/or on proliferation of the primary tumor cell population between the assessment made in presence and in absence of the genetically engineered T cell expressing Chimeric Antigen Receptors (a CAR-T cell) tested and relate the data obtained to values indicative of the genetically engineered T cell expressing Chimeric Antigen Receptors (a CAR-T cell) activity for reducing/increasing viability and/or proliferation of the primary cell population.
A68. An *in vitro* method of identifying subjects susceptible to immune checkpoint immunotherapy treatment, comprising:
   (a) providing a sample comprising at least one T cell from a subject having a cancer;
   (b) providing a sample comprising at least one cancer cell, e.g., from the subject;
   (c) forming an *ex vivo* reaction mixture comprising the at least one T cell, the at least one cancer cell, and a bispecific T cell engager antibody (BiTE), under conditions (e.g., for a period of time) sufficient to allow the T cell to kill cancer cells, thereby producing the cancer-killing T cell
   (d) determining the pharmacological activity of the cancer-killing T cells obtained in step (c) by dose response and/or pharmacodynamic parameters of cancer-killing T cells and tumor cells, selected from EC50, Emax, AUC, Effective E:T Ratios, Basal E:T Ratios, or kinetic parameters;
   (e) determining the pharmacological activity of the cancer-killing T cells repeating steps (c) and (d) by dose response or evaluating a single high saturating dose in combination with immune check point inhibitors, individually, or in combinations, or bispecific or multispecific antibody constructs combining immune checkpoint inhibitors, including the combination of all immune checkpoint inhibitors;
   (f) determining the expression levels of immune checkpoint molecules in both the tumor cells and T cells in the reaction mixture of step (c), comparing basal levels with levels after incubation;
   (g) identifying subjects susceptible to immune checkpoint immunotherapy treatment, whereby the bispecific T cell engager antibody (BiTE) incubation is only a reagent to activate T cells, by assessment of either of the following 2 criteria or a combination of them:
      i. step (d) reveals a resistant tumor cell population in the samples from the subject (i.e. incubation with the bispecific T cell engager antibody (BiTE) does not kill all tumor cells), and addition of one or more immune checkpoint inhibitors in (e) reverts resistance of tumor cell population;
      ii. step (f) reveals an increase in the expression level of an immune checkpoint molecule in either the tumor cells and/or T cells in the reaction mixture of step (c) after incubation, relative to basal levels prior incubation,
   and wherein observance of both (i) and (ii) is indicative of a subject more susceptible to immune checkpoint immunotherapy treatment.
A69. An *in vitro* method of identifying subjects susceptible to immune checkpoint immunotherapy treatment, comprising:
   (a) providing a sample comprising at least one T cell from a subject having a cancer;
   (b) providing a sample comprising at least one cancer cell, e.g., from the subject;
   (c) forming an *ex vivo* reaction mixture comprising the at least one T cell, the at least one cancer cell, and a bispecific T cell engager antibody (BiTE), under conditions (e.g., for a period of time) sufficient to allow the T cell to kill cancer cells, thereby producing the cancer-killing T cell
   (d) Isolating the activated T cells, by FACS or magnetic-beads or other methods, adding them to a cancer cell, e.g., from the subject, forming an *ex vivo* reaction mixture comprising under conditions (e.g., for a period of time) sufficient to allow the activated T cells to kill cancer cells; and;
   (e) determining the pharmacological activity of the cancer-killing T cells obtained in step (d) by dose response and/or pharmacodynamic parameters of cancer-killing T cells and tumor cells, selected from EC50, Emax, AUC, Effective E:T Ratios, Basal E:T Ratios, or kinetic parameters and;
   (f) determining the pharmacological activity of the cancer-killing T cells repeating steps (d) and (e) by dose response or evaluating a single high saturating dose in combination with immune check point inhibitors, individually, or in combinations, or bispecific or multispecific antibody constructs combining immune checkpoint inhibitors, including the combination of all immune checkpoint inhibitors;
   (g) determining the expression levels of immune checkpoint molecules in both the tumor cells and T cells in the reaction mixture of step (d), comparing basal levels with levels after incubation;
   (h) identifying subjects susceptible to immune checkpoint immunotherapy treatment, whereby the bispecific T cell engager antibody (BiTE) incubation is only a reagent to activate T cells, by assessment of either of the following 2 criteria or a combination of them:
      i. step (e) reveals a resistant tumor cell population in the samples from the subject (i.e. incubation with BiTE-activated isolated T cells does not kill all tumor cells), and addition of one or more immune checkpoint inhibitors in (f) reverts resistance of tumor cell population;
      ii. step (g) reveals an increase in the expression level of an immune checkpoint molecule in either the tumor cells and/or T cells in the reaction mixture of step (d) after incubation, relative to basal levels prior incubation,
   and wherein observance of both (i) and (ii) is indicative of a subject more susceptible to immune checkpoint immunotherapy treatment.
A70. An *in vitro* method of identifying subjects susceptible to immune checkpoint immunotherapy treatment to be combined with a bispecific T cell engager antibody (BiTE) immunotherapy, for decreasing resistance of said subject to said BiTE immunotherapy, comprising:
   (a) providing a sample comprising at least one T cell from a subject having a cancer;
   (b) providing a sample comprising at least one cancer cell, e.g., from the subject;
   (c) forming an *ex vivo* reaction mixture comprising the at least one T cell, the at least one cancer cell, and the the bispecific T cell engager antibody (BiTE), being identical to BiTE of the immunotherapy, e.g., under conditions (e.g., for a period of time) sufficient to allow the T cell to kill cancer cells, thereby producing the cancer-killing T cell;
   (d) determining the pharmacological activity of the cancer-killing T cells obtained in step (c) by dose response and/or pharmacodynamic parameters of cancer-killing T cells and tumor cells, selected from EC50, Emax, AUC, Effective E:T Ratios, Basal E:T Ratios, or kinetic parameters;
   (e) determining the pharmacological activity of cancer-killing T cells obtained in step (c) by by dose response or evaluating a single high saturating dose in combination with immune check point inhibitors, individually, or in combinations, or bispecific or multispecific antibody constructs combining immune check point inhibitors, including the combination of all immune check point inhibitors;
   (f) determining the expression levels of immune checkpoint molecules in both the tumor cells and T cells in the reaction mixture of step (c), comparing basal levels with levels after incubation,
   (g) identifying subjects susceptible to immune checkpoint immunotherapy treatment to be combined with a bispecific T cell engager antibody (BiTE) immunotherapy, by assessment of either of the following 2 criteria or a combination of them:
      i. step (d) reveals a resistant tumor cell population in the samples from the subject (i.e. incubation with the bispecific T cell engager antibody (BiTE) does not kill all tumor cells), and addition of one or more immune checkpoint inhibitors in (e) reverts resistance of tumor cell population;
      ii. step (f) reveals an increase in the expression level of an immune checkpoint molecule in either the tumor cells and/or T cells in the reaction mixture of step (c) after incubation, relative to basal levels prior incubation;
   and wherein observance of both (i) and (ii) is indicative of a subject more susceptible to immune checkpoint immunotherapy treatment to be combined with a bispecific T cell engager antibody (BiTE) immunotherapy.
A71. An *in vitro* method of identifying subjects susceptible to immune checkpoint immunotherapy treatment to be combined with a bispecific T cell engager antibody (BiTE) immunotherapy, for decreasing resistance of said subject to said BiTE immunotherapy, comprising:
   (a) providing a sample comprising at least one T cell from a subject having a cancer;
   (b) providing a sample comprising at least one cancer cell, e.g., from the subject;
   (c) forming an *ex vivo* reaction mixture comprising the at least one T cell, the at least one cancer cell, and the bispecific T cell engager antibody (BiTE), being identical to the BiTE of the immunotherapy, e.g., under conditions (e.g., for a period of time) sufficient to allow the T cell to kill cancer cells, thereby producing the cancer-killing T cell;
   (d) Isolating the activated T cells, by FACS or magnetic-beads or other methods, adding them to a cancer cell, e.g., from the subject, forming an *ex vivo* reaction mixture comprising under conditions (e.g., for a period of time) sufficient to allow the activated T cells to kill cancer cells; and;
   (e) determining the pharmacological activity of the cancer-killing T cells obtained in step (d) by dose response and/or pharmacodynamic parameters of cancer-killing T cells and tumor cells, selected from EC50, Emax, AUC, Effective E:T Ratios, Basal E:T Ratios, or kinetic parameters and;
   (f) determining the pharmacological activity of the cancer-killing T cells repeating steps (d) and (e) by dose response or evaluating a single high saturating dose in combination with immune check point inhibitors, individually, or in combinations, or bispecific or multispecific antibody constructs combining immune checkpoint inhibitors, including the combination of all immune checkpoint inhibitors;
   (g) determining the expression levels of immune checkpoint molecules in both the tumor cells and T cells in the reaction mixture of step (d), comparing basal levels with levels after incubation;
   (h) identifying subjects susceptible to immune checkpoint immunotherapy treatment, in combination with the BiTE, by assessment of either of the following 2 criteria or a combination of them:
      i. step (e) reveals a resistant tumor cell population in the samples from the subject (i.e. incubation with BiTE-activated isolated T cells does not kill all tumor cells), and addition of one or more immune checkpoint inhibitors in (f) reverts resistance of tumor cell population;
      ii. step (g) reveals an increase in the expression level of an immune checkpoint molecule in either the tumor cells and/or T cells in the reaction mixture of step (d) after incubation, relative to basal levels prior incubation,
   and wherein observance of both (i) and (ii) is indicative of a subject more susceptible to immune checkpoint immunotherapy treatment for decreasing resistance of said subject to said BiTE immunotherapy.
A72. An *in vitro* method of identifying subjects susceptible to immune checkpoint immunotherapy treatment to be combined with a cellular immunotherapy such a CAR-T to treat a subject, for decreasing resistance of said subject to said cellular immunotherapy, comprising:
   (a) providing a sample comprising at least one T cell selected from the group consisting of a tumor infiltrated lymphocyte (TIL), marrow infiltrated lymphocyte (MIL), a genetically engineered T cell, a CAR-T cell, or an activated T cell obtainable according to step (c) of the method of A1 or A2, or step (d) of the method of A3 and a genetically engineered T cell expressing Chimeric Antigen Receptors obtainable according to step (e) of the method of A1, step (f) of the method of A2, or step (g) of the method of A3, from a subject having a cancer;
   (b) providing a cancer cell, e.g., from the subject;
   (c) forming an *ex vivo* reaction mixture comprising (a) and (b), under conditions (e.g., for a period of time) sufficient to allow the T cells to kill cancer cells, thereby producing the cancer-killing T cell; and
   (d) determining the pharmacological activity of cancer-killing T cells obtained in step (c) by dose response and/or pharmacodynamic parameters of cancer-killing T cells and tumor cells, selected from EC50, Emax, AUC, Effective E:T Ratios, Basal E:T Ratios, or kinetic parameters;
   (e) determining the pharmacological activity of cancer-killing T cells obtained in step (c) by dose response or evaluating a single high saturating dose in combination with immune check point inhibitors, individually, or in combinations, or bispecific or multispecific antibody constructs combining immune check point inhibitors, including the combination of all immune check point inhibitors, either by full dose responses or evaluating a single high saturating dose.
   (f) determining the expression levels of immune checkpoint molecules in both the tumor cells and T cells in the reaction mixture of step (c), comparing basal levels with levels after incubation,
   (g) identifying subjects susceptible to immune checkpoint immunotherapy treatment in combination with the cellular therapy, by assessment of either of the following 2 criteria or a combination of them:
      i. step (d) reveals a resistant tumor cell population in the samples from the subject (i.e. incubation with T cell therapy does not kill all tumor cells), and addition of one or more immuno checkpoint inhibitors in (e) reverts resistance of tumor cell population;
      ii. step (f) reveals an increase in the expression level of an immune checkpoint molecule in either the tumor cells and/or T cells in the reaction mixture of step (c) after incubation, relative to basal levels prior incubation,
   and wherein observance of both (i) and (ii) is indicative of a subject more susceptible to immune checkpoint immunotherapy treatment to be combined with a cellular immunotherapy.
A73. The method of any of A68 to A72, wherein in step (e) of A68, A70 and A72, and step (f) of A69 and A71, the immune check point immunotherapy is added either from the beginning of the incubation or sequentially after a certain amount of time sufficient for the T cells to become activated killing tumor cells.
A74. The method of any of A68 to A73, wherein the different incubation times are evaluated, and any single incubation time can be used to identify subjects susceptible to immune check point immunotherapy, alone or in combination with other drugs.
A75. The method of any of A68 to A74, wherein the immune checkpoint molecule is selected from the group consisting of PDL-1, PDL-2, B7-1 (CD80), B7-2 (CD86), 4-1BBL, Galectin, ICOSL, GITRL, OX40L, CD155, B7-H3, PD1, CTLA-4, 4-1BB, TIM-3, ICOS, GITR, LAG-3, KIR, OX40, TIGIT, CD160, 2B4, B7-H4 (VTCN1), HVEM (TNFRSF14 or CD270), BTLA, KIR, MHC class I, MHC class II, GAL9, VISTA, LAIR1, and A2aR, or combinations of these immune checkpoint molecules in bispecific or multispecific antibody formats.
A76. The method of any of A68 to A75, wherein the immune checkpoint molecule is PD-1.
A77. The method of any of A68 to A76, wherein the method is performed using an automated fluorescence based platform.
A78. The method of any of A68 to A77, wherein the method is performed using flow cytometry.
A79. The method of any of A68 to A78, wherein the bispecific T cell engager antibody (BiTE) has a first element providing affinity for the T cell and a second element having affinity for the cancer cell, wherein the first element binds to a T cell and does not bind to a substantial number of cancer cells and wherein the second element binds to a cancer cell and does not bind to a substantial number of T cells.
A80. The method of A79, wherein the first element binding to T cell comprises one or more of the following cell receptors: CD8, CD3, CD4, α/β T cell receptor (α/β TCR), CD45RO, and/or CD45RA.
A81. The method of any of A79 or A80, wherein the second element binds to one or more of the following cell receptors: CD20, CD28, CD30, CD33, CD52; EpCAM, CEA, gpA33, mucin, TAG-72, carbonic anhydrase IX, PSMA, folate binding protein; ganglioside selected from: GD2, GD3, or GM2; Lewis-Y2, VEGF, VEGFR, αVβ3, α5β1, ErbB1/EGFR, ErbB2/HER2, ERbB3, c-MET, IGF1R, EphA3, TRAIL-R1, TRAIL-R2, RANKL, FAP, tenascin, CD123, CD19, and/or BCMA.
A82. The method of any of A68 to A81, wherein the T cell engager antibody (BiTE) is selected from the group consisting of BsMAb CD19/CD3, BsMAb CD20/CD3, BsMAb CD22/CD3, BsMAb CD123/CD3, BsMAb CD33/CD3 BsMAb CD3/CD28, BsMAb BCMA/CD3, and BsMAb EpCAM/CD3.
A83. The method according to any of A68 to A82, wherein Chimeric Antigen Receptors recognize a neoantigen of a cancer cell.
A84. The method of any of A68 to A83, wherein the sample of step (a) and the sample of step (b) are from the same subject.
A85. The method of any of A68 to A84, wherein step (a) and step (b) comprise providing one sample comprising both the cancer cell and the T cell.
A86. The method of any of A68 to A85, wherein the sample (a) is selected from: whole blood, peripheral blood, bone marrow, lymph node, spleen, a primary tumor and a metastasis.
A87. The method of any of A68 to A86, wherein the sample (a) is derived from a tissue with a microenvironment, wherein substantially no components have been removed or isolated from the sample.
A88. The method of any of A68 to A87, wherein the subject is an adult or a pediatric subject.
A89. The method of any of A68 to A88, wherein the cancer of sample (b) is a hematological cancer selected from: Hodgkin's lymphoma, Non-Hodgkin's lymphoma (B cell lymphoma, diffuse large B cell lymphoma, follicular lymphoma, mantle cell lymphoma, marginal zone B-cell lymphoma, Burkitt lymphoma, lymphoplasmacytic lymphoma, hairy cell leukemia), acute myeloid leukemia, chronic myeloid leukemia, myelodysplastic syndrome, multiple myeloma, chronic lymphocytic leukemia or acute lymphocytic leukemia.
A90. The method of any of A68 to A88, wherein the cancer is a solid cancer selected from: ovarian cancer, rectal cancer, stomach cancer, testicular cancer, cancer of the anal region, uterine cancer, colon cancer, rectal cancer, renal-cell carcinoma, liver cancer, non-small cell carcinoma of the lung, cancer of the small intestine, cancer of the esophagus, melanoma, Kaposi's sarcoma, cancer of the endocrine system, cancer of the thyroid gland, cancer of the parathyroid gland, cancer of the adrenal gland, bone cancer, pancreatic cancer, skin cancer, cancer of the head or neck, cutaneous or intraocular malignant melanoma, uterine cancer, brain stem glioma, pituitary adenoma, epidermoid cancer, carcinoma of the cervix squamous cell cancer, carcinoma of the fallopian tubes, carcinoma of the endometrium, carcinoma of the vagina, sarcoma of soft tissue, cancer of the urethra, carcinoma of the vulva, cancer of the penis, cancer of the bladder, cancer of the kidney or ureter, carcinoma of the renal pelvis, spinal axis tumor, neoplasm of the central nervous system (CNS), primary CNS lymphoma, tumor angiogenesis, metastatic lesions of said cancers, or combinations thereof.
A91. The method of any of A68 to A90, wherein the cancer is not melanoma.
A92. The method of any of A68 to A91, wherein the subject providing sample (a) and/or sample (b):
   (i) has not received a prior treatment for the cancer;
   (ii) has received one or more previous treatments for the cancer; or
   (iii) has minimal residual disease (MRD).
A93. A method for treating a subject having cancer comprising providing a bispecific T cell engager antibody (BiTE) or a T cell selected from the group consisting of a tumor infiltrated lymphocyte (TIL), a genetically engineered T cell, a CAR-T cell, an activated T cell obtainable according to the step (c) of the method of A1 or A2, or step (d) of A3, and a genetically engineered T cell expressing Chimeric Antigen Receptors obtainable according to step (e) of the method of A1, step (f) of the method of A2, or step (g) of the method of A3, in combination with an inhibitor of at least one immune checkpoint molecule selected in the method of A60 to A81 as target for decreasing resistance to a cancer therapy.
A94. The method of A93, wherein the inhibitor of at least one immune checkpoint molecule is selected from the group consisting of Nivolumab, Pembrolizumab and Pidilizumab.
A95. The method of any of A93 or A94, wherein the inhibitor of at least one immune checkpoint molecule is Nivolumab.
A96. The method of any of A93 to A95, further comprising administering a third therapeutic agent or procedure.
A97. The method of A96, wherein the third therapeutic agent or procedure is chosen from one or more of: chemotherapy, a targeted anti-cancer therapy, an oncolytic drug, a cytotoxic agent, an immune-based therapy, a cytokine, a surgical procedure, a radiation procedure, an agonist of T cells (agonistic antibody or fragment thereof or an activator of a costimulatory molecule), an inhibitor of an inhibitory molecule (immune checkpoint inhibitor), an immunomodulatory agent, a vaccine, or a cellular immunotherapy.
A98. An *in vitro* method of evaluating susceptibility of a subject to develop Cytokine-Release Syndrome (CRS) to a bispecific T cell engager antibody (BiTE) immunotherapy treatment, comprising:
   (a) providing a sample comprising at least one T cell from a subject having a cancer;
   (b) providing a sample comprising at least one cancer cell, e.g., from the subject;
   (c) forming an *ex vivo* reaction mixture comprising the at least one T cell, the at least one cancer cell, and the bispecific T cell engager antibody (BiTE), being identical to BiTE of the immunotherapy treatment, e.g., under conditions (e.g., for a period of time) sufficient to allow the T cell to kill cancer cells, thereby producing the cancer-killing T cell; and
   (d) determining the pharmacological activity of the cancer-killing T cells obtained in step (c) by dose response and/or pharmacodynamic parameters of cancer-killing T cells and tumor cells, selected from EC50, Emax, AUC, Effective E:T Ratios, Basal E:T Ratios, Basal E:T Ratios, or kinetic parameters;
   (e) determining the expression levels of multiple cytokines in the ex *vivo* reaction mixture, in supernatant and/or intracellular compartments, at basal and several time points; and
   (f) evaluating susceptibility of a subject to develop Cytokine-Release Syndrome, by analyzing the results of (e) cytokine levels as a function of (d) cancer-killing activity, wherein a high expression value of anti-inflammatory cytokines in the sample, relative to (i.e. as a function of) its relative cancer-killing activity compared with other patient samples, is indicative of less susceptibility to develop Cytokine-Release Syndrome or wherein a low expression value of pro-inflammatory cytokines in the sample, relative to (i.e. as a function of) its relative cancer-killing activity compared with other patient samples, is indicative of less susceptibility to develop Cytokine-Release Syndrome.
A99. An *in vitro* method of evaluating susceptibility of a subject to develop Cytokine-Release Syndrome (CRS) to a Cellular therapy such as a CAR-T therapy, comprising:
   (a) providing a sample comprising at least one T cell selected from the group consisting of a tumor infiltrated lymphocyte (TIL), marrow infiltrated lymphocyte (MIL), a genetically engineered T cell, a CAR-T cell, or an activated T cell obtainable according to step (c) of the method of A1 or A2, or step (d) of the method of A3 and a genetically engineered T cell expressing Chimeric Antigen Receptors obtainable according to step (e) of the method of A1 step (f) of the method of A2, or step (g) of the method of A3;
   (b) providing a sample comprising at least one cancer cell from a subject having a cancer;
   (c) forming an *ex vivo* reaction mixture comprising the sample of step (a) and the sample of step (b); e.g., under conditions (e.g., for a period of time) sufficient to allow said T cells to kill cancer cells; and
   (d) determining the pharmacological activity of the cancer-killing T cells obtained in step (c) by dose response and/or pharmacodynamic parameters of cancer-killing T cells and tumor cells, selected from EC50, Emax, AUC, E:T Ratios, , or kinetic parameters;
   (e) determining the expression levels of multiple cytokines in the *ex vivo* reaction mixture, in supernatant and/or intracellular compartments, at basal and several time points; and
   (f) evaluating susceptibility of a subject to develop Cytokine-Release Syndrome, by analyzing the results of (e) cytokine levels as a function of (d) cancer-killing activity, wherein a high expression value of anti-inflammatory cytokines in the sample, relative to (i.e. as a function of) its relative cancer-killing activity compared with other patient samples, is indicative of less susceptibility to develop Cytokine-Release Syndrome or wherein a low expression value of pro-inflammatory cytokines in the sample, relative to (i.e. as a function of) its relative cancer-killing activity compared with other patient samples, is indicative of less susceptibility to develop Cytokine-Release Syndrome.
A100. The method of A98 or A99, wherein the treatment evaluated for susceptibility of a subject to develop Cytokine-Release Syndrome (CRS) is a combination among BiTEs, Cellular Therapies, and other immunotherapies or other non-immuno therapies.
A101. The method of any of A98 to A100, wherein the cytokine is selected from the group consisting of IL-1a, IL1β, IL-1Ra, IL-2, IL-3, IL-4, IL-5, IL6, IL-7, IL-8, IL-9, IL-10, IL-12, IL12p70, IL-13, IL-15, IL-16, IL-17A, IL-17F, IL-18, IL-22, IP10, IFN-y, TNF-α.
A102. The method of any of A98 to A101, wherein the pharmacological parameter is Area Under the Curve (AUC) and levels of cytokine for IL-10 and/or INF-γ, and their relationship is non-linear enabling selection of subjects with high cancer cell killing activity and moderate cytokine release.
A103. The method of any of A98 to A102, wherein the pharmacological parameter is Area Under the Curve (AUC) and levels of cytokine for IL-10 and/or INF-γ, and their relationship is non-linear enabling selection of lower doses for subjects predicted with high cancer cell killing activity and high cytokine release, whereby such lower doses decrease the probability of suffering Cytokine Release Symdrome.
A104. The method of any of A98 to A103, wherein the pharmacological parameter is high Effective E:T Ratio coinciding with high levels of cytokine IL-13, an anti-inflammatory cytokine, indicative of high cancer-killing activity and low probability of cytokine release syndrome.
A105. The method of any of A98 to A104, wherein sequential time measurements identify dependent processes, such as cytokines induced by other cytokines, or short time vs longer time cytokine level variations, where any of these parameters (e.g. shorter time cytokines) may have higher clinical prediction capacity.
A106. The method of any of A98 to A105, wherein the method is performed using an automated fluorescence based platform.
A107. The method of any of A98 to A106, wherein the method is performed using flow cytometry.
A108. The method of any one of A98 to A107, wherein the bispecific T cell engager antibody (BiTE) has a first element providing affinity for the T cell and a second element having affinity for the cancer cell, wherein the first element binds to a T cell and does not bind to a substantial number of cancer cells and wherein the second element binds to a cancer cell and does not bind to a substantial number of T cells.
A109. The method of A108, wherein the first element binding to T cell comprises one or more of the following cell receptors: CD8, CD3, CD4, α/β T cell receptor (TCR), CD45RO, and/or CD45RA.
A110. The method of A108 or A109, wherein the second element binds to one or more of the following cell receptors: CD20, CD28, CD30, CD33, CD52; EpCAM, CEA, gpA33, mucin, TAG-72, carbonic anhydrase IX, PSMA, folate binding protein; one or more of a ganglioside selected from: GD2, GD3, or GM2; Lewis-Y2, VEGF, VEGFR, αVβ3, α5β1, ErbB1/EGFR, ErbB2/HER2, ERbB3, c-MET, IGF1R, EphA3, TRAIL-R1, TRAIL-R2, RANKL, FAP, tenascin, CD123, CD19, and/or BCMA.
A111. The method of any of A98 to A110, wherein the T cell engager antibody (BiTE) is selected from the group consisting of BsMAb CD19/CD3, BsMAb CD20/CD3, BsMAb CD22/CD3, BsMAb CD123/CD3, BsMAb CD33/CD3 BsMAb CD3/CD28, BsMAb BCMA/CD3, and BsMAb EpCAM/CD3.
A112. The method according to any of A98 to A111, wherein Chimeric Antigen Receptors recognize a neoantigen of a cancer cell.
A113. The method of any of A98 to A112, wherein the sample of step (a) and the sample of step (b) are from the same subject.
A114. The method of any of A98 to A113, wherein step (a) and step (b) comprise providing one sample comprising both the cancer cell and the T cell.
A115. The method of any of A98 to A114, wherein the sample (a) is derived from a tissue with a microenvironment, wherein substantially no components have been removed or isolated from the sample, selected from: whole blood, peripheral blood, bone marrow, lymph node, a biopsy of a primary tumor, or a biopsy of a metastasis or spleen.
A116. The method of any of A98 to A115, wherein the subject is an adult or a pediatric subject.
A117. The method of any of A98 to A116, wherein the cancer of sample (b) is a hematological cancer selected from: Hodgkin's lymphoma, Non-Hodgkin's lymphoma (B cell lymphoma, diffuse large B cell lymphoma, follicular lymphoma, chronic lymphocytic leukemia, mantle cell lymphoma, marginal zone B-cell lymphoma, Burkitt lymphoma, lymphoplasmacytic lymphoma, hairy cell leukemia), acute myeloid leukemia, chronic myeloid leukemia, myelodysplastic syndrome, multiple myeloma, or acute lymphocytic leukemia.
A118. The method of any of A98 to A117, wherein the cancer is a solid cancer selected from: ovarian cancer, rectal cancer, stomach cancer, testicular cancer, cancer of the anal region, uterine cancer, colon cancer, rectal cancer, renal-cell carcinoma, liver cancer, non-small cell carcinoma of the lung, cancer of the small intestine, cancer of the esophagus, melanoma, Kaposi's sarcoma, cancer of the endocrine system, cancer of the thyroid gland, cancer of the parathyroid gland, cancer of the adrenal gland, bone cancer, pancreatic cancer, skin cancer, cancer of the head or neck, cutaneous or intraocular malignant melanoma, uterine cancer, brain stem glioma, pituitary adenoma, epidermoid cancer, carcinoma of the cervix squamous cell cancer, carcinoma of the fallopian tubes, carcinoma of the endometrium, carcinoma of the vagina, sarcoma of soft tissue, cancer of the urethra, carcinoma of the vulva, cancer of the penis, cancer of the bladder, cancer of the kidney or ureter, carcinoma of the renal pelvis, spinal axis tumor, neoplasm of the central nervous system (CNS), primary CNS lymphoma, tumor angiogenesis, metastatic lesions of said cancers, or combinations thereof.
A119. The method of any of A98 to A118, wherein the cancer is not melanoma.
A120. The method of any of A98 to A119, wherein the subject providing sample (a) and/or sample (b):
   (i) has not received a prior treatment for the cancer;
   (ii) has received one or more previous treatments for the cancer; or
   (iii) has minimal residual disease (MRD).
A121. The method of any of A1 to A54 or A98 to A120, wherein when the method is applied to samples of solid tumor is performed using 3D cell culture constructs built to mimic the microenvironment architecture of solid tumors, selected from: spheroids, extracellular matrix gels, synthetic scaffolds, rotary cell culture systems, or on low/non-adherent culture plastics
A122. Use of an Artificial Environment (AE) consisting in a plasma fraction, an erythrocyte fraction or a combination thereof, free from leucocytes, in the method of A1 to A54 as one of the components of the ex vivo reaction mixture comprising a least one T cell, at least one cancer cell and a bispecific T cell engager antibody (BiTE).
A123. Use of an Artificial Environment (AE) consisting in a plasma fraction, an erythrocyte fraction or a combination thereof, free from leucocytes, as one of the components in the method of A63 to A121.
A124. An *in vitro* method of producing a genetically engineered T cell expressing Chimeric Antigen Receptors (a CAR-T cell) or a CAR-T cell preparation:
   (a) providing a sample comprising at least one T cell from a subject having a cancer;
   (b) providing a sample comprising at least one cancer cell;
   (c) genetically engineering the T cell to produce Chimeric Antigen Receptors (CAR) on the surface of the T cell, thereby producing at least one CAR-T cell; and
   (d) forming an *ex vivo* reaction mixture comprising the at least one CAR-T cell and the at least one cancer cell under conditions and for a period of time sufficient to allow the at least one CAR-T cell to acquire a surface marker from at least one cancer cell, thereby producing at least one trogocytotic CAR-T cell;
   (e) selecting at least one trogocytotic CAR-T cell having acquired a cell surface marker from at least one cancer cell, thereby obtaining at least one selected trogocytotic CAR-T cell.
A125. The method of A124, wherein said surface marker is a membrane fluorescent dye or a fluorescently labelled antibody.
A126. The method of A124 or A125, wherein the trogocytotic CAR-T cells is a doblet, wherein the doblet is a trogocytotic CAR-T cell attached to a leukemic cell.
A127. The method of A124, further comprising:
   (f) isolating or enriching the at least one selected CAR-T cell using a fluorescently labeled molecule that binds to i) one or more cancer antigens ii) one or more markers of trogocytotic CAR-T cells, or both i) and ii).
A128. The method of A124, comprising:
   (a) providing a sample comprising at least one T cell from a subject having a cancer;
   (b) providing a sample comprising at least one cancer cell, adding a membrane dye or a cell tracker dye;
   (c) genetically engineering the T cell to produce Chimeric Antigen Receptors (CAR) on the surface of the T cell, thereby producing at least one CAR-T cell;
   (d) forming an *ex vivo* reaction mixture comprising the at least one CAR-T cell and the at least one cancer cell labelled with a membrane dye or a cell tracker dye from (b), under conditions and for a period of time sufficient to allow the at least one CAR-T cell to acquire a surface marker from at least one cancer cell, thereby producing at least one trogocytotic CAR-T cell;
   (e) selecting at least one CAR-T cell having acquired a cell surface marker from at least one cancer cell, thereby obtaining at least one selected trogocytotic CAR-T cell; and
   (f) isolating or enriching the selected trogocytotic CAR-T cells using markers for T cells or CAR-T cells, combined with a marker of trogocytotic CAR-T cells.
A129. The method of A124, wherein said marker of trogocytotic CAR-T cells is a membrane dye or a cell tracker dye.
A130. The method of any one of A124-A129, wherein the selecting step (e) of A124 is based on a parameter selected from the group consisting of increased cancer cell killing activity, reduced toxicity, reduced off-target effect, increased viability, increased proliferation and Effective E:T ratio.
A131. The method of any one of A124-A130, wherein the selecting step (e) of A124 comprises using a fluorescently labeled compound that binds to i) one or more cancer antigens, or diffuses into the cancer cell membrane or ii) one or more markers of trogocytotic CAR-T cells, or both i) and ii); or comprises using a bead coated with an antibody or fragment thereof that binds to i) one or more cancer antigens or ii) one or more markers of trogocytotic CAR-T cells, or both i) and ii).
A132. The method of any one of A124-A131, wherein the at least one trogocytotic CAR-T cell or at least one trogocytotic CAR-T cell preparation comprises one or more CD8+ T cells and/or one or more CD25+ T cells, and/or one or more CD8+/CD25+ T cells and/or one or more CD4+/CD25+ T cells, and or one or more cytotoxic T lymphocytes (CTLs) or one or more tumor infiltrating lymphocytes (TILs) or marrow infiltrated lymphocytes (MILs) and/or one or more trogocytotic T cells.
A133. The method of any of A124-A132, wherein the ex *vivo* reaction mixture further comprises one or multiple agents that enhance T cell activity.
A134. The method of A133, wherein the agent that enhances T cell activity is selected from the group consisting of a chemotherapy drug, a targeted anti-cancer therapy, an oncolytic drug, a cytotoxic agent, an immune-based therapy, a cytokine, an agonist of T cells, agonistic antibody or fragment thereof, an activator of a costimulatory molecule, an inhibitor of an inhibitory molecule, an inhibitor of an immune checkpoint inhibitor, an immunomodulatory agent and a vaccine.
A135. The method of A134, wherein the inhibitors of the immune checkpoint inhibitor is an inhibitor from the group consisting of PDL-1, PDL-2, B7-1 (CD80), B7-2 (CD86), 4-1BBL, Galectin, ICOSL, GITRL, OX40L, CD155, B7-H3, PD1, CTLA-4, 4-1BB, TIM-3, ICOS, GITR, LAG-3, KIR, OX40, TIGIT, CD160, 2B4, B7-H4 (VTCN1), HVEM (TNFRSF14 or CD270), BTLA, KIR, MHC class I, MHC class II, GAL9, VISTA, LAIR1, and A2aR.
A136. The method of A134, wherein the inhibitors of the immune checkpoint inhibitor comprise one or more from the group consisting of ipilimumab, tremelimumab, MDX-1106, MK3475, CT-011, AMP-224, MDX-1105, IMP321 and MGA271.
A137. The method of any of A133 or A134, wherein the agents that enhances T cell activity comprises molecules constructed combining fragments of these molecules enhancing T cell activity, antibodies constructed combining fragments of these antibodies enhancing T cell activity, bispecific or multispecific antibodies combining recognition arms of several immune checkpoint inhibitors selected from the group consisting of PD1-PDL1, PD1-PDL2, PD1-LAG3 and PD1-TIM3.
A138. The method of A134, wherein the agonist of T cells comprises an antibody or fragment thereof to CD137, CD40, and/or glucocorticoid-induced TNF receptor (GITR).
A139. The method of A134, wherein the immunomodulatory agent comprises one or more of the group consisting of lenalidomide, ibrutinib and bortezomib.
A140. The method of A133, wherein the agent that enhances T cell activity enhances and/or restores the immunocompetence of T cells.
A141. The method of A134, wherein the immunomodulatory agent is an inhibitor of MDSCs and/or Treg cells.
A142. The method of A134, wherein the immunomodulatory agent activates an immune response to a tumor specific antigen.
A143. The method of A134, wherein the immunomodulatory agent is a vaccine against targets selected from the group consisting of gp100, MUC1 and MAGEA3.
A144. The method of A134, wherein the immunomodulatory agent is a cytokine, or a recombinant cytokine selected from the group consisting of GM-CSF, IL-7, IL-12, IL-15, IL-18 and IL-21.
A145. The method of A134, wherein the immunomodulatory agent is a modulator of a component (e.g., enzyme or receptor) associated with amino acid catabolism, signalling of tumor-derived extracellular ATP, adenosine signalling, adenosine production, chemokine and chemokine receptor, recognition of foreign organisms, or kinase signalling activity.
A146. The method of A134, wherein the immunomodulatory agent is selected from the group consising of an inhibitor of IDO, COX2, ARG1, ArG2, iNOS, phosphodiesterase or PDE5; a TLR agonist; and a chemokine antagonist.
A147. The method of any one of A124-A146, wherein the selecting step (e) of A124 or A126 and/or the enriching step (f) of A125 or enriching step (f) of A126 comprises using fluorescence activated cell sorting (FACS).
A148. The method of any one of A124-A147, further comprising evaluating the activity of the at least one selected trogocytotic CAR-T cell.
A149. The method of A148, wherein evaluating comprises:
   (a) providing a CAR-T cell or a CAR-T cell preparation thereof obtainable according to the method of any of A124-A148;
   (b) providing a sample of cancer cells, wherein the cancer cells are from the same subject;
   (c) contacting the CAR-T cell or the CAR-T cell preparation thereof with the cancer cells for a period of time sufficient to allow the CAR-T cell to kill the cancer cells;
   (d) determining the level of cancer cells after step (c), and optionally determining the level of CAR-T cells after step (c); and optionally,
   (e) determining the ratio of either cancer cell to CAR-T cell, or CAR-T cell to cancer cell, from step (d).
A150. The method of any of A124-A149, further comprising
   (i) separating selected CAR-T cells into single CAR-T clones and
   (ii) evaluating the activity of the single CAR-T clones,
   (iii) expanding the single CAR-T clones to generate one or more preparations of expanded CAR-T clones.
   (iv) selecting an expanded CAR-T clone, wherein the selected expanded CAR-T clone is defined by having an Effective E:T Ratio higher than 1:5 between the number of cells of the CAR-T clone (E) and the number of target cancer cells (T).
A151. The method of any one of A124-A150, wherein the sample of step (a) and the sample of step (b) of A124 are from the same subject.
A152. The method of any one of A124-A151, wherein step (a) and step (b) of A124 comprise providing one sample comprising both the at least one cancer cell and the at least one T cell.
A153. The method of any one of A124-A152, wherein the sample of step (a) of A124 is selected from: whole blood, peripheral blood, bone marrow, lymph node, spleen, a primary tumor and a metastasis.
A154. The method of any one of A124-A153, wherein the sample of step (a) of A124 is derived from a tissue with a microenvironment, wherein substantially no components have been removed or isolated from the sample.
A155. The method of any one of A124-A154, wherein the subject is an adult or a pediatric subject.
A156. The method of any one of A124-A155, wherein the cancer of the sample of step (b) of A124 is a hematological cancer selected from: Hodgkin's lymphoma, Non-Hodgkin's lymphoma (B cell lymphoma, diffuse large B cell lymphoma, follicular lymphoma, mantle cell lymphoma, marginal zone B-cell lymphoma, Burkitt lymphoma, lymphoplasmacytic lymphoma, hairy cell leukemia), acute myeloid leukemia, chronic myeloid leukemia, myelodysplastic syndrome, multiple myeloma, chronic lymphocytic leukemia and acute lymphocytic leukemia.
A157. The method of any one of A124-A155, wherein the cancer is a solid cancer selected from: ovarian cancer, rectal cancer, stomach cancer, testicular cancer, cancer of the anal region, uterine cancer, colon cancer, rectal cancer, renal-cell carcinoma, liver cancer, non-small cell carcinoma of the lung, cancer of the small intestine, cancer of the esophagus, melanoma, Kaposi's sarcoma, cancer of the endocrine system, cancer of the thyroid gland, cancer of the parathyroid gland, cancer of the adrenal gland, bone cancer, pancreatic cancer, skin cancer, cancer of the head or neck, cutaneous or intraocular malignant melanoma, uterine cancer, brain stem glioma, pituitary adenoma, epidermoid cancer, carcinoma of the cervix squamous cell cancer, carcinoma of the fallopian tubes, carcinoma of the endometrium, carcinoma of the vagina, sarcoma of soft tissue, cancer of the urethra, carcinoma of the vulva, cancer of the penis, cancer of the bladder, cancer of the kidney or ureter, carcinoma of the renal pelvis, spinal axis tumor, neoplasm of the central nervous system (CNS), primary CNS lymphoma, tumor angiogenesis, metastatic lesions of said cancers, or combinations thereof.
A158. The method of any one of A124-A157, wherein the subject providing the sample of step (a) and/or the sample of step (b) of A124:
   (i) has not received a prior treatment for the cancer;
   (ii) has received one or more previous treatments for the cancer; or
   (iii) has minimal residual disease (MRD).
A159. A composition comprising a CAR-T cell or CAR-T cell preparation thereof obtainable according to the method of any of A124-A158.
A160. A pharmaceutical composition comprising the composition of A159 and a pharmaceutically acceptable carrier.
A161. The pharmaceutical composition according to A160 for use in Adoptive Cancer Therapy for treating a subject, wherein the subject is the same subject as that of step (a) of A124, and/or wherein the subject is the same subject as that of step (b) of A124, and/or wherein the subject is different from the subject as that as step (a) or (b) of A124.
A162. The pharmaceutical composition for use according to A161 in Adoptive Cancer Therapy for treating a subject suffering (i) an hematological cancer selected from: Hodgkin's lymphoma, Non-Hodgkin's lymphoma (B cell lymphoma, diffuse large B cell lymphoma, follicular lymphoma, mantle cell lymphoma, marginal zone B-cell lymphoma, Burkitt lymphoma, lymphoplasmacytic lymphoma, hairy cell leukemia), acute myeloid leukemia, chronic myeloid leukemia, myelodysplastic syndrome, multiple myeloma, chronic lymphocytic leukemia or acute lymphocytic leukemia, or (ii) a solid cancer selected from: ovarian cancer, rectal cancer, stomach cancer, testicular cancer, cancer of the anal region, uterine cancer, colon cancer, rectal cancer, renal-cell carcinoma, liver cancer, non-small cell carcinoma of the lung, cancer of the small intestine, cancer of the esophagus, melanoma, Kaposi's sarcoma, cancer of the endocrine system, cancer of the thyroid gland, cancer of the parathyroid gland, cancer of the adrenal gland, bone cancer, pancreatic cancer, skin cancer, cancer of the head or neck, cutaneous or intraocular malignant melanoma, uterine cancer, brain stem glioma, pituitary adenoma, epidermoid cancer, carcinoma of the cervix squamous cell cancer, carcinoma of the fallopian tubes, carcinoma of the endometrium, carcinoma of the vagina, sarcoma of soft tissue, cancer of the urethra, carcinoma of the vulva, cancer of the penis, cancer of the bladder, cancer of the kidney or ureter, carcinoma of the renal pelvis, spinal axis tumor, neoplasm of the central nervous system (CNS), primary CNS lymphoma, tumor angiogenesis, metastatic lesions of said cancers, or combinations thereof.
A163. A method for treating a subject having cancer comprising providing a CAR-T cell or a CAR-T cell preparation thereof obtainable according to the method of any one of A124-A158, the composition of A159 or the pharmaceutical composition of A160, and administering an effective amount of the CAR-T cell, the CAR-T cell preparation, composition or pharmaceutical composition to the subject.
A164. The method of A163, comprising:
   (a) providing a sample from the subject, wherein the sample comprises a T cell and a cancer cell;
   (b) genetically engineering the T cell to produce Chimeric Antigen Receptors (CAR) on the surface of the T cell, thereby producing at least one CAR-T cell; and
   (c) forming an *ex vivo* reaction mixture comprising the at least one CAR-T cell and the at least one cancer cell under conditions and for a period of time sufficient to allow the at least one CAR-T cell to acquire a surface marker from at least one cancer cell, thereby producing at least one trogocytotic CAR-T cell;
   (d) selecting at least one trogocytotic CAR-T cell having acquired a cell surface marker from at least one cancer cell, thereby obtaining at least one selected trogocytotic CAR-T cell.
   (e) administering an effective amount of the selected trogocytotic CAR-T cells to the subject.
A165. The method of any of A163 or A164, further comprising administering to the subject a second therapeutic agent or procedure.
A166. The method of A165, wherein the second therapeutic agent or procedure is selected from the group consisting of chemotherapy, a targeted anti-cancer therapy, an oncolytic drug, a cytotoxic agent, an immune-based therapy such as immune check point inhibitors, a cytokine, a surgical procedure, a radiation procedure, an agonist of T cells, an agonistic antibody or fragment thereof or an activator of a costimulatory molecule, an inhibitor of an inhibitory molecule, an inhibitor of an immune checkpoint inhibitor, an immunomodulatory agent, a vaccine and a cellular immunotherapy.
A167. An *in vitro* method of identifying subjects susceptible to immune checkpoint immunotherapy treatment to be combined with a cellular immunotherapy such a CAR-T to treat a subject, for decreasing resistance of said subject to said cellular immunotherapy, comprising:
   (a) providing a sample comprising selected trogocytotic CAR-T cells of A124;
   (b) providing a cancer cell from a subject having a cancer;
   (c) forming an *ex vivo* reaction mixture comprising (a) and (b), under conditions and for a period of time sufficient to allow the selected trogocytotic CAR-T cells to kill cancer cells;
   (d) determining the pharmacological activity of the selected trogocytotic CAR-T cells by dose response and/or pharmacodynamic parameters of the selected trogocytotic CAR-T cells and tumor cells, selected from EC50, Emax, AUC, Effective E:T Ratios, Basal E:T Ratios, or kinetic parameters;
   (e) determining the pharmacological activity of the selected trogocytotic CAR-T cells by dose response or evaluating a single high saturating dose in combination with immune check point inhibitors, individually, or in combinations, or bispecific or multispecific antibody constructs combining immune check point inhibitors, including the combination of all immune check point inhibitors, either by full dose responses or evaluating a single high saturating dose;
   (f) determining the expression levels of immune checkpoint molecules in both the tumor cells and the selected trogocytotic CAR-T cells in the reaction mixture of step (c), comparing basal levels with levels after incubation,
   (g) identifying subjects susceptible to immune checkpoint immunotherapy treatment in combination with the cellular therapy, by assessment of either of the following 2 criteria or a combination of them:
      i. step (d) reveals a resistant tumor cell population in the samples from the subject and addition of one or more immuno checkpoint inhibitors in (e) reverts resistance of tumor cell population;
      ii. step (f) reveals an increase in the expression level of an immune checkpoint molecule in either the tumor cells and/or T cells in the reaction mixture of step (c) after incubation, relative to basal levels prior incubation,
   and wherein observance of both (i) and (ii) is indicative of a subject more susceptible to immune checkpoint immunotherapy treatment to be combined with a cellular immunotherapy.
A168. An *in vitro* method of evaluating susceptibility of a subject to develop Cytokine-Release Syndrome (CRS) to a Cellular therapy such as a CAR-T therapy, comprising:
   (a) providing a sample comprising selected trogocytotic CAR-T cells of A124;
   (b) providing a sample comprising at least one cancer cell from a subject having a cancer;
   (c) forming an *ex vivo* reaction mixture comprising (a) and (b), under conditions and for a period of time sufficient to allow the selected trogocytotic CAR-T cells to kill cancer cells;
   (d) determining the pharmacological activity of the selected trogocytotic CAR-T cells by dose response and/or pharmacodynamic parameters of the selected trogocytotic CAR-T cells and tumor cells, selected from EC50, Emax, AUC, survival, basal E:T Ratios, Effective E:T ratios or kinetic parameters;
   (e) determining the expression levels of multiple cytokines in the ex *vivo* reaction mixture, in supernatant and/or intracellular compartments, at at least one high dose or with multiples doses, at basal and several time points; and
   (f) evaluating susceptibility of a subject to develop Cytokine-Release Syndrome, by analyzing the results of (e) cytokine levels as a function of (d) cancer-killing activity, wherein a high expression value of anti-inflammatory cytokines in the sample, relative to its relative cancer-killing activity compared with other patient samples, is indicative of less susceptibility to develop Cytokine-Release Syndrome or wherein a low expression value of pro-inflammatory cytokines in the sample, relative to its relative cancer-killing activity compared with other patient samples, is indicative of less susceptibility to develop Cytokine-Release Syndrome.
A169. The method of A168, wherein the dose response curves of the level of cytokines at different time points, for multiple cytokines, as a function of cancer-killing activity, is fitted to a multivariate mathematical function that predicts the probability that the patient may develop clinical Cytokine-Release Syndrome.
A170. The method of A169, wherein instead of a dose response curve a single high concentration is used.
A171. The method of any one of A168-A170 where the cytokines evaluated are NKG2A, IL-2, IL-4, IL-10, IL-6, IL-17A, TNF-α, sFas, sFasL, IFN-y, granzyme A, granzyme B, perforin and granulysin.
A172. The method of any one of A168-A171 where the cytokines evaluated are granulosin, Granzyme A, Granzyme B, IL-10, IL-17A, perforin, sFAS, sFASL and TNF-a.
A173. The method of any one of A168-A172, wherein the method predicts patients with an appropriate balance of activity versus toxicity in terms of CRS and wherein the prediction is based on a Precision Medicine Test for CAR-T treatments.
A174. The method of A173, wherein the prediction is based on selecting thresholds for extreme profiles without any clinical correlation to validate said thresholds, classifying patient samples into extremes (e.g. 10-20%) of very high activity, or very los activity, and very high probability of CRS, or very low probability of CRS.
A175. The method of A174, wherein the prediction is based on a clinical correlation between the ex vivo results and the clinical outcomes of the patients, resulting in a mathematical function and/or algorithm that assigns for every patient sample a probability of developing CRS and being responsive to the CAR-T treatment.
A176. The methods of any one of A174-A175, wherein optimal CAR-T doses are also recommended for the individual patient.
A177. The method of A176, wherein the patient would develop CRS and wherein a lower dose is recommended to said patient, wherein at said recommended dose said patient have a lower probability of developing CRS and preserves an acceptable activity.
A178. The method of any one of A98, A100-A106, wherein the method predicts patients with an appropriate balance of activity versus toxicity in terms of CRS and wherein the prediction is based on a Precision Medicine Test for BiTE treatments.
A179. The method of A178, wherein the prediction is based on selecting thresholds for extreme profiles without any clinical correlation to validate said thresholds, classifying patient samples into extremes (e.g. 10-20%) of very high activity, or very los activity, and very high probability of CRS, or very low probability of CRS.
A180. The method of A179, wherein the prediction is based on a clinical correlation between the ex vivo results and the clinical outcomes of the patients, resulting in a mathematical function and/or algorithm that assigns for every patient sample a probability of developing CRS and being responsive to the BiTE treatment.
A181. The methods of any one of A178-A180 wherein optimal BiTE doses are also recommended for the individual patient.
A182. The method of A181, wherein the patient would develop CRS and wherein a lower dose is recommended to said patient, wherein at said recommended dose said patient have a lower probability of developing CRS and preserves an acceptable activity.

Some of these methods select highest activity fractions/clones of CAR-T cells or to evaluate patient responsiveness to CAR-T alone or combined with other cancer therapies can also be applied to normal CAR-T using other types of T cells than BiTE-activated T cells.

In an embodiment, the BiTE-activated T cells represent normal, standard T cells, such as those commonly used to make CARTs. An example is peripheral blood (PB) T cells, the most common source of T cells for CARTs. These CART-PB could be such as those described in the Examples, where the same type of T cell is present in PB and BM of the same patient and thus the same T cell type can be present in CART-PB and CART-ICT (derived from BM). This is likely to occur because BiTE activates all types of T cells by proximity to the tumor cell, and transduction of a CAR into a T cell can be performed in either resting (standard method) or activated (e.g. BiTE-activated) T cells. Therefore, in an embodiment the CART can be a standard CART.

In an embodiment, the cancer-killing T cell is a CART generated on a tumor-specific antigen T cell. In another embodiment, the cancer-killing T cell is a standard CART on a standard type of T cell, such as PB T cells.

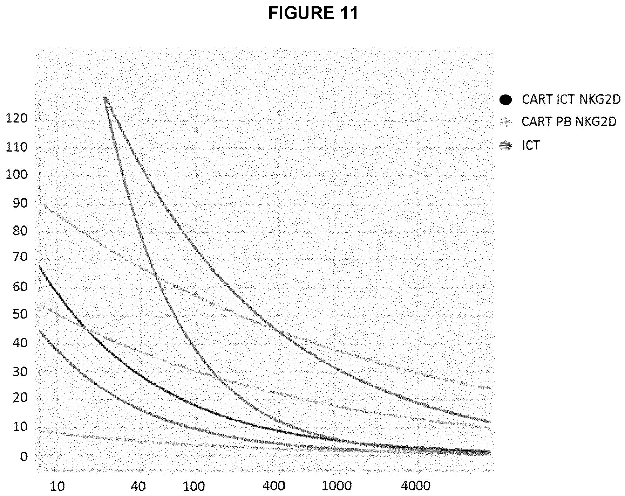

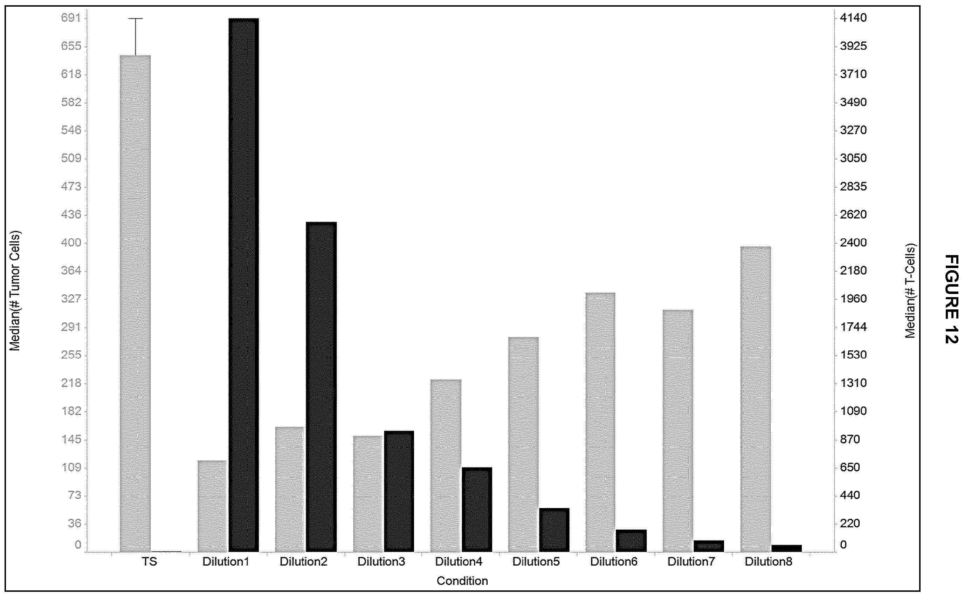

In another embodiment, the trogocytotic CART cells include singlets and doblets, as defined in the Examples 6 and 7, shown in Figures 11 and 12. Interestingly, Figure 11 shows that among the trogocytotic CART cells there is a substantial population of doblets, representing a leukemic cell attached to a CART cell. Panel C shows a forward scatter vs pulse width plot where doblets are identified as the vertical group of dots shifted to the right. Doblets presumably arise when the CART-CD19 forms an immune synapse with the leukemic cell, after which the T cell delivers the toxic cytokines to the intracellular component of the leukemic cell which kills it by cell lysis. On the contrary, Example 7 Figure 12 shows another CART on an AML sample where most trogocytotic CARTs are singlets not doblets. Thus, singlets or doblets may be detected depending on factors such as sample, CART type, effector:target (T cell to tumor cell) ratios, cell density, etc.

In another embodiment, the cancer-killing T cell is a CART generated on a tumor-specific antigen T cell. In another embodiment, the cancer-killing T cell is a standard CART on a standard type of T cell, such as PB T cells. Example 15 shows the use of Effective E:T Ratios to analyze the relationship of cytokines in supernatant vs activity of these normal CART-NKG2D on PB T Cells. Notably, this example shows that using the more standard AUC (Area Under the Curve) values there is much less correlation between CART activity and cytokine secretion than using Effective E:T Ratios. This example shows how to apply Effective E:T Ratios to normal CART activity assessment.

The PM Test ex vivo for standard of care chemotherapy drugs and their combinations is described below for reference; this is the description included as Annex to every patient report. An equivalent development shall be performed for CARTs, and add it to the chemotherapy drugs PM Test described below in 4 sections; Introduction, Fundamentals of the cellular assay, Methodology, Description and interpretation of the results.

### 1. Introduction.

The purpose of this description is to provide basic information about the PharmaFlow PM test for specialist physicians, summarizing its fundamentals and the essential features of the methodology used, as well as the scope and limitations of the results provided by PharmaFlow PM. More extensive and detailed information can be found in the following peer reviewed publication relating to the PharmaFlow PM: "Pharmacological Profiles of Acute Myeloid Leukemia treatments in patient samples by automated flow cytometry; a bridge to individualized medicine", published in 2013 in the publication Lymphoma, Myeloma & Leukemia.

One of the differentiating factors of the Pharma Flow test is the "Native Environment" element, in which the whole bone marrow sample is used. Specifically, the sample is incubated for 72 hours with the monotherapy drugs and combinations of treatment protocols, enabling a realistic ex-vivo analysis. The PM test can identify patients as sensitive or resistant to anthracyclines because it measures individualized efficacy of the anthracyclines rather than average efficacy. Historically, treatment has deemed anthracyclines comparable in their efficacy because they perform similarly when clustered into amorphous averages. Thus, because 30% of patients exhibit an extreme response (very sensitive or very resistant), the PM test can help identify the appropriate treatment. The promising results suggested by the theory translate in practice to real results. PharmaFlow PM achieved high clinical correlation in 1st line AML patients treated with CYT+IDA, demonstrating the effectiveness of the test. Figure 1 shows clinical correlation achieved by the PM Test for 1st line CYT+IDA in AML.

Pharma Flow PM AML, for the treatment of Acute Myeloid Leukemia ("AML"), is a Laboratory Developed Test (LDT) that consists of analyzing, directly in a patient's bone marrow sample, the effect of monotherapy drugs and combinations of treatment protocols that are regularly used in clinical practice for the treatment of the disease. PharmaFlow PM analyzes the pharmacological effect (in terms of dose-response) of these treatments in the pathological cells of the patient's fresh, recently extracted, bone marrow sample. In doing so, the test generates a complete pharmacological profile for the individual patient. PharmaFlow PM analyzes the efficacy of the treatments by measuring "cellular depletion" of leukemic cells induced by the given monotherapy or combination treatment.

Through this method, as further detailed below, PharmaFlow PM identifies treatments to which the patient's cellular response is particularly sensitive or resistant in comparison to the response of the representative patient population to the same treatment. This helps the specialist to identify, prior to treatment, potentially effective therapeutic options.

### 2. Fundamentals of the cellular assay.

PharmaFlow, analyzes the response (in terms of cell sensitivity and synergistic effect of drugs) of the leukemic cells in a sample of bone marrow taken from an AML patient to several drugs and drug combinations. The patient's "ex vivo" pharmacological profiles, which are generated according to the test specifications and methodology, identify the drugs and combinatorial treatments to which the patient's pathological cells are especially sensitive or resistant, by comparing to the cellular response to the same drugs and treatments of the representative patient population in which the same test has been previously performed. Thus, the test provides a new, potentially useful tool to inform and provide support to physicians in their treatment decision.

### 2.1. Methodology

### Flow Cytometry

Flow cytometry is the method chosen for the diagnosis and monitoring of patients with hematological malignances. Additionally, it has been validated for the study of cellular death or apoptosis processes induced by drugs. The PharmaFlow Test allows the escalation of flow cytometry technology, with the ability to measure the effect of a high number of drugs and combinations selectively in pathological cells (identified in a similar manner than in the diagnosis of the disease) of an individual patient's sample.

To perform the PharmaFlow PM test, the patient's bone marrow sample is received, and a small aliquot is first analyzed to determine the number of live leukemic cells (LLC) present in the sample. The rest of the sample is diluted with a culture medium, and is divided into 96 well plates, containing the drug treatments (monotherapies and combinations) to be studied. 8 concentrations are studied for each treatment (drug or combination), adjusted to cover each treatment's range of pharmacological activity as tested in multiple patient samples. The plates are later incubated at 37°C and 5% CO2 for 72 hours. Subsequently, the sample is marked with the specific monoclonal antibodies to identify the leukemic cells, together with Anexin V. The presence of this last marker indicates that the cell has entered into apoptosis or programmed death. Therefore, cells that present the phenotype of a leukemic cell and the absence of Anexin V are identified as LLC.

Final output from flow cytometry analysis consists of an accurate count of LLC on each individual well position in the plate. The effect of each drug concentration or combination mixture is primarily estimated from the number of LLC that remains after incubation. This analyte is used further on in the pharmacological analysis of drugs or combinations effect.

### Pharmacodynamic population modelling.

PharmaFlow PM incorporates modern pharmacokinetic and pharmacodynamic population modelling technologies, increasingly used in clinical trials for new drugs, to analyze the test's flow cytometry data. This yields very accurate estimates in complex multiple-variable systems subject to high variability. In the case at hand, by using this technology, PharmaFlow PM generates dose-response models that evaluate the patient's cellular response to increasing drug concentrations in the patient's bone marrow sample, measured as cellular death or depletion. The final model estimated is characterized by a set of pharmacological parameters that describe the effect of the drug or combination.

In addition to the estimation of these parameters, population models offer the analysis of typical population values to put the patient's individual data in context of a patient population, inter-individual variability data associated to each parameter, and relative standard error individually associated to each estimation. Figure 2 illustrates how an individual's performance can be contextualized within a statistically representative population. The graph shows how an individual who requires lower concentrations of cytarabine to lower the number of LLC can be labeled as sensitive to cytarabine, while the inverse can be evaluated as resistant. The ability to compare responses offers an additional tool to select the appropriate treatment for an individual.

The following section describes how this information is set out and used in this pharmacological profile report of the PharmaFlow PM test.

### 2.2. Description and interpretation of results.

PharmaFlow PM generates a report of the ex-vivo activity of single drugs agents and combinations which are regularly used in clinical practice for the treatment of AML.

Graphically, pharmacodynamics models based on the Hill equation are represented by typical sigmoidal curves of measured effect at increasing drug concentrations. These graphs allow a quick interpretation of drug biological effect and a direct comparison with population typical behavior. Individual model functions can be summarized with the value of the Area Under the Curve (AUC) that it is used as a general activity marker (Figure 3).

Treatments scores are calculated using normalized values of the AUC from dose-response model functions of each individual drug included in a clinical treatment, together with the contribution of the synergy from binary combinations which is estimated from sophisticated drugs interaction surface models.

Normalization is assessed with respect to a reference activity range of the population results stored in the database. This is a key aspect of the PharmaFlow PM test as the interpretation of the ex-vivo activity of individual drugs in a patient sample is not just based on the absolute value of the pharmacological parameters, but their reference to a statistically representative patient population.

The PharmaFlow PM test classifies treatments in 5 categories using a color scale range from higher to lower ex vivo activity. The classification is based on the score mentioned above and is done separately for treatments with different numbers of drugs included. The classification includes a lineal factor to compensate the lower probability of getting highest scores for treatments with higher number of drugs.

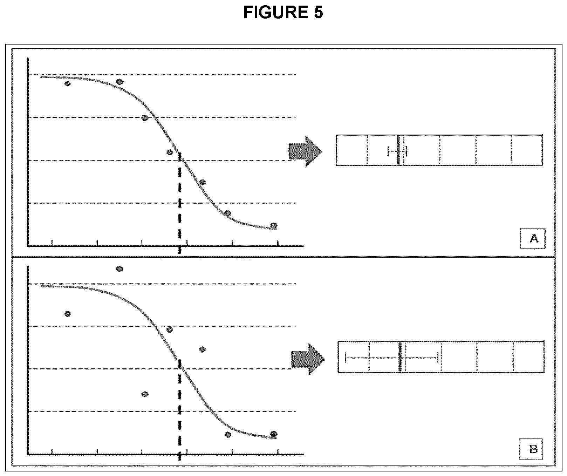

The whole score range (0-100%) is split in 5 parts of 20 points each. Treatments that show an extreme profile of activity are highlighted with a green color for the more sensitive and red color for the extreme resistant cases. 3 different intensities of orange are used for those falling in the intermediate range. Finally, treatments that appear in grey color on the ranking above, are treatments that for different reasons, could either not be assayed or the results obtained are outside confident levels to be reported. Figure 5 shows differences in residual error of model fitting and how it is graphically displayed in horizontal error bars.

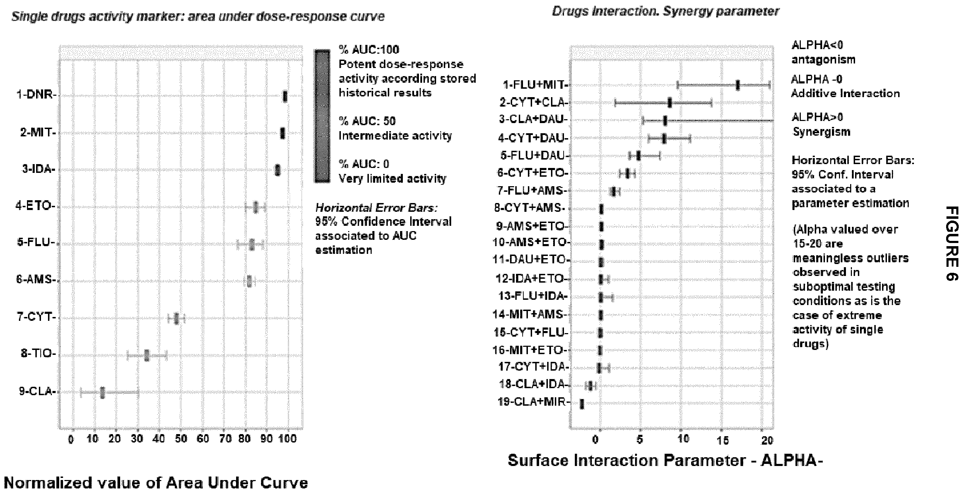

The report includes a section of detailed results on page 4 where individual drug results and synergy parameter values are graphically displayed together with associated confidence interval. The estimation of accurate residual errors and confidence intervals associated with the parameters, allows for the application of quality control criteria to the results provided by the test. Thus, estimations associated to high error levels are automatically discarded. Figure 6 shows a case example of result details section showing individual drugs activity marker (AUC) and confidence interval on the right side and synergy parameter values (alpha) on the right chart also together with associated confidence intervals. The left portion of Figure 6 illustrates how a patient can be sensitive to some drugs and resistant to others. In the example above, the patient is sensitive to the ones marked green as the test yielded a potent dose-response curve and resistant to the ones in red because the test showed very limited activity of the drug lowering the number of LLC. The right side of figure 6 shows the synergy of combinations, which refers to the efficacy of the drugs being used together for the patient.

It is key to point out that the ex vivo evaluation performed by PharmaFlow PM does not directly correspond to clinical activity in the patient, and no direct clinical translation or direct correlation of the test results with the patient's clinical outcome is claimed or shall be necessarily interpreted or assumed of the results of the test. The test only reports the efficacy of the treatments on the leukemic cells of the patients in the bone marrow sample, as described above. Although this is a key factor for the efficacy of the treatment, it does not always directly translate in efficacy in the patient. The reason for that is that several important factors affecting drug efficacy in the patient are not and cannot be taken into account ex vivo by PharmaFlow PM, such as treatment activity pattern in time, the pharmacokinetics of absorption, distribution, metabolism and excretion, among other things that may impact the drug's efficacy in vivo (i.e.: in the patient).

For this reason, although PharmaFlow PM provides the specialist with all the results regarding the patient's response to all tested treatments, recommendations are solely based on the ex vivo activity of treatments that show an extreme profile compared to the population, i.e., extremely sensitive or extremely resistant. The reason for that is that these extreme values of ex vivo activity have a greater likelihood of correlating with clinical activity in the patient, because without considering the other factors affecting drug efficacy mentioned above, when the ex vivo activity shows an extreme profile it is more likely to prevail over these other factors than non-extreme profiles.

Consequently, the test will recommend treating the patient with green color treatment options, and the avoidance of red color treatments. Treatments ranked orange have average efficacy (not extreme profiles), and consequently the test information is deemed to be less reliable in these cases, as other factors are more likely to prevail over the cellular efficacy.

Likewise, providing physicians with information with respect to all treatments, enables them to make adequate decisions among the listed or recommended treatments, based on the actual status and profile of their patient, as, for example, some fragile patients may not tolerate well some treatments that show high ex vivo efficacy.

In an embodiment, the number and tumor-killing activity of trogocytotic T cells, separate from other T cells in the mixture, is considered instead of total T cells for ther embodmients mentioned above regarding ICHKs. Where trogocytotic means those T cells that acquire fluorescent probes from the tumor cells, either antibodies or membrane dyes, including without limitation singlets and doublets as described in Examples 6 and 7. In an embodiment, trogocytotic T cells are isolated (e.g. by FACS sorting) and their tumor-killing activity measured independently of other T cells in the mixture, as shown in Example 8. In another embodiment, the combination with ICHK measures the increase in numbers and/or increase in tumor-killing activity (e.g. the Effective E:T Ratio) of trogocytotic T cells.

In an embodiment the T cells that represents a cell therapy is not a CART cell. In another embodiment the T cells that represents a cell therapy are ICT (Immuno coaching T cells, i.e. a BiTE-activated T cell). In another embodiment the T cells that represents a cell therapy are Tumor-specific antigen T cells. In another embodiment the T cells that represents a cell therapy are selected by surface markers such as CD4, CD8, CD25, CD69, NKG2D. In another embodiment the T cells that represents a cell therapy are CD8+ and NKG2D+ and CD25+.

In embodiments, the selecting and/or enriching step comprises using fluorescence activated cell sorting (FACS) to isolate trogocytotic T cells.

In embodiments, the CAR-T cell or preparation comprises one or more NKG2D T cells. In embodiments, the CAR-T cell or preparation comprises one or more trogocytotic T cells.

In embodiments, the separating step comprises isolation of trogocytotic CAR-T cells. In embodiments, the separating step comprises isolation of trogocytotic CAR-T cells that contain the CART clones with higher tumor-killing activity.

In embodiments, the CAR-T cell preparation comprises cells that effectively kill cancer cells at a high target cell per T cell whereby the T cell counted is only a trogocytotic T cell.

In embodiments the CAR-T cell purified, sorted, enriched, expanded, and/or selected are trogocytotic CAR-T cells.

In embodiments the tumor cells are labelled with a fluorochome that enbales measuring trogocytotic CAR-T cells as a measure of tumor-killing activity.

In another embodiment a PM Test ex vivo can be developed by evaluation of the tumor-killing activity of these drugs and combinations mentioned above. In an embodiment the PM Test ex vivo can follow the methodology and format of the PM Test ex vivo for AML chemotherapy described above.

In embodiments, the CAR-T cell is a T cell, e.g., a cytotoxic T lymphocyte, e.g., a CD8+ T cell e.g. a NKG2D+ T cell.

In embodiments the CAR-T cells are trogocytotic CAR-T cells, purified away from other cells in the mixture.

In embodiments, among the trogocytotic CART cells there can be a substantial population of doblets, representing a leukemic cell attached to a CART cell. Example 6 Figure 11 Panel C shows a forward scatter vs pulse width plot where doblets are identified as the vertical group of dots shifted to the right. Doblets presumably arise when the CART-CD19 forms an immune synapse with the leukemic cell, after which the T cell delivers the toxic cytokines to the intracellular component of the leukemic cell which kills it by cell lysis. It is interesting that trogocytotic markers also include doblets, since both classes of CART cells are supposed to include the best tumor-killing CARTs cells. In embodiments, most trogocytotic CART cells are singlets, as described for a NKG2D CART in AML in example 7 Figure 12 right panel.

In embodiments, selection, purification, and/or enrichment of trogocytotic CAR-T cells may include doblets formed by a CAR-T cell attached to a cancer cell with cancer cell markers.

In embodiments, the CAR-T cells are not expanded and are administered directly to patients without expansion.

In another embodiments CAR-T cells comprises a detectable amount of a immunomodulatory agent such as immune check point antibodies.

In certain embodiments, the pharmaceutical composition comprises a detectable (e.g., trace) amount of an immunomodulatory agent, e.g., immune check point inhibitor (ICHK) antibodies described herein. In embodiments, the ICHK is present at a concentration of less than 10% by weight, e.g., less than 10%, 9%, 8%, 7%, 6%, 5%, 4%, 3%, 2%, 1%, 0.5%, 0.1%, 0.05%, 0.01%, or less by weight (e.g., but no less than 0.0001% by weight).

The term "Effective E:T Ratio" represents a T cell cancer-killing ratio, while the term "basal E:T ratio merely reflects an initial stoichiometry of a patient sample without any relationship to the actual activity of said T cell in killing said cancer cells".

Example 1 describes generating CAR-T on BiTE-activated T cells.

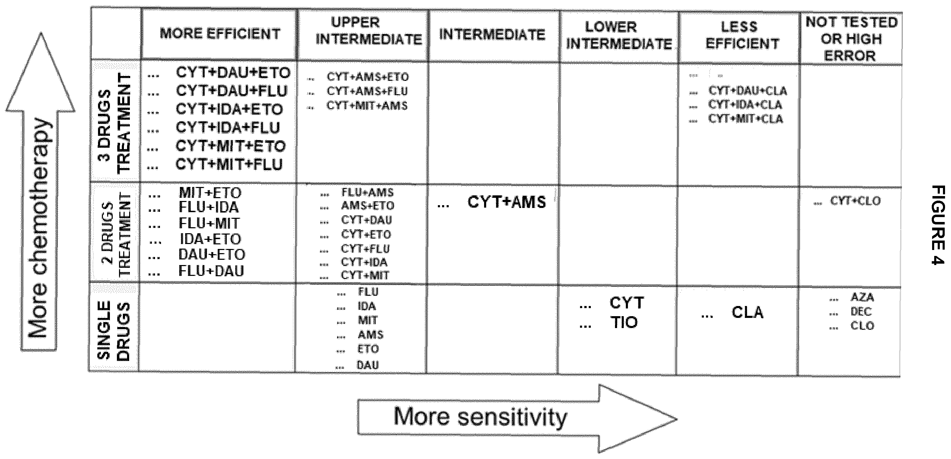

Examples 2-5 describe different methods for testing cellular responsiveness of primary cell populations to cellular immunotherapies such as CAR-Ts. Examples 2 and 3 describe measurement of the efficacy and activity of CART-ICT on B cell malignancies and AML, respectively. Example 4 describes measurement of the efficacy and activity of CART cells of NKG2D in a solid tumor, melanoma. Example 5 describes the development of a Precision Medicine Test ex vivo for a NKG2D CART in AML samples. In Figures 1-6 we have described an existing PM Test ex vivo for chemotherapies in AML we have developed and is currently being used to guide AML patient treatment.

Example 5 shows the development of a PM Test ex vivo for CAR-Ts for AML, in analogy to the currently approved PM Test ex vivo for standard chemotherapy for AML shown in Figures 1-6.

Examples 6 and 7 describe the identification of trogocytotic CAR-T cells in ALL and AML, respectively. Examples 6 and 7 describe how selecting trogocytotic T cells as those T cells that have cancer cell markers can select for both singlets and doblets. Without wishing to be bound by theory, it is believed that these doblets are actually a T cell attached to a cancer cell by means of an immune synapse. Such doblets would represent a step in the cancer cell killing by the T cell, in which the T cell inserts some toxins into the the cancer cell cytoplasm that kill the cancer cell by cell lysis. Thus, these doblets may represent a part of the best cancer killer T cells that are already in the process of killing these cancer cells.

Example 8 describes identification and FACS sorting of trogocytotic vs non-trogocytotic CAR-T cells in AML, evaluating the tumor-killing activity of each subpopulation validating an enhanced killing activity of trogocytotic vs non-trogocytotic CAR-T cells.

Example 8 describes a case sorting by FACS trogocytotic CAR-T cells that are later confirmed to have enhanced cancer-killing activity. Example 8 describes identification and FACS sorting of trogocytotic vs non-trogocytotic CAR-T cells in AML, evaluating the tumor-killing activity of each subpopulation validating an enhanced killing activity of trogocytotic vs non-trogocytotic CAR-T cells.

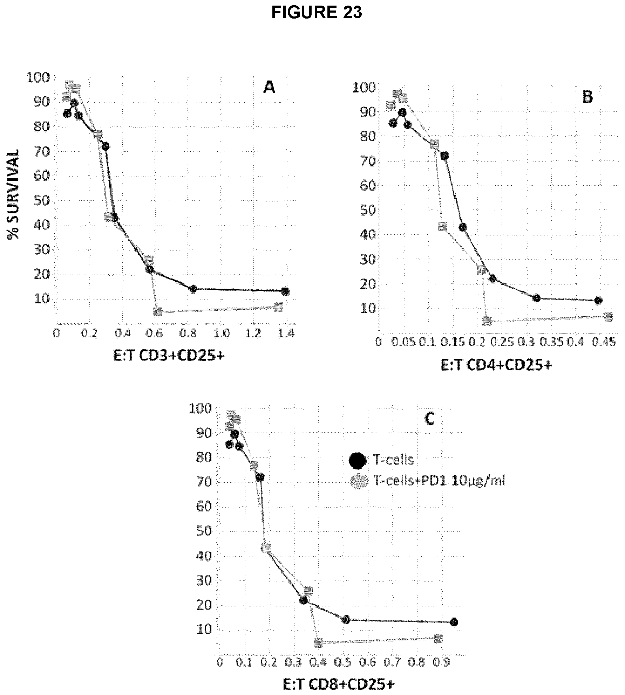

Examples 9-13 describe combinations of immune check points with either BiTEs or CAR-T cells, in either hematological or solid tumors. Examples 9 and 10 describe ex vivo incubation of combinations of BiTE-activated T cells with immune check point inhibitors. Examples 12 and 13 describe combinations of CAR-T cells with immune check point inhibitors. Incubating with immune check point inhibitors may increase the number and/or cancer-killing activity of CAR-T cells. In embodiments, at least one and maybe multiple immunomodulatory agents such as immune check point inhibitors are added to the incubation mixture to facilitate generating best cancer-killing CAR-T cells, for subsequent use in cellular therapy. Example 13 describes the combination of a NKG2D CART with immune check point inhibitors in a solid tumor melanoma sample, specifically with a PDL1 (Figure 23).

Example 14 shows that the BiTE-incubated AML sample with a high Effective E:T Ratio, has a unique phenotype in that it shows high levels of IL13 and IL2 in the supernatant. It is only one sample, but IL13 is interesting because it is involved in an anti-inflammatory response, which could lower the CRS symptoms after an initial T cell killing of cancer cells.

Several examples describe ex vivo combinations of immune check point inhibitors with either BiTE or CART as a method of identifying subjects susceptible to immune checkpoint immunotherapy treatment. Example 9 describes the combination with isolated FACS sorted BiTE-activated T cells, that has been washed 5 times and should not have any BiTE left, mixed with new leukemic cells from the same AML sample never exposed to the BiTE before; In this case the BiTE is a reagent producing activated T cells, and it represents a method of identifying subjects susceptible to monotherapy immune check point therapy. Example 10 describes combining during all the ex vivo incubation a BiTE with an immune check point PD1. It is known that BiTE induces increased expression of immune check points due to releasing interferon-gamma to the medium. Thus, this example represents a method of identifying subjects susceptible to combination therapy of a BiTE with an immune check point. Example 11 is similar to Example 10 in its set up, but adding more immune check points and immunophenotyping, and thus also represents a method of identifying subjects susceptible to combination therapy of a BiTE with an immune check point. Examples 12 and 13 describes combining a CAR-T cell with immune check points, and thus represents a method of identifying subjects susceptible to combination therapy of a CAR-T with one or more immune check points.

Examples 9-13 as described above describes the different alternatives to study ex vivo combination therapies with immune check points; combining with BiTE o CAR-T or activated T cells, in hematological or solid tumors, studying one or multiple immune check points, even studying combining many or all immune check points to generate a better cancer-killer CAR-T cell.

Example 14 describes such a method of evaluating susceptibility to Cytokine-Release Syndrome (CRS) for a BiTE, in this case a CD3xCD123 for AML.

Examples 15 and 16 describes such a method of evaluating susceptibility to Cytokine-Release Syndrome (CRS) for a CAR-T, either in hematological malignancies or solid tumors. Example 15 shows the CRS prediction assay for a CART-NKG2D in hematological malignancies. Example 16 shows the CRS prediction assay for the same CART-NKG2D in a solid tumor, melanoma. In both examples, the prediction assay consists in combining cytokines levels in supernatant with tumor-killing activity for every sample. The AUC (Area Under the Curve) parameterto calculate tumor-killing activity does not correlate with supernatant cytokines, probably because we have only 3 concentrations and the error for AUC calculaton is too large. Calculating the Effective E:T Ratio, the number of tumor cells killed by a single CAR-T on average, results in a significant correlation where higher tumor-killing activity correlates with higher levels of cytokines in supernatant.

Example 17 describes the benefits of AE vs no AE for evaluating the tumor-killing activity of a CAR-T. Examples 18 and 19 describe the benefits of AE vs no AE for 2 different BiTEs.

In the claims, articles such as "a," "an," and "the" may mean one or more than one unless indicated to the contrary or otherwise evident from the context. Claims or descriptions that include "or" between one or more members of a group are considered satisfied if one, more than one, or all of the group members are present in, employed in, or otherwise relevant to a given product or process unless indicated to the contrary or otherwise evident from the context. The invention includes embodiments in which exactly one member of the group is present in, employed in, or otherwise relevant to a given product or process. The invention includes embodiments in which more than one, or all of the group members are present in, employed in, or otherwise relevant to a given product or process.

Furthermore, the invention encompasses all variations, combinations, and permutations in which one or more limitations, elements, clauses, and descriptive terms from one or more of the listed claims are introduced into another claim. For example, any claim that is dependent on another claim can be modified to include one or more limitations found in any other claim that is dependent on the same base claim. Where elements are presented as lists, e.g., in Markush group format, each subgroup of the elements is also disclosed, and any element(s) can be removed from the group. It should be understood that, in general, where the invention, or aspects of the invention, is/are referred to as comprising particular elements and/or features, certain embodiments of the invention or aspects of the invention consist, or consist essentially of, such elements and/or features. For purposes of simplicity, those embodiments have not been specifically set forth *in haec verba* herein. It is also noted that the terms "comprising" and "containing" are intended to be open and permits the inclusion of additional elements or steps. Where ranges are given, endpoints are included. Furthermore, unless otherwise indicated or otherwise evident from the context and understanding of one of ordinary skill in the art, values that are expressed as ranges can assume any specific value or sub-range within the stated ranges in different embodiments of the invention, to the tenth of the unit of the lower limit of the range, unless the context clearly dictates otherwise.

### Examples

### Example 1. Generation of CART-ICT cells in ALL and AML

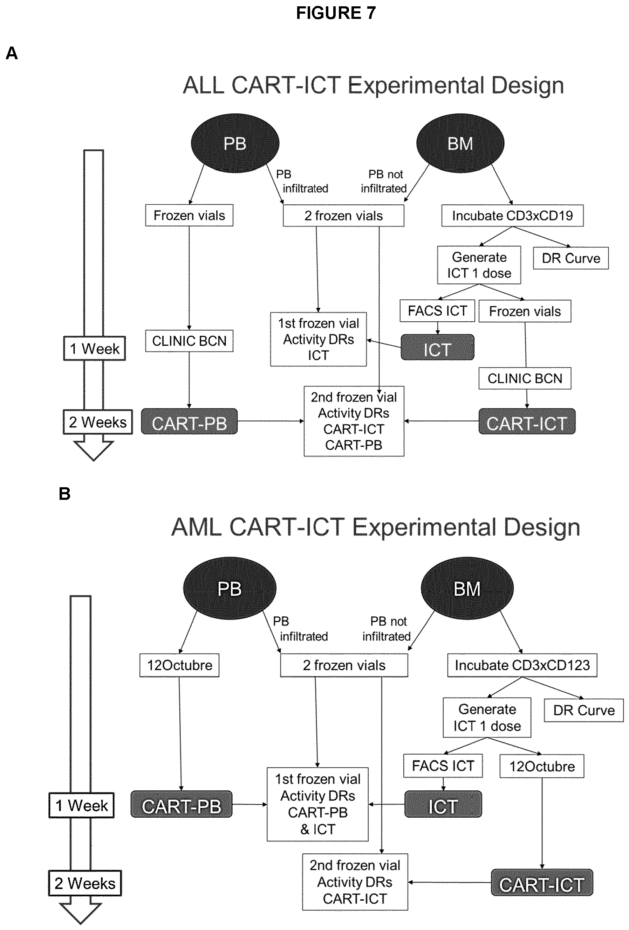

In this example we i) provide a rational depiction to demonstrate that immune coaching T-cells (ICTs) generated with BiTE exposure may have equal or higher activity than standard chimeric antigen receptor (CAR) engineered T-cells, and potentially improved safety and manufacturing issues, ii) demonstrate that the generation of a CAR- T cell with BiTE activated T-cells may generate a more potent response that could combine the targeted antibody efficacy of the CAR and the native tumor antibodies present on the BiTE derived activated T-cells, and iii) propose a method to compare the activity of BiTE derived T-cells with BiTE derived CAR-T cells. To demonstrate this approach in ALL, a set of experiments have been designed and are illustrated in Figure 7A. B-ALL samples were included and Blinatumomab (CD3-CD19 BiTE) was used as the immune coaching factor. Both PB and BM sources will be collected. In the first scenario the PB has not been infiltrated with leukemic cells. In this case, the PB sample will be used to isolate the mononuclear cell population by Ficoll gradient (Histopaque-1077, Sigma. Ref: H8889) and frozen in FBS (Gibco, Ref.10500-6) + 10% DMSO (Sigma, Ref. D4540), which will be cryopreserved and sent to Clinic BCN. There it will be thawed, activated, transfected, expanded and frozen, generating the CART-PB as previously described. The leukemic cells of the BM counterpart will also be isolated by Ficoll gradient. One frozen vial will be used for cytototoxicity assays to evaluate the killing capacity of the bispecific antibody Blinatumomab in an 8 dose-response curve concentration after 120 hours incubation in the same manner as previously described in detail. Prior to analysis, the leukemic cells will labeled with Annexin V FITC (Immunostep, Ref: ANXVF-400T), CD19-PE (clone HIB19, e-Bioscience, Ref. 12-0199-42), CD4-PerCP (clone OKT4, (Biolegend, Ref. 317432), CD5-PECy7 (clone UCHT2, Biologend, Ref. 300622), CD45-PO (Life Technologies, Ref. MHCD4530), CD25-APC (Biolegend, Ref: 302610), CD8-APCCY7 (Biolegend, Ref. 344714).

The concentration of blinatumomab that causes the largest increase in the proliferation of T-cells and the most depletion of the leukemic cell population will be selected as the one with the highest cytotoxic capacity and will be used for generation of BiTE activated T cells, which in this example and in Figure 7 are referred to as ICTs (Immune Coaching T cells). The production of ICTs will be performed in two branches: i) ICTs generated with the most cytotoxic concentration of blinatumomab and sorted by FACS (Fluorescence Activated Cells Sorter), and ii) the rest of the sample will be frozen in FBS + 10% DMSO without previously isolating the ICTs and shipped to generate the CART-ICTs.

To perform the sorting of the ICTs, the cryopreserved sample will be exposed to a concentration of 15ng/mL of the CD3-CD19 bispecific antibody for 120 hours. The resultant cells will be pooled and collected into one aliquot and labeled with CD19-PE, CD5-PECy7, CD45-PO, CD25-APC, and Annexin V-FITC (to monitor the level of apoptosis). Prior to sorting, the labeled cells will be suspended in Binding Buffer with 2% of FBS, 2% Hepes (Sigma, Ref. H3537) and 1% ZellShield (Minerva Biolabs, Ref. 13-0050) at 15×10⁶ cells/mL. The sorted cells will be collected in RPMI-1640 (Sigma, Ref. R0883), 50% FBS, 2% Hepes and 1% ZellShield. Phenotypically, the sorted cells will be CD19-/ CD5+/ CD45+/CD25+/ Annexin V-. To confirm the amount of sorted cells and calculate the number of ICTs, a cell count will be performed using CD19-PE, CD4-PerCP, CD5-PECy7, CD45-PO, CD25-APC, CD8-APCCy7 and Annexin V-CF Blue to monitor the apoptosis.

In order to provide data to demonstrate that ICTs derived after a BiTE exposure have equal or higher activity that standard CAR engineered T-cells and similar activity as immune coaching CAR-T, the autologous previously frozen BM or infiltrated PB will be used to evaluate the B-cell killing activity by the three constructs (CART-PB, CART-ICT and only ICTs). The 3 different T cell Effectors will be added at different ratios against the B-cell target as previously described. The PharmaFlow platform will quantify the activity of these T cells in killing tumor cells by an effective E:T ratio that measures how many tumor cells are killed by every T cell (CD4+ or CD8+).

For the cytotoxic quantification of the ICTs constructs, a second tube of cryopreserved cells from the same patient will be thawed. The leukemic cells will be stained with the cell surface dye PKH67 (Sigma Aldrich, Ref. MIDI67) and incubated in triplicate for 24 hours with the sorted ICTs at 8 different E:T ratios ranging from 10:1 to 0.078:1, the number of targeted stained blast cells will remain constant. For this assay, only the blast cells will be stained with the dye, not the ICTs to discriminate between effector and target. The culture medium to be used will be RPMI-1640, 20% of FBS, 2% Hepes, 1% L-Glutamine (LONZA, Ref. BE17-605) and 1% ZellShield with 0.5µl/well autologous plasma and 0.5µl/well RBC as described in our previous patent application number 62/321 ,964 filed before USPTO. Prior to analysis the multicolor flow cytometry panel previously used for the cell count of ICTs will be added to define both the leukemic cells and ICTs.

An identical approach will be followed using fresh AML samples and the CD3-CD123 bispecific antibody to generate BiTE activated T cells (referred to as ICTs in this example). This example is illustrated in Figure 7B.

**Production of lentiviral vector.** A self-inactivating (SIN) lentiviral vector was generated by a third-generation packaging system in which 293T cells were transiently transfected with the transfer, helpers (pMD.Lg/pRRE) and envelope (pMD2.VGVg) plasmids, obtaining VSV-G-pseudo-typed lentiviral particles. The pMD2.VSVg and the helper pRSV.REV plasmids used were obtained from PlasmidFactory (Bielefeld, Germany).

Transfections were conducted in 293T cells at 50-70% confluence in 150 mm diameter plates following the CaCl₂ DNA precipitation method. Culture medium was replaced with fresh media two hours before transfection. The amounts of plasmids used for a 150 mm plate of 293T cells were: 36 µg of the corresponding transfer plasmid, 9 µg of the pMD2.VSV.G envelope plasmid, 12.5 µg of the pMD.Lg/pRRE helper, 6.25 µg of the pRSV.REV plasmid and 15 µg of pAdVantage plasmid (Promega, Fitchburg, Wisconsin, United States). The pAdVantage plasmid is described that enhances transient protein expression by increasing translation initiation. This mixture was prepared in a final volume of 1,100 µL of 0.1x Tris-EDTA buffer/dH2O (2:1) per plate and then 150 µL of 2.5 M CaCl2 were added. After 15 minutes of incubation at room temperature (RT) in agitation to allow the correct homogenization of the mixture; 1,250 µL of 2X HBS buffer (100 mM HEPES, 281 mM NaCl, 1.5 mM Na2HPO4, pH 7.15) were added dropwise while vortexing at full speed, allowing the formation of Ca2+/DNA- precipitates. Immediately, the total volume was added to 293T cells, which will subsequently phagocyte the precipitates. After 13 hours, culture medium was replaced by fresh medium. Lentiviral supernatants were collected 36 hours post-transfection, filtered through 0.2 µm pore-size filters (Milipore/Merck KGaA, Darmstadt, Germany) and concentrated by ultracentrifugation. Viral pellets were then resuspended in StemSpam medium to concentrate them 500 times, aliquoted and stored at -80°C.

Viral titers were determined by transduction of 293T cells with serial dilutions of the supernatants. 7.5x104 cells/well were seeded in 6-well tissue culture plates the day before. The same day of the titration, cell number in each well was determined. Serial dilutions of the LV supernatants were prepared in IMDM-based complete medium starting from 10-3 to 10-7 and then used to transduce 293T cells. After 10-15 days, cells were collected and analyzed by FACS.

**Manual T-Cell NKG2D CAR cell transduction.** Peripheral blood (PB) mononuclear cells and Immune coached T cells (ICTs) from four AML patients were thawed, washed in PBS, counted and sorted in FACS ARIA fusion flow cytometer device and stained with anti-CD5 and anti-CD25 mAbs. Subsequently, cells were washed in PBS, counted and cultured overnight at 1 × 10⁶ cells/mL in X-VIVO-15 (Lonza, 04-4180) medium supplemented with 250 IU/mL IL2 (130-097-746, Miltenyi Biotec), 5 ng/mL anti-CD3 (clone OKT3; 317303) and 5 ng/mL anti-CD28 antibody (clone 28.2; 302913), both from BioLegend. After 24 hours, transduction was performed on RetroNectin (T100B; Takara Bio, Clontech Laboratories) pre-coated plates using a Multiplicity of infection (MOI) = 5. Two consecutive rounds of lentiviral transduction with NKG2D CAR separated by 6 days were performed. Transduction efficiency was evaluated by FACS with an anti-NKG2D staining and non-viable cells Propidium iodide (PI) exclusion analysis. Then, the cells were harvested for subsequent experiments.

### Example 2. Measurement of the efficacy and activity of CART-ICT cells in B-cell malignancies

Peripheral blood (PB) or bone marrow (BM) samples obtained from hematological patients were plated with their corresponding bispecific antibodies at 8 different concentrations for 120h hours as explained before. All samples were from adult patients, over 18 years of age, who gave informed consent for study participation. The hematological samples were ALL, CLL and AML and the bispecific antibodies used were those that target CD19 malignant cells in ALL and CLL and CD123 pathological population in AML while CD3 targeted the CTLs in each of these 3 hematological malignancies. PB or BM samples were diluted with culture media and plated into the 96-well plates containing the bispecific antibodies.

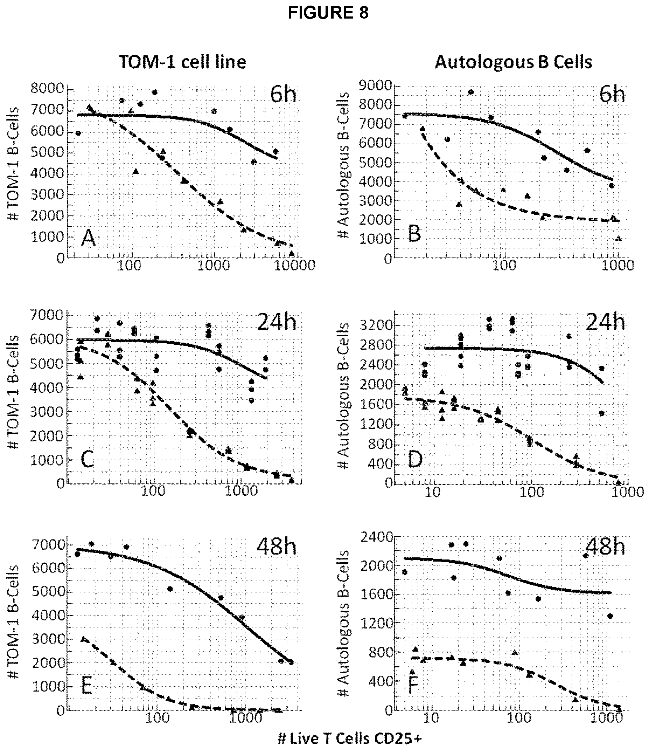

We have used a CLL patient sample to illustrate this example. Peripheral blood mononuclear cells (PBMCs) were harvested from the patient and used for two purposes: i) generation of autologous CAR-T cells, and ii) preserve autologous B-cells to evaluate the CAR-T efficacy. For the first purpose, the PBMCs were isolated as previously described and activated by the use of magnetic beads conjugated with CD3 and CD28 antibodies. These cells were subsequently genetically engineered by viral transduction to express the CAR under good clinical manufacturing practice. These activated T cells were then expanded ex *vivo* for 10-14 days and frozen. The frozen CAR-T cells together with the previously cryopreserved autologous B-cells were thawed and co-culture in a medium containing AIM-V supplemented with 20% FBS at 6 hours, 24 hours and 48 hours. The TOM-1 B-ALL CD19+ cell line was used as a positive control of the CAR-T efficacy. In this example, we have compared the activity of these CAR-T Cells with the activity of activated T-Cells without transfection. In the experiment, different numbers of effector (CAR-T or Activated T-Cells) in a dose-response manner to a fixed number of target (autologous B-cells or TOM-1 CD19+ cell line) B-Cells were used. At 24 hours nine-dose response points in triplicate were used while at 6h and 48h only one replicate was used for the nine-dose response points. After the different incubation times, the antibodies that identified the malignant B-Cell population together with those that define the T cells were added to the plates. In this example, we have used CD45-PO (Invitrogen, Ref. MHCD4530, clone HI30) and CD19-PE (Ebioscience, Ref: 12-0199-42, clone HIB19) for B-Cell identification together with CD5-PECy7 (Biolegend, Ref. 300622, clone UCHT2) and CD25-APCH7 (BD-Pharmingen, Ref: 560225, clone M-A251) forT-Cells. Annexin V-FITC (Immunostep, Ref. ANXVF) was also added to monitor the level of apoptosis and fully quantify the number of live cells. Plates were analyzed using the ExviTech^{®} flow cytometry based platform. The cells were identified based on FSC, SSC and the expression of the different surface markers. Figure 8 illustrates the results obtained. The X axes represents the absolute number of activated CD25+ T Cells for both the CAR-T cell population and the activated T-Cell population and the Y axes display the absolute number of TOM-1 B-Cells (Figures 8A, 8C and 8E) or the absolute number of patient's autologous B-Cells (Figures 8B, 8D and 8F). As can be seen, the CAR-T Cells (dotted line) deplete the B-Cells from both the TOM-1cell line and the autologous B Cells from the patient sample more completely and faster than the activated T-Cells without transfection (solid line). The CAR-T Cells are effective as removing all of the TOM-1 cells even at 6 hours, and were active against the Autologous B cells at this time point, eliminating them at 24 hours.

### Example 3. Measurement of the efficacy and activity of CART-ICT vs CART and ICT cells in hematological malignancies

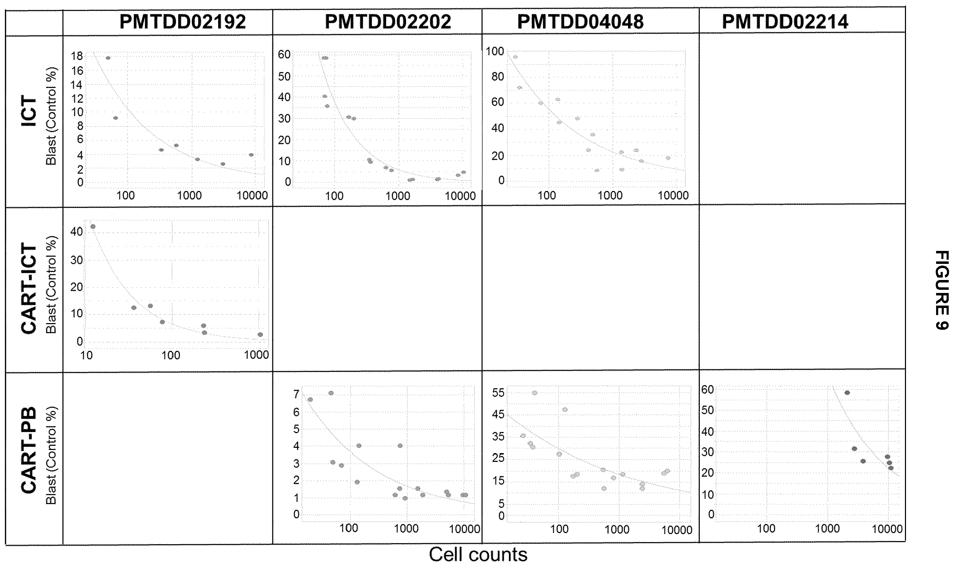

The activity of 3 different potential autologous cell therapies, CART-PB, CART-ICT, and ICT, as defined in Example 1, were compared on 4 AML samples. CART-NKG2D on PB and ICT cells, and ICTs, all autologous on the same AML sample were generated as described in Example 1. Not all 3 constructs could be generated for each AML sample, due to reasons such as transduction efficacy or viability of the cells. Figure 9 shows the ex vivo dose response curve of each of these 3 autologous cell therapies in 4 AML samples. We could generate ICTs for % samples, CART-ICT for only ¼ samples, and CART-PB for % samples. The reasons for not generating all these 3 cell therapy constructs in all 4 samples were diverse. A major problem was the insufficient amount of T cells in these samples, because AML samples have normally more than 90% leukemic cells and few T cells left. We used normal samples from PB and BM, while in the clinical setting CARTs are generated from apheresis with much larger volumes that used in these experiments. We could still generate ICTs and CART-PB on % samples. The problem was on CART-ICT where we could only generate it in ¼ samples. The main reason was difficulties in transducing these ICTs with the virus, because we had not optimized these conditions. Furthermore, the only sample where we could generate the CART-ICT we could not generate the CART-PB to compare them directly. Thus, we show here we can reduce to practice the generation of CART-ICTs, but we cannot compare them directly with CART-PB to confirm an advantage such as enhanced activity.

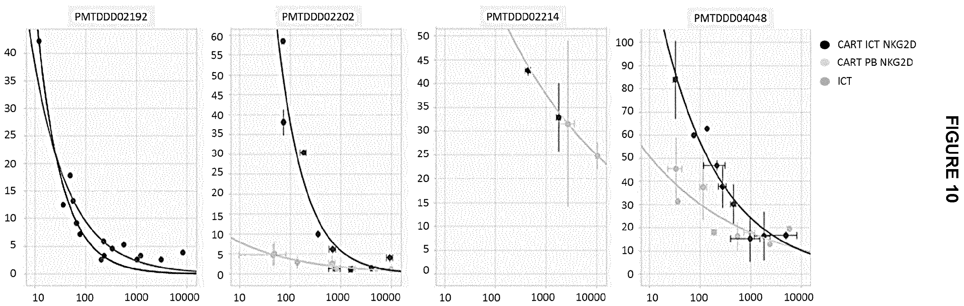

Figure 10 shows the dose response curves for each sample of the 3 cell therapies that could be generated for each of the 4 AML samples. The only possible comparison of CART-ICT is on the leftmost sample (PMTDD02192) with the corresponding ICTs, and both show a similar dose response curve. For samples PMTDD02202 and PMTDD04048 the dose response curves for CART-PB are more active in tumor killing than ICTs, because there are shifted to the left killing tumor cells towards lower Effector:Target ratios (equivalent to a standard concentration parameter).

A direct comparison of CART-ICTs claimed here with standard CART-PB is not possible. An indirect comparison that the activities in sample PMTDD02192 CART-ICT is similar to ICT, and the other samples CART-PB is better or equal to ICT, cannot conclude that CART-PB is better than CART-ICT. The reason is that the interpatient variability in the ex vivo activity of these cell therapy constructs is larger than any of these intrapatient differences, as shown in Figure 11 overlapping all these dose response curves for all these 4 AML patients. Because the activity differences of these dose response curves for different patients is larger than the activity different between CART-PB, ICT, CART-ICT within any sample, it is expected that CART-ICT would be either better or worse than CART-PB for different samples. A large interpatient variability means a Precision Medicine test that identifies the right cell therapy construct for each individual patient would be an important advantage for patient treatment outcome.

### Example 4. Measurement of the efficacy and activity of CART cells in solid tumors; melanoma

The same CART-NKG2D lentiviral vector described in Example 1 was generated following the same methodology, but transduced on a different healthy donor peripheral blood sample T cells. A solid tumor melanoma sample was thawed from a cryopreserved sample obtained from the biobank of Molecular Responses (www.molecularresponses.com). An initial cell count was performed using Annexin V-FITC, CD45-PO, EpCAM-PE, 7-AAD, NKG2D-PECy7 and CD5-APC and a fixed number of 2000 tumor cells per well of the melanoma sample was incubated in RPMI + 20%FBS with 8 increasing concentration of CART-NKG2D diluted 1:2 for 24 h, plus control well without CART-NKG2D. After the incubation time, the plate was processed and labeled with the same monoclonal antibodies cocktail. The PharmaFlow platform was used for quantifying the absolute numbers of live tumoral cells. Figure 12 shows the absolute counts of tumor cells (grey, left vertical axis) and CART-NKG2D cells (black, right vertical axis). Left column shows the number of tumor cells in control wells and right column displays the CART-NKG2D T cells. The following 8 categories show 8 subsequent dilutions of T cells from highest (dilution 1) to lowest (dilution 8). There is a dose-dependent decrease in the number of tumor cells (grey) as we increase CART-NKG2D T cells.

**Production of Lentiviral vector.** The same method as described in Example 1 was followed.

**Automated T NKG2D CAR cell transduction.** Automated TCT was performed on the CliniMACS Prodigy using the Tubing Set TS520 and the TCT process. Where not specified otherwise, reagents and materials were obtained from Miltenyi Biotec. Cell processing was begun within 24 h of product collection. Fresh non-mobilized leukapheresis from healthy donors were obtained and washed with PBS/EDTA + 0,5% HAS (Grifols). Cell labeling with magnetic beads was performed using CliniMACS CD4 Reagent and CliniMACS CD8 Reagent (Miltenyi Biotec) for 30 min at 4-8° C and magnetically selected. Cells were cultured in GMP Medium TexMACS + 50 U/mL MACS GMP Human Recombinant interleukin (IL)-2 in the CentriCult-Unit. Cells were subsequently activated with the polymeric nanomatrix MACS GMPTransAct CD3/CD28 Kit at a final dilution of 1:200 (CD3 Reagent) and 1:400 (CD28 Reagent). One hundred million purified T cells were transduced on day 1 during 48h using a multiplicity of infection (MOI) of 2 with a self-inactivating third-generation lentiviral vector encoding a CAR specific for NKG2D CAR. The NKG2D CAR construct contains full-length NKG2D ectodomain fused to 41BB and the intracellular CD3ζ domains, under the control of EF1α internal promoter and including a mutated woodchuck post-regulatory element (WPRE) and human immunodeficiency virus (HIV) central polypurine tract (cPPT).Vector was pseudotyped with vesicular stomatitis virus (VSV) and concentrated by ultracentrifugation. The ultra-concentrated lentiviral vector thawed at room temperature was diluted in 10 ml of X-VIVO 15 media in a 50 mL transfer bag 200-074-400 CryoMACS, which was then attached to the CliniMACS Prodigy by sterile welding. The vector was automatically transferred in the culture chamber and the vector bag was further rinsed with 20 mL. During culture the temperature and atmosphere is maintained at 37°C with 5% CO2. To remove excess stimulation reagent and LV, culture wash was automatically performed 2 days after stimulation, and culture was switched from static culture to agitated culture. Cultivation volume was increased subsequently through automated feeding to 250 mL. Automated media exchange via centrifugation was executed every day via replacement of a maximum of 180mL of culture medium. After 6 days of cultivation, the media bag was exchanged (GMP Medium TexMACS + 50 U/mL MACS GMP Human Recombinant interleukin (IL)-2). On day 10 after purification, cells were harvested in a final volume of 100 ml, automatically formulated in 0.9% Sodium-Chloride solution supplemented with 0.5% human serum albumin (HAS; Grifols) and transferred into a bag.

### Example 5. Precision Medicine Test ex vivo for a CART-NKG2D in AML samples

Repeating the measurements of the killing activity of CARTs on several samples enables a comparison of the activity of said CART on different samples. When a gradation of different activities is identified, this means the same CART is more sensitive or more resistant to some patient samples. This result can become a Precision Medicine Test whereby the relative sensitivity or resistance to these samples can be interpreted as a potential sensitivity or resistance of the patient clinically if administered said CART. The same CART-NKG2D from Example 4 was used, from the same healthy donor cells and produced with the same methods.

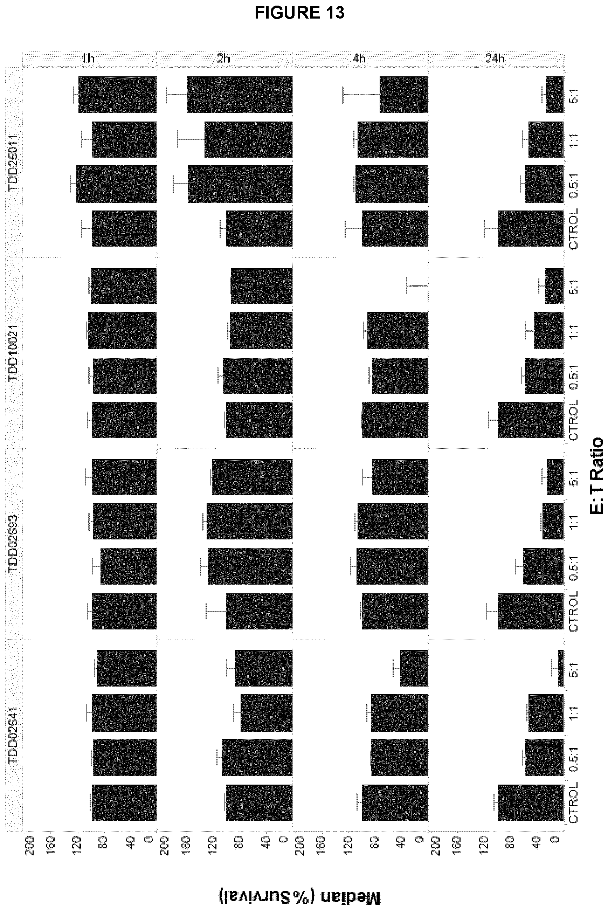

Figure 13 shows the tumor-killing activity of the same CART-NKG2D from a healthy human donor when incubated with the sample of 4 different AML patients, at different incubation times and E:T ratios (Effector:Target). This is an allogenic mixture because the CART cells are derived form a different person than the AML patient samples. The CART-NKG2D kills leukemic cells in all 4 samples at 24 h, but only in 2 samples at 4 h, with little effect at 1 and 2 hours.

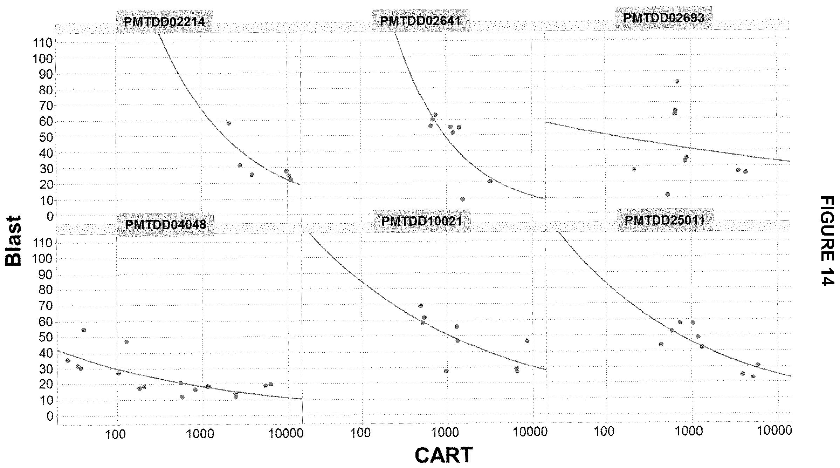

To generate a PM Test, we bring together these 4 AML samples and the 3 AML samples from Example 3, each of these groups using a different donors T cell to generate the CART-NKG2D. To quantitate these tumor-killing activities, we have fitted these results to dose response curves, shown below for each sample in Figure 14.

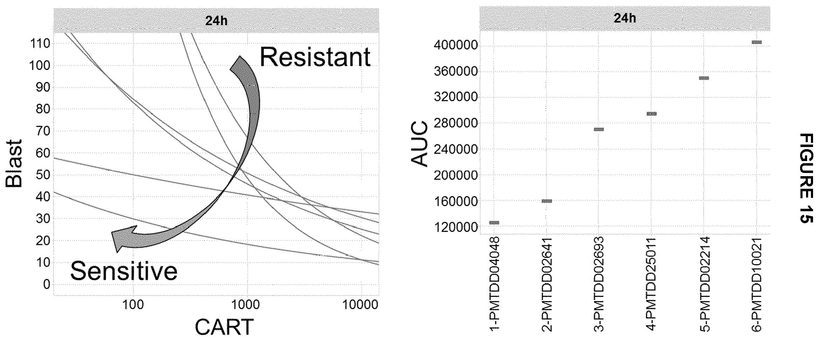

To derive a Precision Medicine ex vivo test from these results, we need to rank these samples in order of tumor-killing activity by the drug candidate (in this case the CART-NKG2D). Samples on which the CART shows highest activity represent samples most sensitive to this treatment ex vivo, and the prediction would be that they would be also the most clinically sensitive patients for said CART treatment. Conversely, lowest activity samples would represent patients more likely to be resistant clinically to this treatment. To compare and rank these samples on their CART tumor-killing activity, Figure 15 left panel shows the dose response curves overlapped. The arrow points towards the direction from less active to more active samples, and hence from patients clinically predicted more resistant (less active) to more sensitive (more active). This visual gradation can be converted to a quantitative ranking of activities by using pharmacodynamic parameters derived from the dose response curve fitting. An example is to calculate the AUC (Area Under the Curve) to estimate the overall activity of the CART on each sample, ranking the patient samples according to their AUC, as shown in Figure 15 right panel. This ranking would correspond to a probability of predicting the clinical response of the patient; the lowest AUC value represents the predicted most sensitive patient, while the highest AUC value represents the predicted most resistant patient.

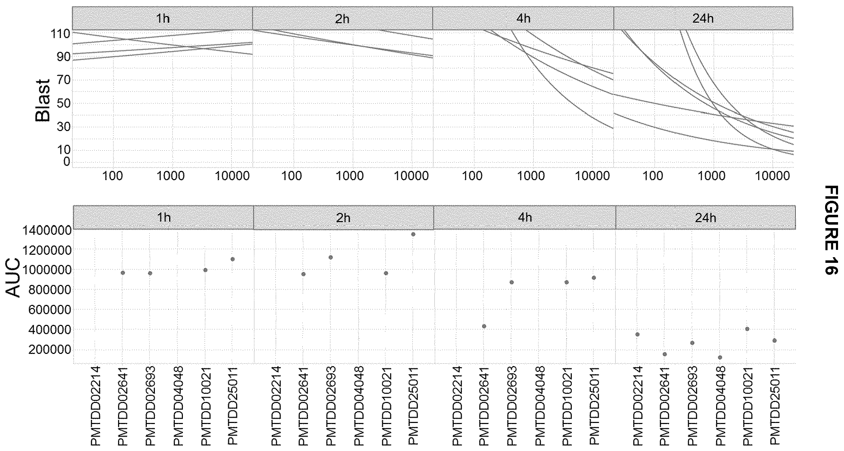

Figure 16 shows the dose response curves at increasing incubation time points of the same CART-NKG2D on the same AML samples. There is a clear pattern of increasing tumor-killing activity at increasing incubation times. This means that the numbers of both the CART effector T cell population, as the leukemic target cell population, are dynamic, i.e. change over time. This means the dose response curves as shown overlapped in Figure 15 for 24 h represent only a given time point, that may not reflect accurately the clinical response in patients treated with this CART. The pharmacokinetics of the CART will also affect the clinical response. For example, if the CART cells can attack in proximity the tumor cells for a short-limited amount of time, then the samples for which the CART can kill tumor cells quickly, at a short time, would represent patients most likely to be sensitive to this treatment. Conversely, if the CART can attack the tumor cells of the patient for a long amount of time inside the patient, e.g. long residence time in bone marrow for AML, then fast acting and slower acting CART cells may show a similar clinical response. Given the dynamic nature of these CART effects ex vivo, the most appropriate method of data analysis would be measuring activity at multiple time points and applying dynamic models to fit the experimental results. There are dynamic models that use differential equations known in the art that can describe the behavior of both CART and tumor cell populations overtime.

### Example 6. Identifying trogocytotic CART cells on B-ALL

Paired samples of peripheral blood (PB) and bone marrow (BM) from the same patient with B- Acute Lymphoblastic Leukemia (B-ALL) were collected and cryopreserved in vials containing approximately 20 million cells. From the PB T Cells a CART-CD19 T cell was transduced, expanded and cryopreserved. The PB CART-CD19 and the B-ALL BM vial were thawed and an initial evaluation was performed using the following monoclonal antibodies cocktails: Annexin V-FITC, CD19-PE, CD45-PECy7 and CD5-PerCP-Cy5.5, for B-ALL BM leukemic cells, and GFP (FITC), Annexin-PB, CD19-PE, CD45-PECy7 and CD5-PerCP-Cy5.5, for the CART-CD19 T cells.

A fixed number of BM leukemic cells was stained with DiD membrane dye and then were mixed with an increasing number of CART-CD19 in a 1:2 dilution. Evaluation of trogocytosis and activity after 1 hour and 24 hours of incubation at 37°C in 5% CO₂, respectively, was evaluated with the following staining: GPF (FITC), CD19-PE, CD5-PerCPCy5.5, Annexin V-PB and DiD (APC and APCCy7) in the PharmaFlow platform.

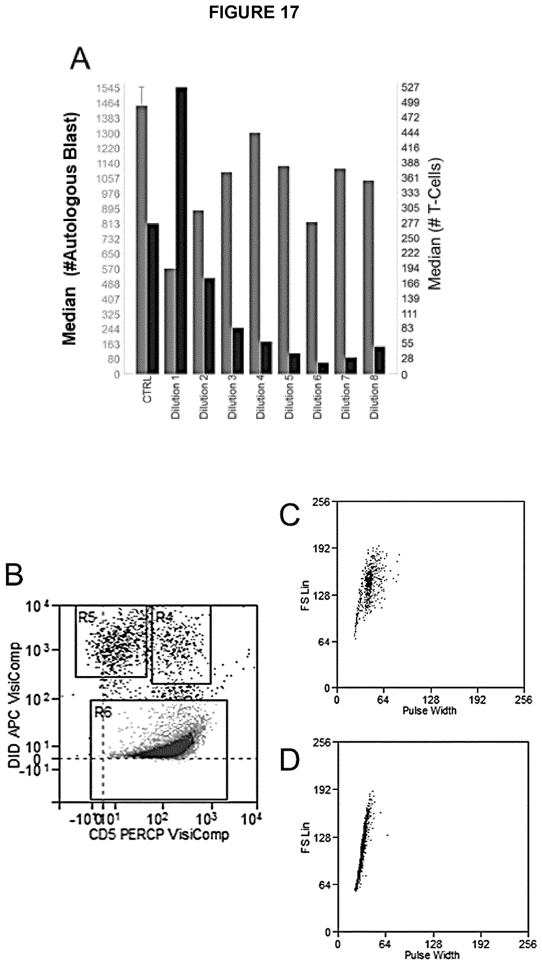

Figure 17A shows the number of CART-CD19 T Cells and leukemic cells, showing that at higher number of CART-CD19 they kill B-ALL autologous leukemic cells at 24h. The autologous BM sample was stained with DiD membrane dye. Panel (B) flow cytometry dot plot shows marker CD5 of T cells, including CART-CD19, versus the DiD dye (y axis). There are 3 areas delineated by rectangles; R6 represent leukemic cells labelled with DiD and CD5 negative. Among the CD5+ T cells there are two subgroups, R6 captures most T cells that are CD5+ and DiD-, while R4 represents trogocytotic CART-CD19 T cells CD5+ and also DiD+. Surprisingly, among the trogocytotic CART cells there is a substantial population of doblets, representing a leukemic cell attached to a CART cell. Panel C and panel D show a forward scatter vs pulse width plot where doblets (C) and singlets (D) are identified as the vertical group of dots shifted to the right. Doblets presumably arise when the CART-CD19 forms an immune synapse with the leukemic cell, after which the T cell delivers the toxic cytokines to the intracellular component of the leukemic cell which kills it by cell lysis. It is interesting that trogocytotic markers also include doblets, since both classes of CART cells are supposed to include the best tumor-killing CARTs cells.

### Example 7. Identifying trogocytotic CART cells on AML

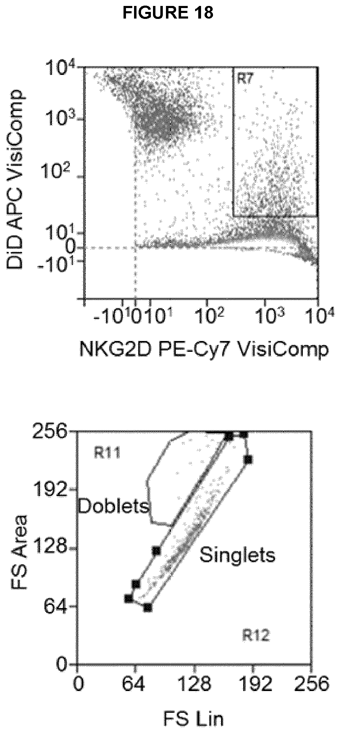

A CART-NKG2D was generated from a healthy donor PB sample following the methods described in Example 4. This CART-NKG2D showed good activity at killing the leukemic cells of AML sample TDD10021 from example 4 and was selected for this experiment. A cryopreserved vial of AML sample TDD02641 was thawed and labelled with the membrane dye DiD. A cryopreserved bag containing 600 million CART-NKG2D was thawed and incubated with the AML sample for 1 hour. An E:T ratio of 5:1 was used for the experiments. The following antibody panel was used to discriminate each population: CD8-FITC, CD33-PE, CD5-PerCP Cya5.5, NKG2D PE-Cya7, Annexin-V PB, CD45PO and the DiD. Blast cells were identified as DiD+/CD33++/CD45 weak. By contrast, CART-NKG2D were identified as DiD-/CD33-/CD45++/CD5+/NKG2D++, Trogocytotic CART-NKG2D T cells were observed after 1 hour incubation, as shown in R7 gate of Figure 18, where a population of cells CD5+ NKG2D+ and DID+ can be observed. Right panel shows that almost all of these Trogocytotic CART-NKG2D are singlets with very few doblets. These doblets may represent a leukemic cell attached to a CART cell.

### Example 8. Selection of CART best clones by trogocytosis

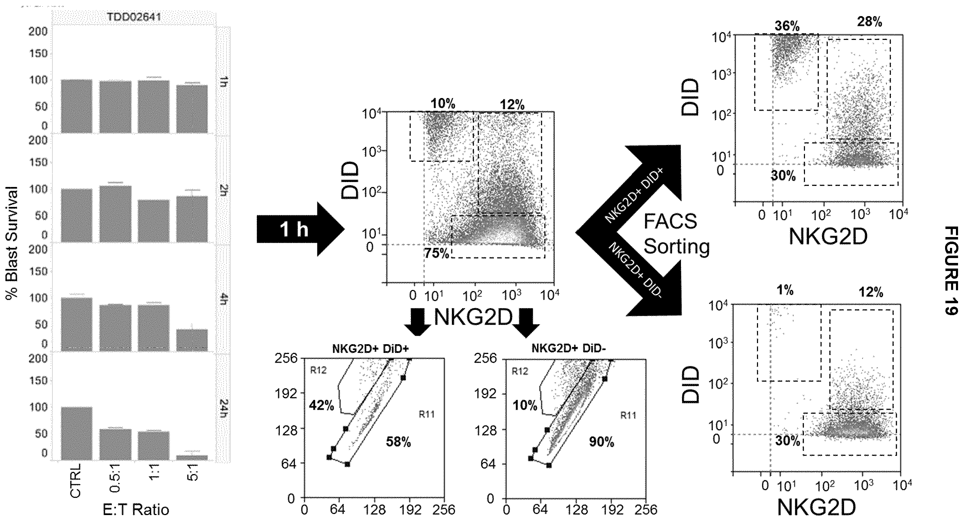

The same CART-NKG2D from Example 4 was used, from the same healthy donor cells and produced with the same methods. This CART-NKG2D showed good activity at killing the leukemic cells of AML sample TDD02641 from example 3 and was selected for this experiment. A cryopreserved vial of AML sample TDD02641 was thawed and labelled with the membrane dye DiD. A cryopreserved bag containing 600 million CART-NKG2D was thawed and incubated with the AML sample for 1 hour. An E:T ratio of 5:1 was used for the experiments. The following antibody panel was used to discriminate each population: CD8-FITC, CD33-PE, CD5-PerCP Cya5.5, NKG2D PE-Cya7, Annexin-V PB, CD45PO and the DiD. Blast cells were identified as DiD+/CD33++/CD45 weak. By contrasts CART-NKG2D were identified as DiD-/CD33-/CD45++/CD5+/NKG2D++. Figure 19 left shows the tumor-killing timeline, where there is no killing detected at 1 h. Nonetheless, trogocytotic CART-NKG2D T cells were observed after 1 hour incubation, as shown in Figure 19 middle, where a population of cells CD5+ NKG2D+ and DID+ can be observed. As shown in Figure 19 middle two lower plots, the DID+ population contained 42% doblets, while the DID- contain only 10% doblets. Trogocytotic T cells (DID+ CD5+ +NKG2D+) were sorted in a FACSAria Fusion cell sorter (BD) based on their positivity for both DID and CD5 (Figure 19 right) as well as FSC/SSC. Not surprisingly, there is a 36% leukemic cells in the sorted trogocytotic CART population, which may correspond to the doblets shown in middle panel lower plot: these doblets are composed of paired leukemic cells with CART cells, attached supposedly by an immune synapse that is step in the tumor killing process by T cells.

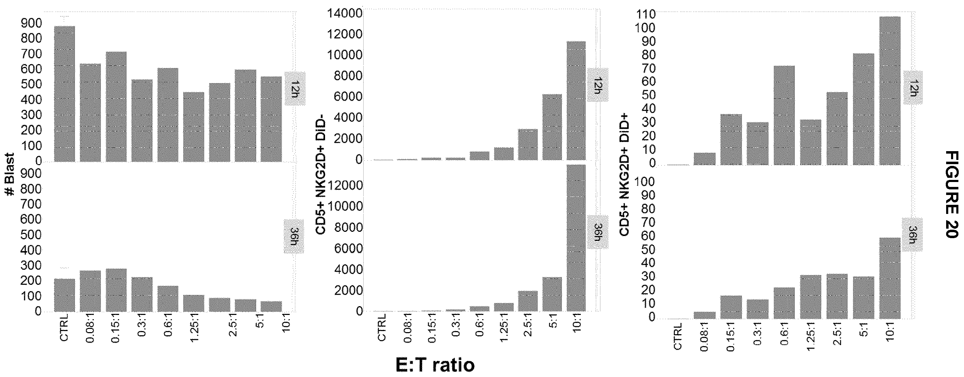

The FACS sorted trogocytotic vs non-trogocytotic CARTs were then co-cultured with RPMI + FBS 20% for 12 h and 36 h with the same AML sample thawed before to test the cytotoxicity activity of DID+ vs DID- CD5+ CART-NKG2D T cells. Figure 20 shows the activity of the non-trogocytotic DID- CART cells. Tumor cells decrease as CART cells increase at 36 h (left bottom) but not at 12 h (left top), showing cytotoxic activity. The number of CART-NKG2D+ DID- T cells reaches 14.000 at 36 h (middle bottom), and at this condition there is still a minor population of 80 NKG2D+ DID+ T cells (right bottom).

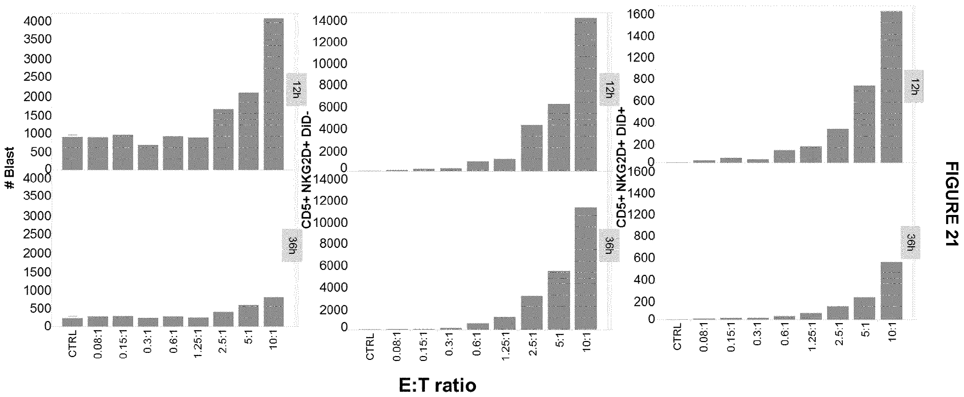

FACS sorting of trogocytotic CART-NKG2D+ DID+ T cells was contaminated by doblets of CART-NKG2D attached to leukemic cells. Doblets presumably arise when the CART-NKG2D forms an immune synapse with the leukemic cell, after which the T cell delivers the toxic cytokines to the intracellular component of the leukemic cell which kills it by cell lysis. It is interesting that trogocytotic markers also include doblets, since both classes of CART cells are supposed to include the best tumor-killing CARTs cells. In the sorted CART-NKG2D T cells there were a 30% of leukemic cells. As a result, when we incubated the DID+ vs DID- CART-NKG2D T cells with the same AML sample, the number of leukemic cells in the DID+ mixture actually increased. Figure 21 left panels shows the number of leukemic cells increasing at higher number of CART-NKG2D+DID+ T cells after 12 and 36 h incubation. This is contrary to the expectation that these trogocytotic CARTs are better killers. However, because there is a 30% leukemic cells in this sorted population, and we dispense a maximum of 10 CARTs for each leukemic cell, if in these 10 CARTs there are 3 leukemic cells, then there is a total of 4 leukemic cells for 10 CARTs; this means adding 10 CARTs for each leukemic cell we are multiplying by 3 the number of leukemic cells in that well, which explains why the absolute number increases rather than decreases as we increase the number of CARTs. It is interesting that in this trogocytotic CART-NKG2D+DID+ sorted population, at 36 h there are 12.000 CART-NKG2D that are DID- for only 600 that retain the phenotype NKG2D+DID+.

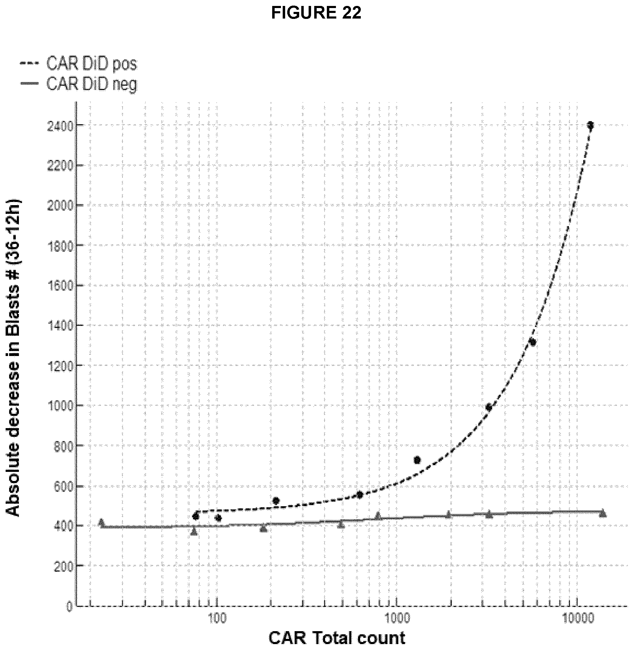

To calculate the killing activity of the sorted trogocytotic CART-NKG2D+ DID+ shown in Figure 21, we need to subtract the number of leukemic cells added due to a 30% contamination of the sorted CART-NKG2D+ DID+. Figure 22 shows this result in terms of the variation in the number of leukemic cells as we increase the number of CART cells. The constant variation that can be observed in Figure 22 for non-trogocytotic CARTs should represent the loss of viability (spontaneous dead) of the leukemic cells between 12 to 36 h. Hence, we have first calculated the dose response curves for leukemic cells vs total CARTs of each population (trogocytotic and non trogocytotic) at 12 h incubation. Second, interpolating within these dose response curves, calculate the leukemic cells that should have been at 12 h for each value of CART numbers measured at 36 h. Third, calculate the difference between these extrapolated leukemic cell numbers at 12 h and the real observed leukemic cell numbers at 36 h. Perform a fitting with such a delta difference in leukemic cell numbers versus the value of CART numbers at 36 h. The result is shown in Figure 22, where the trogocytotic CARTs show a clear enhanced tumor-killing activity relative to the non-trogocytotic CARTs.

### Example 9. Measurement of activity of purified activated T cells in presence and absence of an immune checkpoint inhibitor PD1 in AML

This example describes the use of a bispecific antibody, CD3xCD123 (Creative Biolabs), as BiTE, on blast cells from an AML sample. The sample was from an adult patient, over 18 years of age, who gave informed consent for study participation. T-Lymphocytes were generated from a frozen AML sample after 120-hour incubation at 37°C in humidified air containing 5% CO2 and the presence of the CD3xCD123 BITE. After this period, activated (CD25+) T-cells (CD8+ and CD4+) were sorted by fluorescence activated cell sorting (FACS sorting) using a FACS Aria III flow cytometer (BD). The purity of the sorted cell populations was higher than 99%. Once the effector T-cells were purified, A new vial from the same patient was thawed to evaluate the cytotoxicity of the sorted populations.

The effector T-cells (CD8+CD25+ or CD4+CD25+) were mixed with a constant number of blast cells at different effector:target (E:T) ratios in presence of absence of Nivolumab (Anti-PD1 antibody) to generate a dose response curve at different E:T ratios. Both cell types (effectors and targets) were seeded for another 24h incubation at 37°C in humidified air containing 5% CO2 without the presence of the CD123xCD3 BITE.

Figure 23 illustrates the results obtained. The X axes represents the Effector:Target ratio of the activated CD25+CD3+ T cells (Figure 23A), CD4+CD25+ T cells (Figure 23B) and CD8+CD25+ T cells (Figure 23C) and the Y axes display the normalized percentage of survival of the leukemic cells. As can be seen, the activated T cells in presence of 10µg/ml of Nivolumab (grey line) leave fewer cells than activated T cells alone (black line) at equal E:T ratios. Because after the T-cell activation with the CD123xCD3 BITE some of the T-cells can acquire PD-1 expression, the addition of the anti PD-1 antibody can inhibit the negative T-cell regulation by PD-1. In addition, the presence of the anti PD-1 antibody fully kill all the leukemic cells (Emax=0) in contrast to the activated T-cells that still leave a proportion of leukemic cells alive (Emax=12). This example demonstrates the importance of the addition of immune checkpoints to the activated T-cells to significant improve the efficacy of the generated T-cells after a BITE exposure.

### Example 10. Measurement of T-cell activity with BiTE in presence and absence of immune checkpoint inhibitor PD1 for CLL

Despite the clinical improvement with the use of BiTEs in treating hematological malignancies, a remarkable proportion of patients are still resistant. The development of rational combination therapies aims to overcome the resistance to bispecific antibody treatments and the immune checkpoint blockade is one of the more promising approaches to overcome this bispecific antibody resistance. Hence, we can measure in these resistant immunosuppressed populations which immune checkpoint proteins are expressed.

This example describes the use of Blinatumumab as the BiTE, tested in combination with Nivolumab (an Anti-PD1 antibody) on B-cells from a CLL sample. The sample was from an adult patient, over 18 years of age, who gave informed consent for study participation.

Figure 24 shows how in a CLL (Chronic Lymphatic Leukemia) PB (peripheral blood) sample that was resistant to blinatumomab (CD3-CD19 BiTE) the addition of an anti-PD1 antibody (Nivolumab) increased the number of CD8+ (panel A) and CD4+ (panel B) activated T-cells. In panels A and B, the solid lines represent the BiTE alone and the dashed lines are the BiTE plus Nivolumab (BiTE+PD1). It can be seen how the presence of nivolumab increased the number of both CD4+ and CD8+ cell populations over the BiTE alone. Additionally, as seen in panel C, the killing efficacy of those T cells, in the presence of Nivolumab (dotted line) shifted the EC50 towards the left from the BiTE alone (solid line). These results reflected a greater level of T-cell activation and subsequent B-cell depletion with the combination of the BiTE and the immune checkpoint inhibitor, showing an overall improvement of the T-cell response.

This demonstrates how a biomarker assay that could guide the selection of which immune checkpoint inhibitors would benefit each patient could be developed. Future work comparing the ex vivo response to the clinical response would confirm the validity of the biomarker assay.

### Example 11. Combination BiTE with Immune Check Point in AML by dual expression and functional criteria

It is common to measure the expression levels of immune check points (ICHKs) on tumor samples to identify patients likely to respond to ICHK treatment. We can combine this criterion with an ex vivo functional criteria, as shown in Figure 19, to predict which ICHK is best to combine with a BiTE treatment.

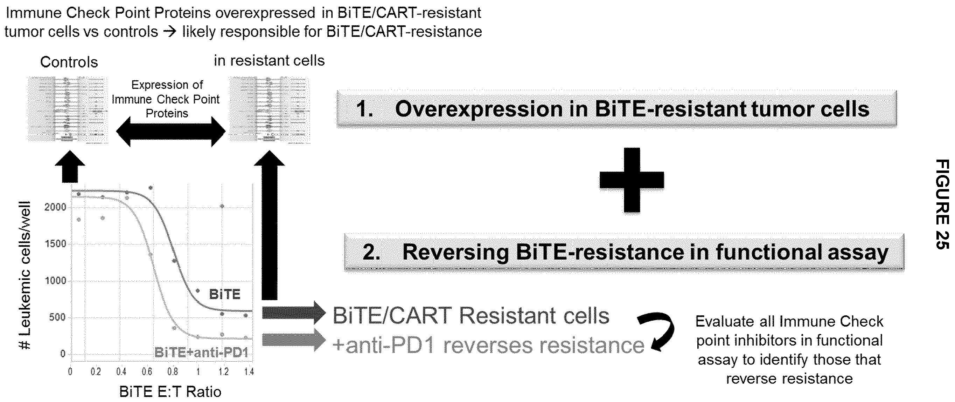

Figure 25 shows a dose response curve for a BiTE (black) inducing depletion of leukemic cells (Y axis). In this case is the CD3xCD123 Blinatumumab on a CLL sample. There is a subpopulation of resistant leukemic cells not killed by the BiTE incubation black arrow and test Resistant cells). Because these leukemic cells are resistant to BiTE-activated T cells, they are probably immunosuppressed, most likely by enhanced expression of ICHKs. We tested this hypothesis by adding anti-PD1 (Nivolumab) throughout the BiTE incubation, resulting in a dose response curve (grey) that reverted the resistance of the BiTE only curve (black) killing most of the resistant leukemic cells. Incubation with BiTEs is known to induce expression of ICHKs due to secretion of interferon-gamma, that may not be expressed initially in the patient sample.

We can combine a dual criteria shown in Figure 25; adding ICHK such as PD1 to revert functional ex vivo resistance, and also measure the expression levels of ICHKs in control wells and in the BiTE-resistant leukemic cells. These 2 criteria should be consistent:
1. Overexpression of ICHK in BiTE-resistant tumor cells. Comparing ICHK expression in control wells vs maximum effect of BiTE, we expect the ICHK responsible for resistance to be overexpressed in the resistant leukemic cell population relative to the control untreated wells. There could actually be several ICHKs expressed in control well, but only one or few may be responsible for resistance to BiTE, and these ones are expected to be overexpressed in the resistant leukemic cells.
2. Reversing BiTE-resistance in ex vivo functional assay. Adding a high dose of the anti-ICHK that is responsible for BiTE-resistance is expected to reverse resistance increasing significantly leukemic cell killing. We expect that the ICHKs that reverse resistance in the functional assay are the same that are overexpressed in the resistant leukemic cell population.

Requiring both criteria maximizes the likelihood to predict correctly which ICHK should be combined with the BiTE for each individual patient. Therefore, this method represents a Precision Medicine Test to predict ICHK-BiTE combinations.

The method consists on measuring the activity of the BiTE in a patient sample alone, adding each potential ICHK and also their combinations that represent drugs or drug candidates accessible to the patient. Minimally each ICHK should be added at a single high dose. In each of these conditions, the expression levels of these ICHKs should be measured in control untreated wells, and wells incubated with BiTE alone or BiTE+ICHK combinations. The dual criteria mentioned above can then be applied to the results.

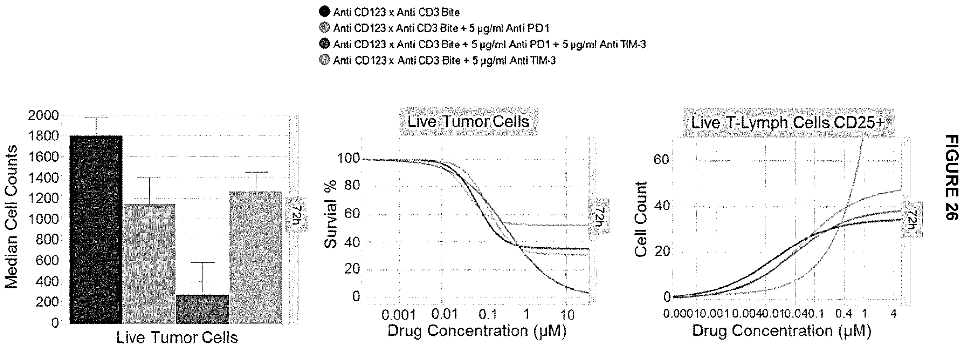

Figure 26 shows an example of this approach. An AML BM sample incubated for 72 h with a CD3xCD123 BiTE, alone or in combination with a single high dose of either PD1, TIM3, or both PD1+TIM3. Left panel shows the number of leukemic cells in each condition. There are approximately 1.800 BiTE-resistant leukemic cells. Adding PD1 or TIM3 lowers the number of resistant tumor cells to 1.100, still a large number of resistant leukemic cells. Adding both PD1+TIM3 at the same time lowers the number of leukemic cells to about 200, almost reversing the resistant phenotype. Middle panel shows the functional dose response curves, showing that indeed both PD1+TIM3 are required to reverse BiTE resistance. Right panel shows the dose response curve for activated (CD25+) T cells for each condition. Note that they are all similar, except adding TIM3 that increases the proliferation of activated T cells, but without increasing their tumor-killing activity (middle panel). This is an example that expression of ICHKs such as TIM3 may have different effects that conferring resistance, and thus a dual criteria combining expression and functional effects is best to discern the right combination partner for each patient sample. Notably, the combination PD1+TIM3 does not change the number of activated T cells (right panel), but it does reverse the resistant tumor cells inducing nearly complete tumor killing (left and middle panels). Thus, the combination of these 2 ICHKs should induce a higher killing activity on the same number of activated T cells induced by the BiTE.

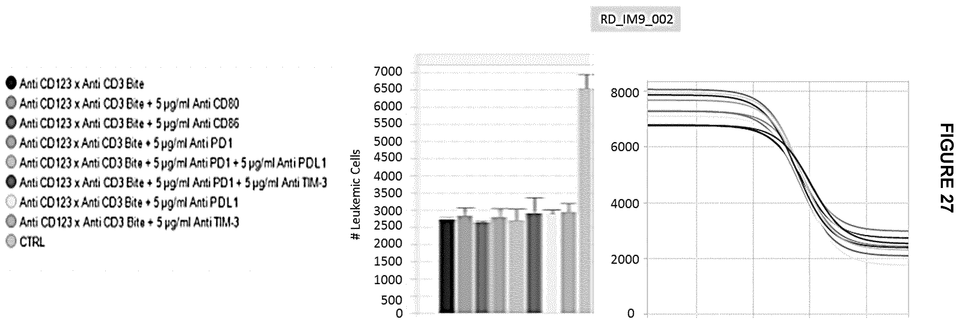

Patient samples are always very heterogenous, and it is expected that for some samples combining a BiTE with a single ICHK would be sufficient, other such as Figure 26 may require 2 ICHKs, other may require multiple ICHKs, and other samples may have a resistant mechanism that does not rely on ICHKs. This interpatient variability means we should apply systematically this approach to identify the right combination of a BiTE with a ICHK for each patient sample testing as many ICHKs and combinations as possible. Single ICHKs seems necessary. Two ICHKs is reasonable because there are multiple multispecific antibodies in development with 2 ICHK recognition arms. Three or more ICHKs can become difficult in practical terms. Figure 27 shows an AML patient sample for which none of the ICHK tested, alone or in combination, can reverse the BiTE-resistant leukemic cells. The dose response curves also show similar overlapped dose response curves for all conditions adding multiple single and double ICHKs. This represents a patient sample where this approach cannot find any suitable combination of the BiTE with ICHKs.

### Example 12. Combination CART with Immune Check Point in AML

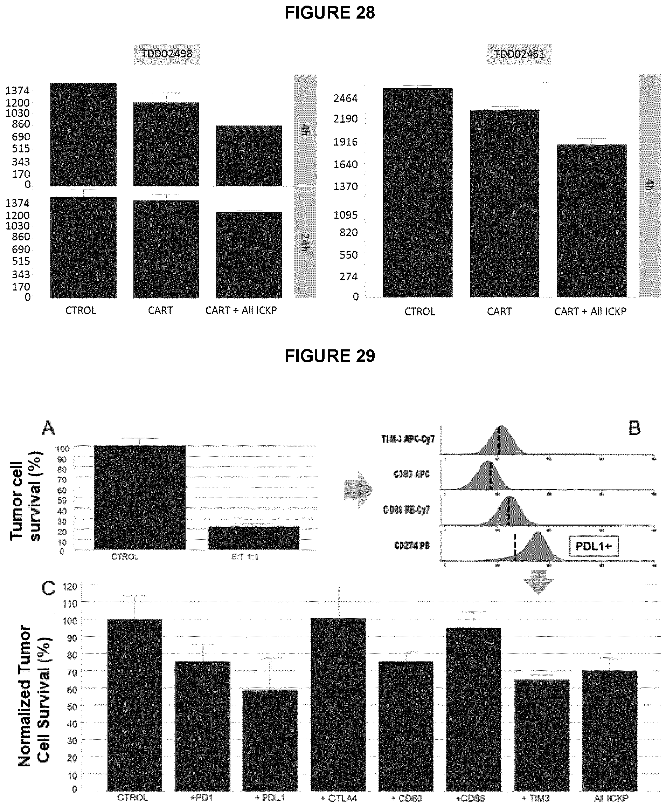

The same approach described in Example 11 above for BiTEs can be used for CARTs, another immunotherapy suitable to be combined with ICHKs. The same CART-NKG2D from Example 4 was used, from the same healthy donor cells and produced with the same methods. Figure 28 shows 2 AML samples incubated with CART-NKG2D already described in Example 3. The tumor-killing activity of these 2 AML samples was evaluated already in Example 6. For each sample, we show the number of tumor cells, in the left column for untreated control wells, in the middle column for CART-NKG2D treated wells at a high dose (5:1 ratio of CART:tumor cells), and in the right column for the CART-NKG2D adding multiple ICHKs at once. The ICHKs were PD1, PDL1, CTLA4, CD80, CD86, TIM-3 and NKG2A. For both samples the CART had a partial effect, that further enhance albeit also only partially by all ICHKs.

Expression studies showed no detectable expression of any of these ICHKs in any of these two AML samples. It is known that AML samples express little ICHKs. However, the fact that adding all ICHKs had a detectable effect may mean some of them are present at low, undetectable levels by the labeled antibodies used, but which may still be functional.

### Example 13. Combination of CART with Immune Check Point in Melanoma

The same CART-NKG2D from Example 4 was used, from the same healthy donor cells and produced with the same methods. The CART-NKG2D was used in combination with several ICHK's in a solid tumor melanoma sample, the same used in Example 4 evaluating its tumor-killing activity. The same vial of the solid tumor from melanoma sample was used and a fixed number of 2000 tumor cells per well was incubated at 37°C and 5% CO₂ in RPMI + 20%FBS for 24 hours with the same number of CART-NKG2D T cells (ratio 1:1). The tumor cells alone were used as control. After the incubation time, the plate was processed, labeled with Annexin V-FITC, CD45-PO, EpCAM-PE, 7-AAD, NKG2D-PECy7 and CD5-APC and analyzed in the PharmaFlow platform. Figure 29 panel A shows the percentage of tumor cell survival (Y-axis) in control wells (only tumor cells, target cells) vs CART-NKG2D (effector) and tumor cells (target) (E:T ratio 1:1).

One aliquot of the same melanoma sample was used for establishing the basal expression of PDL-1 , CTLA4, CD80, CD86 and TIM3 ICK's at time 0 hours (Figure 29, panel B). The monoclonal antibodies cocktail used included Annexin V-FITC, 7-AAD, CD45-PO, EpCAM-PE, PDL1 (CD274)-BV421, CD86-PECy7, CD80-APC and TIM3-APCCy7.

Figure 29, panel C shows other aliquot of the same tumor sample that was incubated for 24 hours with a fixed number of 2000 tumor cells and CART-NKG2D T cells, in a ratio 1:1, along with a fixed final concentration of 10µg/ml of PD-1, PDL-1, CTL4, CD80, CD86 and TIM3 ICHK's either alone or all together per well. The fixed 1:1 ratio of tumor cells and CART-NKG2D T cells was used as control. After the incubation period the plate was processed, labeled with Annexin V-FITC, CD45-PO, EpCAM-PE, 7-AAD, NKG2D-PECy7, CD5-APC and PDL1-PB and analyzed in the PharmaFlow platform. The control condition was normalized to better perform the analyses of the rest of conditions with ICHK's.

### Example 14. Cytokine Release Syndrome prediction for BiTE in AML

There have been many efforts to develop a predictive PM Test to identify patients likely to suffer from Cytokine Release Syndrome when treated with BiTEs. Most efforts measured levels of cytokine in supernatants, mostly in CARTs not BiTEs, but do not study these values relative to their tumor-killing activity on each sample. Yet, the concept of a therapeutic window implies that the toxicity of a drug should be balanced with its efficacy. In cancer, it is very typical that enhanced tumor-killing activity corresponds to an enhance toxicity. Thus, we have analyzed the level of cytokines in supernatants as a function of BiTE tumor-killing activity. A CD3xCD123 BiTE was used on AML samples.

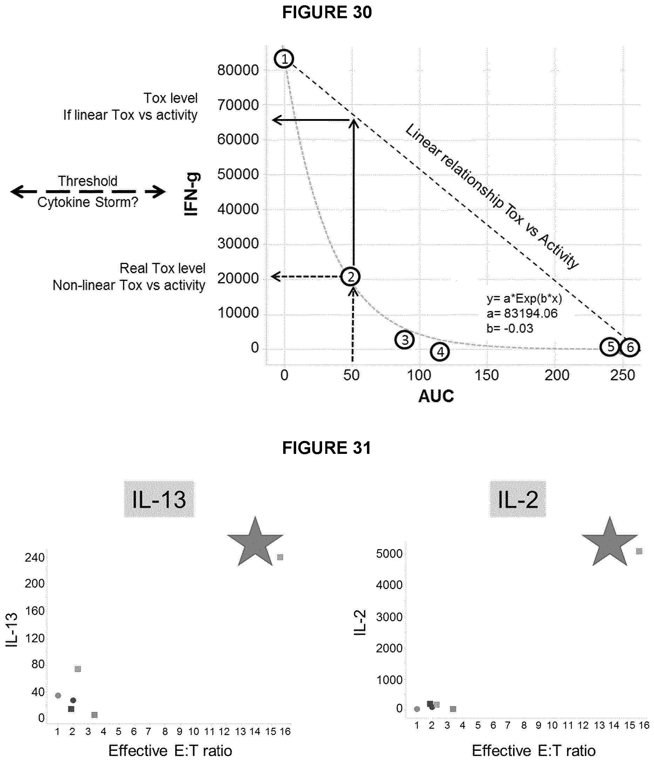

Figure 30 shows the levels of interferon-gamma versus the BiTE activity calculated by the AUC (Area Under the Curve) from the dose response curves of the BiTE incubation in each sample. Let's assume interferon-gamma represents the toxicity induced by Cytokine Release Syndrome, although it may not be the best cytokine to study. If it was a linear relationship, such as the dotted line, that relationship would be obvious. However, their relationship is not linear, it follows a curved relationship, such as at very high AUC (no BiTE activity) there is no cytokine release. As we increase the activity (lower AUC) in other samples, we increase toxicity (cytokine release) but at a lower rate, and thus those samples could have a good efficacy to toxicity ratio. At maximal activity (lowest AUC) the cytokine levels peak and the balance efficacy vs toxicity may no longer be beneficial for the patient. There might even be a certain threshold for Cytokine Release Syndrome to occur in patients frOm this analysis, as illustrated in Figure 30.

Figure 31 shows the levels of cytokines IL-13 and IL-2 versus the Effective E:T Ratios for the same samples discussed for Figure 30. There is a very high level of these 2 cytokines achieved in the supernatant by only 1 sample, and this sample has a very high Effective E:T Ratio. Although it is only 1 sample, the extreme separation from other samples and the potential association of a high Effective E:T Ratio with high levels of these two cytokines could be very interesting. High Effective E:T Ratio means the BiTE-activated T cell for that sample kills many tumor cells very efficiently. Figure 31 shows that that sample kills 16 leukemic cells per activated T cell, compared with the rest of samples that kill less than 4 leukemic cell per activated T cell. We hypothesize that these high tumor-killing activated T cells are no other than professional killers, the patient's own tumor-selective T cells trained and optimized to kill those tumor cells, albeit immunosuppressed by the tumor microenvironment. If this hypothesis is right, we may expect a lesser CRS for these patients because these are the native T cell killers and not artificial CARTs. Hence the potential relevance of this high killing T cells secreting high levels of IL-13; this cytokine is commonly involved in anti-inflammatory responses and may lower the probability of CRS. This would be consistent with this sample killing tumor cells by reactivated tumor-selective T cells, the patient's own native T cells that kill tumor cells without excessive toxicity such as CRS.

### Example 15. Cytokine Release Syndrome prediction for CART in Hematological malignancies

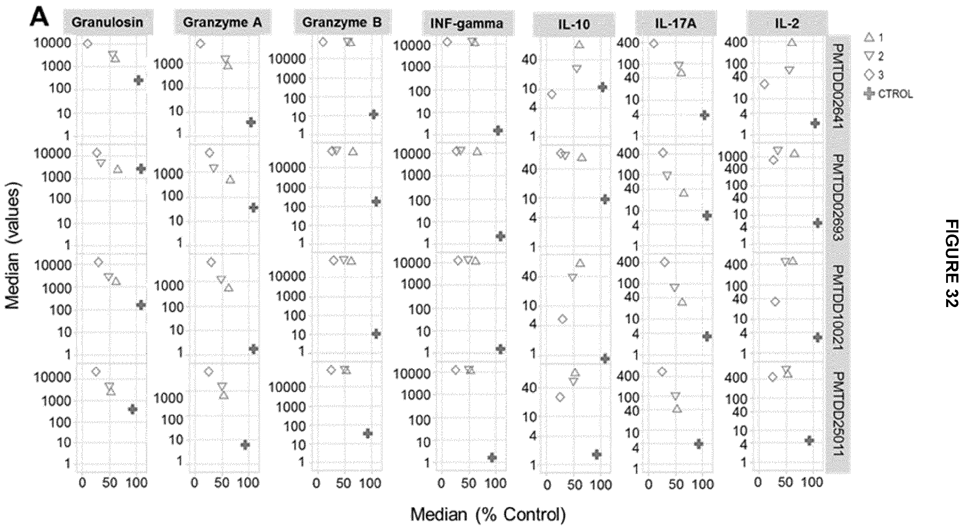

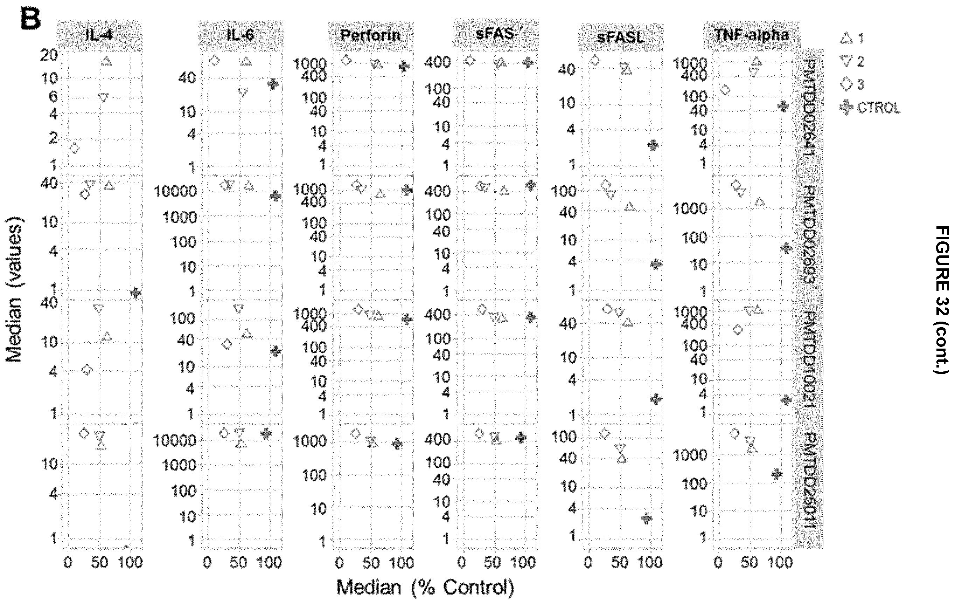

The same CART-NKG2D from Example 4 was used, from the same healthy donor cells and produced with the same methods. The CART-NKG2D and 4 AML BM samples described in Example 5 were incubated to measure both tumor-killing activity and cytokines releases in the supernatant. The CART was produced from a healthy donor, and used at three different Effector to Target (E:T) ratios and the corresponding control against 4 AML. After 24h incubation, the supernatant of each experiment was recovered and tested with the LEGENDplex^{™} Human CD8/NK Panel. This panel allows simultaneous quantification of 13 human proteins, including IL-2, IL-4, IL-10, IL-6, IL-17A, TNF-α, sFas, sFasL, IFN-γ, granzyme A, granzyme B, perforin and granulysin. In the Figure 32, each row corresponds to a different AML sample and each column to a different cytokine. Inside each graph the 3 different CART concentrations are represented corresponding the diamond to the E:T of 5:1, the inverted triangle to the E:T of 1:1, the triangle to the E:T of 0,5:1 and the cross the control. In the X-axis is represented the % survival, the percentage of the number of live cells versus the control. The control represented 100% of the live cells and this number gradually decrease with the increasing concentrations of the CART NKG2D. The Y-axis represents the concentrations of each of the proteins in pg/ml detected by Flow cytometry.

As can be seen in Figure 32A and B, increasing numbers of the CART NKG2D cells reduces the % of live leukemic cells and increase the number of most of the cytokines. There is a gradual increase of the cytokines in a dose dependent manner of the CART NKG2D, more evident for granulosin, Granzyme A, Granzyme B, IL-10, IL-17A, perforin, sFASL and TNF-a. Many of these cytokines are associated with cytokine release syndrome (CRS). This type of analysis simultaneously analyzes both the activity of the CART and the doses of pro-inflammatory cytokines associated to CRS. In this sense, interestingly in the comparison sample to sample the numbers of TNF-a are higher in two samples vs the other two that could be associated to a higher degree of CRS. These patterns need to be correlated with clinical data in terms of efficacy and also Cytokine Release Syndrome toxicity levels to derive a prediction algorithm.

### Example 16. Cytokine Release Syndrome prediction for CART in solid tumors: melanoma

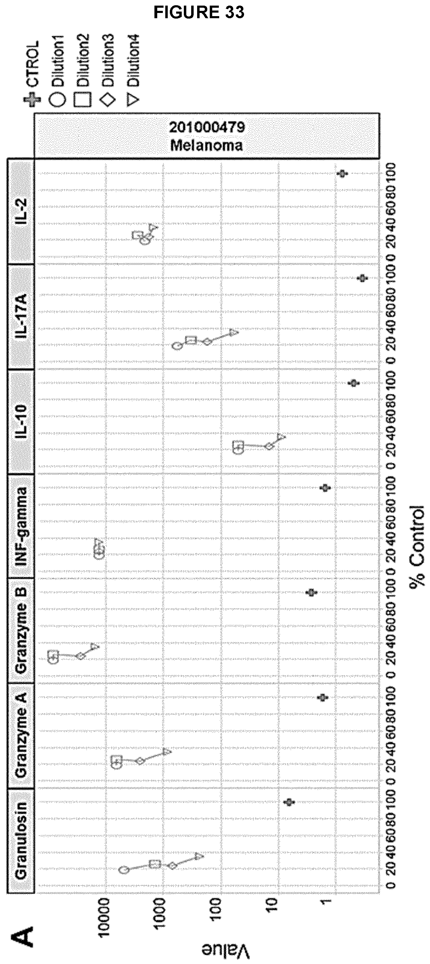

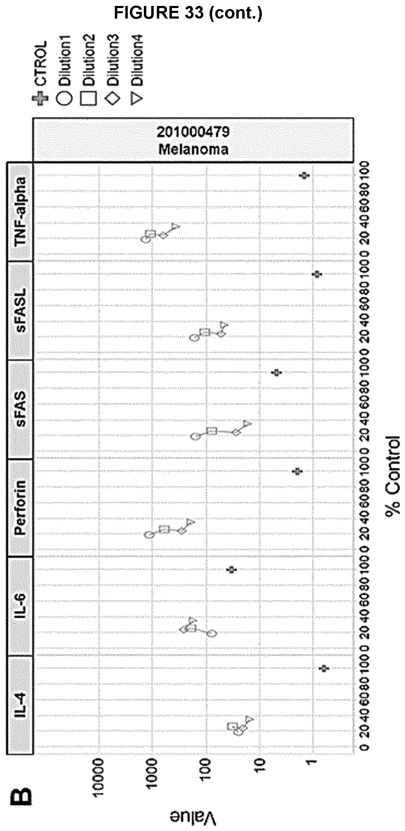

The same CART-NKG2D from Example 4 was used, from the same healthy donor cells and produced with the same methods. The same CART NKG2D and the same melanoma sample described in Example 4 were incubated to measure both tumor-killing activity and cytokines releases in the supernatant. The CART was used at four different Effector to Target (E:T) ratios and the corresponding control against the melanoma sample. After 24h incubation, the supernatant of each experiment was recovered and tested with the LEGENDplex^{™} Human CD8/NK Panel. This panel allows simultaneous quantification of 13 human proteins, including IL-2, IL-4, IL-10, IL-6, IL-17A, TNF-α, sFas, sFasL, IFN-γ, granzyme A, granzyme B, perforin and granulysin. In the Figure 33A and B, each column corresponds to a different cytokine. Inside each graph the 4 different CART concentrations correspond to the 4 smaller dilutions (dilutions 1-4, with higher CART proportions) from Example 4 because these are the supernatants from Example 4. In the X-axis is represented the % survival, the percentage of the number of live cells versus the control. The control represented 100% of the live cells and this number gradually decrease with the increasing concentrations of the CART NKG2D (i.e. decreasing dilutions). The Y-axis represents the concentrations of each of the proteins in pg/ml detected by Flow cytometry.

As can be seen in the Figure 32, increasing numbers of the CART NKG2D cells reduces the % of live leukemic cells and increase the number of most of the cytokines. There is a gradual increase of the cytokines in a dose dependent manner of the CART NKG2D. This pattern is more evident for granulosin, Granzyme A, Granzyme B, IL-10, IL-17A, perforin, sFAS, sFASL and TNF-a. These are the same cytokines a CART dose dependent increase was observed in the previous Example 15, with the only exception of sFAS that in the previous Example 15 its levels were too high outside the detection range. Both Examples use the same CART-NKG2D, but in this example on a melanoma sample while in Example 15 was on 4 AML samples. This clear and consistent pattern across different samples, even hematological malignancy vs solid tumor samples, suggests this approach of associating cytokine supernatant levels to tumor-killing activity may enable a reliable consistent PM Test for CRS for CAR-T treatments. Nonetheless, these patterns need to be validated in a larger cohort of patient samples, and correlated with clinical data in terms of efficacy and also CRS toxicity levels to derive a prediction algorithm.

### Example 17. Effect of Artificial Environment on CART tumor-killing activity

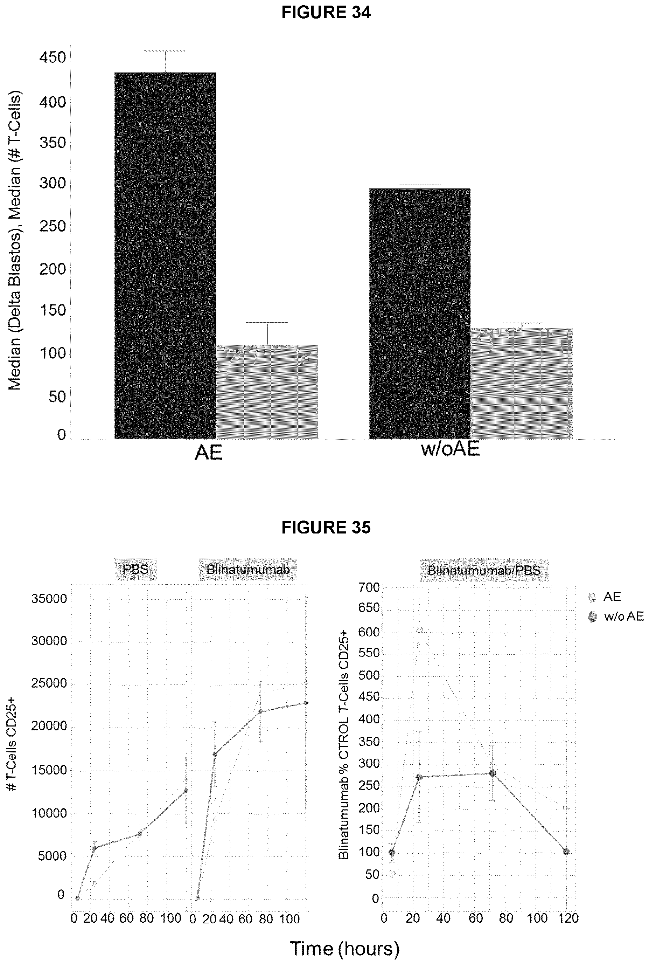

A CART-CD19 generated in an equivalent manner to Example 1 was incubated with a B-ALL sample already described in Example 6 at a 1:1 CART to tumor cell ratio, with or without AE (Artificial Environment). AE were added in combination at a concentration of 1 µl/60 µl. 0,5 µl from a pooled RBC plus 0,5 µl from a pooled plasma from B-ALL. Both conditions (with and w/o AE) were incubated 24h. **Figure 34** compares the median delta leukemic cells and the median number of CARTs for both conditions. Black bars represent the difference between the absolute number of blast cells between the basal level 0h and the 24h post-incubation and the grey bars represent the number of CART-CD19. The median number of CARTs is similar at the basal levels and post incubation, since we dispensed equal CART numbers. However, the median number of leukemic cells killed was significantly higher in the presence of the AE that in its absence. Thus, removing AE alters negatively the activity of CARTs in these ex vivo assays, and thus AE should be preserved in these assays.

### Example 18. Artificial Environment effect of the ex vivo activity of Blinatumomab (CD3×CD19 bispecific)

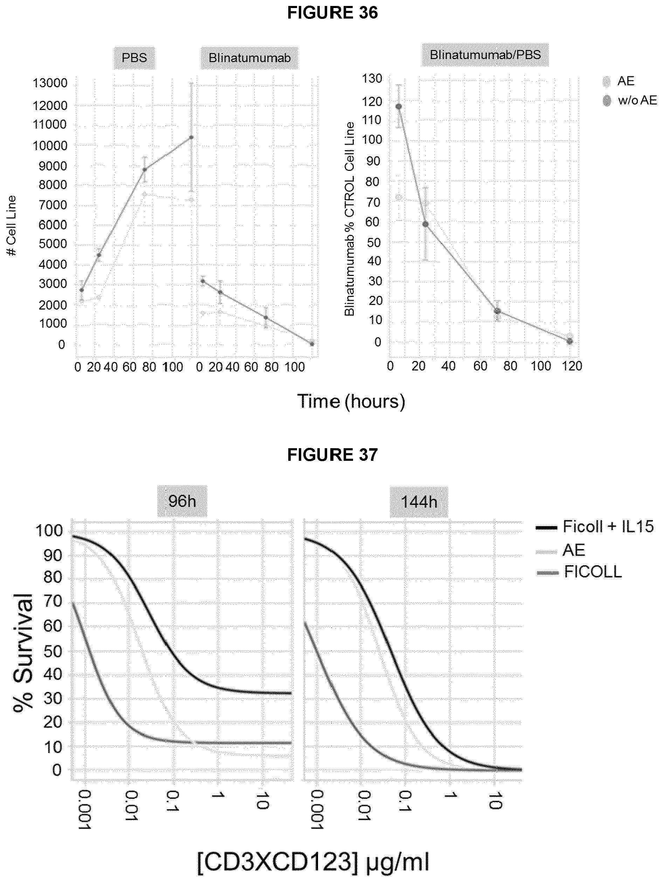

The bispecific antibody Blinatumomab (CD3xCD19) was incubated with or without Artificial Environment, at a single high dose at different time points (6, 12, 24, 72, 120 h). Blinatumomab-induced activation of T cells and the concomitant depletion of tumor cells was measured. A healthy donor buffy coat for T cells and a tumor cell line were used. The results are shown below in Figures 35 and 36, respectively.

Figure 35 shows the absolute number of activated T Cells (CD5+CD25+) over time. The left panel represents the control wells with only PBS incubating with Artificial Environment (AE, grey) and without AE (black). Note that in this sample there are activated T cells expanding over time, prior to adding Blinatumomab. There is a difference at 24 h where activated T cells expand better without AE (black). The middle panel represents the Blinatumomab incubated activated T cells. There is a difference at 24 h where activated T cells expand better without AE (black). The right panel shows the ratio of activated T cells incubating with Blinatumomab vs control PBS, the fold over of T cell activation induced by Blinatumomab. There is a very large difference in the activity of Blinatumomab ex vivo incubating with AE (grey) or without AE (black) at 24 h, 600 vs 270 fold vs control. Blinatumomab is more active with AE.

Figure 36 shows also a significant difference with vs without AE at short times, 6 h, likely related to the increased viability of tumor cells with AE we have observed.

In conclusion, the ex vivo assay to evaluate the activity of Blinatumomab in inducing T cell activation and killing of tumor cells show important and significant differences (with vs without AE). These differences are large at initially and converge overtime. Blinatumomab is more active in AE, and thus artificially removing AE results in an artificial decrease of its activity.

### Example 19. Artificial Environment effect of the ex vivo activity of bispecific antibody CD3×CD123

In this example, the ex vivo activity of a bispecific antibody (a CD3xCD123 bispecific antibody, specific for AML) is artificially modified by removing AE. The mechanism of action of this antibody is similar to Blinatumomab, both sharing the CD3, but Blinatumomab uses a CD19 arm that recognizes ALL; and CD3xcD123 uses a CD123 arm that recognizes AML. Dose response curves were measured at different times, comparing AE with removing AE using a Ficoll.

Figure 37 shows the AE for AML samples show consistently a lower activity than removing the AE with a Ficoll. Adding IL15 to Ficoll does not fix this problem. Thus, removing AE artificially alters the ex vivo activity of this bispecific antibody.

### Citation List

### Non-patent literature

- Adams JL et al. (2015). Big opportunities for small molecules in immuno-oncology. Nat Rev Drug Discov 14(9):603-22.
- Benien, P. and Swami, A. (2014) 3D tumor models: history, advances and future perspectives, Future Oncol 10:1311-1327.
- Bennett TA et al. (2014). Pharmacological profiles of acute myeloid leukemia treatments in patient samples by automated flow cytometry: a bridge to individualized medicine. Clin. Lymphoma Myeloma Leuk. 14(4):305-18.
- Borrello I et al. (2016) Marrow-Infiltrating Lymphocytes - Role in Biology and Cancer Therapy. Front Immunol. 7:112.
- Buil-Bruna N et al. (2016). Bringing Model-Based Prediction to Oncology Clinical Practice: A Review of Pharmacometrics Principles and Applications. The Oncologist 21(2):220-32.
- Chothia C et al. (1987). Canonical structures for the hypervariable regions of immunoglobulins. J Mol Biol 196(4):901-17.
- Costa EC el al. (2017) 3D tumor spheroids: an overview on the tools and techniques used for their analysis. Biotechnol Adv 34(8):1427-1441.
- Dai H et al. (2016). Chimeric Antigen Receptors Modified T-Cells for Cancer Therapy. J Natl Cancer Inst. 108(7). pii: djv439.
- Eastwood D et al. (2013) Severity of the TGN1412 trial disaster cytokine storm correlated with IL-2 release. Br J Clin Pharmacol. 76(2):299-315.
- Fennema, E et al. (2013) Spheroid culture as a tool for creating 3D complex tissues, Trends Biotechnol 31:108-115.
- Finco D et al. (2014) Cytokine release assays: current practices and future directions. Cytokine. 66(2):143-55.
- Hamid O et al. (2013). Safety and tumor responses with lambrolizumab (anti-PD-1) in melanoma. N Engl J Med 369(2):134-44.
- Kabat EA et al. (1991). Sequences of Proteins of Immunological Interest. US Department of Health and Human Services Fifth Edition. NIH Publication No. 91-3242.
- Larbi A and Fulop T (2014). From "truly naive" to "exhausted senescent" T cells: when markers predict functionality. Cytometry Part A 85(1): 25-35.
- Martin F et al. (1994). The affinity-selection of a minibody polypeptide inhibitor of human interleukin-6. EMBO J 13(22):5303-9.
- Maus MV et al. (2014). Adoptive immunotherapy for cancer or viruses. Annu Rev Immunol 32:189-225.
- McConnell SJ et al. (1995). Tendamistat as a scaffold for conformationally constrained peptide libraries. J Mol. Biol 250(4):460-70.
- Montes M et al. (2005). Optimum in vitro expansion of human antigen-specific CD8 T cells for adoptive transfer therapy. Clin Exp Immunol 142(2):292-302.
- Morgan RA et al. (2016). Genetic Modification of T Cells. Biomedicines. 20;4(2). pii: E9.
- Mould DR et al. (2013). Basic Concepts in Population Modeling, Simulation, and Model-Based Drug Development - Part 2: Introduction to Pharmacokinetics Modeling Methods. CPT Pharmacometrics Syst Pharmacol 2(4):e38.
- Nam, KH et al. (2015) Biomimetic 3D Tissue Models for Advanced High-Throughput Drug Screening, J Lab Autom 20: 201-215.
- Olbrich H et al. (2017) Reconstructing the immune system with lentiviral vectors. Virus Genes. doi: 10.1007/s11262-017-1495-2.
- Pardoll DM. (2012). The blockade of immune checkpoints in cancer immunotherapy. Nat Rev Cancer 12(4):252-64.
- Rosenberg SA et al. (1988). Use of tumor-infiltrating lymphocytes and interleukin-2 in the immunotherapy of patients with metastatic melanoma. A preliminary report. N Eng J Med 19(25):1676-80.
- Scott AM et al. (2012). Monoclonal antibodies in cancer therapy. Cancer Immun 12:14.
- Serafini P et al. (2008). Myeloid-derived suppressor cells promote cross-tolerance in B-cell lymphoma by expanding regulatory T cells. Cancer Res 68(13):5439-49.
- Song DG et al. (2012). CD27 costimulation augments the survival and antitumor activity of redirected human T cells in vivo. Blood 119(3):696-706.
- Spiess C et al. (2015). Alternative molecular formats and therapeutic applications for bispecific antibodies. Mol Immunol 67:95-106.
- Tramontano A et al. (1994). The making of the minibody: an engineered beta-protein for the display of conformationally constrained peptides. J Mol. Recognit 7(1):9-24.
- Upton RN et al. (2014). Basic Concepts in Population Modeling, Simulation, and Model-Based Drug Development: Part 3-Introduction to Pharmacodynamic Modeling Methods. CPT Pharmacometrics Syst Pharmacol 3(1): e88.
- Vessillier S et al. (2015) Cytokine release assays for the prediction of therapeutic mAb safety in first-in man trials--Whole blood cytokine release assays are poorly predictive for TGN1412 cytokine storm. J Immunol Methods. 424:43-52.
- Guidance for Industry Population Pharmacokinetics (1999). US Department of Health and Human Services Food and Drug Administration; Center for Drug Evaluation and Research (CDER) and Center for Biologies Evaluation and Research (CBER).
- http://www.fda.gov/downloads/drugs/guidancecomplianceregulatoryinformation/guidances/uc m072137.pdf).

## Claims

1. An *in vitro* method of producing a genetically engineered T cell expressing Chimeric Antigen Receptors (a CAR-T cell) or a CAR-T cell preparation:
(a) providing a sample comprising at least one T cell from a subject having a cancer;
(b) providing a sample comprising at least one cancer cell;
(c) forming an ex *vivo* reaction mixture comprising the at least one T cell, the at least one cancer cell, and a bispecific T cell engager antibody (BiTE) or a multispecific antibody under conditions and for a period of time sufficient to allow the at least one T cell to become activated and kill at least one cancer cell, thereby producing at least one activated T cell;
(d) selecting the activated T cell, based on at least a parameter selected from the group consisting of: increased cancer cell killing activity, reduced toxicity, reduced off-target effect, increased viability, increased proliferation, and Effective E:T ratio; and
(e) genetically engineering the activated T cell to produce Chimeric Antigen Receptors (CAR) on the surface of the activated T cell, thereby producing at least one CAR-T cell.

2. The method according to claim 1, wherein the bispecific T cell engager antibody (BiTE) or the multispecific antibody have a first element providing affinity for the T cell and a second element having affinity for the cancer cell, wherein the first element binds to a T cell and does not bind to a substantial number of cancer cells and wherein the second element binds to a cancer cell and does not bind to a substantial number of T cells.

3. The method according to claim 1 or 2, wherein the selection of the activated T cell, is based on the parameter Effective E:T ratio, and wherein the activated T cell has an Effective E:T ratio higher than 1:5 between the number of activated T cells (E) and the number of target cancer cells (T) after exposure to the bispecific T cell engager antibody (BiTE) or to the multispecific antibody.

4. The method according to any one of claims 1 to 3, further comprising evaluating the activity of the CAR-T cell or CAR-T cell preparation, wherein evaluating comprises:
(f) providing a CAR-T cell or a CAR-T cell preparation thereof obtainable according to the method of claim 1;
(g) providing a sample of cancer cells, wherein the cancer cells are from the same subject;
(h) contacting the CAR-T cell or the CAR-T cell preparation thereof with the cancer cells for a period of time sufficient to allow the CAR-T cell to kill the cancer cells; and
(i) determining the level of cancer cells after step (c),
wherein a decrease in the level or amount of cancer cells, relative to a reference level, is indicative of increased cell killing activity, or wherein a reduced change or no substantial change in the level or amount of cancer cells relative to a reference level, is indicative of decreased cell killing activity.

5. A composition comprising a CAR-T cell or CAR-T cell preparation thereof obtainable according to the method of any one of claims 1 to 4.

6. The composition according to claim 5, wherein the CAR-T cell requires (i) and at least one of (ii), (iii), or (iv): (i) has cytotoxic activity toward a cancer cell, and (ii) comprises at least 100 copies of a cancer cell surface marker, including a membrane cell marker on the cancer cell; and/or (iii) comprises a detectable amount of a bispecific T cell engager antibody (BiTE) or of a multispecific antibody; and/or (iv) comprises a detectable amount of agents enhancing T cell activity such as immune check point inhibitors.

7. A pharmaceutical composition comprising the composition of claim 5 or 6 and a pharmaceutically acceptable carrier.

8. The pharmaceutical composition according to claim 7 for use in Adoptive Cancer Therapy for treating a subject, wherein the subject is the same subject as that of step (a) of claim 1, and/or wherein the subject is the same subject as that of step (b) of claim 1, and/or wherein the subject is different from the subject as that as step (a) or (b) of claim 1.

9. A CAR-T cell or a CAR-T cell preparation thereof obtainable according to the method of any one of claims 1 to 4, or the composition of claim 5 or 6, for use in the treatment of a subject having cancer.

10. The CAR-T cell, or the CAR-T cell preparation, or the composition, for use according to claim 9, wherein the selection of the activated T cell is based on the parameter Effective E:T ratio, and wherein the activated T cell has an Effective E:T ratio higher than 1:5 between the number of activated T cells (E) and the number of target cancer cells (T) after exposure to the bispecific T cell engager antibody (BiTE) or to the multispecific antibody.

11. The CAR-T cell, or the CAR-T cell preparation, or the composition, for use according to claim 9 or 10, further comprising administering to the subject a second therapeutic agent or procedure.

12. The CAR-T cell, or the CAR-T cell preparation, or the composition, for use according to claim 11, wherein the second therapeutic agent or procedure is selected from the group consisting of: chemotherapy, a targeted anti-cancer therapy, an oncolytic drug, a cytotoxic agent, an immune-based therapy such as immune check point inhibitors, a cytokine, a surgical procedure, a radiation procedure, an agonist of T cells, an inhibitor of an inhibitory molecule, an immune checkpoint inhibitor, an immunomodulatory agent, a vaccine, and a cellular immunotherapy.

13. An *in vitro* method of identifying subjects susceptible to immune checkpoint immunotherapy treatment to be combined with a CAR-T cellular immunotherapy, to treat a subject, for decreasing resistance of said subject to said cellular immunotherapy, comprising:
(a) providing a sample comprising at least one T cell selected from the group consisting of a tumor infiltrated lymphocyte (TIL), marrow infiltrated lymphocyte (MIL), a genetically engineered T cell, a CAR-T cell, or an activated T cell obtainable according to step (c) of the method of claim 1 and a genetically engineered T cell expressing Chimeric Antigen Receptors obtainable according to step (e) of the method of claim 1, from a subject having a cancer;
(b) providing a cancer cell;
(c) forming an *ex vivo* reaction mixture comprising (a) and (b), under conditions and for a period of time sufficient to allow the T cells to kill cancer cells, thereby producing the cancer-killing T cell; and
(d) determining the pharmacological activity of cancer-killing T cells obtained in step (c) by dose response and/or pharmacodynamic parameters of cancer-killing T cells and tumor cells, selected from the group consisting of: EC50, Emax, AUC, Effective E:T Ratios, Basal E:T Ratios, and kinetic parameters;
(e) determining the pharmacological activity of cancer-killing T cells obtained in step (c) by dose response or evaluating a single high saturating dose in combination with immune check point inhibitors, individually, or in combinations, or bispecific or multispecific antibody constructs combining immune check point inhibitors, including the combination of all immune check point inhibitors, either by full dose responses or evaluating a single high saturating dose;
(f) determining the expression levels of immune checkpoint molecules in both the tumor cells and T cells in the reaction mixture of step (c), comparing basal levels with levels after incubation;
(g) identifying subjects susceptible to immune checkpoint immunotherapy treatment in combination with the cellular therapy, by assessment of either of the following 2 criteria or a combination of them:
i. step (d) reveals a resistant tumor cell population in the samples from the subject because incubation with T cell therapy does not kill all tumor cells, and addition of one or more immune checkpoint inhibitors in (e) reverts resistance of tumor cell population;
ii. step (f) reveals an increase in the expression level of an immune checkpoint molecule in either the tumor cells and/or T cells in the reaction mixture of step (c) after incubation, relative to basal levels prior incubation,
and wherein observance of both (i) and (ii) is indicative of a subject more susceptible to immune checkpoint immunotherapy treatment to be combined with a cellular immunotherapy.

14. The method according to claim 13, wherein the immune checkpoint molecule is selected from the group consisting of: PDL-1, PDL-2, B7-1 (CD80), B7-2 (CD86), 4-1BBL, Galectin, ICOSL, GITRL, OX40L, CD155, B7-H3, PD1, CTLA-4, 4-1BB, TIM-3, ICOS, GITR, LAG-3, KIR, OX40, TIGIT, CD160, 2B4, B7-H4 (VTCN1), HVEM (TNFRSF14 or CD270), BTLA, KIR, MHC class I, MHC class II, GAL9, VISTA, LAIR1, and A2aR, and combinations of said immune checkpoint molecules in bispecific or multispecific antibody formats.

15. The method according to claim 13 or 14, wherein the bispecific T cell engager antibody (BiTE) or the multispecific antibody have a first element providing affinity for the T cell and a second element having affinity for the cancer cell, wherein the first element binds to a T cell and does not bind to a substantial number of cancer cells and wherein the second element binds to a cancer cell and does not bind to a substantial number of T cells.

16. The method according to any one of claims 13 to 15, wherein the sample (a) is selected from the group consisting of: whole blood, peripheral blood, bone marrow, lymph node, spleen, a primary tumor and a metastasis.

17. A bispecific T cell engager antibody (BiTE) or a multispecific antibody or a T cell selected from the group consisting of a tumor infiltrated lymphocyte (TIL), a genetically engineered T cell, a CAR-T cell, an activated T cell obtainable according to the step (c) of the method of claim 1, and a genetically engineered T cell expressing Chimeric Antigen Receptors obtainable according to step (e) of the method of claim 1, in combination with an inhibitor of at least one immune checkpoint molecule selected in the method of any one of claims 13 to 16 as target for decreasing resistance to a cancer therapy, for use in the treatment of a subject having cancer.

18. The combination for use of claim 17, wherein the inhibitor of at least one immune checkpoint molecule is selected from the group consisting of Nivolumab, Pembrolizumab and Pidilizumab.

19. The combination for use according to claim 17 or 18, further comprising administering a third therapeutic agent or procedure.

20. The method according to any one of claims 1 to 4, wherein when the method is applied to samples of solid tumor, the method is performed using 3D cell culture constructs built to mimic the microenvironment architecture of solid tumors, selected from the group consisting of: spheroids, extracellular matrix gels, synthetic scaffolds, rotary cell culture systems, and low/non-adherent culture plastics.
